# FASCICULE DE BREVET EUROPEEN

(11) **EP 2 433 965 B1**
(45) Date de publication et mention de la délivrance du brevet: **15.03.2017**
(21) Numéro de dépôt: 11178479.9
(22) Date de dépôt: 15.12.2005
(51) Int. Cl.: C07K 16/00, C07K 16/28, C07K 16/30

(54) **Production de formats d'anticorps et applications immunologiques de ces formats**
Herstellung von Antikörperformaten und immunologische Anwendungen dieser Formate
Production of antibody formats and immunological applications of said formats

(30) Priorité: 16.12.2004 FR 0413433
(43) Date de publication de la demande: 28.03.2012
(62) Demande divisionnaire de: 05826565.3
(73) Titulaire: Centre National de la Recherche Scientifique (C.N.R.S.), 75016 Paris (FR); INSERM (Institut National de la Santé et de la Recherche Médicale), 75013 Paris (FR); Université d'Aix-Marseille, 13007 Marseille (FR); Université de Montpellier, 34090 Montpellier (FR); Institut Regional du Cancer de Montpellier - Val d'Aurelle, 34298 Montpellier (FR); Institut Paoli Calmettes, 13009 Marseille (FR)
(72) Inventeur: Baty, Daniel, 13009 Marseille (FR); Behar, Ghislaine, 13015 Marseille (FR); Chartier, Martine, 13011 Marseille (FR); Pelegrin, André, 34070 Montpellier (FR); Teillaud, Jean-Luc, 75004 Paris (FR); Teulon, Isabelle, 34980 Saint Gely du Fesc (FR)
(74) Mandataire: Cabinet Plasseraud

(56) Documents cités:
- WO-A2-03/035694
- KIPRIYANOV S M ET AL: "SYNERGISTIC ANTITUMOR EFFECT OF BISPECIFIC CD19 X CD3 AND CD19 X CD16 DIABODIES IN A PRECLINICAL MODEL OF NON-HODGKIN'S LYMPHOMA", THE JOURNAL OF IMMUNOLOGY, THE AMERICAN ASSOCIATION OF IMMUNOLOGISTS, US, vol. 169, no. 1, 1 juillet 2002 (2002-07-01), pages 137-144, XP001094164, ISSN: 0022-1767
- MUYLDERMANS S: "SINGLE DOMAIN CAMEL ANTIBODIES: CURRENT STATUS", JOURNAL OF BIOTECHNOLOGY, ELSEVIER SCIENCE PUBLISHERS, AMSTERDAM, NL, vol. 74, no. 4, 1 janvier 2001 (2001-01-01), pages 277-302, XP008019929, ISSN: 0168-1656
- FLEIT H B ET AL: "HUMAN NEUTROPHIL FC-GAMMA RECEPTOR DISTRIBUTION AND STRUCTURE", PROCEEDINGS OF THE NATIONAL ACADEMY OF SCIENCES OF THE UNITED STATES OF AMERICA, vol. 79, no. 10, 1982, pages 3275-3279, ISSN: 0027-8424
- WIRTHMUELLER U ET AL: "SIGNAL TRANSDUCTION BY FC-GAMMA-RIII CD16 IS MEDIATED THROUGH THE GAMMA CHAIN", JOURNAL OF EXPERIMENTAL MEDICINE, vol. 175, no. 5, 1992, pages 1381-1390, ISSN: 0022-1007
- GHISLAINE BEHAR ET AL: "Isolation and characterization of anti-Fc[gamma]RIII (CD16) llama single-domain antibodies that activate natural killer cells", PROTEIN ENGINEERING, DESIGN AND SELECTION JANUARY 2008 OXFORD UNIVERSITY PRESS GB,, vol. 21, no. 1, 1 January 2008 (2008-01-01), pages 1-10, XP002646408, DOI: 10.1093/PROTEIN/GZM064 [retrieved on 2007-12-11]
- HOLLIGER P ET AL: "DIABODIES: SMALL BIVALENT AND BISPECIFIC ANTIBODU FRAGMENTS", PROCEEDINGS OF THE NATIONAL ACADEMY OF SCIENCES, NATIONAL ACADEMY OF SCIENCES, US, vol. 90, no. 14, 15 July 1993 (1993-07-15) , pages 6444-6448, XP002014058, ISSN: 0027-8424, DOI: 10.1073/PNAS.90.14.6444
- VELY F ET AL: "A NEW SET OF MONOCLONAL ANTIBODIES AGAINST HUMAN FCGAMMARII (CD32) AND FCGAMMARIII (CD16): CHARACTERIZATION AND USE IN VARIOUS ASSAYS", HYBRIDOMA, LIEBERT, NEW YORK, NY, US, vol. 16, no. 6, 1 January 1997 (1997-01-01), pages 519-528, XP009046827, ISSN: 0272-457X
- GRUEL N ET AL: "BYPASSING TUMOR-SPECIFIC AND BISPECIFIC ANTIBODIES: TRIGGERING OF ANTITUMOR IMMUNITY BY EXPRESSION OF ANTI-FCGAMMAR SCFV ON CANCER CELL SURFACE", GENE THERAPY, NATURE PUBLISHING GROUP, GB, vol. 8, no. 22, 1 November 2001 (2001-11-01), pages 1721-1728, XP009011865, ISSN: 0969-7128, DOI: 10.1038/SJ.GT.3301575
- C. Rozan ET AL: "Single-Domain Antibody-Based and Linker-Free Bispecific Antibodies Targeting Fc RIII Induce Potent Antitumor Activity without Recruiting Regulatory T Cells", Molecular Cancer Therapeutics, vol. 12, no. 8, 1 August 2013 (2013-08-01) , pages 1481-1491, XP055156453, ISSN: 1535-7163, DOI: 10.1158/1535-7163.MCT-12-1012

## Description

L'invention se rapporte à la production de formats d'anticorps et à leurs applications immunologiques, plus particulièrement en immunothérapie et en immunodiagnostic.

On rappelle que les molécules d'anticorps sont des immunoglobulines (Ig) appartenant à 5 classes : IgM, IgG, IgD, IgE et IgA. De manière générale, ces molécules comprennent une chaîne lourde (H) et une chaîne légère (L) qui est soit la chaîne kappa (κ), soit la chaîne lambda λ).

Chaque classe d'immunoglobulines comporte un type propre de chaîne H, chaîne µ pour l'IgM, y pour l'IgG, δ pour l'IgD, ε pour l'IgE et α pour l'IgA.

Chaque chaîne est formée de domaines, chacun possédant une liaison disulfure interne. Une chaîne L possède deux domaines et une chaîne H, 4 domaines. La séquence du domaine comprenant l'extrémité aminée de chaque chaîne est variable (régions VH et VL), celle des autres domaines est constante (CH1, CH2 et CH3 de la chaîne H, et CL de la chaîne L).

Les régions variables V comportent des régions de séquences hypervariables appelées CDR dont l'ensemble détermine la complémentarité.

Dans les chaînes H, les deux premiers domaines (VH-CH1) sont suivis d'une région charnière. Dans une immunoglobuline, la chaîne L est reliée à la chaîne H par une liaison disulfure pour former un hétérodimère. Cet hétérodimère est relié au même hétérodimère par plusieurs liaisons disulfures au niveau de la région charnière pour former l'immunoglobuline. Par clivage à l'aide d'une protéase au niveau de la charnière, on obtient deux fragments : le fragment Fab (domaine fixant l'antigène, composé des domaines VL-CL et VH-CH1 ) et le fragment Fc (domaine effecteur, composé des domaines (CH2-CH3)₂).

L'invention concerne plus spécialement des fragments d'anticorps et différents formats d'anticorps élaborés à partir de ces fragments, en particulier des formats d'anticorps chimérisés ou humanisés, multispécifiques et/ou multivalents.

Les « formats d'anticorps » tels que visés par l'invention correspondent à différentes combinaisons des domaines et régions des types mentionnés ci-dessus.

Par « anticorps chimérisé », on entend un domaine VH d'origine animale fusionné aux régions constantes d'une immunoglobuline humaine.

Par « anticorps humanisé », on entend un domaine VH humain sur lequel on a greffé les régions hypervariables (CDRs) d'un VH d'origine animale, fusionné aux régions, constantes d'une Ig humaine.

Ces anticorps reconnaissent les épitopes de cibles correspondant à une molécule donnée. Ces épitopes peuvent être différents, et appartenir à des cibles différentes ou à la même cible. Ainsi, « anticorps bispécifique » désigne un format possédant deux VH différents liant deux cibles différentes ; « anticorps biépitopique » correspond à un format possédant deux VH différents liant deux épitopes différents sur la même cible.

La « valence » correspond au nombre de fois où le même VH se trouve sur le fragment considéré.

La spécificité de reconnaissance des anticorps pour atteindre une cible déterminée a été exploitée pour le diagnostic et la thérapie de différentes pathologies, et tout particulièrement dans le cas de l'oncologie, où la cible peut être un antigène associé aux tumeurs, un récepteur de facteur de croissance, un produit d'oncogène ou de gène "suppresseur de tumeur" muté, voire une molécule liée à l'angiogenèse ou une molécule exprimée également sur les cellules non tumorales, mais absente des cellules progénitrices (comme dans le cas du CD20).

Après plus de 20 années de travaux expérimentaux, l'immunociblage des tumeurs par des anticorps monoclonaux connaît actuellement un développement important.

Ainsi, les résultats de différentes études cliniques ont démontré, récemment, les possibilités thérapeutiques de certains anticorps et ont conduit à leur approbation par la FDA et à l'obtention d'AMM européenne.

Cette progression est due pour beaucoup à l'utilisation d'anticorps recombinants dits de «seconde génération» :
- anticorps humanisés, comme l'Herceptine, un anticorps anti-HER2/Neu utilisé en association avec la chimiothérapie dans certains carcinomes du sein,
- anticorps chimériques comme le Rituximab, un anticorps anti-CD20 utilisé dans le traitement de lymphomes B folliculaires.

Grâce à l'ingénierie génétique, il est en effet possible de «greffer» les régions variables ou hypervariables d'anticorps de souris sur des molécules d'Ig humaines.

De nouvelles techniques permettent désormais d'obtenir des anticorps entièrement humains soit par sélection de domaines variables humains exprimés sur des phages (technique dite du «Phage display»), soit en utilisant des souris transgéniques produisant des anticorps humains.

Par ailleurs, pour stimuler le système immunitaire et favoriser ainsi le contact entre la cellule cible tumorale et une cellule effectrice, on a utilisé le concept d'anticorps bispécifiques. Il s'agit donc de construire un anticorps doté d'une double spécificité : cet anticorps doit être capable de fixer une molécule produite à la surface des cellules tumorales (telles que le CEA, HER2/Neu, le G_{D2}...) et une molécule exprimée à la surface de cellules effectrices de l'immunité, cellules NK, lymphocytes T tueurs ou CTL, polynucléaires neutrophiles, monocytes et macrophages (telles que les récepteurs Fc...). Une variante à cette stratégie consiste à construire un anticorps liant une molécule produite à la surface de la cellule tumorale et une molécule présentant des propriétés directes ou indirectes de cytotoxicité (radio-élément, toxine, pro-drogue).

Jusqu'à présent la plupart des anticorps bispécifiques étaient développés en couplant 2 fragments d'anticorps par voie biochimique. Cette technique est cependant rarement développée à l'échelle industrielle. Quelques anticorps bispécifiques ont été développés par voie génétique, tels que les anticorps bispécifiques de type scFv («diabodies »). Mais ils restent difficiles à produire dans *E. coli* sous forme soluble et ils sont de plus très peu efficaces en termes d'ADCC.

La demande WO03/035694 décrit des anticorps de camélidés.

Muyldermans S., Reviews in Molecular Biotechnology 74 (2001) p277-302, est une revue sur les VHH de camélidés.

Holliger P. et al. PNAS, vol90 (1993), p6444-6448 décrit les «diabodies», anticorps bispécifiques ou bivalents obtenus par association de deux scFv.

Kipriyanov S. et al., J Immunology vol.169, no. 1 (2002) p137-144, décrit notamment un anticorps bispécifique obtenu à partir d'un scFv anti-CD16.

Wirthmueller U. et al., J Exp. Med. vol 175 (1992) p1381-1390, décrit un anticorps monoclonal anti-CD16.

Gruel N. et al. Gene Therapy, vol 8 (2001), p1721-1728, décrit un scFv anti-CD16 fusionné à la région transmembranaire du récepteur PDGFR.

Vely F. et al. Hybridoma, vol 16 (1997), p519-528 décrit des anticorps monoclonaux anti-CD16.

Dans le cadre de la recherche d'anticorps candidats pour générer des formats d'anticorps pour l'immunothérapie et disposer notamment d'anticorps multi-spécifiques, les inventeurs ont orienté leurs travaux vers des anticorps particuliers, dépourvus de chaîne légère, identifiés chez les camélidés (chameau, dromadaire, lama) (*Hamers-Casterman et al., 1993).*

Des domaines variables d'anticorps simple chaîne lourde de camélidés (VHH), reconnaissant spécifiquement un type d'antigènes, ont été sélectionnés à partir d'animaux immunisés et ont permis de concevoir divers formats d'anticorps chimérisés ou humanisés pouvant être produits à partir de constructions plasmidiques. Il s'est avéré que les différents formats étaient compatibles pour permettre la production de n'importe quel autre VHH ou VHH humanisé, ou VH humain.

L'invention a pour but de fournir des formats d'anticorps comportant une partie ou la totalité des domaines de VHH, ayant les propriétés de reconnaissance de cibles et d'épitopes recherchés, tels que définis dans les revendications.

Elle également un procédé de production de ces différentes constructions.

Selon encore un autre aspect, l'invention décrit les applications immunothérapeutiques et immunodiagnostiques des différents formats rendus accessibles.

L'invention a ainsi pour objet les formats d'anticorps comprenant une partie ou la totalité des domaines de VHH de camélidés, notamment de lamas, fusionnés à des régions constantes d'anticorps humains, tels que définis dans les revendications.

Selon un premier mode de réalisation de l'invention, les formats d'anticorps sont de type Fab et sont caractérisés par l'association de deux domaines VHH, identiques ou différents, l'un des domaines étant fusionné à la région constante Cκ ou Cλ d'une immunoglobuline humaine, l'autre à la région constante CH1 d'une immunoglobuline humaine.

Selon un deuxième mode de réalisation de l'invention, les formats d'anticorps sont de type Fab' et sont caractérisés par l'association de deux domaines VHH, identiques ou différents, l'un des domaines étant fusionné à la région constante Cκ ou Cλ d'une immunoglobuline humaine, l'autre à la région constante CH1 suivie d'une région charnière H d'une immunoglobuline humaine.

Ces formats d'anticorps chimérisés ou humanisés sont de type monospécifique/bivalent, bispécifique/monovalent et biépitopiquc/monovalent.

Selon un troisième mode de réalisation de l'invention, les formats d'anticorps sont de type F(ab')₂ et sont caractérisés par l'association de deux formats de type Fab' tels que définis ci-dessus.

Ces formats d'anticorps chimérisés ou humanisés possèdent une région charnière H, "Hinge" d'une immunoglubuline humaine et permettent les combinaisons monospécifique/tétravalent, bispécifique/bivalent et biépitopique/bivalent.

Selon un quatrième mode de réalisation de l'invention, les formats d'anticorps sont de type F(ab')₂ et sont caractérisés par l'association de deux Fab' obtenus par réduction des formats de type F(ab')₂ ci-dessus.

Ces formats d'anticorps chimérisés ou humanisés possèdent une région charnière H "Hinge" d'une immunoglobuline humaine et permettent les combinaisons de monospécifique/tétravalent à tétraspécifique/monovalent ou tétraépitopique/monovalent, incluant toutes les possibilités intermédiaires.

Selon un cinquième mode de réalisation de l'invention, les formats d'anticorps sont de type (HCH2CH3)₂ (H, représentant la région charnière "Hinge" d'une immunoglobuline humaine, CH2 et CH3, représentant le second et troisième domaine constant d'une chaîne lourde d'une Ig humaine, et sont caractérisés par l'association de deux VHH identiques, chacun étant fusionné à la région H-CH2-CH3 d'une Ig humaine.

Ces formats d'anticorps chimérisés ou humanisés permettent les combinaisons monospécifique/bivalent.

Selon un sixième mode de réalisation de l'invention, les formats d'anticorps sont de type mAb* (ce type désigne les domaines variables d'origine remplacés par tout ou partie des domaines de VHH, fusionnés à des régions constantes d'anticorps humains) et sont caractérisés par l'association de deux VHH, identiques ou différents, l'un étant fusionné à la région Cκ ou Cλ humaine, l'autre à la région CH1-H-CH2-CH3 d'une Ig humaine.

Ces formats d'anticorps chimérisés ou humanisés permettent les combinaisons de type monospécifique/tétravalent et bispécifique/bivalent et biépitopique/bivalent.

Dans ces différents formats, l'immunoglobuline est une IgG, correspondant à une isoforme IgG1, IgG2, IgG3 ou IgG4 humaine, ou une IgA humaine correspondant à une isoforme IgA1, IgA2, ou toute autre Ig humaine.

Le présent mémoire décrit des fragments d'anticorps VHH de camélidés, notamment de lamas. Il s'agit en particulier de fragments caractérisés en ce qu'il s'agit d'une partie ou de la totalité de fragments anti-antigène carcinoembryonnaire (anti-CEA en abrégé) ou anti-récepteur FcγRIII (anti-CD16 en abrégé).

Les fragments d'anticorps anti-CEA répondent plus particulièrement à une séquence en acides aminés choisie dans le groupe comprenant les séquences SEQ ID N° 77, SEQ ID N° 78, SEQ ID N° 79, SEQ ID N° 80 et SEQ ID N° 105.

Les fragments d'anticorps anti-CD16 répondent de manière préférée à une séquence en acides aminés choisie dans le groupe comprenant les séquences SEQ ID N° 73, SEQ ID N° 74, SEQ ID N° 75, SEQ ID N° 76, SEQ ID N° 103 et SEQ ID N° 104.

Ces fragments anti-CD16 constituent des produits nouveaux et à ce titre entrent également dans le champ de l'invention.

L'invention décrit aussi les CDRs de ces fragments VHH.

L'invention décrit également un procédé de production d'anticorps chimérisés ou humanisés, multispécifiques et/ou multivalents pour l'immunothérapie ou l'immunodiagnostic, caractérisé en ce qu'il comprend l'utilisation de formats d'anticorps définis ci-dessus.

Il s'agit plus spécialement des domaines variables de VHH anti-CEA et anti-CD16 avantageusement produits selon un protocole comprenant
- l'immunisation de camélidés, notamment de lamas avec, comme immunogène, un CEA ou un CD16,
- la purification des lymphocytes B récupérés à partir du sang,
- la construction de banque de VHH, et
- l'isolement des VHH à partir de banque.

La construction de la banque comprend
- l'extraction des ARN totaux des lymphocytes B,
- la transcription inverse des ARN pour obtenir les ADNc correspondants,
- l'amplification par PCR des gènes codant pour les régions variables des anticorps simple chaîne lourde anti-CD16 et anti-CEA,
- la ligation de fragments d'ADN VHH obtenus par coupure, par des enzymes, des ADN amplifiés avec un phagemide.

Les VHH sont isolés à partir des banques par la technique de phage display et purifiés.

Lesdits domaines variables de VHH anti-CEA et anti- CD16 sont avantageusement produits selon un protocole comprenant
- l'immunisation de camélidés, notamment de lamas avec, comme immunogène,
   un CEA ou un CD 16,
- la purification des lymphocytes B récupérés à partir du sang,
- la construction de banque de VHH, et
- l'isolement des VHH à partir de la banque.

De manière avantageuse, la construction de la banque comprend
- l'extraction des ARN totaux des lymphocytes B,
- la transcription inverse des ARN pour obtenir les ADNc correspondants,
- l'amplification par PCR des gènes codant pour les régions variables des anticorps simple chaîne lourde anti-CD 16 et anti-CEA,
- la ligation de fragments d'ADN VHH, obtenus par coupure par des enzymes des ADN amplifiés, avec un phagemide.

Les VHH sont isolés à partir des banques par la technique de phage display et purifiés.

Les différents VHH ont été validés en termes de spécificité et d'affinité comme illustré par les exemples.

Les gènes des VHH sélectionnés ont ensuite été introduits dans des vecteurs d'expression, notamment des plasmides, pour produire différents formats d'anticorps chimérisés multispécifiques et/ou multivalents (anti-CEA/anti-CD16), capables de se lier aux cellules tumorales exprimant le CEA à leur surface et de recruter les cellules effectrices du système immunitaire (monocytes, macrophages, NK, polynucléaires neutrophiles...) qui expriment le CD16.

L'invention décrit également les vecteurs d'expression des formats d'anticorps définis ci-dessus.

Elle décrit plus spécialement des vecteurs d'expression, notamment des plasmides renfermant entre deux sites uniques d'enzymes de restriction les promoteurs, les séquences signal, les séquences nucléotidiques capables de coder pour les domaines VHH définis ci-dessus, les régions constantes d'une Ig humaine, ou pour des domaines VH humains, les régions CDRs d'un VHH, les régions constantes d'une Ig humaine.

Les plasmides selon le présent mémoire sont capables d'exprimer en quantités élevées les formats d'anticorps définis ci-dessus, sous des formes solubles dans des bactéries et les régions codant pour les domaines d'anticorps peuvent être facilement transférées dans d'autres systèmes d'expression procaryotes ou encore eucaryotes.

L'invention décrit ainsi des plasmides **pCκCH1γ1-TAG** (SEQ ID N°98 et SEQ ID N°112) **et pCκCH1γ1**(SEQ ID N°100 et SEQ ID N°114) permettant la production des anticorps de type Fab selon le premier mode de réalisation de formats d'anticorps défini ci-dessus.

Ces plasmides sont plus spécialement caractérisés par l'insertion des séquences nucléotidiques codant pour la région légère Cκ, et la région constante lourde CH1 d'une Ig dans le **plasmide p55Flag/RBS/35cmyc6HisGS** (SEQ ID N°94 et SEQ ID N°110).

L'invention décrit également les plasmides **pCκCH1γ1-TAG** (SEQ ID N°99 et SEQ ID N°113) **et pCκCH1Hγ1** (SEQ ID N°101 et SEQ ID N°115) permettant la production des anticorps de type Fab' et F(ab')₂ selon les deuxième, troisième et quatrième mode de réalisation de formats d'anticorps défini ci-dessus.

Ces plasmides sont plus spécialement caractérisés par l'insertion des séquences nucléotidiques codant pour la chaîne lourde CH1 et la région Hinge (H) d'une Ig dans **p55CκFlag/RBS/35cmyc6HisGS** (SEQ ID N°97 et SEQ ID N°111).

L'invention décrit encore les plasmides **pHCH2CH3γ1-TAG** (SEQ ID N°95) **et pHCH2CH3γ1** (SEQ ID N°96) permettant la production des anticorps de type (HCH2CH3)₂ selon le cinquième mode de réalisation de formats d'anticorps défini ci-dessus.

Ces plasmides sont plus spécialement caractérisés par l'insertion des séquences nucléotidiques codant pour la région Hinge (H) et les régions constantes CH2 et CH3 d'une Ig dans **p55Flag/RBS/35cmyc6HisGS**.

L'invention décrit aussi le plasmide **pMabγ1*** (SEQ ID N°102 et SEQ ID N°116) permettant la production des anticorps de type mAb* selon le sixième mode de réalisation de l'invention.

Ce plasmide est plus spécialement caractérisé par l'insertion des séquences nucléotidiques codant pour la région constante lourde CH1, la région Hinge et les régions constantes CH2 et CH3 d'une Ig dans **pCκCHγ-TAG.**

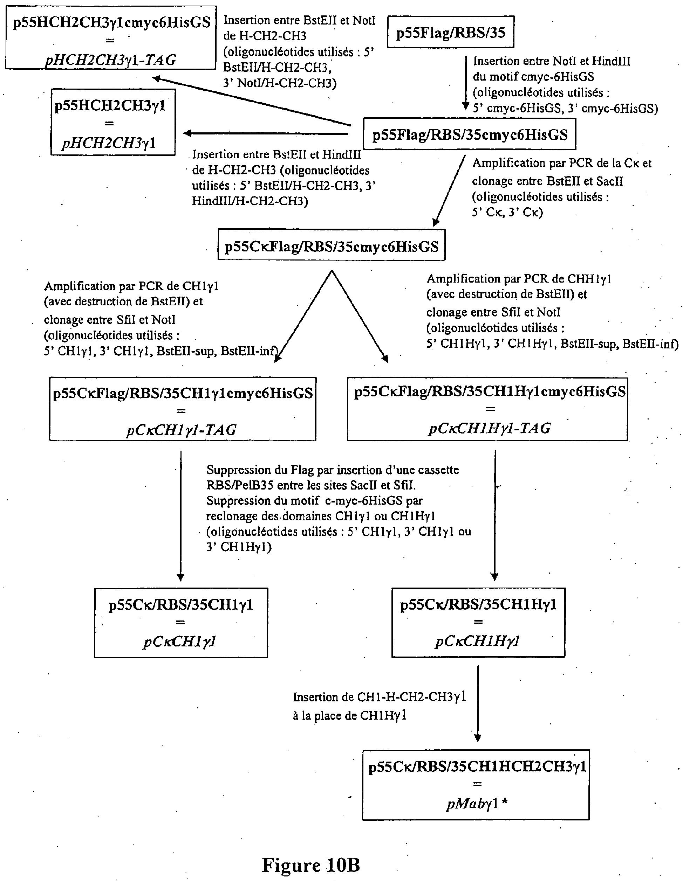

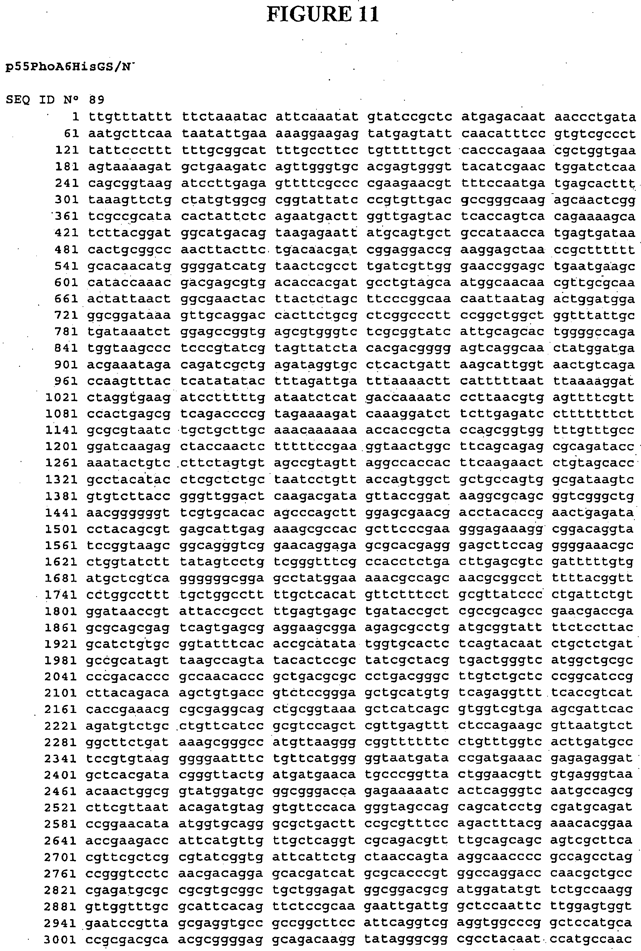

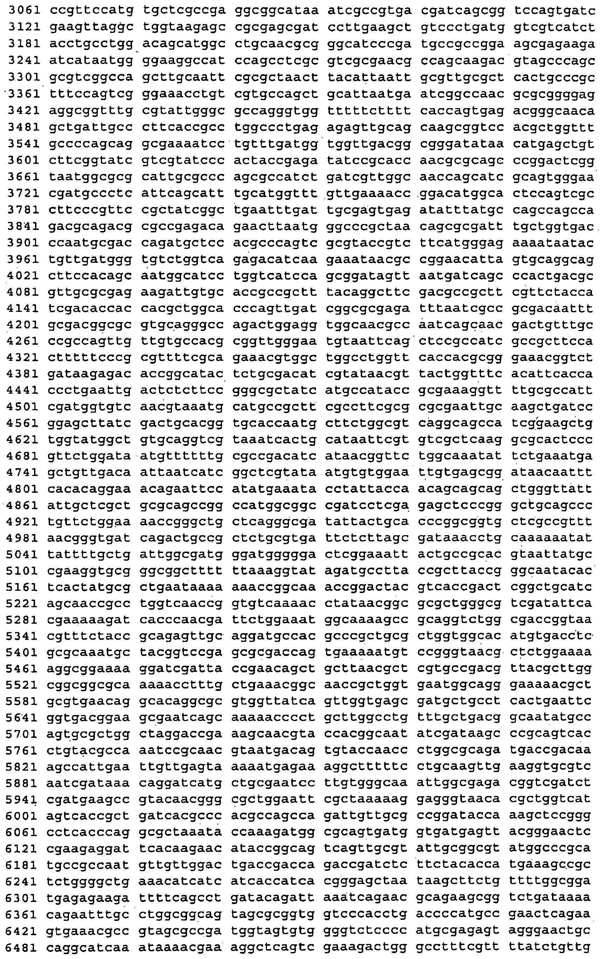

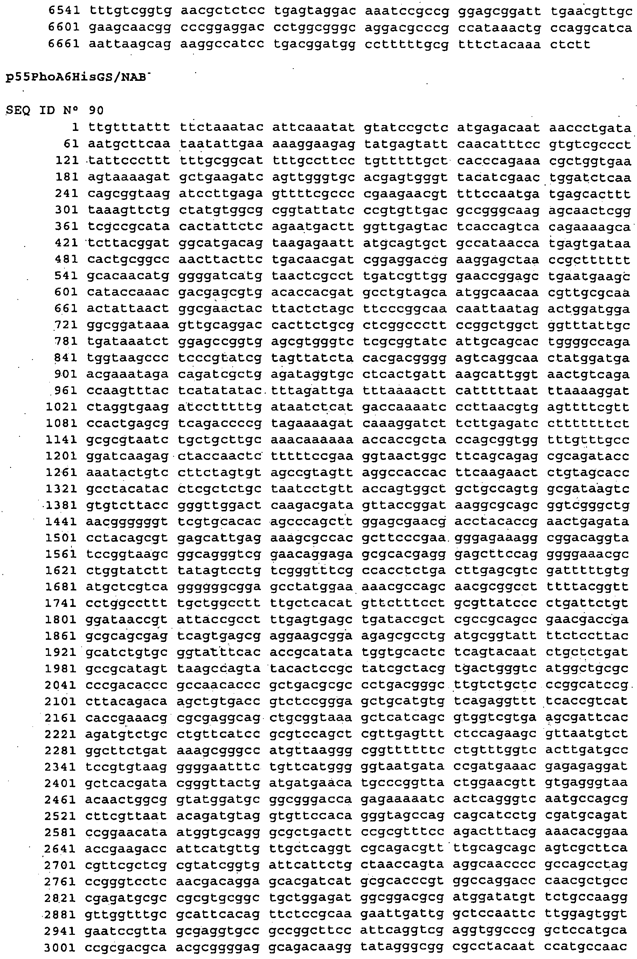

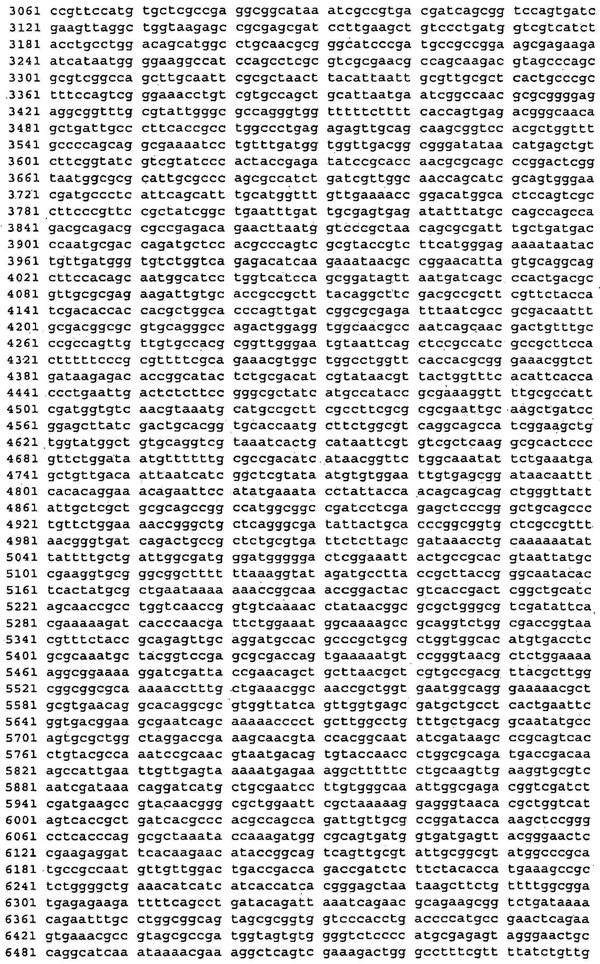

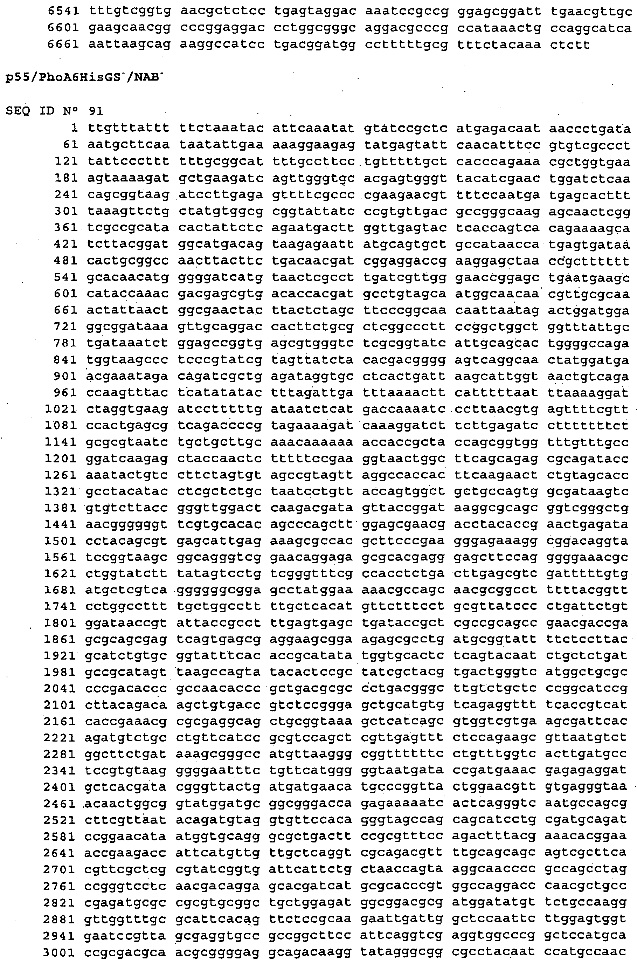

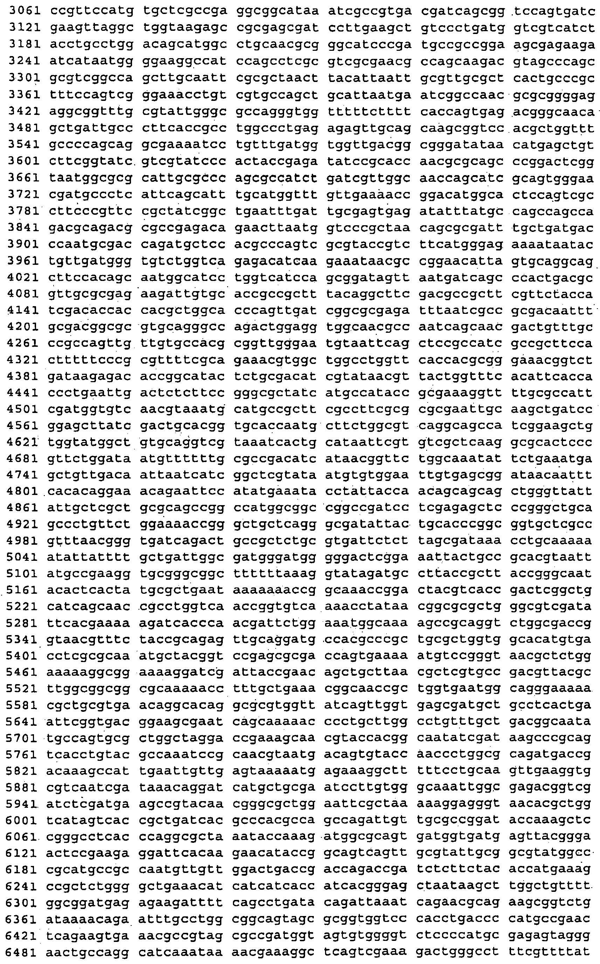

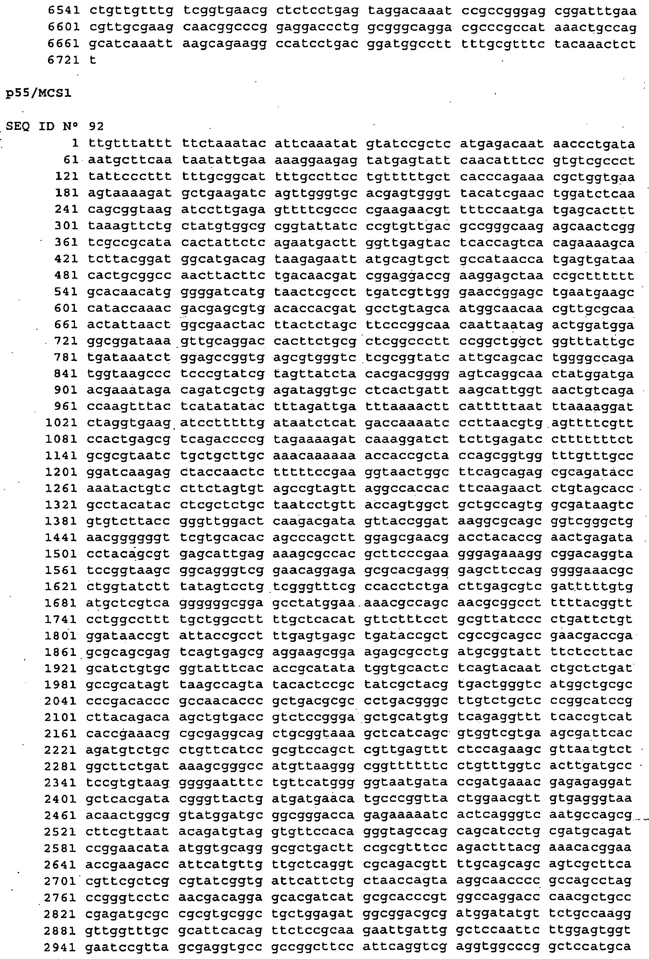

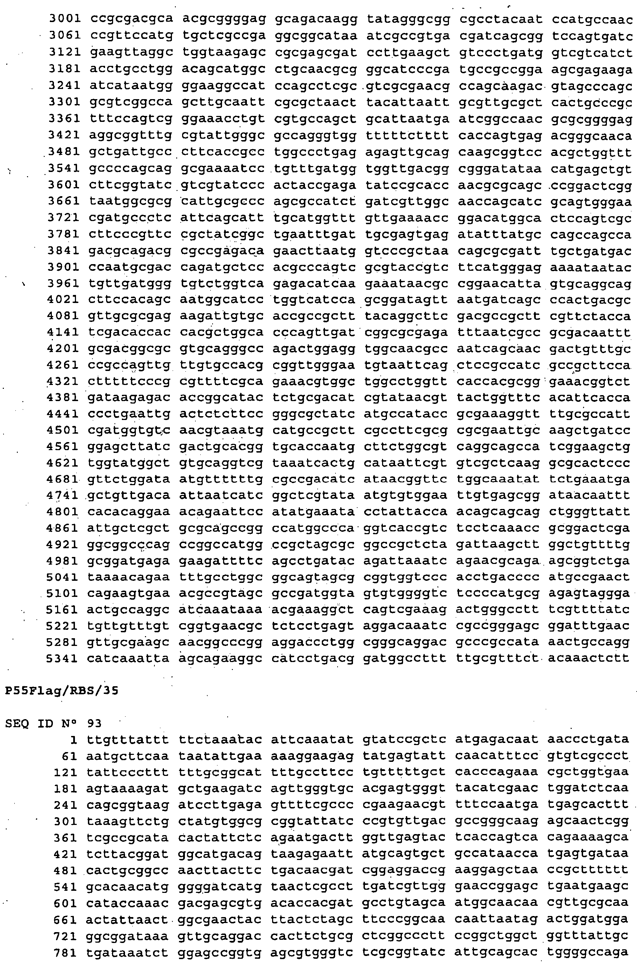

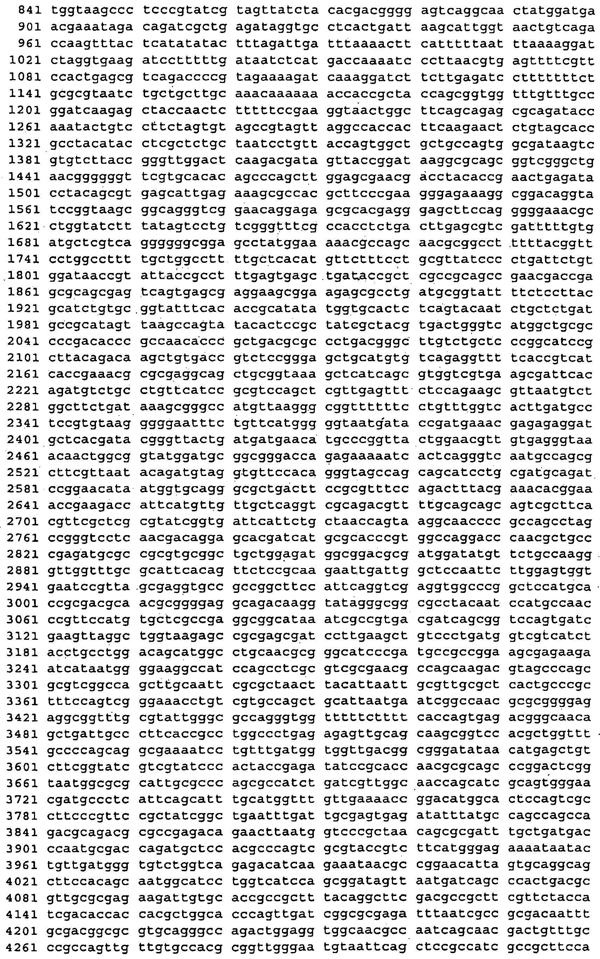

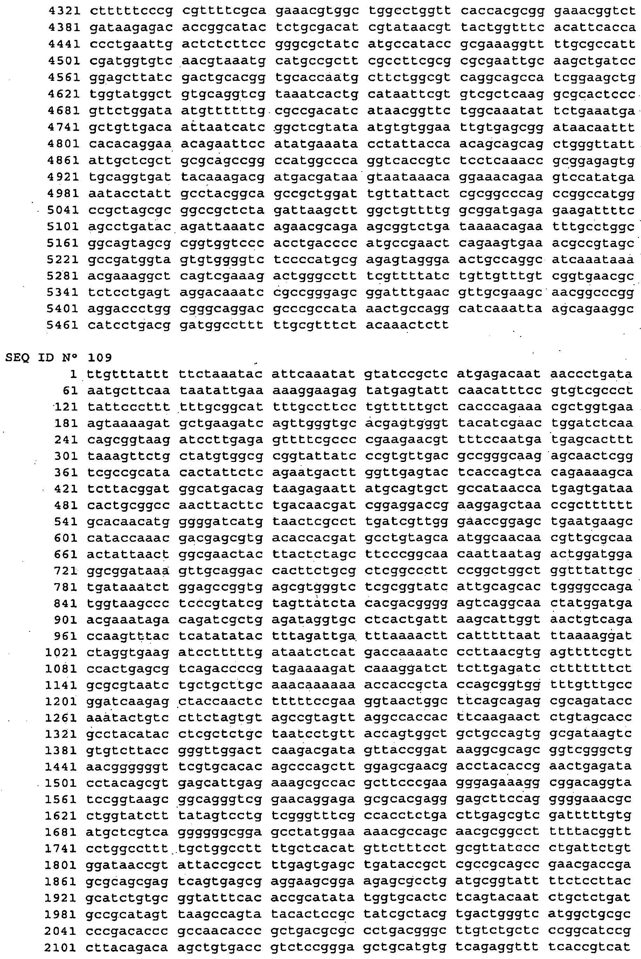

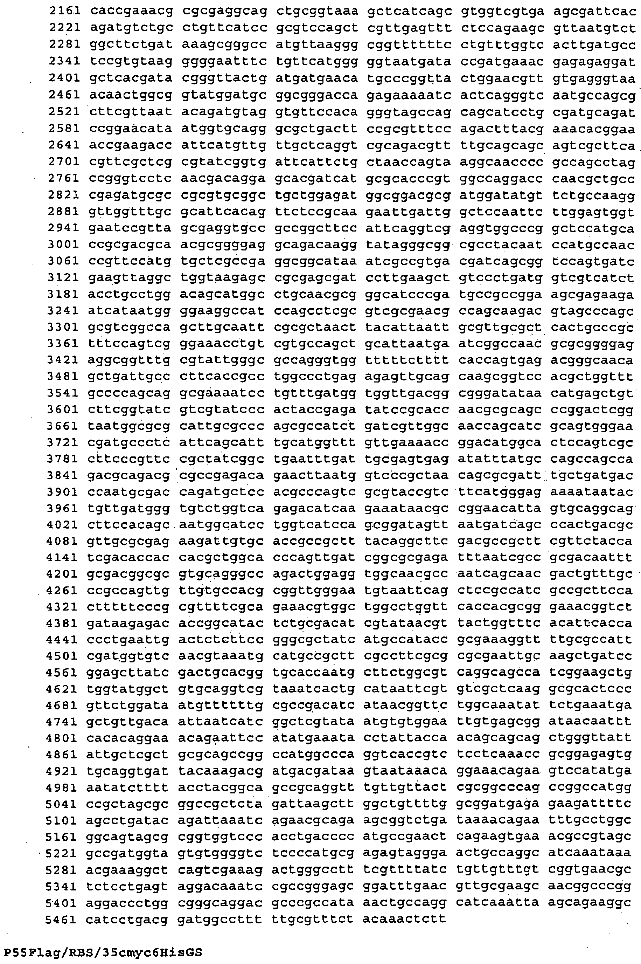

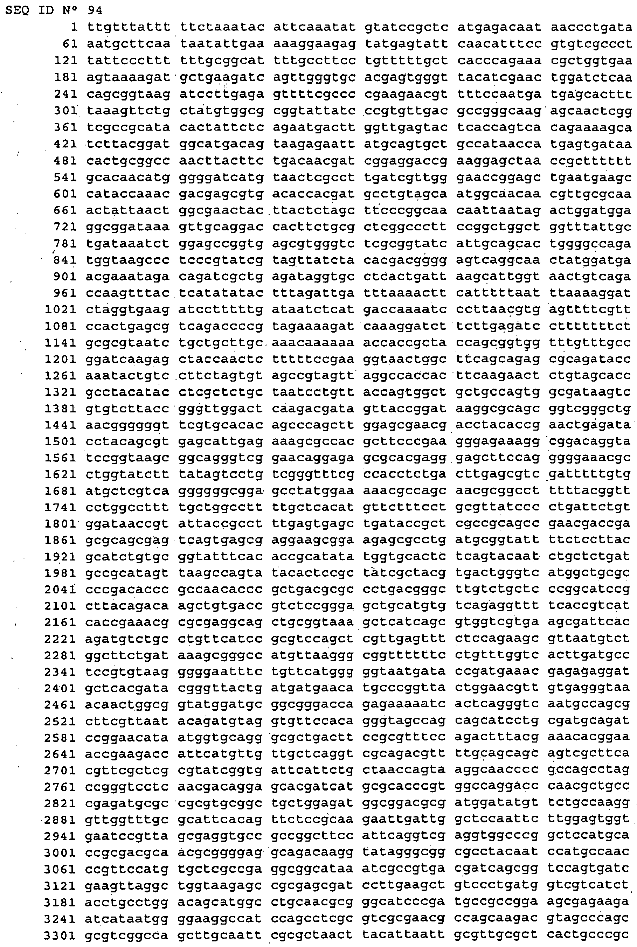

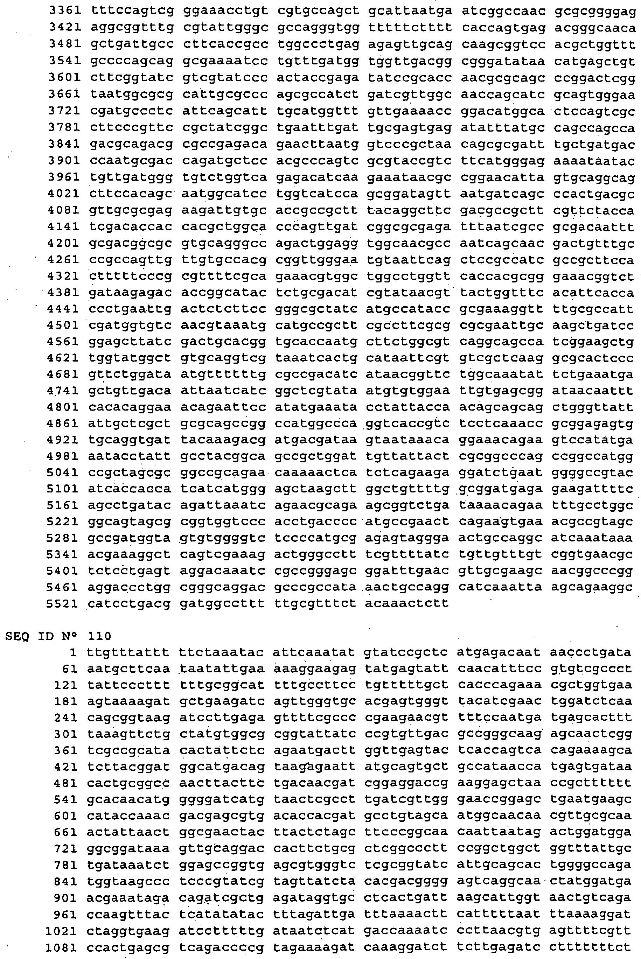

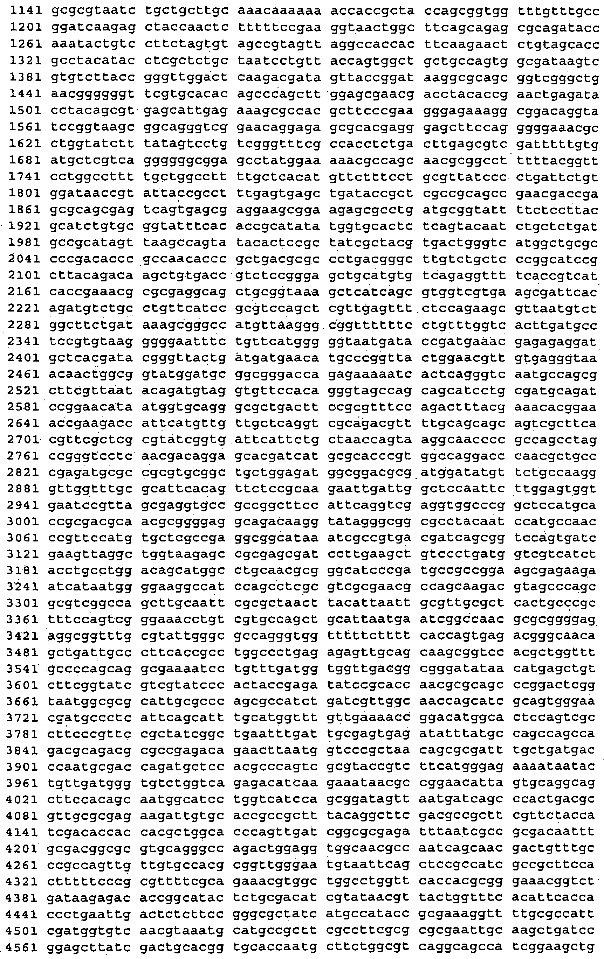

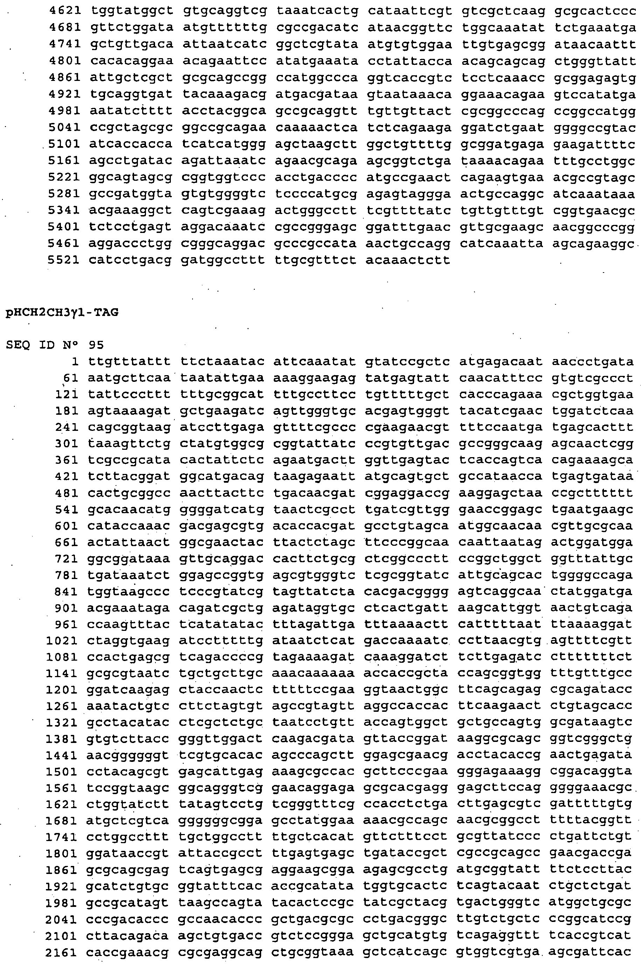

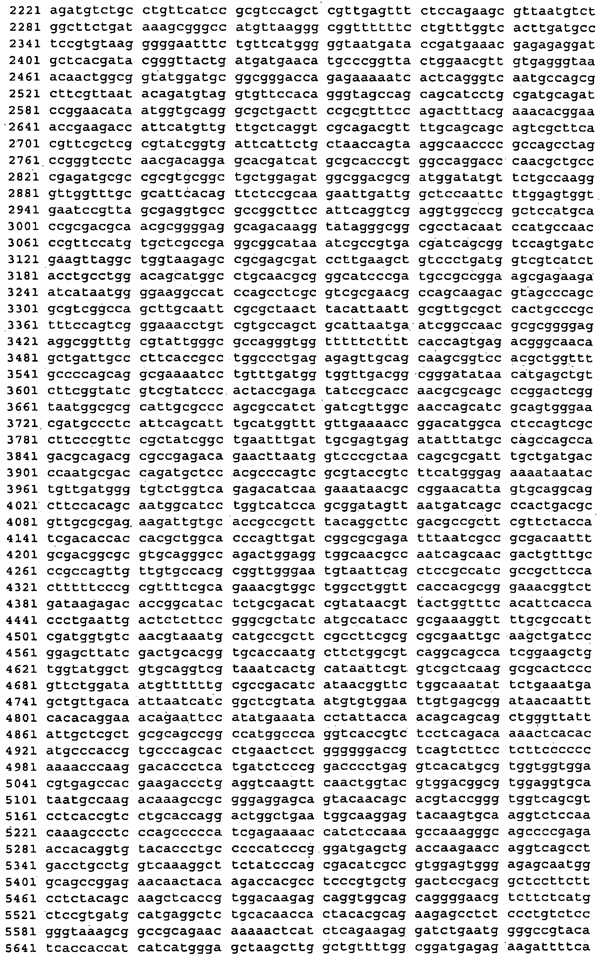

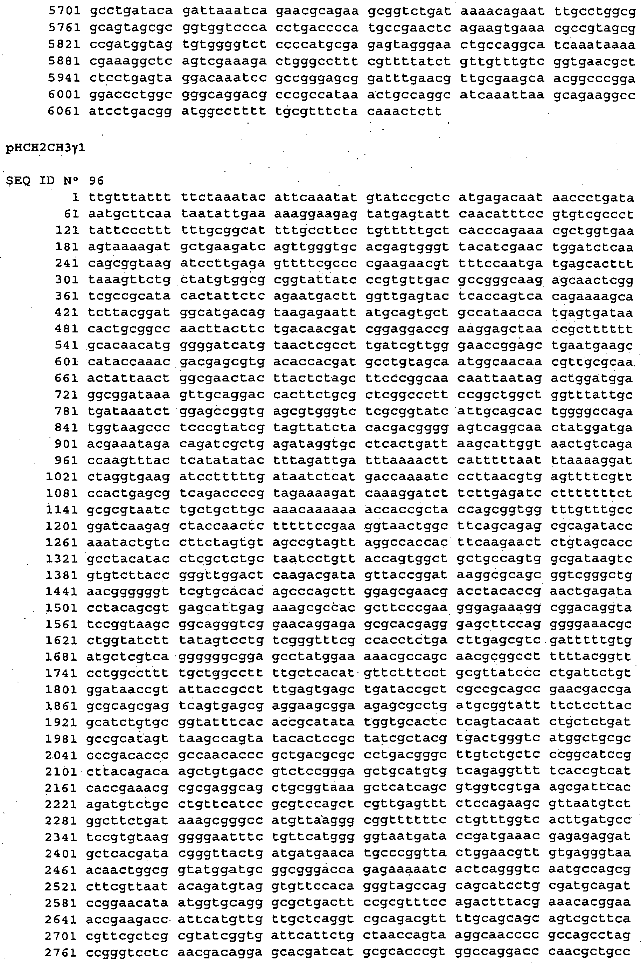

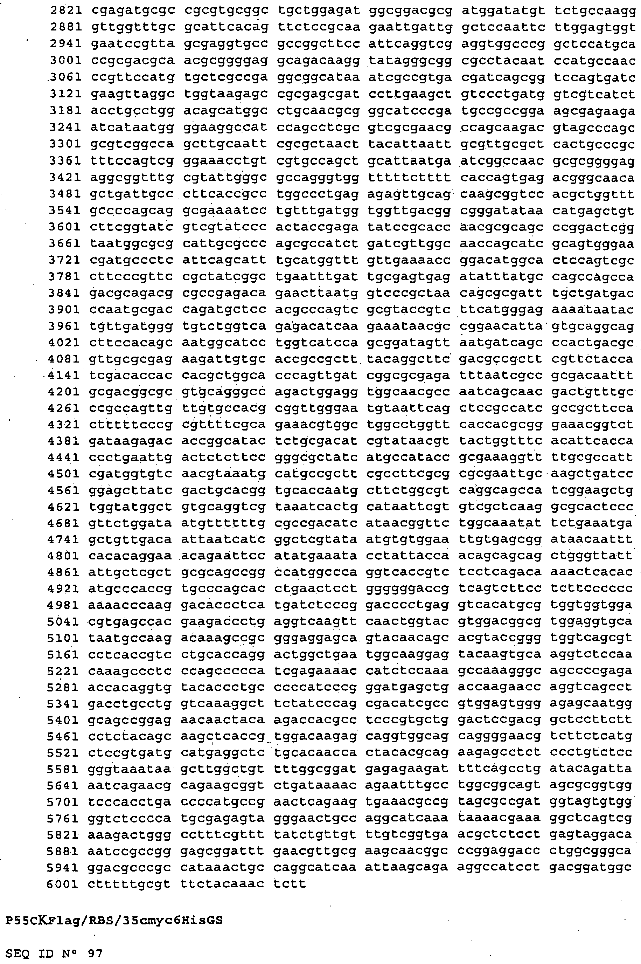

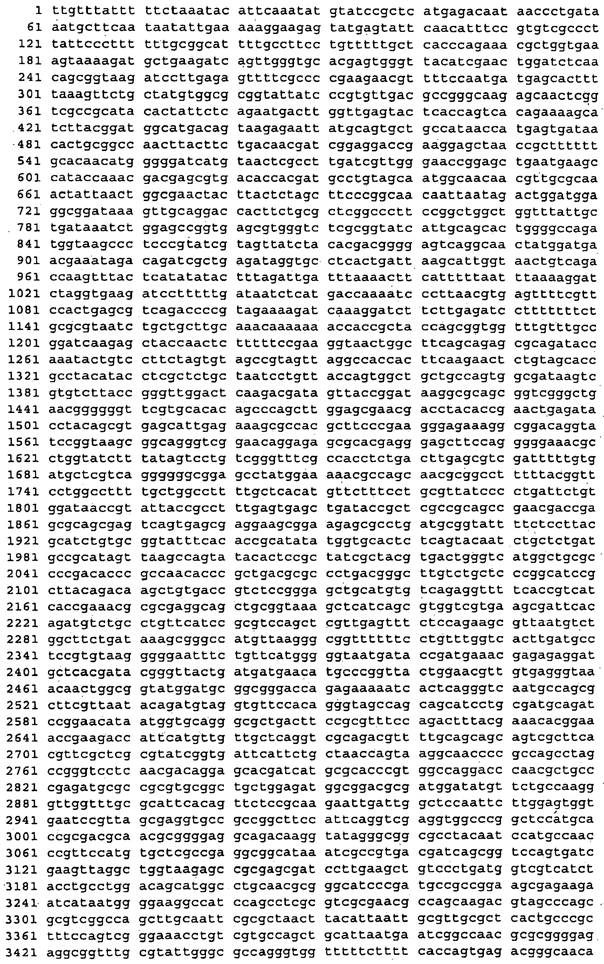

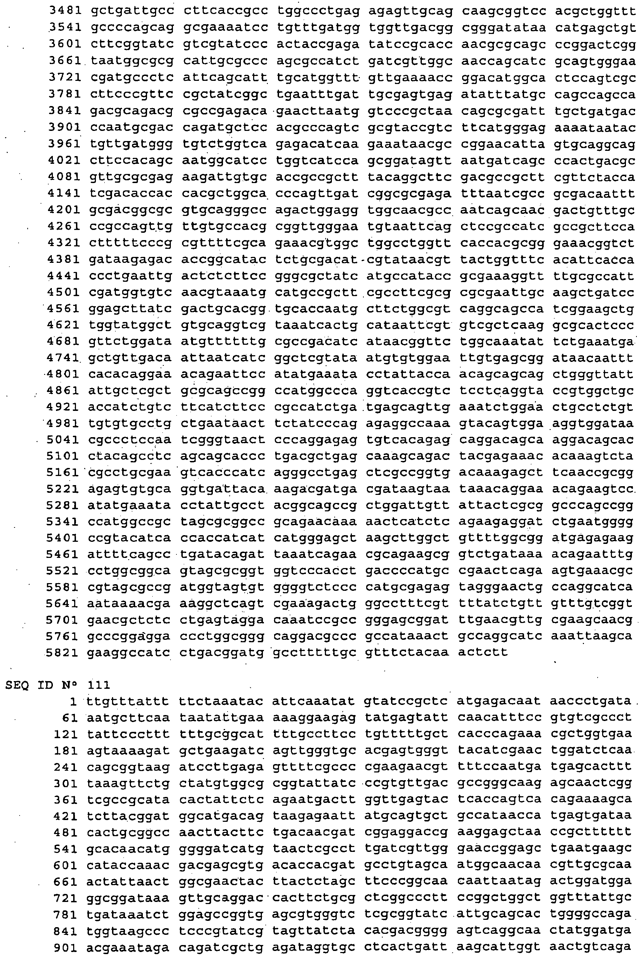

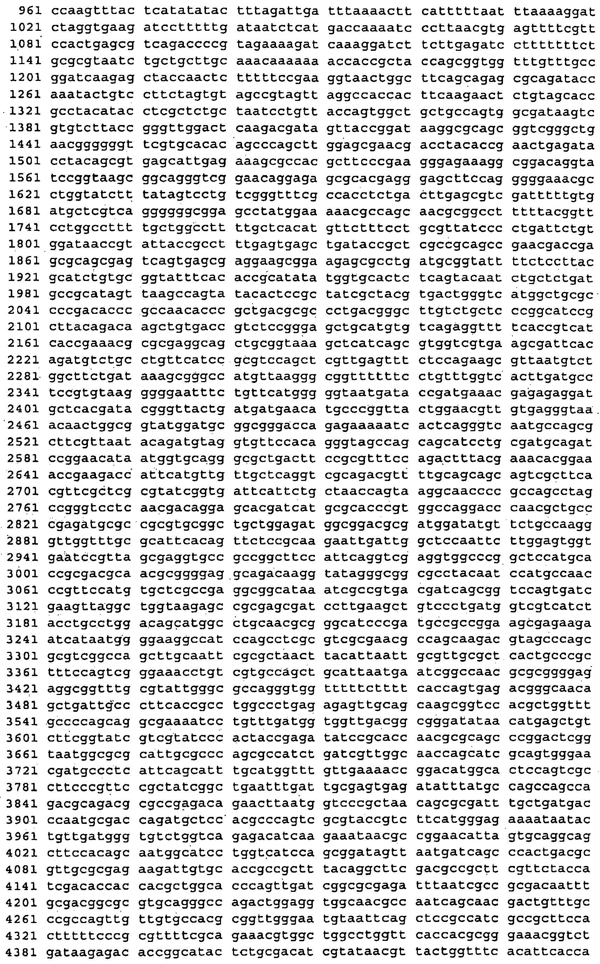

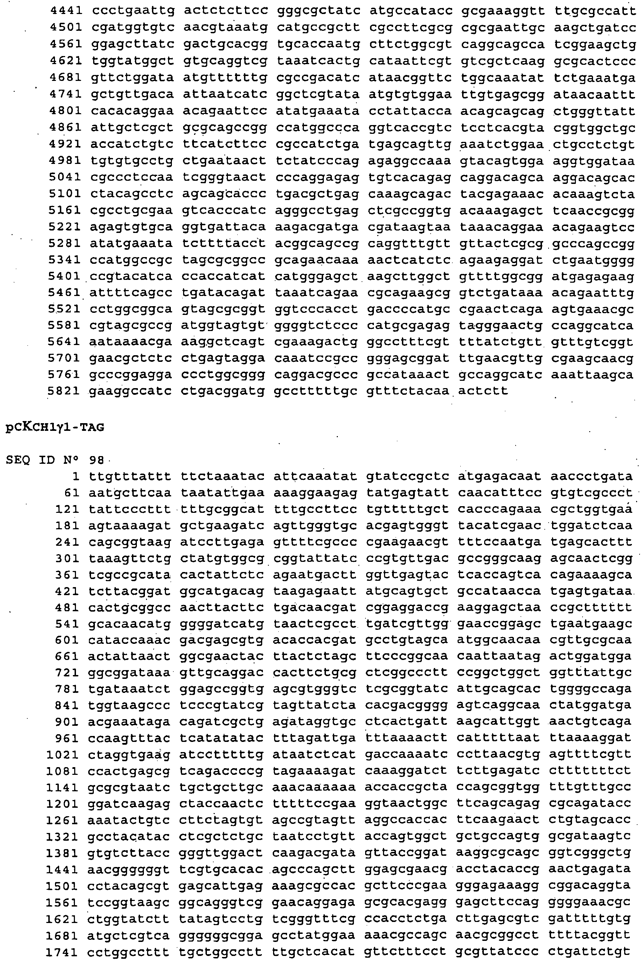

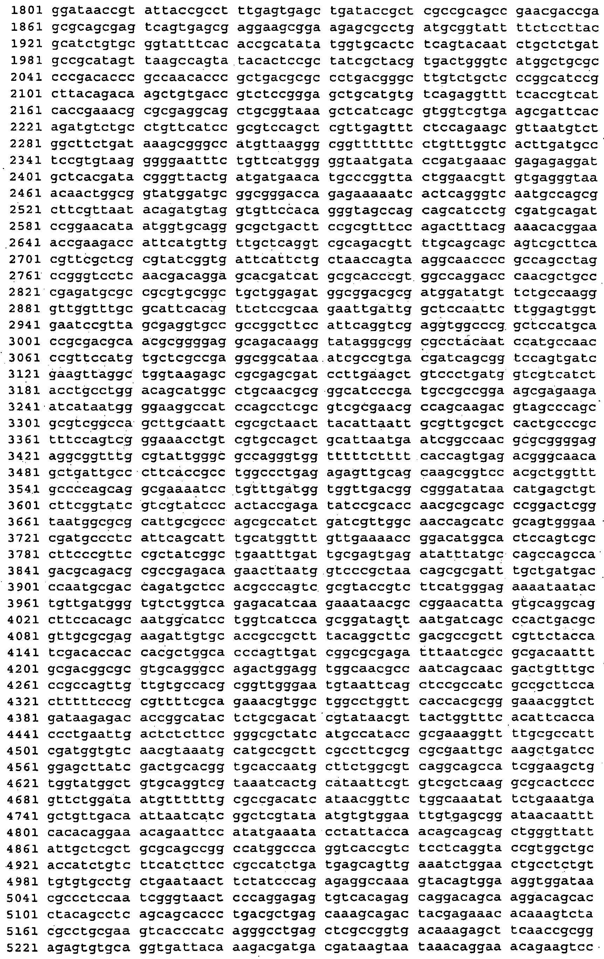

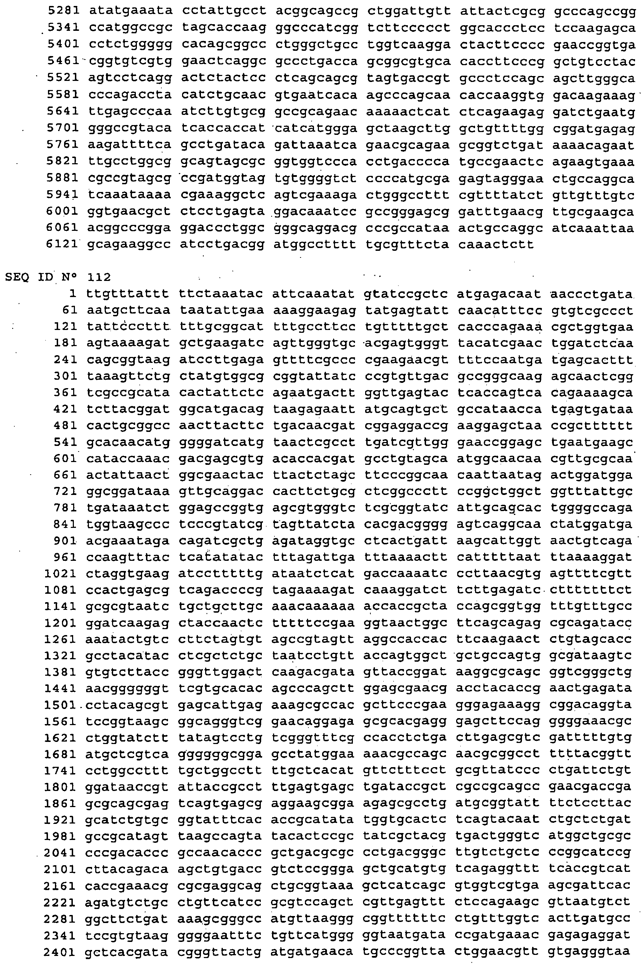

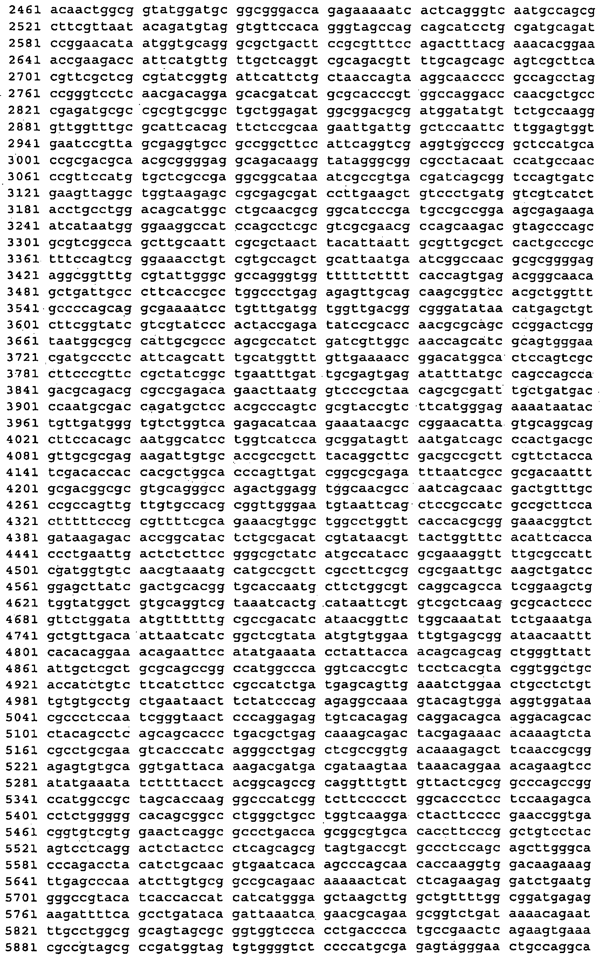

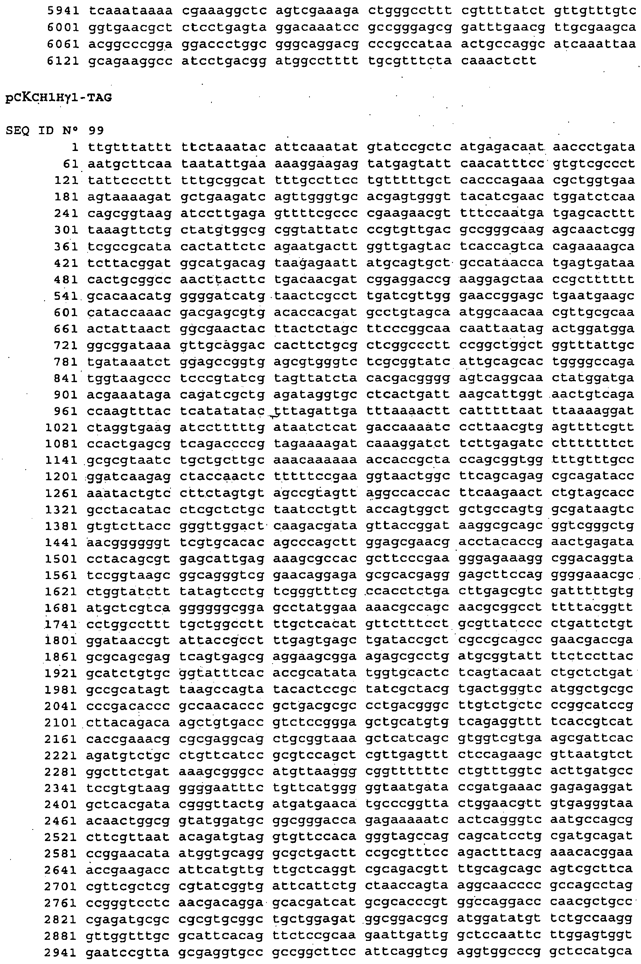

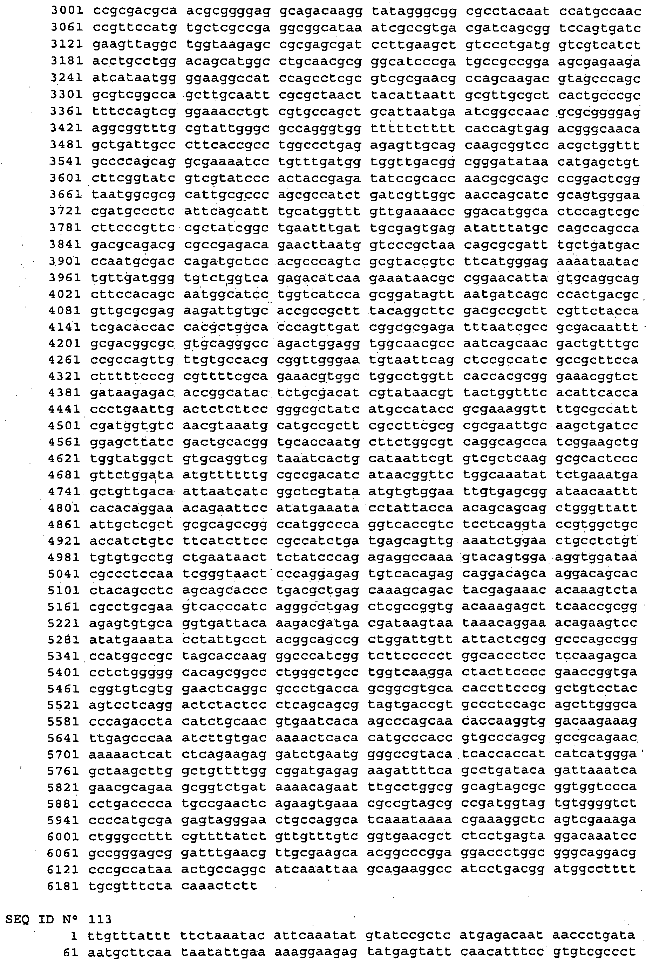

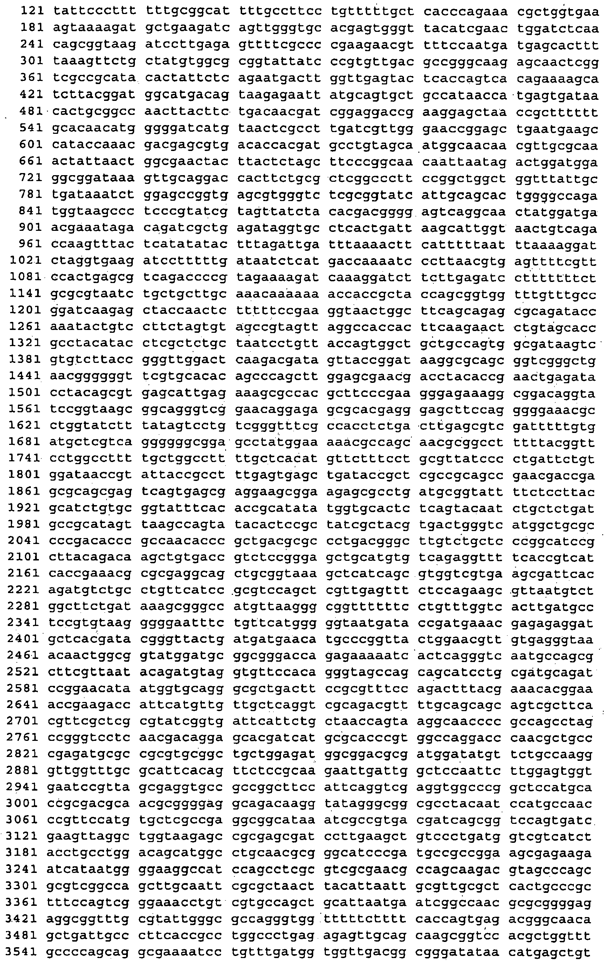

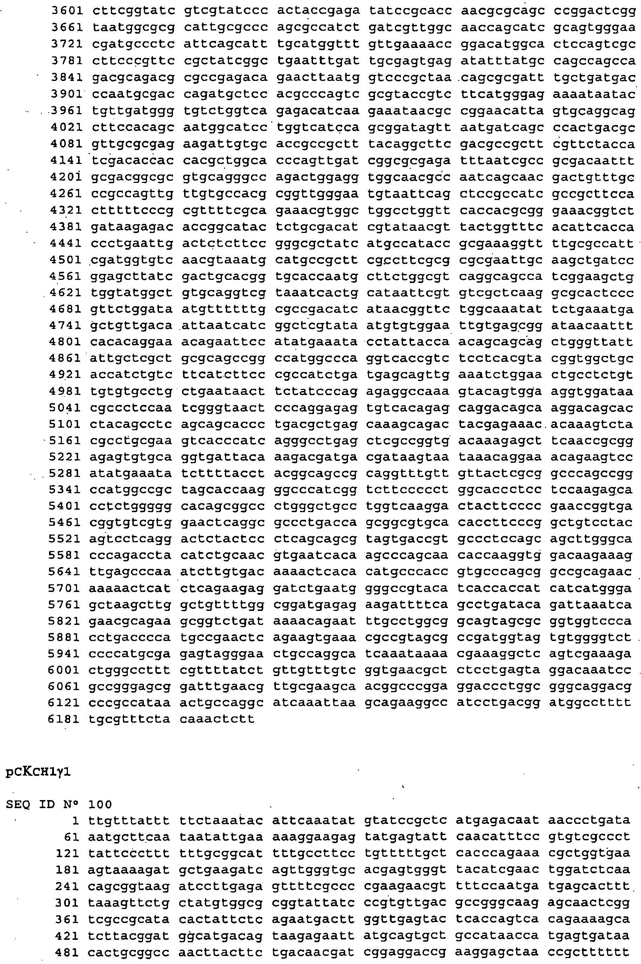

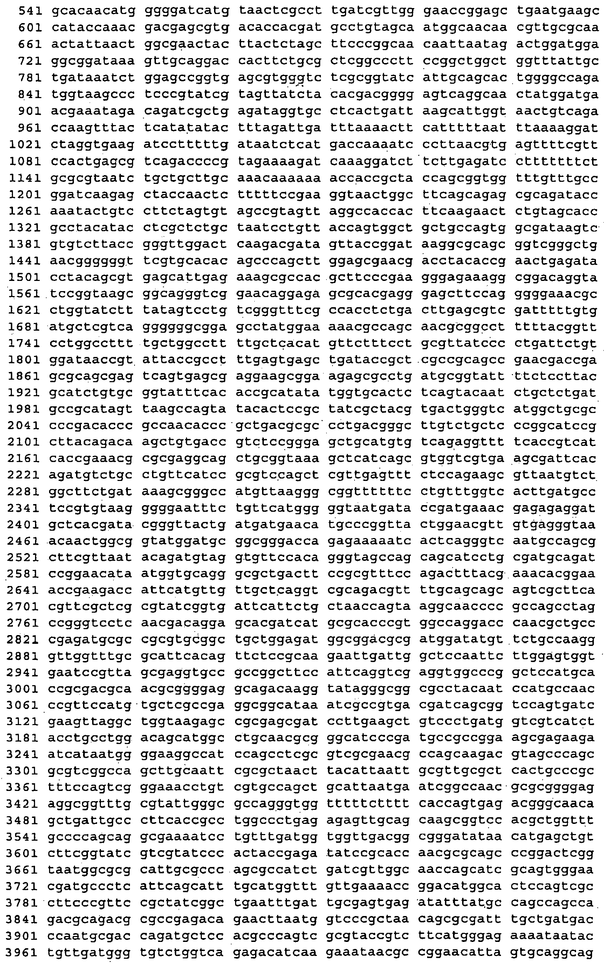

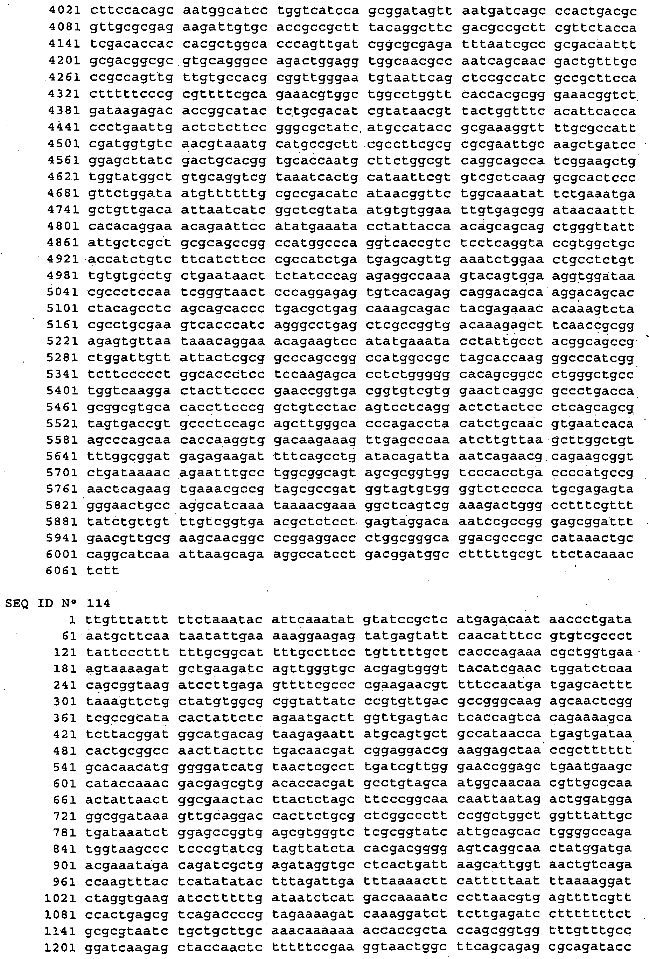

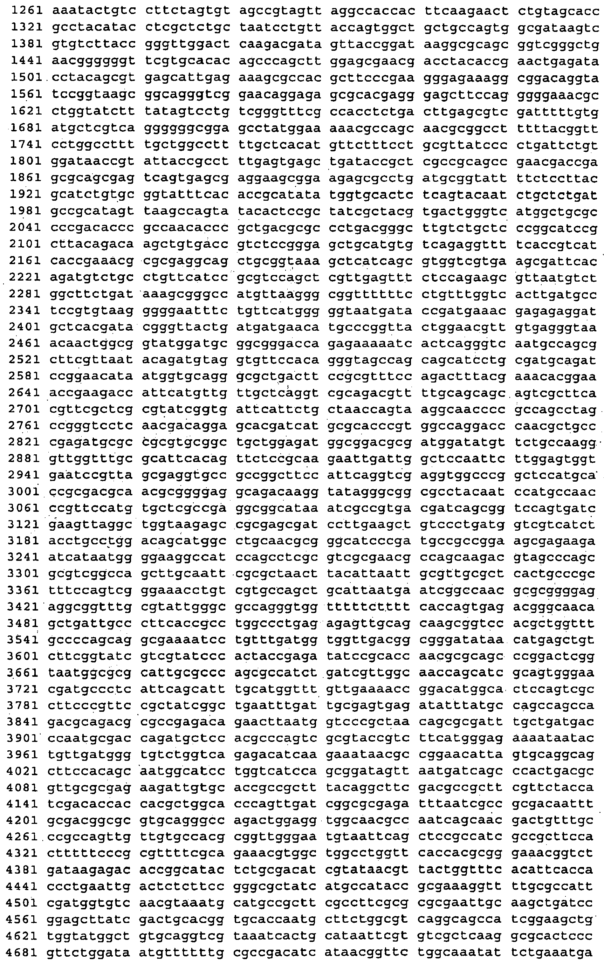

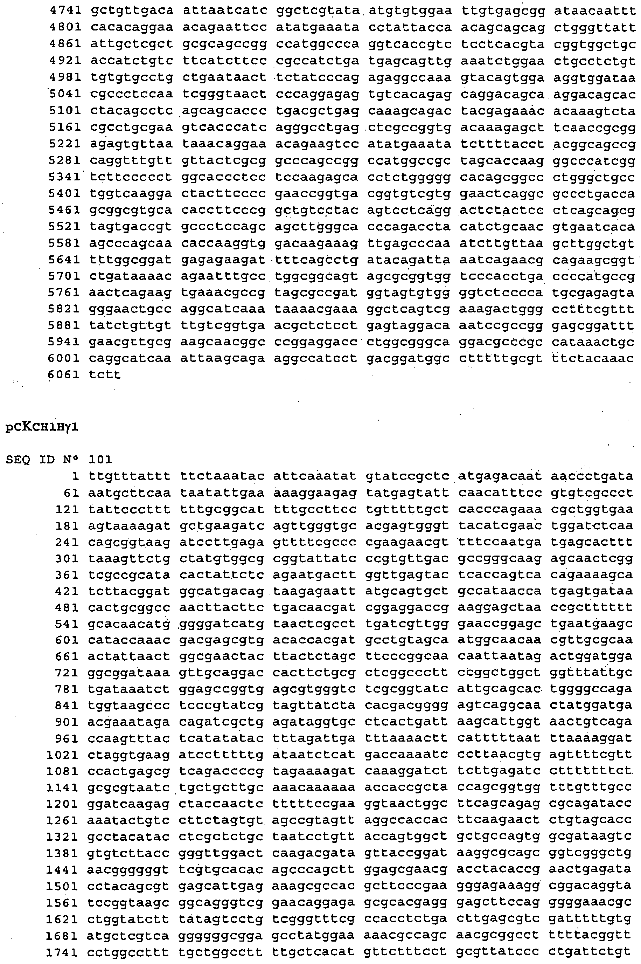

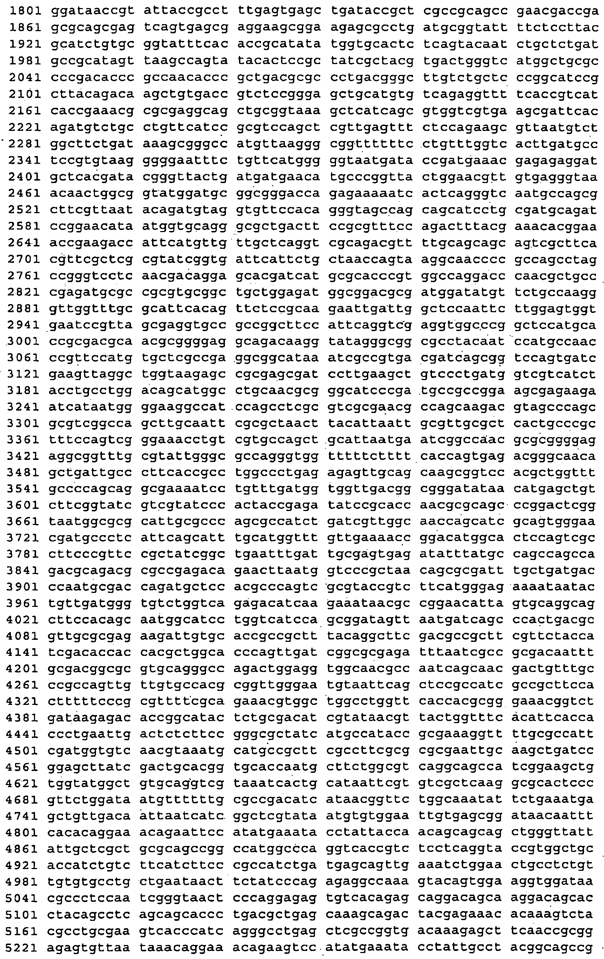

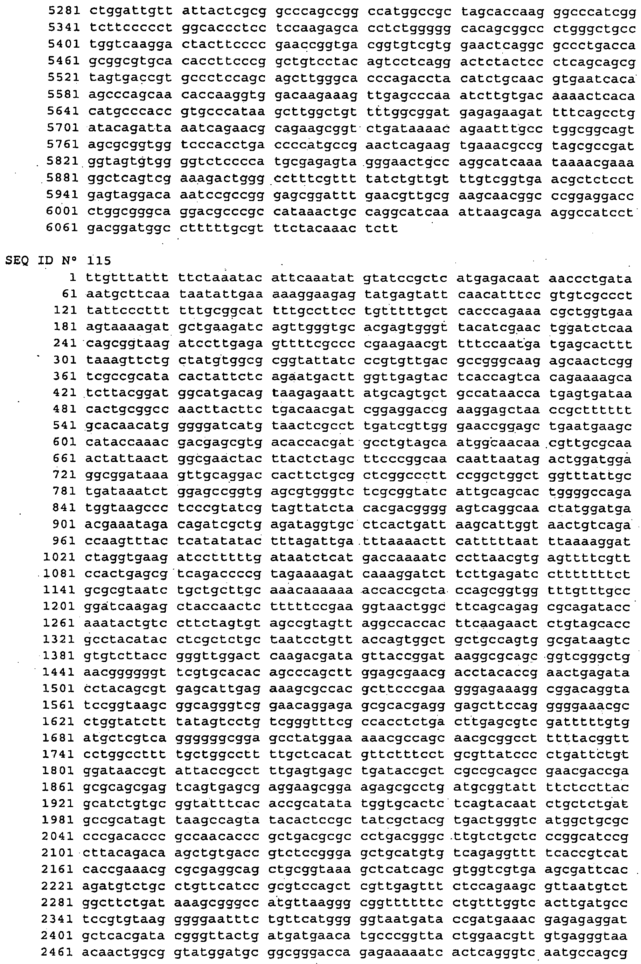

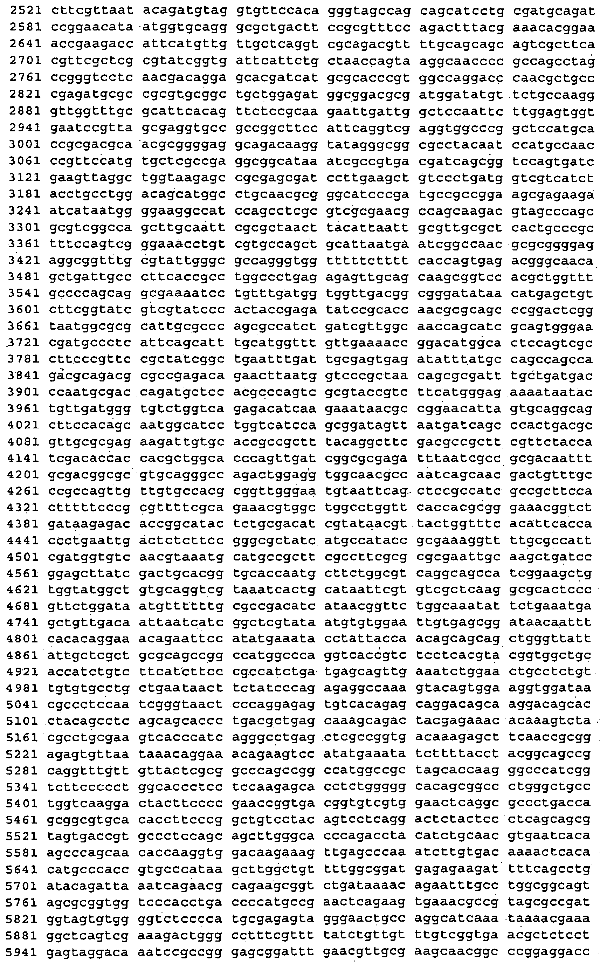

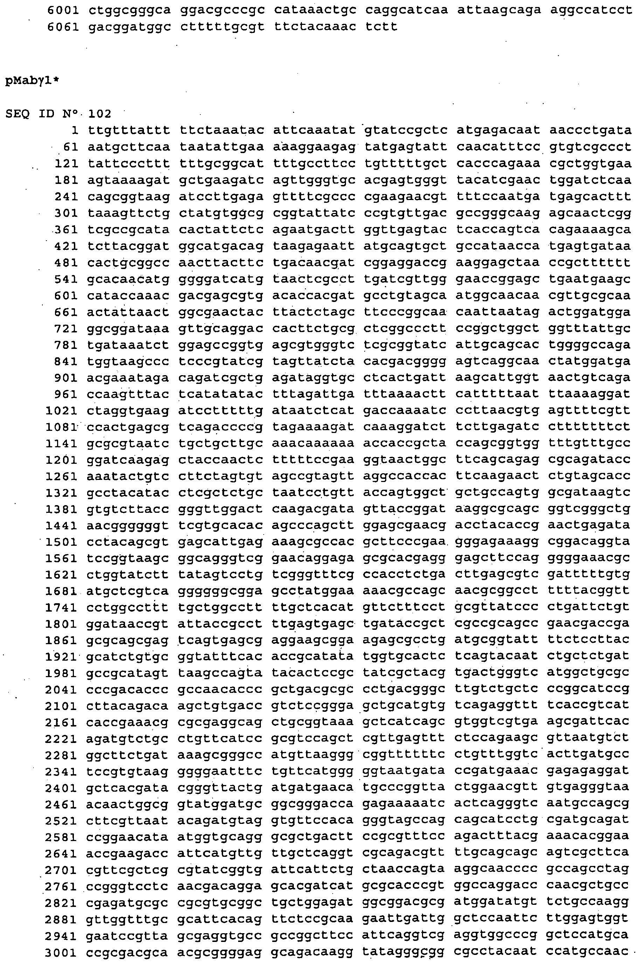

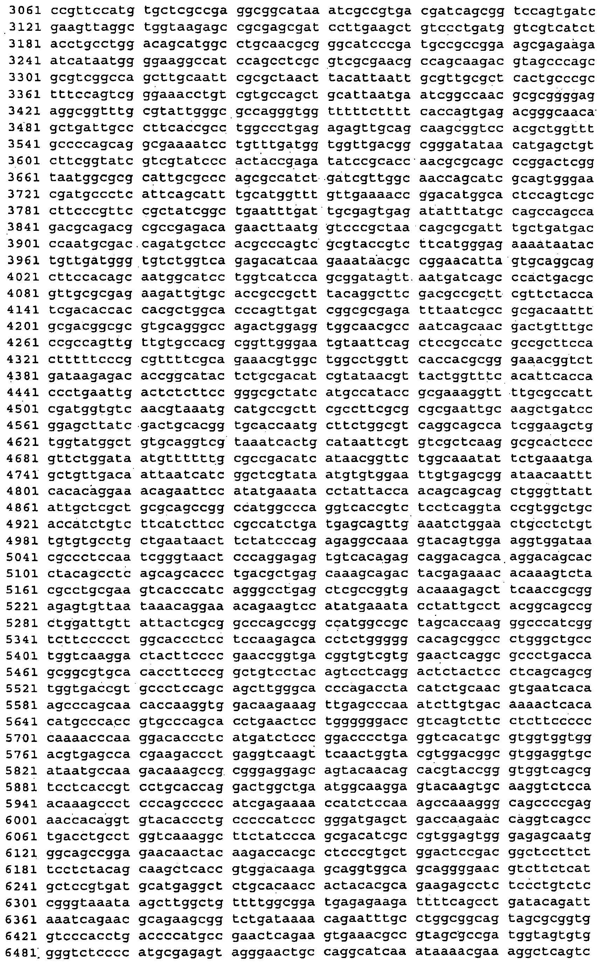

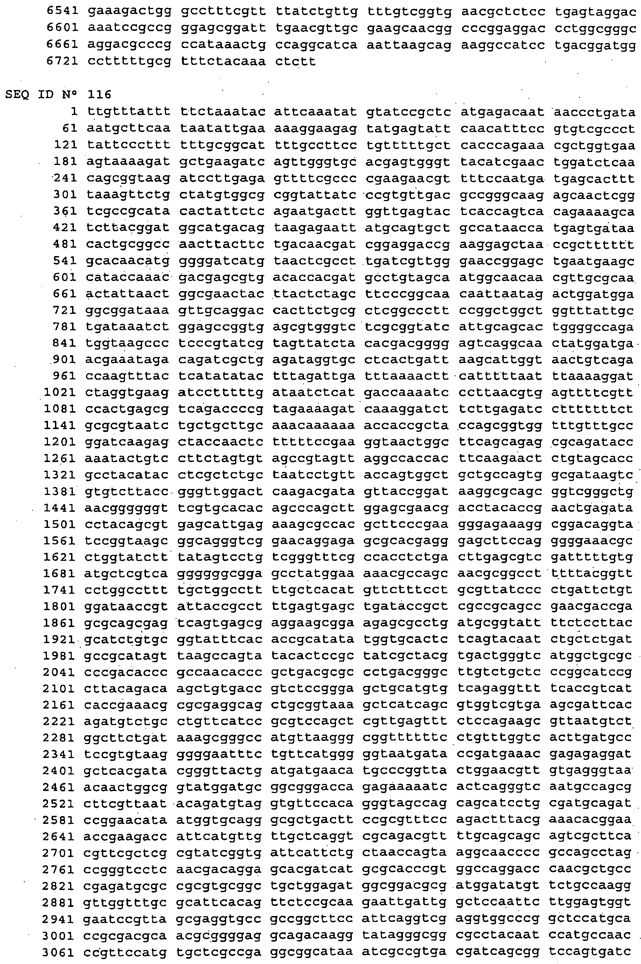

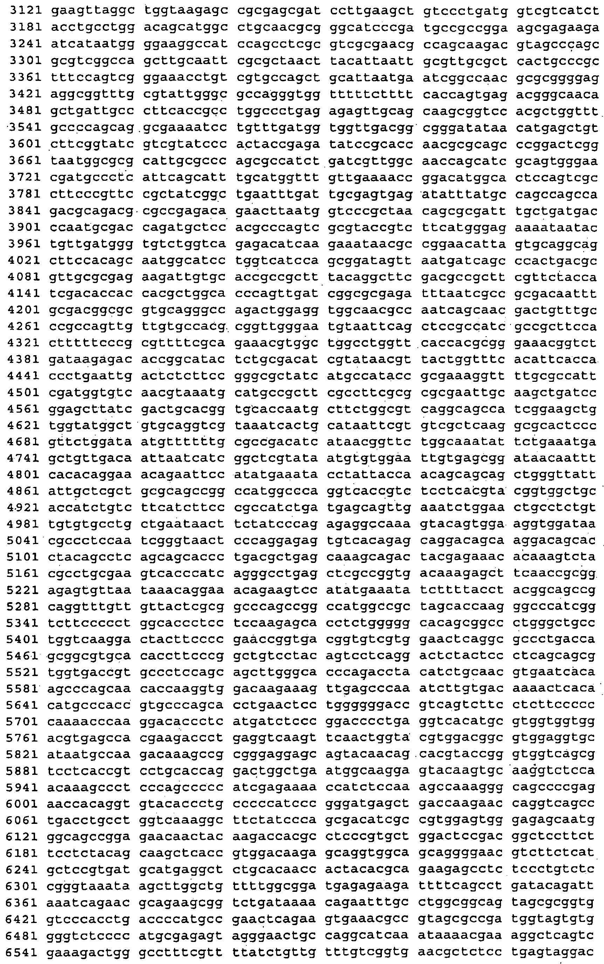

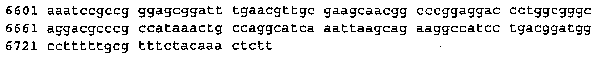

Les schémas de ces plasmides sont illustrés sur la figure 10B et leurs séquences nucléotidiques dans la figure 11. Les plasmides intermédiaires utilisés pour la construction des plasmides ci-dessus également décrits. Il s'agit plus spécialement des plasmides **p55PhoA6HisGS/N⁻** (SEQ ID N°89), **p55PhoA6HisGS/NAB⁻** (SEQ ID N°90), **p55/MCS1** (SEQ ID N°92), **p55Flag**/**RBS**/**35** (SEQ ID N°93 et SEQ ID N°109), **p55Flag/RBS/35cmyc6HisGS** (SEQ ID N°94 et SEQ ID N°110) et **p55CκFlag/RBS/35cmyc6HisGS** (SEQ ID N°97 et SEQ ID N°111) construits pour développer les plasmides définis plus haut.

Les domaines CH1, CH2, CH3, H d'une Ig dans ces plasmides appartiennent à IgG1, IgG2, IgG3 ou IgG4, ou encore à IgA, ou toute autre Ig.

Les gènes codant pour les VHH ou les VH humains sont introduits entre les sites uniques dans les différents plasmides. Ces gènes peuvent être remplacés par des gènes codant pour des VHH humanisés par greffage des CDRs des VHH sur des VH humains.

De façon plus générale, les plasmides décrits peuvent être conçus pour renfermer des séquences nucléotidiques codant pour d'autres VHH que les VHH anti-CEA ou anti-CD16, ou pour d'autres VH humains, ou pour d'autres VHH humanisés, capables de se fixer à n'importe quelle molécule.

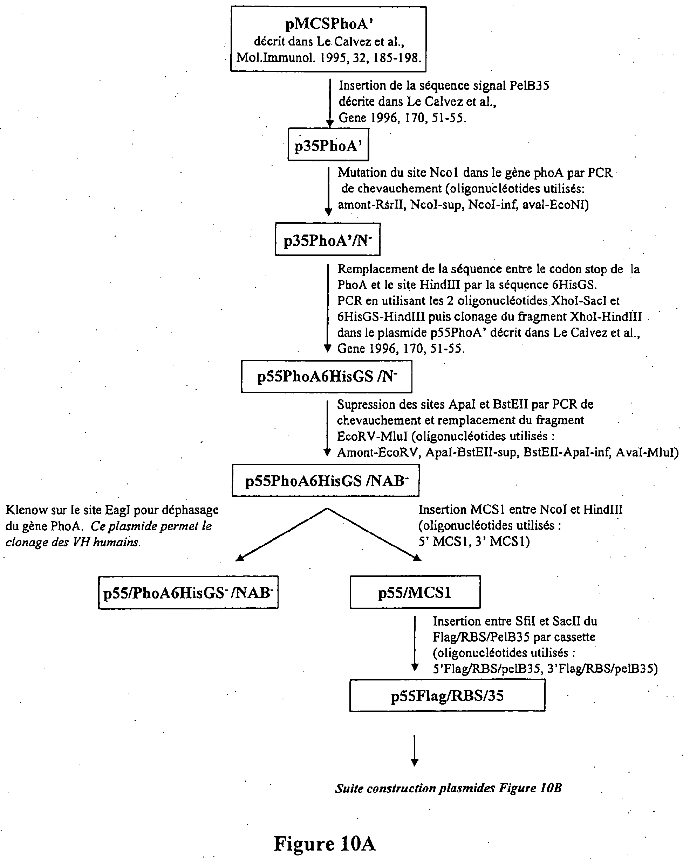

L'invention décrit encore le plasmide **p55/PhoA6HisGS⁻/NAB⁻** (SEQ ID N°91) caractérisé en ce qu'il comprend les séquences nucléotidiques pour produire des domaines VH humains fusionnés à la phosphatase alcaline selon le schéma des figures 10A et 11.

Une méthode pour sélectionner les fragments variables humains des chaînes lourdes d'immunoglobulines (VH) et isoler les clones les mieux produits et les mieux sécrétés a été développée.

De manière avantageuse, ces VH humains servent de matrice pour y greffer les CDR des VHH précédemment sélectionnés afin d'humaniser les régions variables.

Les formats d'anticorps définis ci-dessus présentent un grand intérêt en immunothérapie et pour l'immunodiagnostic. Ils sont en effet capables de reconnaître des molécules différentes ou de lier deux épitopes différents sur une même molécule, et permettent en outre d'avoir accès à de nouveaux épitopes non reconnus par les anticorps conventionnels. Ils peuvent être aisément humanisés, ce qui ouvre des perspectives avantageuses, pour disposer d'anticorps de faible immunogénécité après injection chez l'homme. Leur obtention sous une forme soluble constitue une caractéristique additionnelle d'intérêt pour ces anticorps. Leurs applications en immunodiagnostic et en immunothérapie font donc également partie de l'invention.

D'autres caractéristiques et avantages de l'invention seront donnés dans les exemples qui suivent dans lesquels il est fait référence aux figures 1 à 13, qui représentent, respectivement,
- Les figures 1 et 2, les séquences en acides aminés (SEQ ID N°73 à 76, 103 et 104) et en nucléotides (SEQ ID N°81 à 84, 106 et 107) de 4 clones de VHH anti-CD16 et les séquences en acides aminés (SEQ ID N°77 à 80 et 105) et en nucléotides (SEQ ID N°85 à 88 et 108) de 4 clones anti-CEA isolés selon l'invention,
- les figures 3 et 4, les résultats par FACS montrant la spécificité de 8 VHH analysés, et des anticorps bispécifiques correspondant,
- la figure 5, les résultats par FACS montrant l'accessibilité sur cellules des anticorps bispécifiques,
- la figure 6, les résultats d'essais de compétition par ELISA entre 2 VHH anti-CD16 et des anticorps monoclonaux anti-CD16,
- la figure 7, les profils de compétitions sur cellules par FACS de 2 VHH anti-CD16 et des anticorps monoclonaux anti-CD16,
- la figure 8, les résultats d'activation du CD16A par 2 VHH anti-CD16, et par l'anticorps bispécifique anti-CEA 17/anti-CD16 c21 de type F(ab')₂,
- la figure 9, les résultats de lyse cellulaire par les cellules NK activées par les anticorps bi-spécifiques,
- les figures 10A et 10B, des constructions de plasmides selon l'invention,
- la figure 11, les séquences de plasmides de l'invention,
- les figures 12A et 12B, des formats d'anticorps de type Fab, Fab', F(ab')₂, (HCH2CH3)₂ et mAb*,
- la figure 13, des gels d'électrophorèse de fragments d'anticorps de type Fab, Fab' et F(ab')2 au cours des différentes étapes de leur purification.

### Exemple 1 :

### Immunisation des lamas, titration des sérums et purification des lymphocytes B.

Un lama femelle a été immunisé avec la région extracellulaire du récepteur FcγRIIIB humain recombinant (CD16B) (décrit dans la publication : Teillaud *C et al.,* 1993).REF

Un lama mâle a été immunisé avec la région extracellulaire de l'antigène carcino-embryonnaire humain recombinant (CEA) (décrit dans la publication : Terskikh *et al.,1993,* et dans le brevet : Terskikh *A et al.,* 1993).REF

Les animaux ont été immunisés tous les mois avec 500 µg de chacun des immunogènes. Cent ml de sang ont été prélevés 15 jours après chaque immunisation. Pour chacun des échantillons prélevés, une titration des sérums et des anticorps purifiés (IgG1, 2 et 3) a été réalisée pour détecter la présence d'anticorps contre les différents immunogènes. Les lymphocytes B ont ensuite été purifiés sur gradient de Ficoll (histopaque-1077, Sigma-Aldrich), puis lavés 2 fois avec du PBS.

### Construction des banques de VHH : purification des ARN totaux, transcription inverse, PCR1, PCR2 et clonage dans le phagemide pHen1.

### Construction des banques de VHH :

### Purification des ARN totaux :

Les ARN totaux des lymphocytes B sont extraits selon la méthode utilisant l'isothiocyanate de guanidium (Chomczynski et Sacchi, 1987) REF. Après des extractions phénol/chloroforme en milieu acide, les ARN totaux sont précipités à l'éthanol. La qualité des ARN et leur quantification sont évaluées sur gel d'agarose 1%. Ils sont ensuite convertis en ADNc par transcription inverse.
*Transcription inverse et PCR :*
Séquences SEQ ID N° 1 à 9 des oligonucléotides utilisés :
   3' CH2FORTA4
      SEQ ID N°1 : CGCCATCAAGGTACCAGTTGA
   3'CH2-2
      SEQ ID N°2 : GGTACGTGCTGTTGAACTGTTCC
   3' RC-IgG2
      SEQ ID N°3 : GGAGCTGGGGTCTTCGCTGTGGTGCG
   3' RC-IgG3
      SEQ ID N°4 : TGGTTGTGGTTTTGGTGTCTTGGGTT
   5'VH1-Sfi
      SEQ ID N°5 :
   5'VH2-Sfi
      SEQ ID N°6 :
   5'VH3-Sfi
      SEQ ID N°7 :
   5'VH4-Sfi SEQ ID N°8
   3'VHH-Not
      SEQ ID N°9 : CACGATTCTGCGGCCGCTGAGGAGAC(AG)GTGACCTGGGTCC

Cinq µg d'ARN total sont hybridés avec 1 pmole d'oligonucléotide 3' CH2FORTA4 (Arbabi *Ghahroudi et al.,* 1997)REF ou CH2-2 spécifique du domaine CH2 des IgG simple chaîne lourde de lama rétrotranscrits avec 150 U de superscript II (BRL) pendant 30 min à 50°C. Les oligonucléotides spécifiques des régions charnières des IgG 2 et 3, 3' RC-IgG2 et 3' RC-IgG3, peuvent aussi être utilisées. Les ADNc simples brins sont purifiés sur billes (BioMag^{R} Carboxyl Terminator, Polyscience Inc) et élués avec 17 µl de 10mM Tris-acétate pH 7,8.

### Conditions de PCR1 :

Quatre µl d'ADNc sont amplifiés par PCR avec 0,5 U de Dynazyme Extend DNA polymerase (Finnzymes), 10 pmoles de la même amorce 3' CH2FORTA4 ou CH2-2 et 10 pmoles des 4 amorces 5'VH1-4 Sfi spécifiques du domaine VH des IgG humaines, dans un volume de 50 µ1. (94°C, 3 min; 94°C, 1 min; 60°C, 1 min; 72°C, 1min : 37 cycles puis 72°C, 10 min).

Trois fragments d'ADN sont amplifiés : un fragment d'environ 900 pb codant pour les domaines VH-CH1-CH2 des IgG1 ; et 2 fragments d'environ 600 bp codant pour les domaines VHH-CH2 des IgG2 et 3.

### Conditions de PCR2 :

Les fragments de 600 pb sont purifiés sur gel d'agarose 1% ( kit « Qiaquick gel extraction », Qiagen) puis amplifiés par PCR avec 1 U de Deep Vent (Biolabs) et 10 pmoles des 4 amorces 5'VH1-4 Sfi spécifiques du domaine VH des IgG humaines et 10 pmoles de l'amorce 3' VHH-NotI.
(94°C, 3 min; 94°C, 45 sec ; 65°C, 45 sec; 72°C, 45 sec ; 15 cycles, puis, 94°C, 45 sec; 60°C, 45 sec; 72°C, 45 sec ; 15 autres cycles, puis 72°C, 10 min).

Les fragments d'environ 400 pb codant pour les VHH sont purifiés sur gel d'agarose 1% ( kit « Qiaquick gel extraction », Qiagen) rassemblés et précipités par l'éthanol. Ils sont ensuite coupés par les enzymes de restriction Ncol et Notl, ou Bgll et NotI (Biolabs) pour être clonés dans le phagemide pHen1 (Hoogenboom *et al.,* 1991)REF aux sites NcoI et NotI ou Sfil et NotI.

### Préparation du vecteur :

Vingt µg de phagemide pHen1 sont digérés dans un volume de 300 µl avec 50 U de Sfil en présence de BSA, à 50°C, 16 h ; ou avec 50 U de Ncol en présence de BSA, à 37°C, 16 h. Le phagemide linéarisé est purifié sur gel d'agarose 0,7% (kit « Qiaquick gel extraction », Qiagen). L'ADN élué est ensuite coupé par 50 U de NotI à 37°C dans un volume de 200 µl, 16 h. L'enzyme est détruite par la chaleur 15 min à 65°C et l'ADN est extrait au phénol/chloroforme et précipité à l'éthanol. Le pHen1 coupé est contrôlé sur gel d'agarose 0,7%, quantifié et ajusté à 200 ng/µl.

### Préparation des fragments d'ADN VHH :

Cinq µg de fragments VHH sont coupés dans un volume de 300 µl avec 50 U de Bgll et NotI en présence de BSA, à 37°C, 16 h ; ou avec 50 U de Ncol et Notl en présence de BSA, à 37°C, 16 h. Les enzymes sont dénaturées à 65°C, 15 min ; puis les ADN sont extraits au phénol/chloroforme et précipités à l'éthanol en présence de 10 µg de glycogène (Roche). Les fragments VHH coupés par Ncol et NotI sont purifiés sur gel d'agarose 1% puis contrôlés sur gel d'agarose 2%, quantifiés et ajustés à 100 ng/µl.

### Ligature :

Cent cinquante ng de pHen1 digérés par Sfil et NotI sont ligaturés avec 60 ng de fragment VHH digéré par Bg1I et NotI dans un volume de 20 µl avec 2000 U de T4 DNA ligase (Biolabs) à 16°C, 17 h.

La ligase est inactivée à 65°C, 15 min, et le produit de ligature est coupé par 20 U de XhoI (Biolabs) pour éliminer le vecteur résiduel non ligaturé, 37°C, 4 h. Six ligatures sont ainsi réalisées. Les produits de ligature sont ensuite rassemblés en 2 tubes et extraits au phénol/chloroforme, précipités en présence de 10 µg de glycogène et repris dans 2 x 18 µl H₂O ultrapure. Deux µl sont utilisés par électroporation.

La banque de VHH du lama mâle (réf. : 080101) représente 5,4 10⁶ clones et la banque de VHH du lama femelle (réf. : 010301) 10⁶ clones.

**Isolement des VHH à partir des banques par la technique de phage-display.**

### Sélection des VHH anti-CEA et anti-CD16 :

Les différents VHH ont été isolés par la technique du phage-display.

### Production des banques de phages :

Dix µl du stock d'une banque 080101 ou 010301 (cellules TG1 transformées avec les phagemides) sont inoculés dans 50 ml de (2TY, 100 ug/ml d'ampicilline, 2% glucose) et incubés à 37°C jusqu'à une DO₆₀₀ égale à 0,5. Cinq ml de la culture sont alors infectés avec 5 ml de M13KO7 à 10¹³ pfu/ml, 30 min, 37°C, sans agitation. Après centrifugation, le culot de phages est repris dans 25 ml de (2TY, 100 µg/ml d'ampicilline, 25 µg/ml kanamycine). La culture est incubée 16 h à 30°C avec agitation. Les phages sont ensuite précipités avec 1/5 vol de 2,5 M NaCl / 20% PEG 6000 et concentrés 25 fois dans du PBS.

### Sélection des VHH :

Deux cents µl de billes recouvertes de streptavidine (Dynabeads M-280, Dynal) sont équilibrés avec 1 ml de lait 2%PBS pendant 45 min à température ambiante avec agitation sur une roue. 10¹² phages de la production précédemment décrite sont aussi équilibrés avec du lait 2%-PBS dans un volume final de 500 µl pendant 60 min à température ambiante avec agitation sur une roue.

Les billes sont compactées avec un aimant, re-suspendues avec 250 µl de lait 2%/PBS et incubées avec 200 µl d'antigène biotinylé pendant 30 min à température ambiante sur une roue. 150, 75 et 25 nM final d'antigène biotinylé sont utilisés au 1^{er}, 2^{ème} et 3^{ème} tour, respectivement.

Aux 450 µl de billes/antigène-biotine on rajoute les 500 µl de phages pendant 3 h à température ambiante avec agitation sur une roue. Le mélange billes / antigène-biotine / phages est lavé 5 fois avec 800 µl de lait 4%-PBS puis transféré dans un nouveau tube eppendorf. Cinq autres lavages sont réalisés avec 800 µl de PBS-Tween 0,1% puis le mélange est transféré dans un nouveau tube eppendorf. Enfin, 5 lavages sont réalisés avec 800 µl de PBS.

Les phages anticorps fixés sur les billes/antigène-biotine sont re-suspendus avec 200 µl de PBS et incubés 30 min à 37°C, sans agitation, avec 1 ml de TG1 rendues compétentes pour la fixation des phages au pili (cellules compétentes : à partir d'une culture de TG1 en 2YT sur la nuit, on réalise une dilution au 1/100 et on inocule 50 ml de 2YT à 37°C sous agitation jusqu'à une DO₆₀₀ proche de 0,5). A chaque sélection, les phages sont comptés et amplifiés pour un nouveau tour de sélection.

### Comptage des sélections :

On réalise des dilutions de 1 µl de cellules TG1 transfectées avec les phages (voir ci-dessus) de 10⁻² à 10⁻⁵ avec du 2YT. Un, 10 et 100 µl de chaque dilution sont étalés sur boîte de Petri (2YT / ampliciline 100 µg/ml / glucose 2%). Les boîtes sont incubées 16 h à 30°C.

### Etalement de la sélection pour l'isolement des colonies :

On centrifuge les 5 ml de TG1 transfectées pendant 10 min à 3000 g pour concentrer les cellules et on reprend le culot avec 1 ml de 2YT. Deux cent cinquante µl sont étalés par boîte de Petri (12 cm x 12 cm) (2TY/ ampicilline 100 µg/ml / glucose 2% et incubées 16 h à 30°C.

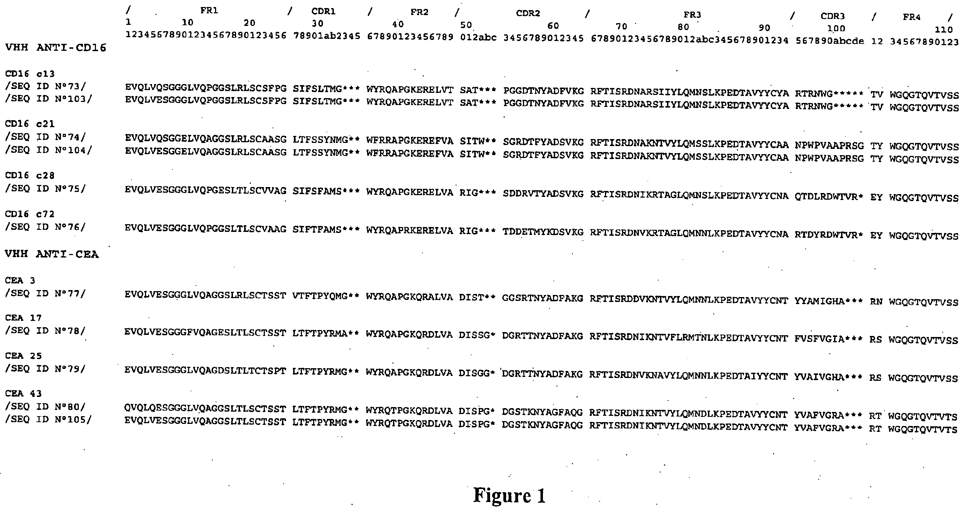

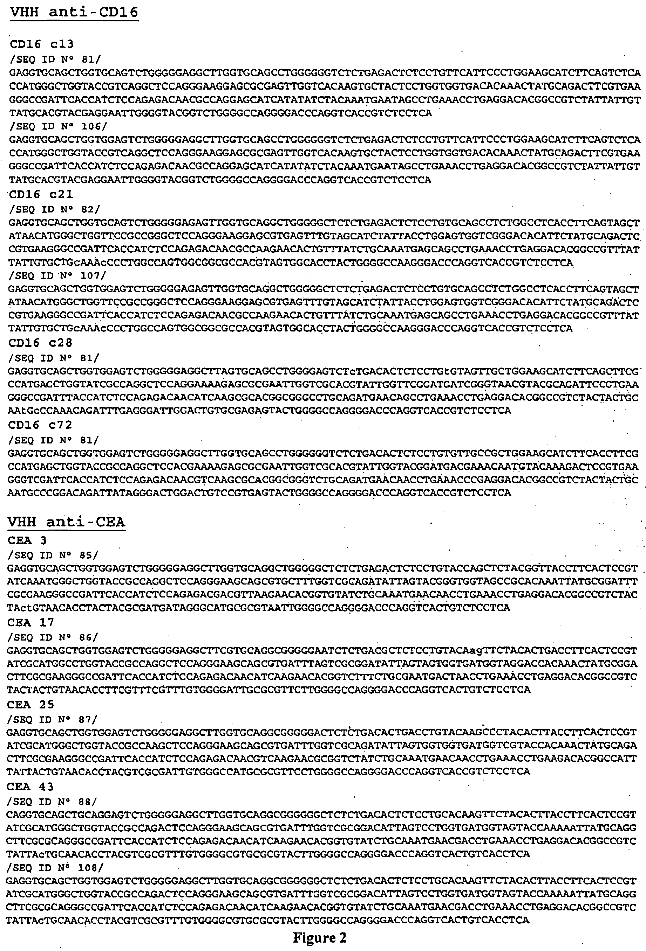

Les VHH suivants ont été isolés par cette méthode : quatre VHH anti-CEA (clones : 3, 17, 23, 43) et quatre VHH anti-CD16 (clones : c13, c21, c28, c72) ont été obtenus dont les séquences en acides aminés et en nucléotides sont indiquées dans les Figures 1 et 2.

### Reclonage, production et purification des VHH et des anticorps bispécifiques. Clonage des VHH :

Les VHH ont été clonés dans le plasmide p55PhoA6HisGS/NAB⁻ (construction décrite dans le paragraphe 1.3.6, voir Figure 10A et 11) entre les sites de restriction Sfil et HindIII.

### Conditions de PCR :

Cinquante ng de VHH ont été amplifiés par PCR avec 1 U de Deep Vent (Biolabs), 10 pmoles des amorces 5' pJF-VH3-Sfi et 3' cmyc-6His/HindIII dans un volume final de 50 µl.
(94°C, 3 min ; 94°C, 45 sec; 52°C, 45 sec; 72°C, 45 sec ; 30 cycles puis, 72°C, 5 min).

Les séquences SEQ ID N° 10 et SEQ ID N°11 des oligonucléotides utilisés sont les suivantes :
5' pJF-VH3-Sfi
   SEQ ID N°10: CTTTACTATTCTCACGGCCATGGCGGCCGAGGTGCAGCTGGTGG
3'cmyc-6His/HindIII
   SEQ ID N°11:

Les produits de PCR sont purifiés sur gel d'agarose 1% ( kit « Qiaquick gel extraction », Qiagen) et coupés avec 20 U de BglI et 20 U de HindIII (Biolabs) 16 h à 37°C. Dix µg de p55PhoA/NAB⁻ sont coupés d'abord avec 50 U de Sfil 16 h à 50°C, puis avec 20 U de HindIII 12 h à 37°C. Les produits de digestion (vecteur et fragments de PCR) sont précipités à l'éthanol. Les ADN sont re-suspendus dans 20 µl d'H₂O et quantifiés sur un gel d'agarose 0,7%.

La ligature est effectuée avec 200 U de T4 DNA ligase (50 ng de p55PhoA/NAB⁻ coupés par Sfil et HindIII et 10 ng de fragment PCR coupés par BglI et HindIII dans un volume de 20 µl, 16h à 16°C. Après inactivation de la T4 DNA ligase 15 min à 65°C, le vecteur non recombinant est éliminé par digestion enzymatique avec 10 U de Xhol, 2 h à 37°C.

Après transformation de la ligature et analyse de quelques colonies recombinantes, les VHH d'intérêt sont produits chez *E.coli.*

### Production des VHH :

Une colonie isolée est inoculée dans 3 ml de 2YT / ampicilline 100 µg/ml / 2% glucose et incubée à 37°C avec agitation. Cinquante ml de 2YT / ampicilline 100 µg/ml / 2% glucose sont ensuite ensemencés avec une dilution de la culture précédente et incubés 16 h à 30°C avec agitation. Quatre cents ml de 2YT/ ampicilline 100 µg/ml sont inoculés avec l'équivalent de 0,1 unité DO_{600,} et incubés à 30°C avec agitation, jusqu'à une DO₆₀₀ de 0,5 à 0,7.La culture est ensuite induite avec 400 µl d'IPTG (isopropyl-β-D-thiogalactopyranoside) 0,1 mM final et cultivée à 30°C pendant 16 h.

### Production des anticorps bispécifiques :

Une colonie isolée, issue d'une transformation des plasmides réalisée dans la souche *d'E. coli* DH5α, est inoculée dans 3 ml de 2YT / ampicilline 100 µg/ml / 2% glucose et incubée à 30°C avec agitation. Cinquante ml de LB / ampicilline 100 µg/ml / 2% glucose sont ensuite ensemencés avec une dilution de la culture précédente et incubés 16 h à 30°C avec agitation. Quatre cents ml de LB / ampicilline 100 µg/ml sont inoculés avec l'équivalent de 0,1 unité D0₆₀₀, et incubés à 30°C avec agitation pendant 2h30, puis la culture est incubée à 20°C avec agitation, jusqu'à une DO₆₀₀ de 0,5 à 0,7. La culture est ensuite induite avec 400 µl d'IPTG (isopropyl-β-D-thiogalactopyranoside) 0,1 mM final cultivée à 20°C pendant 72h.

### Extraction de la fraction soluble du périplasme :

Les cultures à partir desquelles sont produits les VHH ou les anticorps bispécifiques sont centrifugées à 4200 g, 4°C, 40 min. Le culot est repris dans 4 ml de TES glacé (0,2 M Tris-HCl pH 8,0; 0,5 mM EDTA; 0,5 M sucrose). On ajoute ensuite 160 µl de lysozyme (10 mg/ml dans du TES, fraîchement préparé) puis 24 ml de TES froid dilué au 1/2 dans H₂O. Le mélange est incubé 30 min dans la glace.

Après centrifugation à 4200 g, 4°C, 40 min, on récupère le surnageant (correspondant à la fraction périplasmique) et on ajoute 150 µl de DNAse (10 mg/ml) et 5 mM final de MgC12, 30 min à température ambiante. La solution est dialysée 16 h contre le tampon d'équilibre (acétate de sodium 50 mM, NaCl 0,1M pH 7,0).Purification des VHH :
La colonne (BD TALON™ Metal affinity, BD Biosciences Clontech) est équilibrée avec le tampon d'équilibre (acétate de sodium 50 mM, NaCl 0,1M pH 7,0). La fraction périplasmique est déposée sur la colonne. Après lavage de la colonne avec 5 volumes de tampon d'équilibre, le VHH est élué par gradient de pH ou d'imidazole (gradient entre le tampon d'équilibre pH 7,0 et la solution acétate de sodium 50mM pH 5,0 ou la solution d'imidazole 200mM). Chaque fraction est contrôlée sur un gel SDS/PAGE (acrylamide 15%) après coloration au bleu de coomassie. Les fractions d'intérêt sont rassemblées et dialysées contre du PBS. Le VHH est concentré sur membrane (Amicon Ultra 5000MWCO, Millipore) et dosé par la méthode colorimétrique de Lowry à l'aide du kit Biorad Protein Assay.

### Purification des anticorps bispécifiques :

Les anticorps bispécifiques sont purifiés à partir de la fraction soluble du périplasme (Cf extraction de la fraction soluble du périplasme) en deux temps ; d'abord sur une colonne BD TALON (Cf purification des VHH) puis sur une protéine G (HiTrap protein G 5ml, Amersham biosciences).

La colonne « Hi Trap protein G » est équilibrée en PBS (NaCl 137 mM, KCl 2,67 mM, Na₂HPO₄ 1,2 mM, KH₂PO₄ 1,76 mM pH 7,4). Les protéines éluées sur la colonne BD TALON et dialysées en PBS sont déposées sur la protéine G. Après lavage de la colonne avec 5 volumes de PBS, l'anticorps bispécifique est élué avec de la glycine 0,1 M pH 2,7 puis tamponné avec de l'hépès 1 M pH 8. Après contrôle sur gel SDS / PAGE (acrylamide 10%), l'anticorps bispécifique est dialysé en PBS 0,1x, congelé à -80°C et lyophilysé pour être concentré dix fois. Enfin le F(ab')₂ est séparé du Fab' sur une colonne Tricorn Superdex 200 10/300 GL (Amersham Biosciences) équilibrée en PBS.

### Caractérisation fonctionnelle des VHH et des anticorps bispécifiques par ELISA, Biacore, immunofluorescence (cytométrie de flux, FACS) et par tests d'activation du CD16.

### Caractérisation des anticorps anti-CEA et anti-CD 16 par ELISA :

### ELISA des phages-VHH :

Cinq µg/ml d'antigène biotinylé (CEA ou CD16) sont fixés sur une plaque streptavidine (BioBind Assembly Streptavidin Coated, ThermoLabsystems) préalablement saturée en lait 2%-PBS. Cinq 10¹⁰ phages-anticorps sont mis en contact avec l'antigène. La liaison antigène/anticorps est détectée grâce à un ELISA composé d'un anticorps monoclonal dirigé contre la protéine P8 du phage (HRP/anti-M13 monoclonal conjugate, Pharmacia). L'ajout du substrat, 10mg ABTS (2,2'-azino bis (3-ethylbenzo-thiazoline-6-sulphonic acid, diammonium salt) à 20 ml tampon de révélation (18 ml PBS, 1 ml acide citrique 1 M, 1 ml citrate de sodium 1 M, 10 ml H₂O₂ 30%), permet de lire la réaction à 405 nm (Tecan).

### ELISA des VHH :

Cinq µg/ml d'antigène biotinylé sont fixés sur une plaque streptavidine (BioBind Assembly Streptavidin Coated, ThermoLabsystems) préalablement saturée en lait 2%-PBS. Chaque VHH (gamme de 0,001 µg/ml à 1 µg/ml) est lié à l'antigène absorbé dans les micropuits. La liaison est révélée avec un anticorps monoclonal dirigé contre l'étiquette c-myc (Santa Cruz Biotechnology, Inc) dilué au 1/1000 et un anticorps polyclonal de chèvre dirigé contre les IgG de souris couplé à la peroxidase dilué au 1/5000 (ref 55556, ICN) en présence d'ABTS.(2,2'-Azino-di-(3-ethylbenzthiazoline sulfonate)diammonium salt, Roche).

### ELISA des anticorps bispécijiques :

Dix µg /ml d'antigène (rhCD16 ou rhCEA) sont coatés passivement sur une plaque MaxiSorp ( Nunc). Après saturation de la plaque en PBS / lait 4%, l'anticorps bispécifique (F(ab')₂, Fab', Fab) (gamme de 800 à 0,4 nM) est lié à l'antigène adsorbé dans les micropuits. La liaison est révélée soit :
- avec un anticorps monoclonal dirigé contre l'étiquette Flag (anti-Flag M2 mAB, Sigma) dilué au 1/5000 et un anticorps polyclonal de chêvre dirigé contre les IgG de souris couplé à la phosphatase alcaline dilué au 1/5000 (ref 115-055-003, Jackson Immunoresearch) en présence de DNPP (disodium 4-nitrophenyl phosphate hexahydrate).
- avec un anticorps monoclonal dirigé contre l'étiquette c-myc (Santa Cruz Biotechnology) dilué au 1/500 et un anticorps polyclonal de chêvre dirigé contre les IgG de souris couplé à la phosphatase alcaline dilué au 1/5000 (ref 115-055-003, Jackson Immunoresearch) en présence de DNPP
- avec un anticorps polyclonal de chêvre dirigé contre la chaîne légère kappa humaine couplé à la phosphatase alcaline dilué au 1/500 (ref 2060-04, SouthernBiotech) en présence de DNPP.

Mise en évidence de l'accessibilité du VHH CEA 17 quand le VHH CD16 c21 est lié au rhCD16 absorbé dans les micropuits avec du rhCEA biotinylé et de la streptavidine couplée à la phosphatase alcaline diluée au 1/500 (DAKO, cat DO396).

Constantes d'affinité des anticorps anti-CEA et anti-CD16 par Biacore :
Le BIACORE utilise le principe de la résonance plasmonique de surface (SPR) pour suivre en temps réel les interactions entre molécules sans marquage de celles-ci. L'un des partenaires de l'interaction est immobilisé de façon covalente sur un biocapteur alors que l'autre est injecté dans un flux continu. Le principe de détection par SPR permet de suivre les changements de masse à la surface du biocapteur dus à la formation puis à la dissociation des complexes moléculaires. La réponse, quantifiée en unité de résonance (RU) est une indication directe du taux de fixation de l'analyte par la mesure de la variation de l'indice de réfraction. L'enregistrement du signal (un sensorgramme) est traité de façon mathématique pour obtenir les constantes de vitesse d'association, kₐ, de dissociation k_{d} et les constantes d'association K_{A} (K_{A} = ka/kd) et de dissociation à l'équilibre K_{D} (KD = kd/ka).

Les interactions entre le CEA ou le CD16 et les VHH (qui possèdent une étiquette c-myc reconnue par l'anticorps monoclonal 9E10, Santa Cruz Biotechnology, Inc) ont été étudiées sur un BIACORE 3000 muni d 'un biocapteur de type CM5 sur lequel l'anticorps monoclonal 9E10 a été immobilisé de façon covalente suivant la procédure standard de couplage par les amines proposée par BIACORE (activation par NHS/EDC). Le VHH (en tampon : 10 mM HEPES; 150 mM NaCl; 3 mM EDTA; 0,005% surfactant P20) est alors injecté puis une gamme de CEA ou de CD16 est injectée sur le VHH immobilisé sur le 9E10. En parallèle, les injections sont réalisées sur un canal contrôle qui a subi la même chimie de couplage mais sans injection de protéine. Les affinités des VHH sont indiquées dans le Tableau 1 ci-dessous. Des affinités équivalentes sont obtenues à partir des différents formats d'anticorps bispécifiques.

**Tableau 1**

| VHH | ka x10⁵ (1/Ms) | kd x 10⁻³ (1/s) | KA x10⁷ (1/M) | KD x10⁹ (M) |
|---|---|---|---|---|
| Anti-CEA 3 | 1,24±0,014 | 1,68±0,002 | 7,38 | 13,6 |
| Anti-CEA 17 | 1,56±0,014 | 1,3±0,002 | 12 | 8,3 |
| Anti-CEA 25 | 1,13±0,014 | 3,6±0,004 | 3,15 | 31,7 |
| Anti-CEA 43 | 1,78±0,019 | 1,83±0,002 | 9,72 | 10,3 |
| Anti-CD16 cl3 | 0,53±0,07 | 5,67±0,02 | 0,94 | 100,6 |
| Anti-CD16 c21 | 2,86±0,02 | 2,79±0,006 | 10,3 | 9,7 |
| Anti-CD16 c28 | 0,42±0,03 | 3,45±0,006 | 1,22 | 81,9 |
| Anti-CD16 c72 | 0,39±0,02 | 3,7±0,006 | 1,06 | 94,6 |

### Analyse par FACS de la spécificité des VHH anti-CEA et anti-CD16 et des anticorps bispécifiques correspondant :

### Spécificité pour le CEA :

*(Pour que l'immunociblage soit efficace, il est primordial que les anticorps anti-CEA ne reconnaissent pas le NCA, molécule très homologue au CEA qui est exprimée à la surface des granulocytes.)*

La mise en évidence de la liaison antigène-anticorps est effectuée sur la lignée non transfectée MC38 (lignée cellulaire d'un cancer du colon murin), les lignées transfectées avec le CEA (MC38/CEA⁺) ou le NCA (MC38/NCA⁺), la lignée tumorale humaine LS174T (lignée cellulaire d'adénocarcinome colique humain exprimant le CEA à sa surface) et les granulocytes qui expriment à leur surface le NCA.

Les granulocytes sont extraits à partir de sang frais en présence d'héparine, sur gradient de ficoll à deux densités (histopaque 1119 et 1077). Les granulocytes se trouvent à l'interface des 2 histopaques.

Anticorps utilisés pour la fixation aux cellules :
- 35A7, anticorps monoclonal anti-CEA(spécifique du CEA).
- 192, anticorps monoclonal anti-CEA (qui croise avec le NCA).
- 7.5.4 et 3G8 anticorps monoclonal anti-CD16.
- VHH anti-CD16 (c13, c21, c28, c72).
- VHH anti-CEA (3, 17, 25, 43).
- Anticorps bispécifiques anti-CEA/anti-CD16 construits à partir des 8 VHH isolés. Anticorps utilisés pour la révélation :
- 9E10, anticorps monoclonal de souris anti-cmyc (200 µg/ml, utilisé au 1/10ème)se fixant à l'étiquette c-myc des VHH purifiés.
- AP326F, anticorps polyclonal de mouton anti-IgG de souris couplé au FITC (Silenus, utilisé au 1/100ème).
0,5 x 10⁶ cellules sont utilisées par essai. Les VHH et anticorps monoclonaux sont dilués dans 100 µl de PBS-1% BSA.
0,5 x 10⁶ cellules (mesure auto fluorescence des cellules)
0,5 x 10⁶ cellules + anti IgG de souris-FITC 20 µg/ml
0,5 x 10⁶ cellules + anti-9E10 20 µg/ml, puis anti IgG de souris-FITC 20 µg/ml 0,5 x 10⁶ cellules + VHH anti-CEA ou anti-CD16 1 à 5 µg/ml, puis 9E10 20 µg/ml, puis anti IgG de souris-FITC 20 µg/ml

0,5 x 10⁶ cellules + anticorps monoclonaux (35A7, 192, 7.5.4) 20 µg/ml, puis anti IgG de souris-FITC 20 µg/ml

A chaque étape les échantillons sont incubés 45 min, 4°C, dans l'obscurité.

Entre chaque réaction, on effectue un lavage avec 2 ml de PBS / 1% BSA. A la dernière étape les cellules sont reprises avec 0,5 ml de PBS.

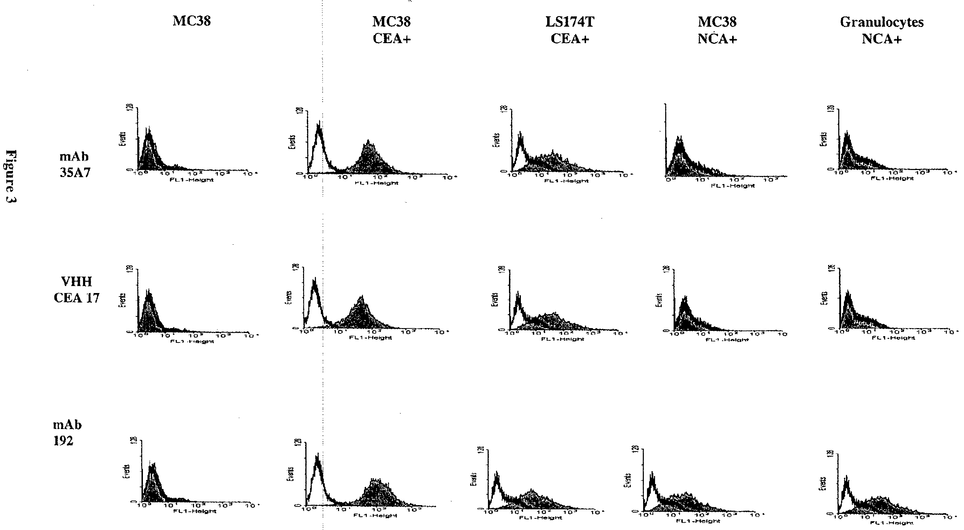

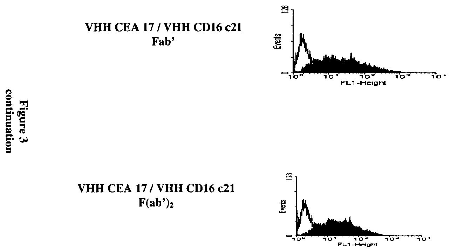

Les résultats par FACS démontrent la spécificité des 4 VHH anti-CEA analysés et des anticorps bispécifiques anti-CEA / anti-CD16 sous forme de Fab, Fab' et F(ab')₂. Ils sont spécifiques du CEA et ne croisent pas avec le NCA. Un exemple est illustré dans la figure 3. Dans cet exemple, les anticorps monoclonaux de référence mAb 35A7 et 192 ne se lient pas sur les cellules MC38 qui n'expriment pas le CEA mais sur les cellules MC38 CEA⁺, et LS174T CEA⁺ qui expriment le CEA sur leur surface. Le mAb 192 lie aussi les cellules MC38 NCA⁺ et granulocytes qui expriment le NCA sur leur surface. Le VHH CEA 17 ne lient que les cellules qui expriment le CEA sur leur surface. Des résultats équivalents sont obtenus avec les autres anticorps VHH anti-CEA (clones 3, 25 et 43). L'anticorps bispécifique VHH CEA 17 / VHH CD16 c21 est spécifique des cellules tumorales à la fois sous forme de Fab' et de F(ab')₂. Des résultats équivalents sont obtenus avec les autres anticorps bispécifiques anti-CEA/anti-CD16 sous forme de Fab, Fab' et F(ab')₂ (dérivant des 8 VHH anti-CEA et anti-CD16).

### Spécificité pour le CD16 :

Pour une reconnaissance efficace des cellules effectrices du système immunitaire, les anticorps anti-CD16 sélectionnés à partir du CD16B doivent aussi reconnaître le CD16A. De plus ils ne doivent pas présenter de réaction croisée avec le CD32 (RFcyHA et RFcγHB).

Les expériences sont réalisées avec des cellules Jurkat (cellules d'une lignée de lymphome T humain; ATCC TIB-152) transfectées ou non avec le gène qui code le CD16A (lignée stable exprimant le CD16A à leur surface ; Vivier *et al.,* 1992), des granulocytes qui expriment le CD16B, des cellules K562 qui n'expriment que le CD32A et des cellules IIA 6huIIB1 qui n'expriment que le CD32B.

Anticorps utilisés pour la fixation aux cellules :
- 3G8, anticorps monoclonal anti-CD16 (RFcγIIIIA/IIIB humain), anticorps anti-site reconnaissant un épitope conformationnel et bloquant la liaison des IgG aux CD16A et CD16B.
- 7.5.4, anticorps monoclonal anti-CD16 (RFcγIIIA/IIIB humain), anticorps reconnaissant un épitope linéaire, localisé hors site de liaison des IgG du CD16 et n'affectant que faiblement cette liaison à fortes concentrations dans des tests de compétition (*Vély et al.,* 1997).
- AT10 anticorps monoclonal anti-CD32 (RFcγIIA/IIB) humain.
- IV.3, anticorps monoclonal anti-CD32 (RFcγIIA) humain.
- VHH anti-CD16 (c13, c21, c28, c72).
- VHH anti-CEA (3, 17, 25, 43).
- 35A7, anticorps monoclonal anti-CEA
- 192, anticorps monoclonal anti-NCA (qui croise avec le CEA)
- Anticorps bispécifiques anti-CEA/anti-CD16 construits à partir des 8 VHH isolés. Anticorps utilisés pour la révélation :
- 9E10, anticorps monoclonal de souris anti-cmyc (200 µg/ml, utilisé au 1/10ème) se fixant à l'étiquette c-myc des VHH purifiés.
- Fab'2 d'un anticorps de chèvre anti-IgG de souris couplé au FITC (F(ab')₂/FITC) utilisé à 20 µg/ml (Jackson Immunoresearch Lab. Inc., 115-096-003).
0,5 x 10⁶ cellules sont utilisées par essai. Les VHH et anticorps monoclonaux sont dilués dans 100 µl de PBS-1% BSA.
0,5 x 10⁶ cellules (mesure autofluorescence des cellules)
0,5 x 10⁶ cellules + (F(ab')₂/FITC) 20 µg/ml
0,5 x 10⁶ cellules + anti-9E10 20 µg/ml, puis (F(ab')₂/FITC) 20 µg/ml 0,5 x 10⁶ cellules + VHH 1 à 5 µg/ml, puis 9E10 20 µg/ml, puis (F(ab')₂/FITC) 20 µg/ml

0,5 x 10⁶ cellules + anticorps monoclonaux ( 35A7, 192, 3G8,7.5.4, AT10, IV.3) 20 µg/ml, puis (F(ab')₂/FITC) 20 µg/ml.

A chaque étape les échantillons sont incubés 45 min, 4°C, dans l'obscurité.

Entre chaque réaction, on fait un lavage avec 2 ml de PBS / 1% BSA. A la dernière étape les cellules sont reprises avec 0,5 ml de PBS

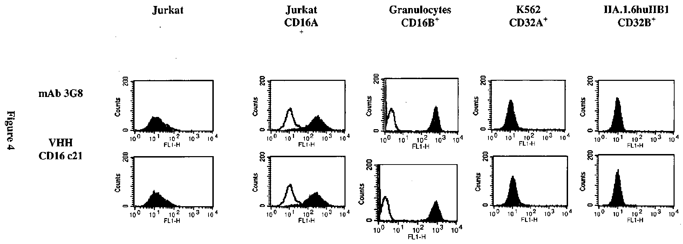

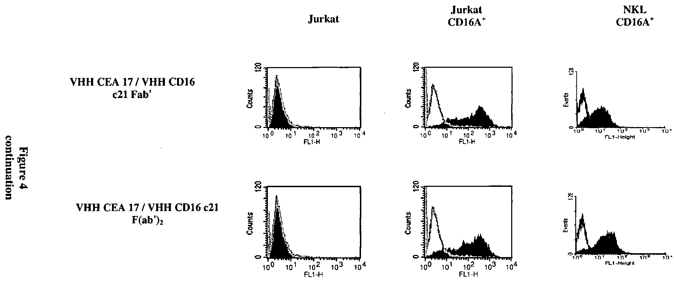

Les résultats par FACS démontrent la spécificité des 4 VHH anti-CD16 analysés et des anticorps bispécifiques anti-CEA / anti-CD16 sous forme de Fab, Fab' et F(ab')₂. Ils sont spécifiques du CD16 et ne croisent pas avec le CD32. Un exemple est illustré dans la figure 4. Dans cet exemple, l'anticorps monoclonal de référence mAb 3G8 se lient pas sur les cellules Jurkat, K562 CD32A⁺ et IIA.1.6huIIB1 CD32B⁺ qui n'expriment pas le CD16 mais sur les cellules Jurkat CD16A⁺, et les granulocytes qui expriment le CD16 sur leur surface. Les VHH CD16 c21 ne lient que les cellules qui expriment le CD16 sur leur surface. Des résultats équivalents sont obtenus avec les autres anticorps VHH anti-CD16 (clones : cl3, c28 et c72). L'anticorps bispécifique VHH CEA 17 / VHH CD16 c21 est spécifique des cellules Jurkat CD16A⁺ et NKL à la fois sous forme de Fab' et de F(ab')₂. Des résultats équivalents sont obtenus avec les autres anticorps bispécifiques anti-CEA/anti-CD16 sous forme de Fab, Fab' et F(ab')₂ (dérivant des 8 VHH anti-CEA et anti-CD16).

### Analyse par FACS de l'accessibilité des anticorps bispécifiques sur cellules :

### L'accessibilité du domaine VHH anti-CD16 :

Cinq 10⁵ cellules LS174T sont incubées pendant 30 min, dans du PBS-BSA 1 % dans la glace, en présence de Fab, Fab' ou F(ab')₂, (gamme de 10 µg/ml à 0,1 µg/ml). Les cellules sont lavées en PBS-BSA 1 %. La fixation du rhCD16 (10µg/ml) sur le domaine VHH anti-CD16 des différents fragments d'anticotps est ensuite détectée, en deux temps, en incubant l'anticorps monoclonal 7.5.4 ou 3G8 (3 µg/ml) avec les cellules pendant 30 min, dans la glace puis en incubant les cellules avec du F(ab')₂ de chèvre anti-IgG de souris (H+L) marqué au FITC (Jackson Immunoresearch Laboratory, cat : 115-096-003), pendant 30 min dans la glace. Après plusieurs lavages, l'immunofluorescence est analysée par cytométrie de flux avec un FACScalibur 4C4 (Becton dickinson) en utilisant le programme Cell Quest Pro.

### L'accessibilité du domaine VHH anti-CEA :

Cinq 10⁵ cellules Jurkat CD16A+ sont incubées pendant 30 min, dans du PBS-BSA 1 % dans la glace, en présence de Fab, Fab' ou F(ab')2, (gamme de 10 µg/ml à 0,1 µg/ml). Les cellules sont lavées en PBS-BSA 1 %. La fixation du rhCEA(10(µg/ml) sur le domaine VHH anti-CEA des Fab, Fab' ou F(ab')₂, est ensuite détectée, en deux temps, en incubant l'anticorps monoclonal 192 (3 µg/ml) avec les cellules pendant 30 min, dans la glace puis en incubant les cellules avec du F(ab')₂ de chèvre anti-IgG de souris (H+L) marqué au FITC (Jackson Immunoresearch Laboratory, cat : 115-096-003), pendant 30 min dans la glace. Après plusieurs lavages, l'immunofluorescence est analysée par cytométrie de flux avec un FACScalibur 4C4 (Becton dickinson) en utilisant le programme Cell Quest Pro.

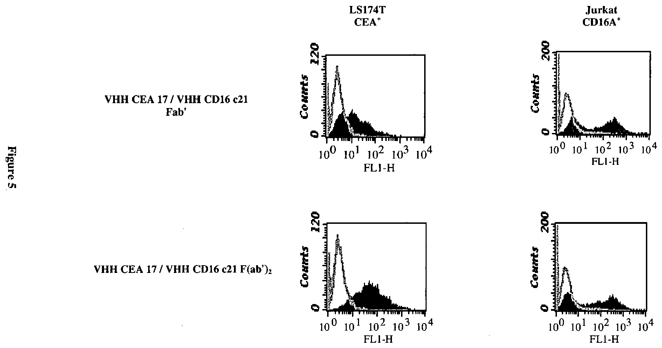

Un exemple est illustré dans la figure 5. L'anticorps bispécifique VHH CEA 17 / VHH CD16 c21 se lie à la fois aux cellules LS174T et Jurkat CD16A⁺. Des résultats équivalents sont obtenus avec les autres anticorps bispécifiques anti-CEA/anti-CD16 sous forme de Fab, Fab' et F(ab')₂ (dérivant des 8 VHH anti-CEA et anti-CD16).

### Essai de compétition entre les VHH anti-CD16 et les anticorps monoclonaux 3G8 et 7.5.4 :

### ELISA :

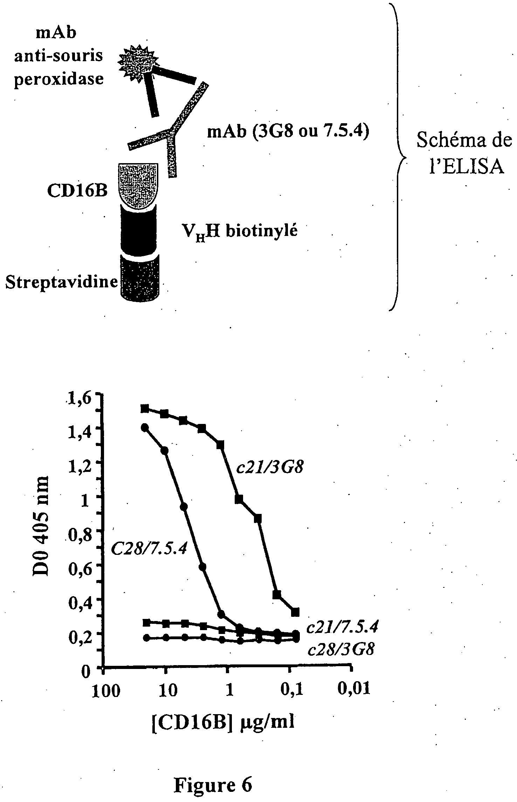

Cinq µg/ml de VHH biotinylé (c21, c28) sont fixés par puits d'une plaque adsorbée avec de la streptavidine (BioBind Assembly Streptavidin Coated, ThermoLabsystems) préalablement saturée en lait 2%-PBS. Le CD16B à une gamme de concentration de 0,07 à 20 µg/ml est ensuite ajouté. Dns un deuxième temps, l'anticorps monoclonal (3G8 ou 7.5.4) à la concentration constante de 5 µg/ml est ajouté. La liaison CD16B-anticorps monoclonal est révélée avec un F(ab')₂ de chèvre anti-IgG de souris couplé à la phosphatase alcaline (Southern Biotechnology, 1030-04) en présence de p-nitrophenylphosphatase (Sigma, N9389)
Les courbes de compétitions en ELISA sont montrées dans la Figure 6. Le VHH CD16 c21 est déplacé par l'anticorps monoclonal 7.5.4. Le VHH CD16 c28 est déplacé par l'anticorps monoclonal 3G8.

### Immunofluorescence indirecte (FACS) :

Cinq 10⁵ cellules Jurkat-CD16A sont incubées pendant 30 min, dans du PBS-BSA 5% dans la glace, en présence des VHH CD16 c21 ou c28 (de 1 à 100 µg/ml). Les cellules sont ensuite incubées avec 0,1 µg/ml de 3G8 ou 1 µg/ml de 7.5.4 pendant 30 min dans les mêmes conditions, puis lavées en PBS-BSA 5%. La fixation de 3G8 ou 7.5.4 est alors détectée en incubant les cellules avec du F(ab')₂ d'un anticorps de chèvre anti-IgG de souris (H+L) marqué au FITC (Jackson ImmunoResearch Laboratories Inc., West Grove, Pennsylvania, USA, cat n°: 115-096-003) pendant 30 min dans la glace. Après plusieurs lavages, l'immunofluorescence est analysée par cytométrie de flux avec un FACScalibur 4C4 (Becton Dickinson, Mountain View, California, USA) en utilisant le programme Cell Quest Pro.

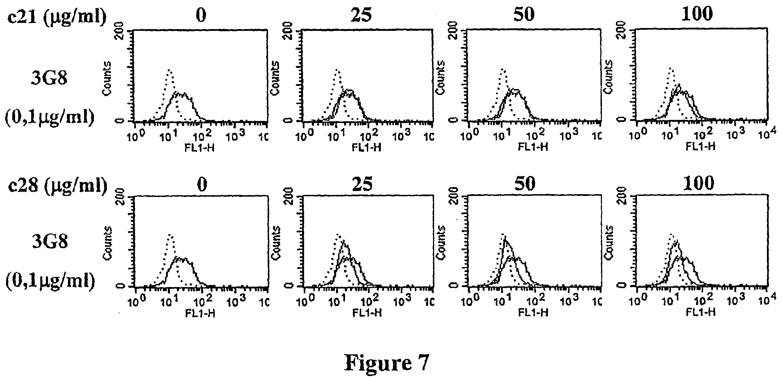

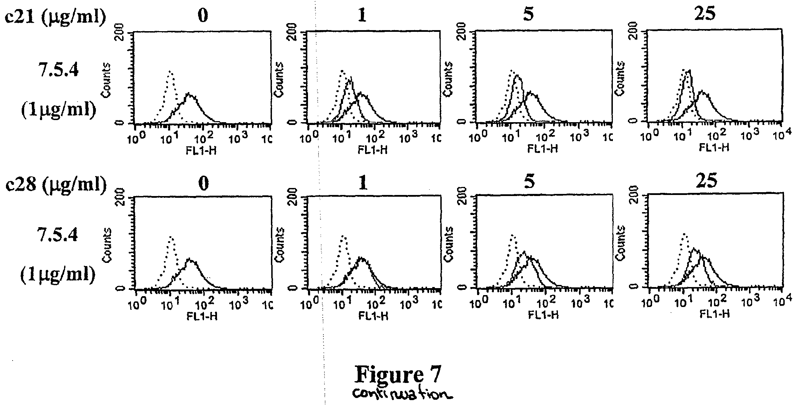

Les profils de compétitions sur cellules sont montrés dans la Figure 7. Le VHH CD16 c21 est déplacé par l'anticorps monoclonal 7.5.4. Le VHH CD16 c28 est déplacé par l'anticorps monoclonal 3G8. A forte dose le VHH CD16 c28 est aussi déplacé par le mAb 7.5.4.

### Activation par les VHH anti-CD16 et par les anticorps bispécifiques des cellules Jurkat CD16⁺ :

Les expériences sont réalisées avec des cellules Jurkat (ATCC TIB-152) transfectées avec le gène qui code le CD16A. Les cellules sont cultivées en RPMI 1640 complémenté avec 10% de FCS, 100 U/ml penicilline, 100 µg/ml streptomycine, 2mM L-glutamine, 0,5 mg/ml G418.

Cinq x 10⁵ cellules sont ensuite incubées 18 h dans des puits de microplaques (250 µl de RPMI contenant 10% FCS, 1% PS, 0,5 mg/ml G418).
Dix ng/ml de phorbol myristate acetate (PMA) (concentration n'induisant pas *per se* la production et la sécrétion d'IL2, mais nécessaire comme " second signal " à cette production) sont ensuite ajoutés, suivis par l'addition de 0,01 à 0,1 µg/ml de VHH biotinylé et de 10 µg/ml de streptavidine (permettant le pontage des VHH) ou d'anticorps bispécifiques non biotinylés.

L'IL2 humaine produite dans les surnageants cellulaires est mesurée par ELISA en utilisant les anticorps du kit R&D (Duoset Human IL2 ; référence : DY202) et de la streptavidine couplée à la phosphatase alcaline (DAKO, DO396) en présence de p-nitrophenylphosphate (Sigma, cat 104-405).

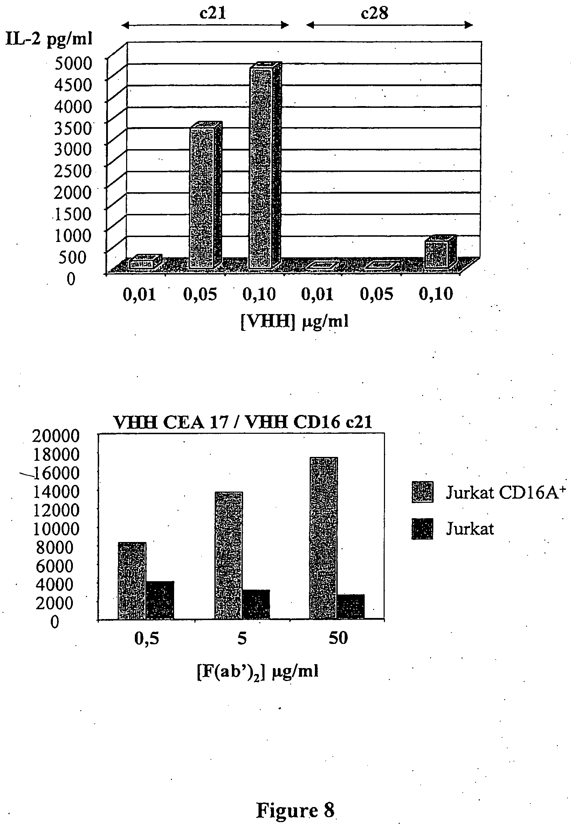

Les résultats d'activation du CD16A (production et sécrétion d'interleukine 2) sont montrés dans la Figure 8. Les deux VHH anti-CD16 c21 et c28 activent la production d'IL2 de cellules Jurkat CD16A⁺. Des quantités supérieures de c28 sont nécessaires pour obtenir une induction de la production et de la sécrétion d'IL2 comparable à celle induite par le c21. L'anticorps bispécifique anti-CEA 17 / anti-CD16 c21 sous forme de F(ab')2 active aussi la production d'IL2 si les cellules Jurkat expriment le CD16A à leur surface et ceci en absence de pontage via la streptavidine. Des résultats équivalents sont obtenus à partir des autres VHH anti-CD16 et des anticorps bispécifiques anti-CEA/anti-CD16

### Lyse des cellules tumorales par les cellules NKL en présence d'anticorps bispécifique :

Pour le test de cytotoxicité des cellules NK, les lignées utilisées sont des cellules NKL (Robertson et al., 1996(12)) provenant d'une leucémie à large lymphocytes granuleux, possédant des propriétés fonctionnelles semblables à celle des NK et dont l'expression du CD16 a été vérifiée préalablement par cytométrie de flux. Les cellules cibles utilisées sont des cellules HeLa provenant d'une leucémie humaine, très sensibles aux NK, des cellules de colon murin C15.4.3 AP (MC38), sensibles au NK et des cellules MC38 transfectées avec du CEA humain et naturellement résistantes aux NK.

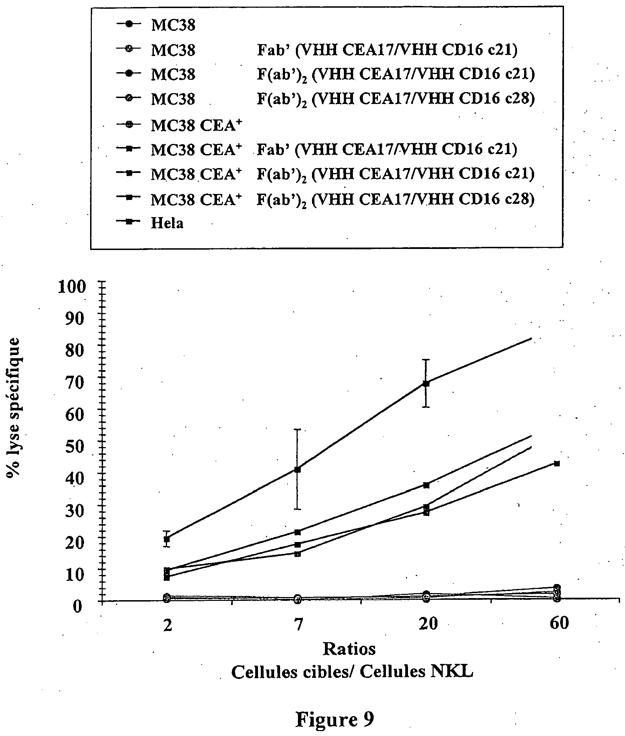

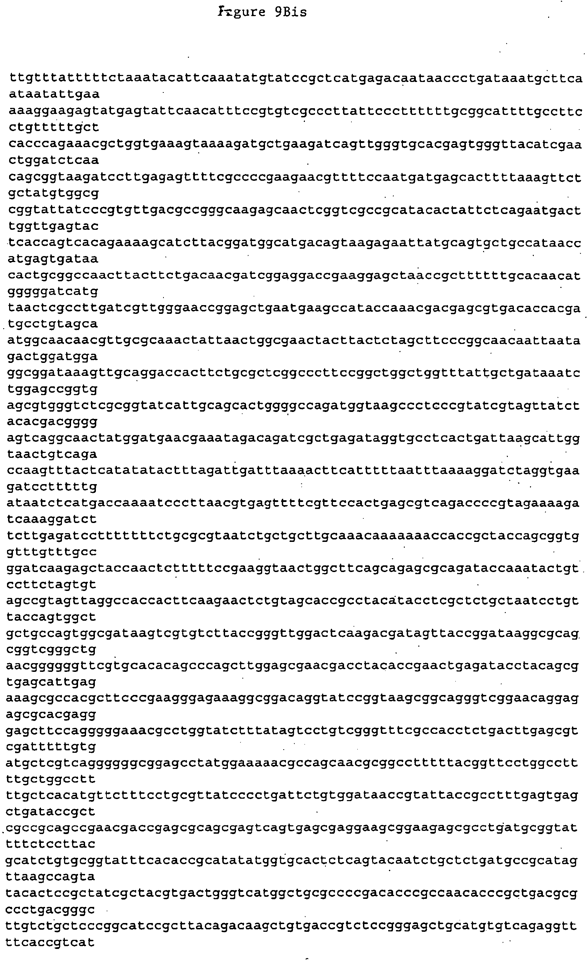

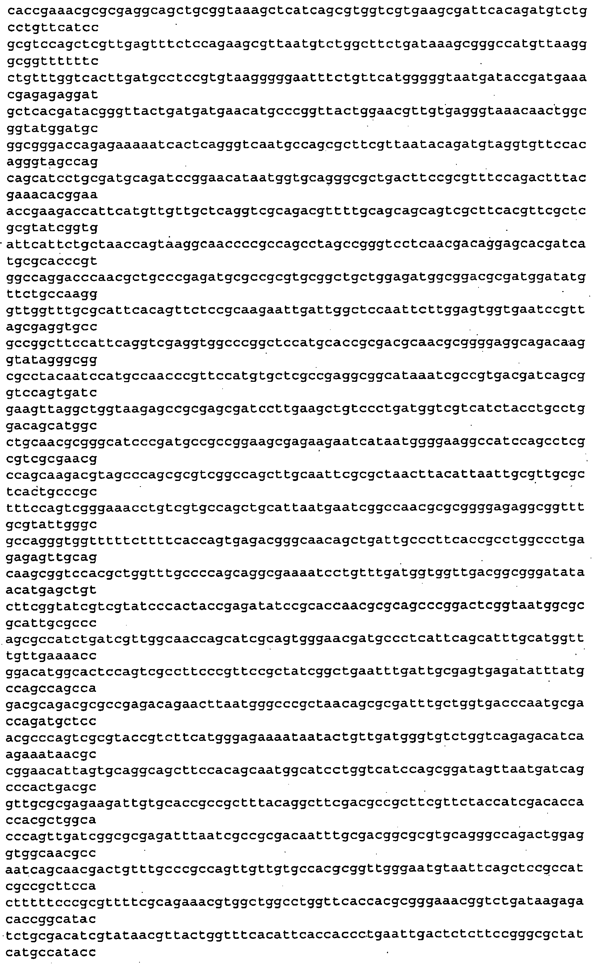

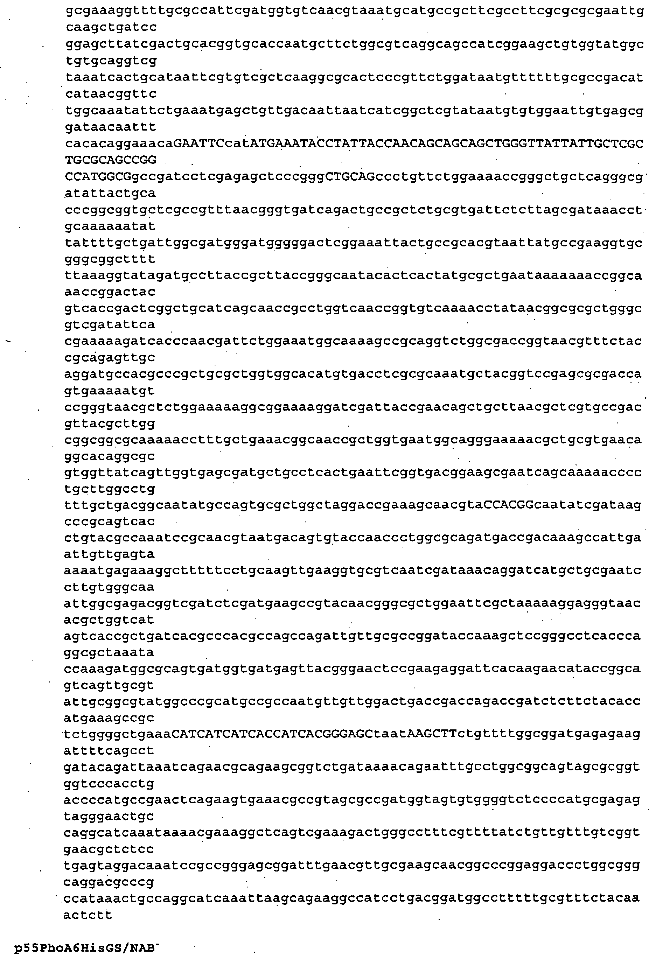

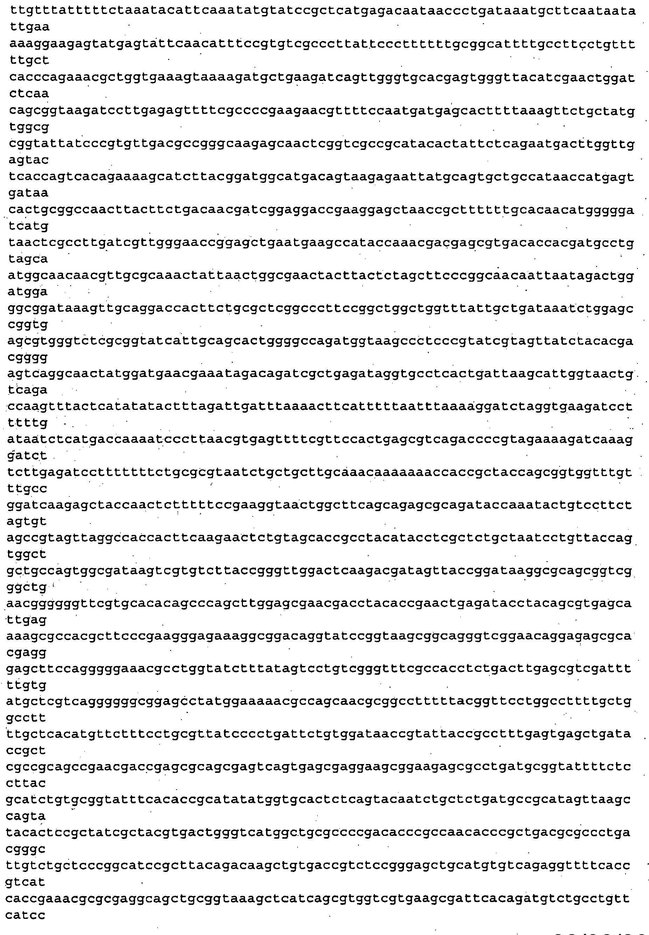

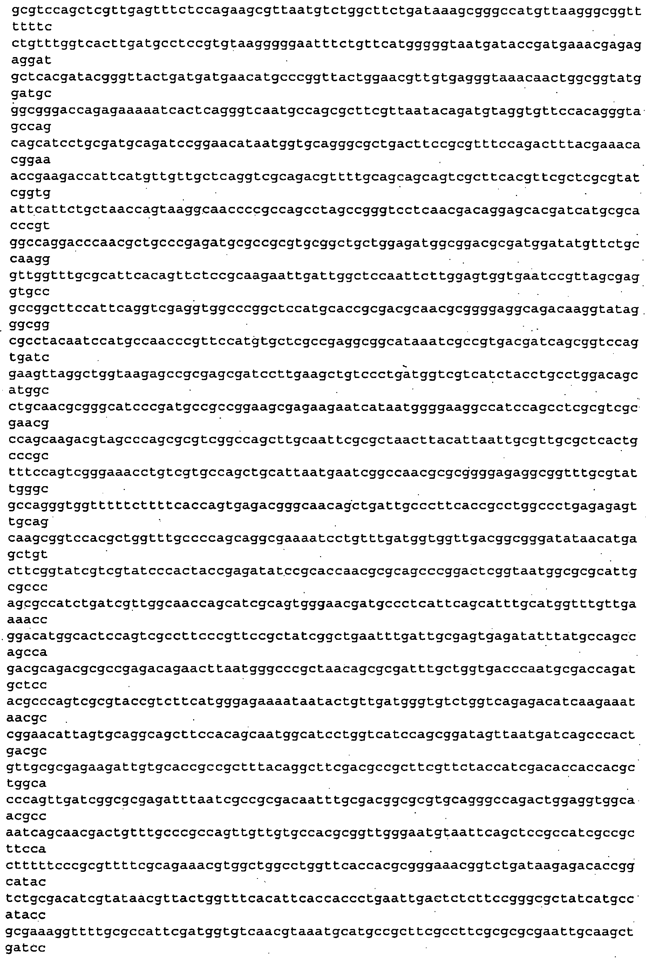

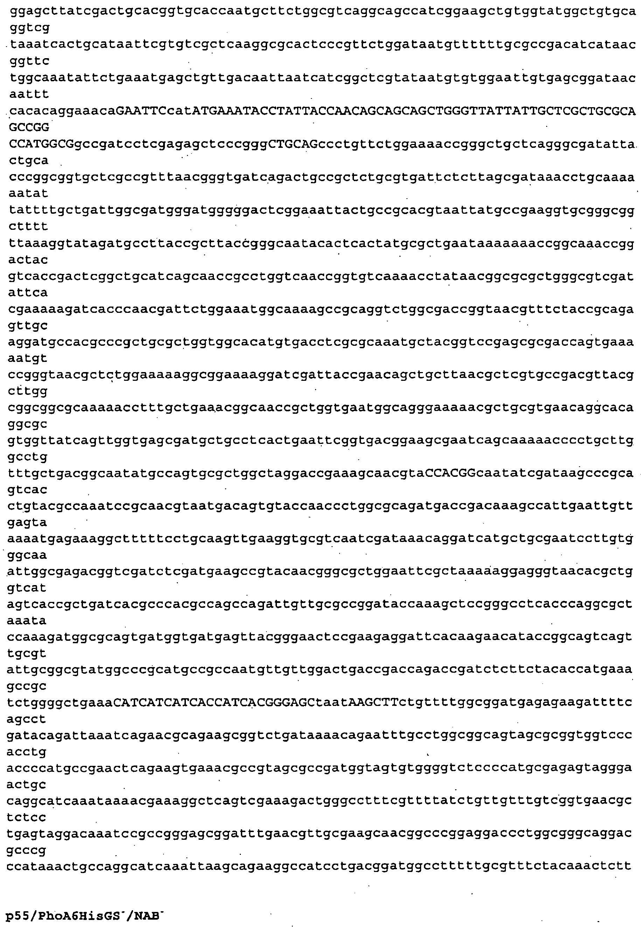

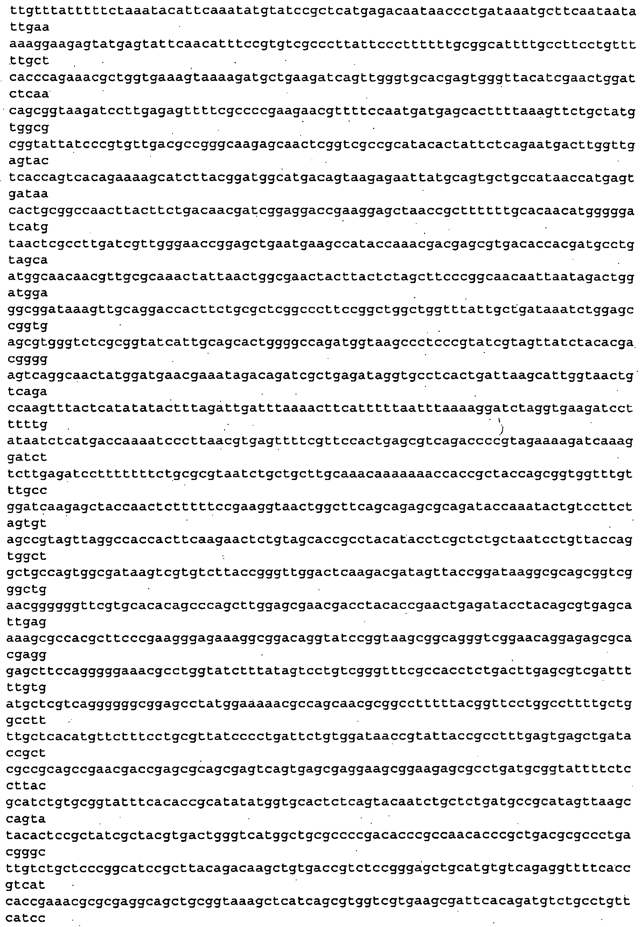

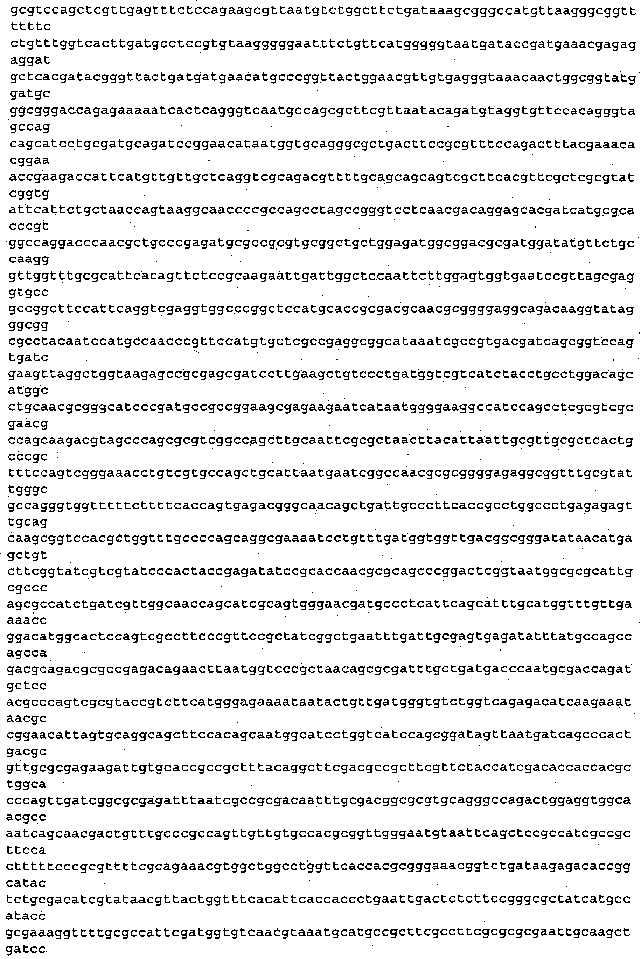

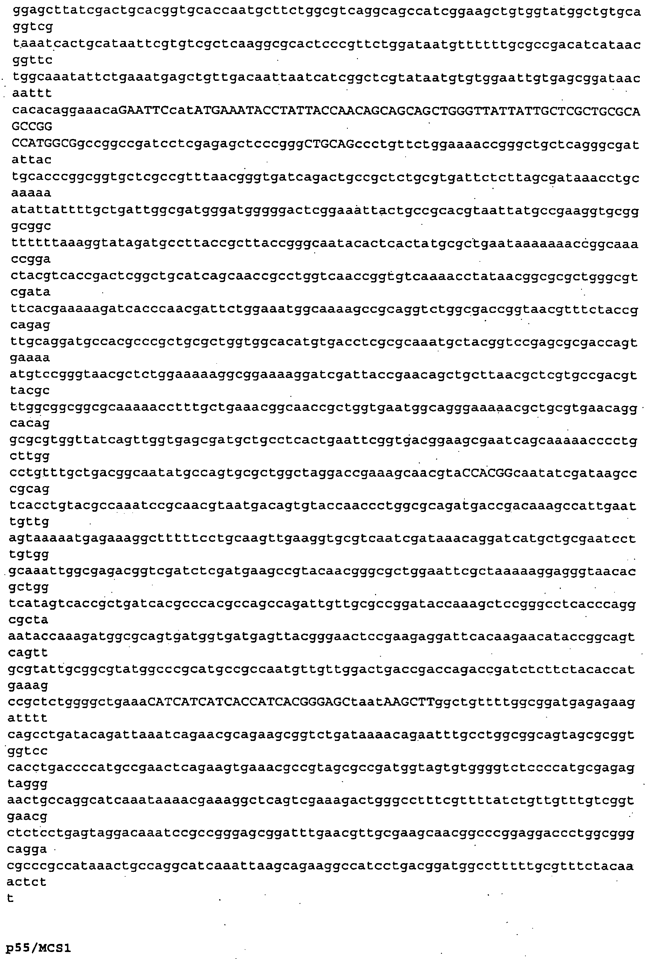

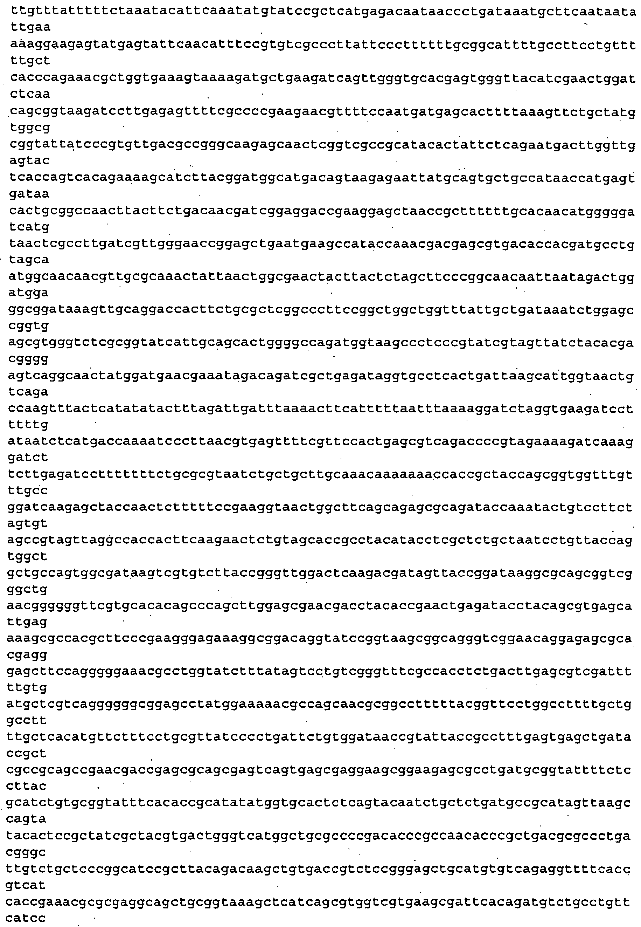

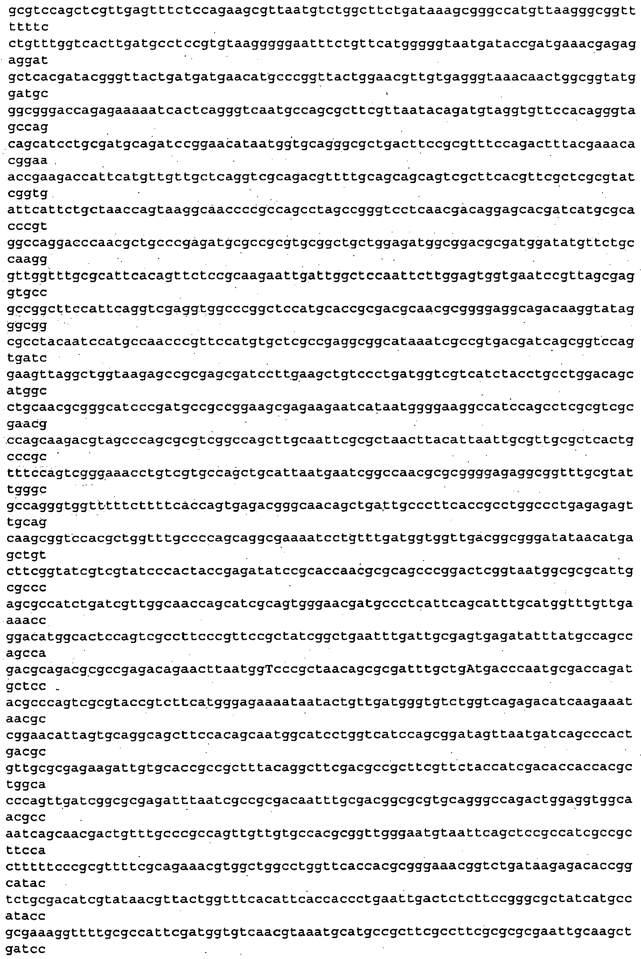

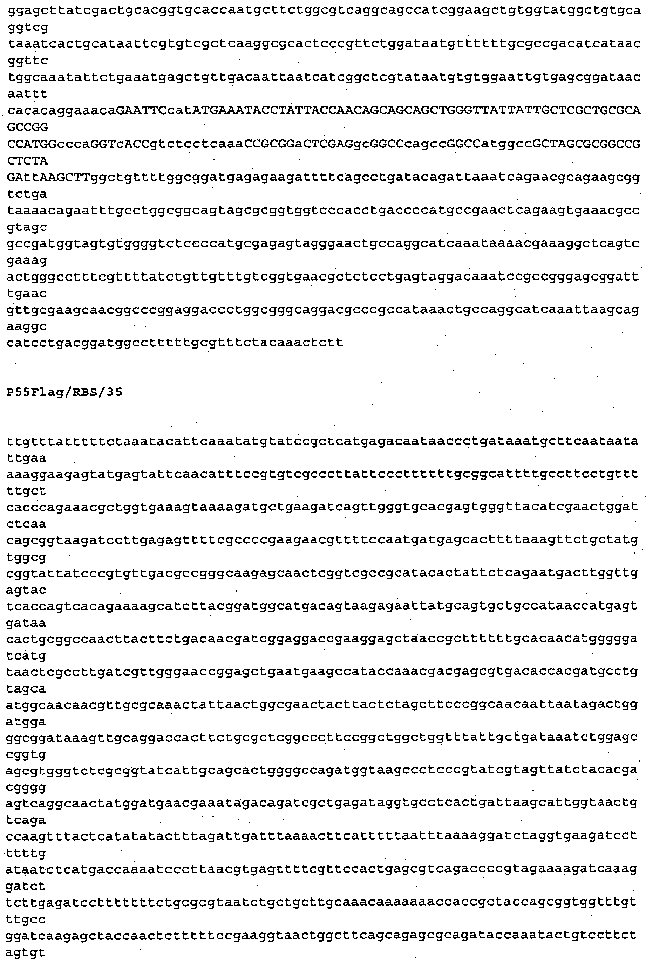

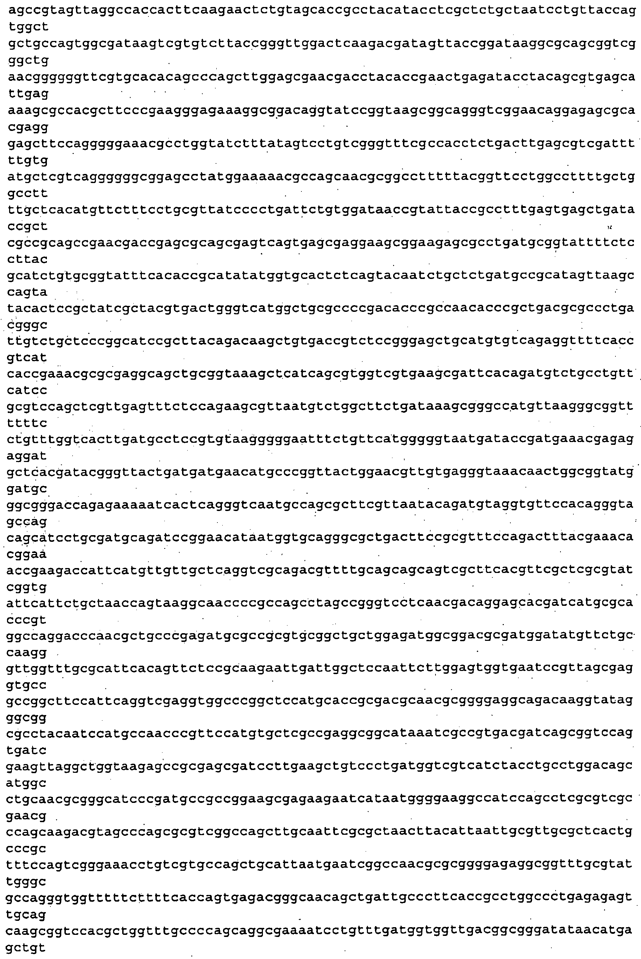

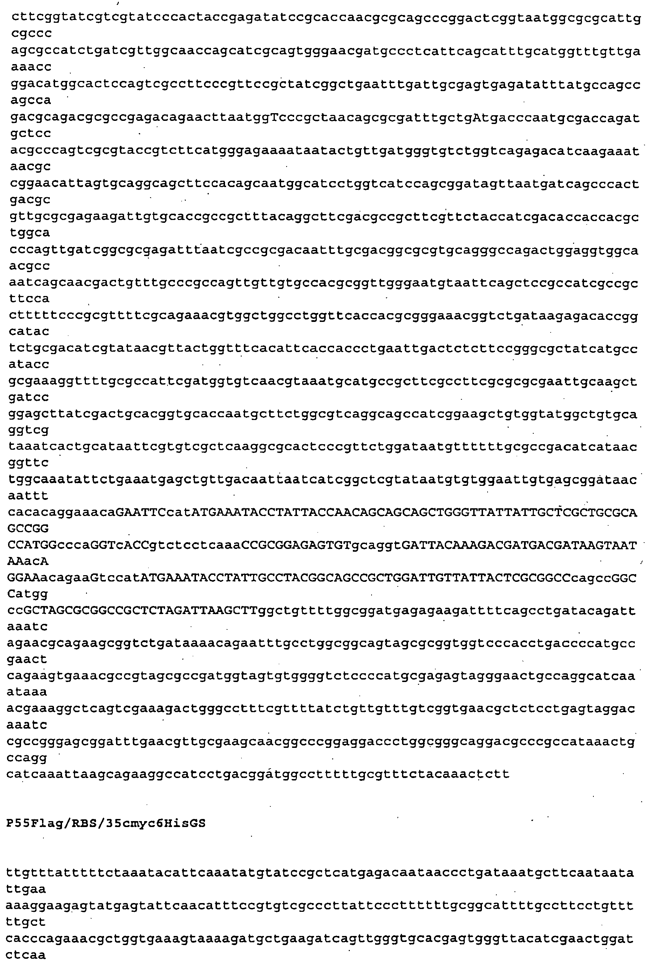

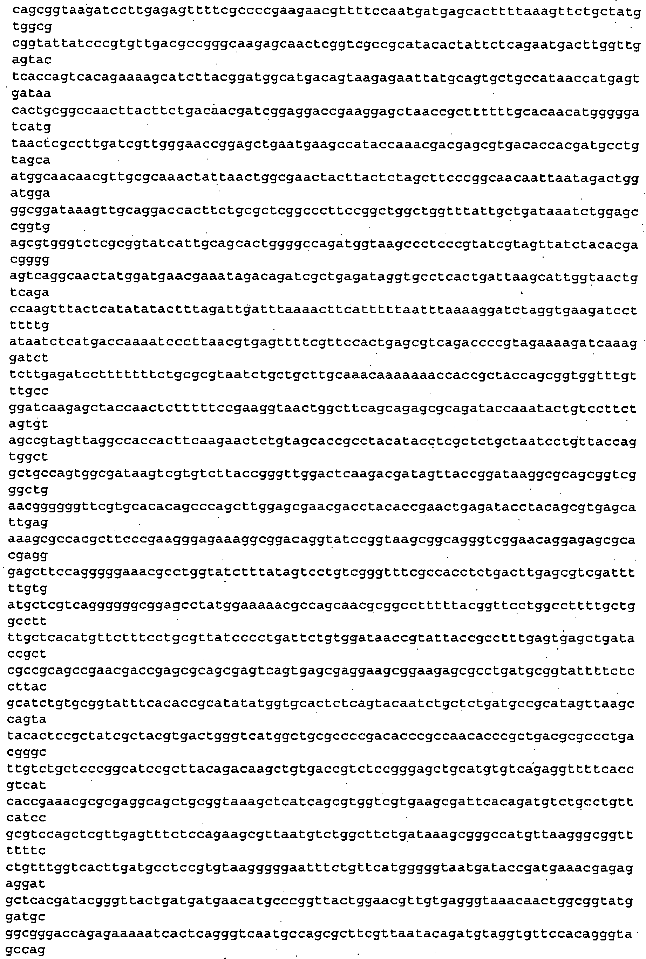

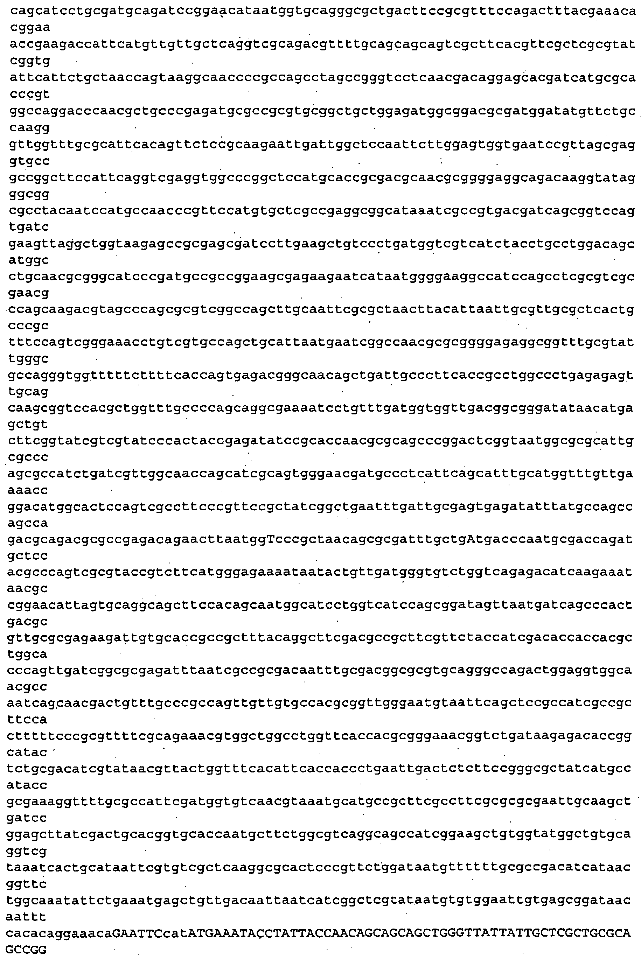

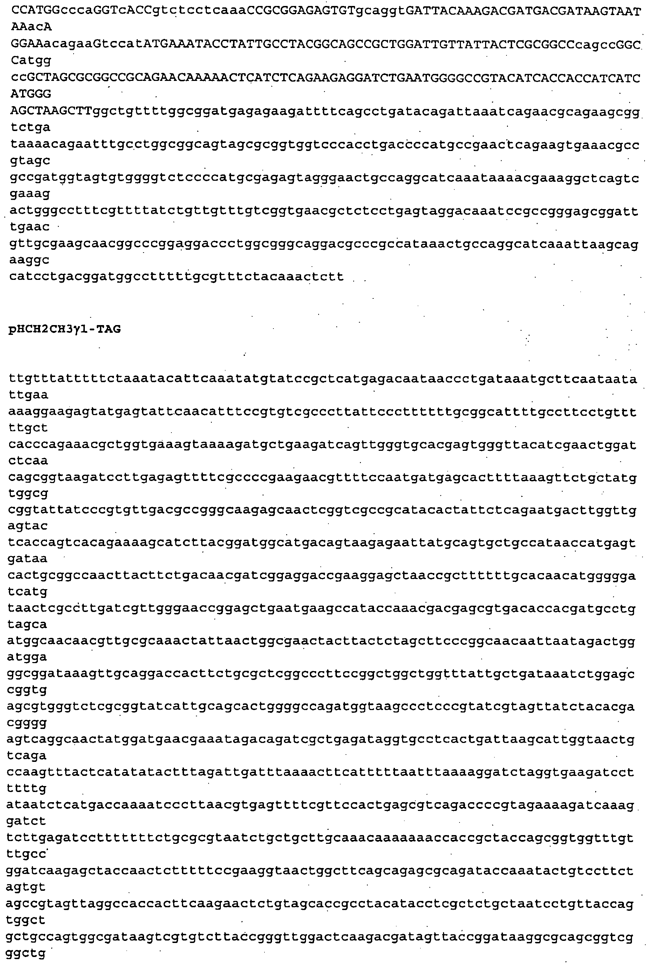

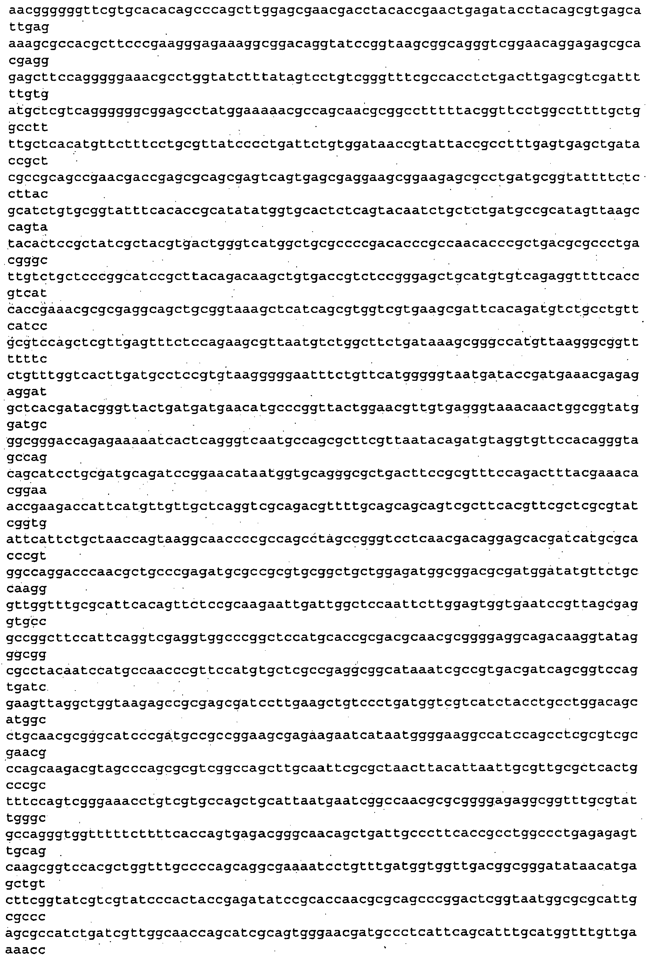

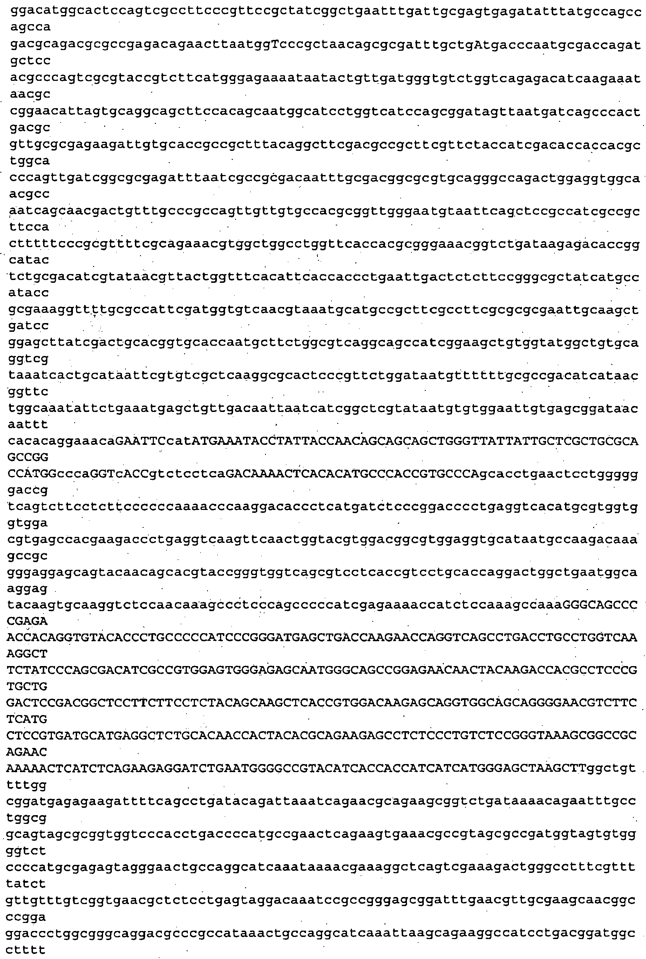

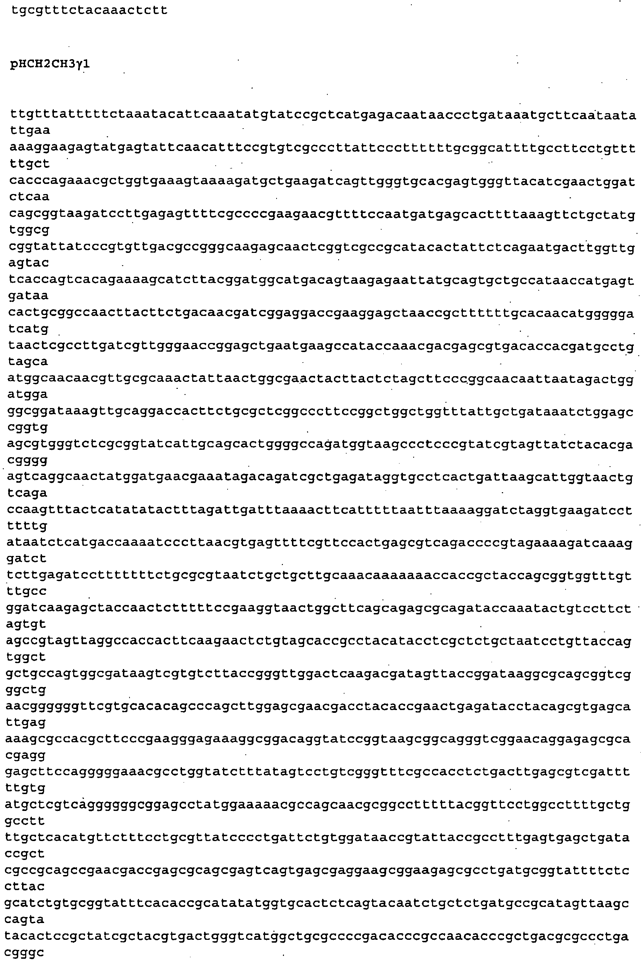

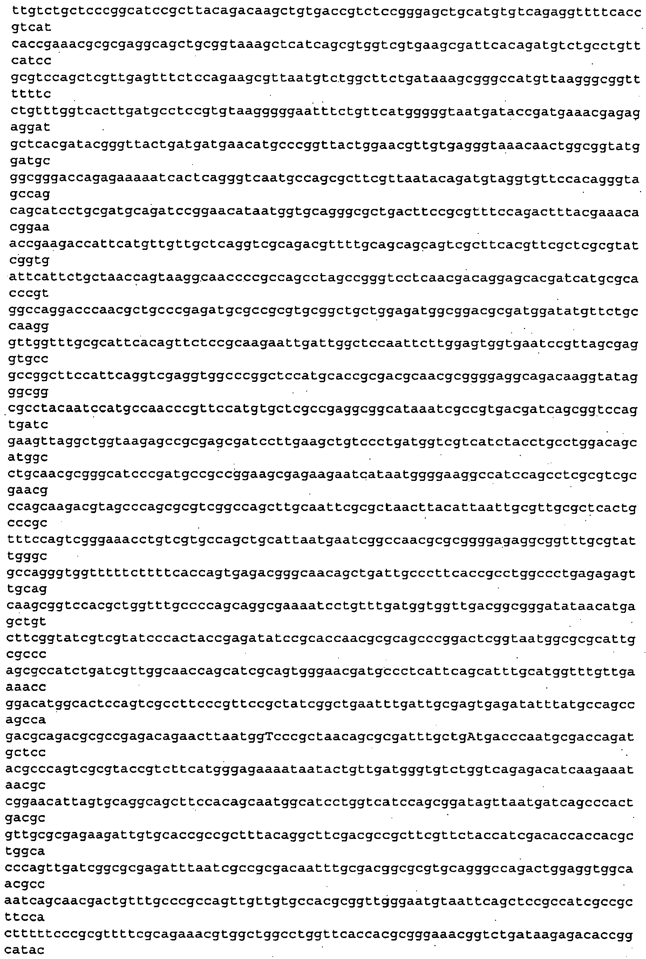

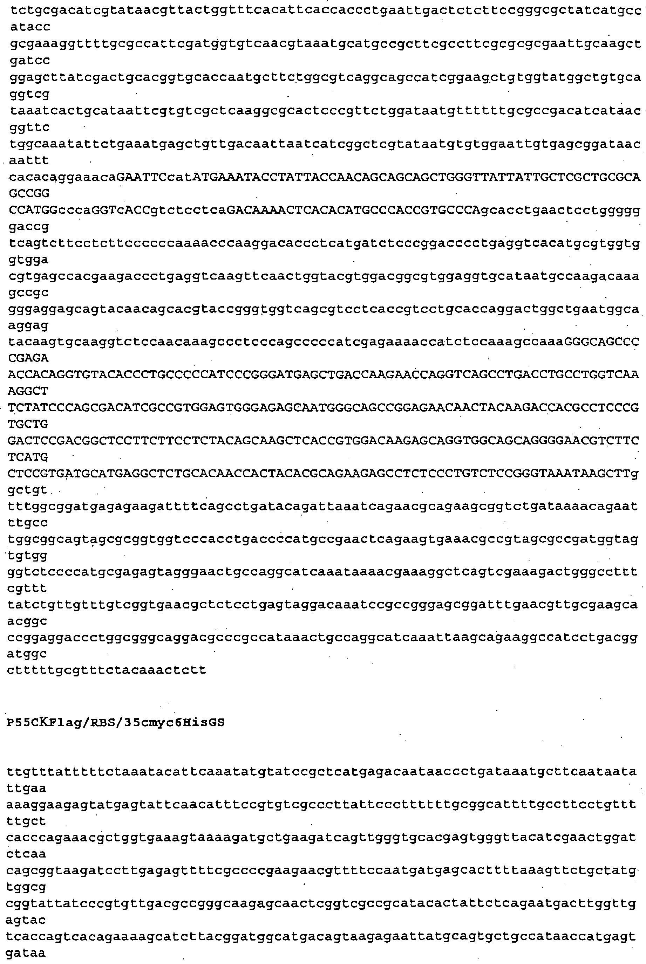

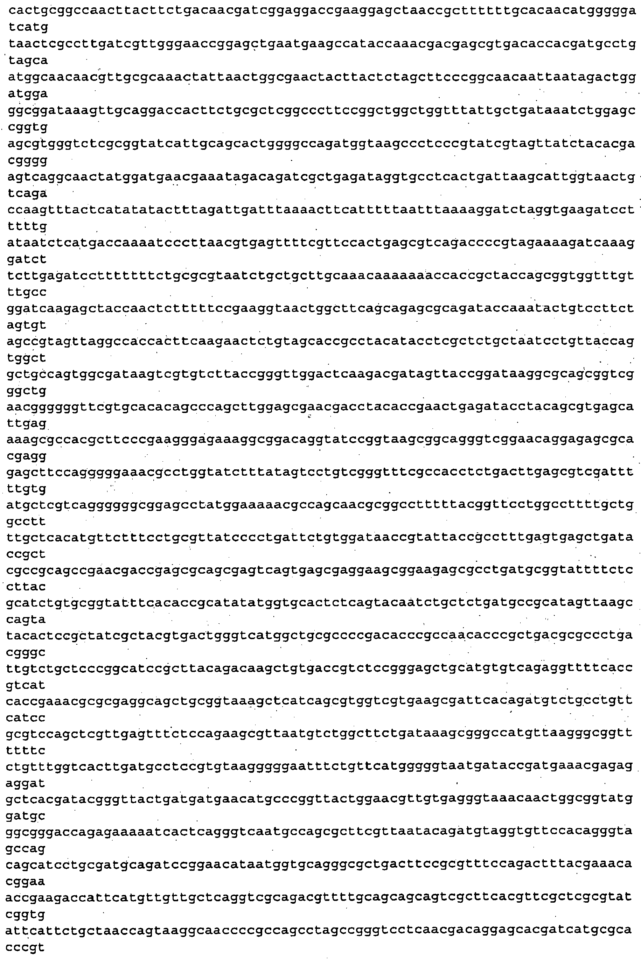

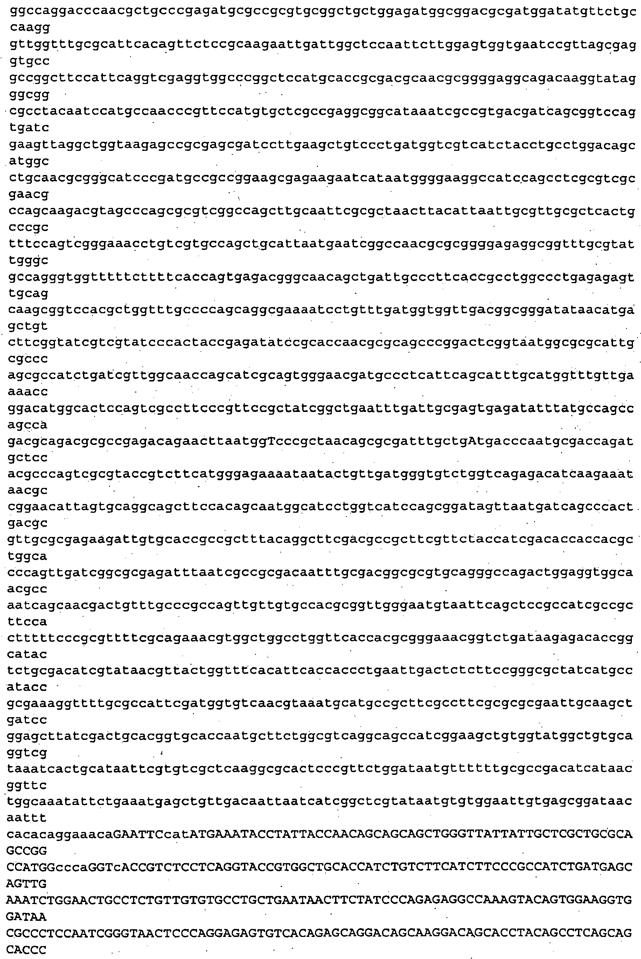

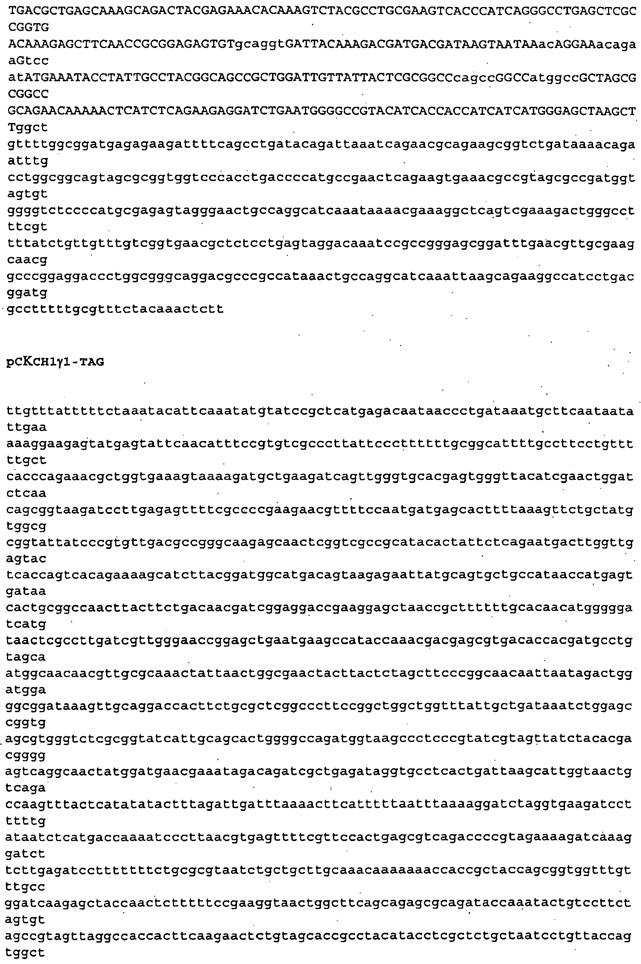

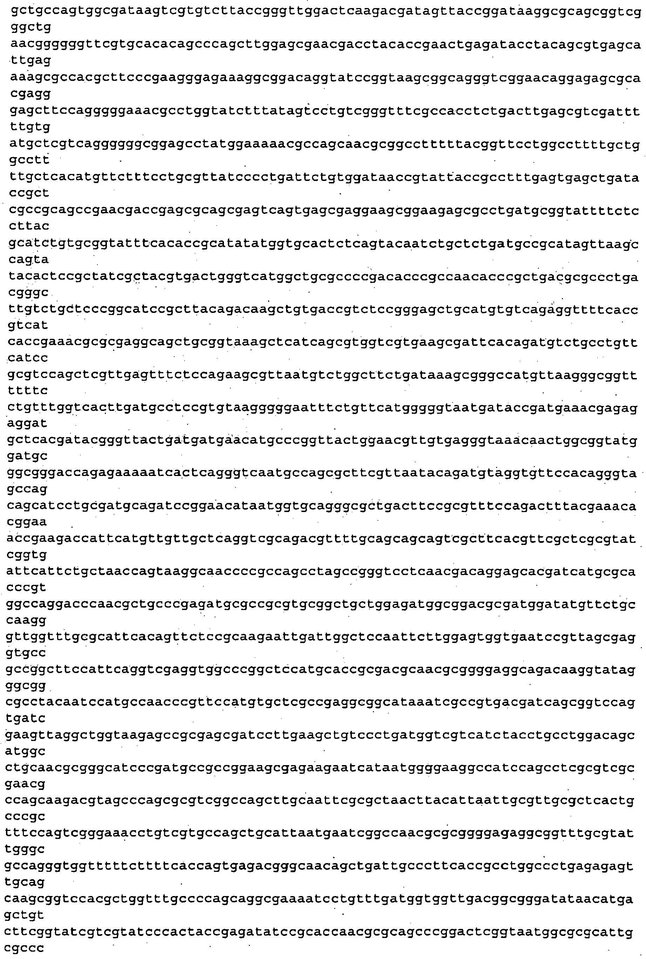

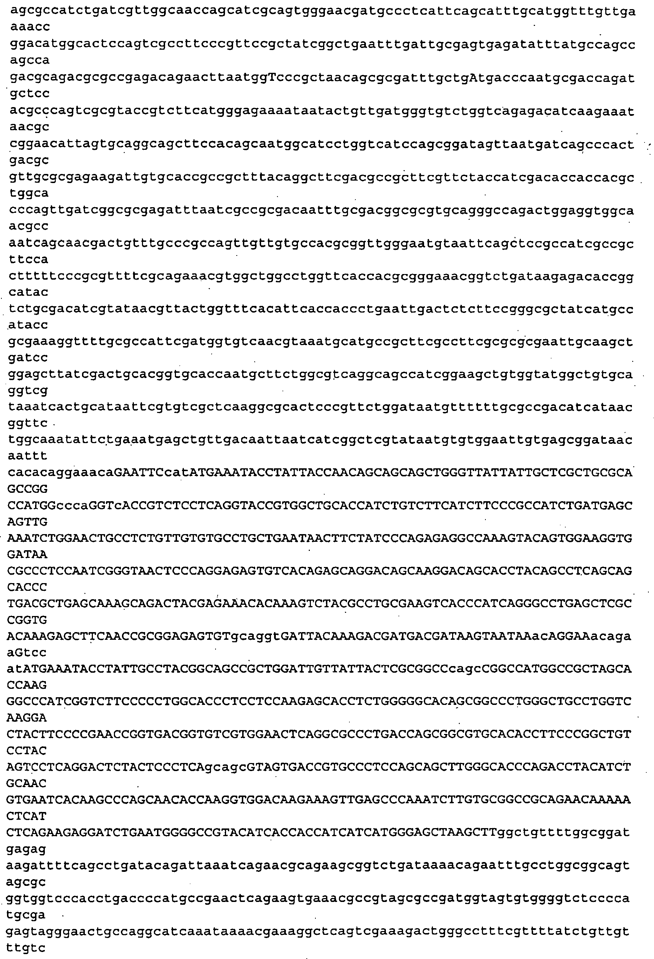

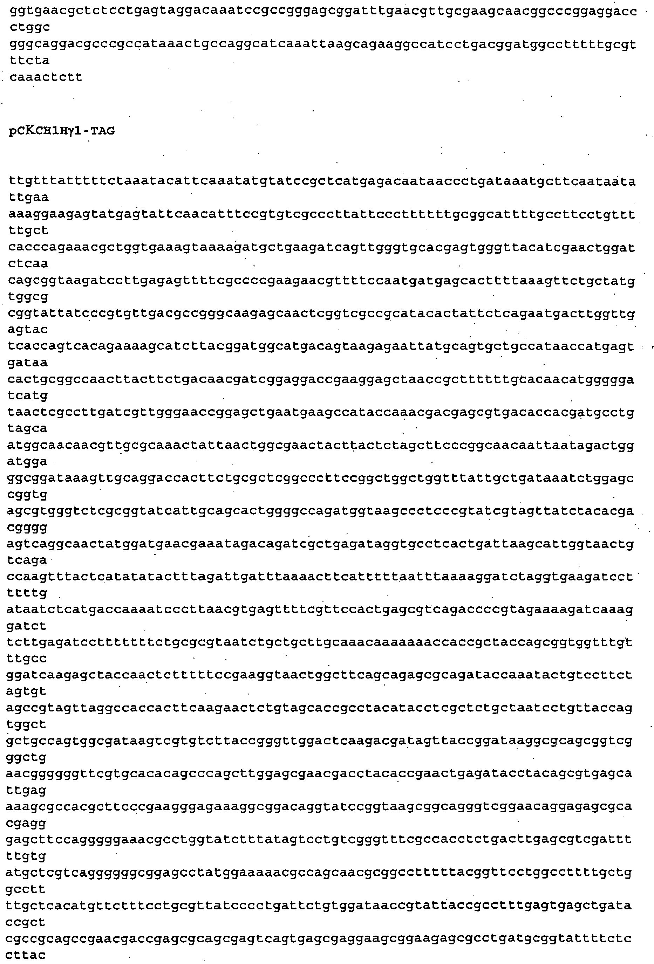

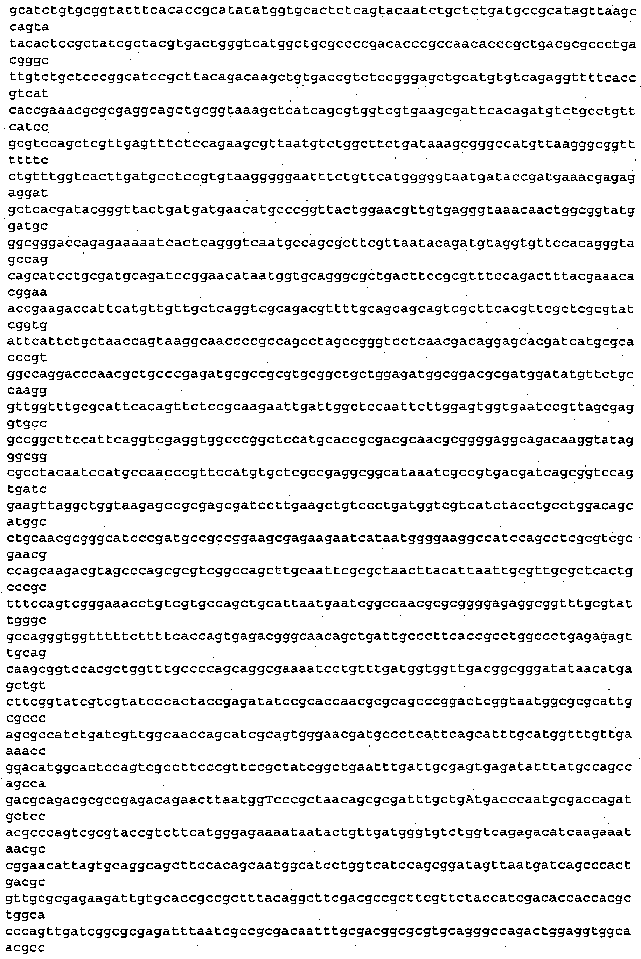

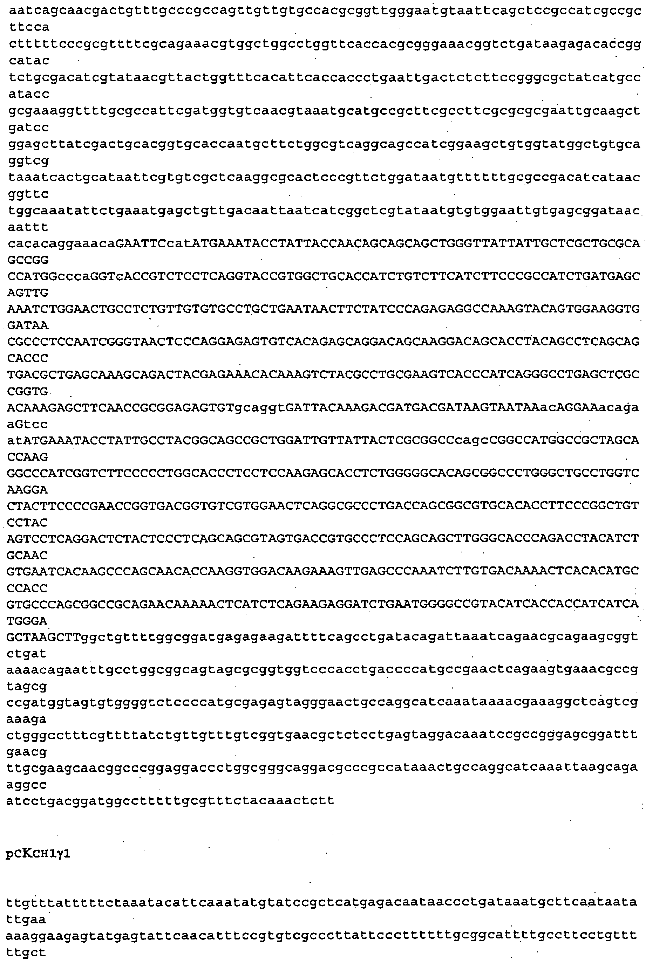

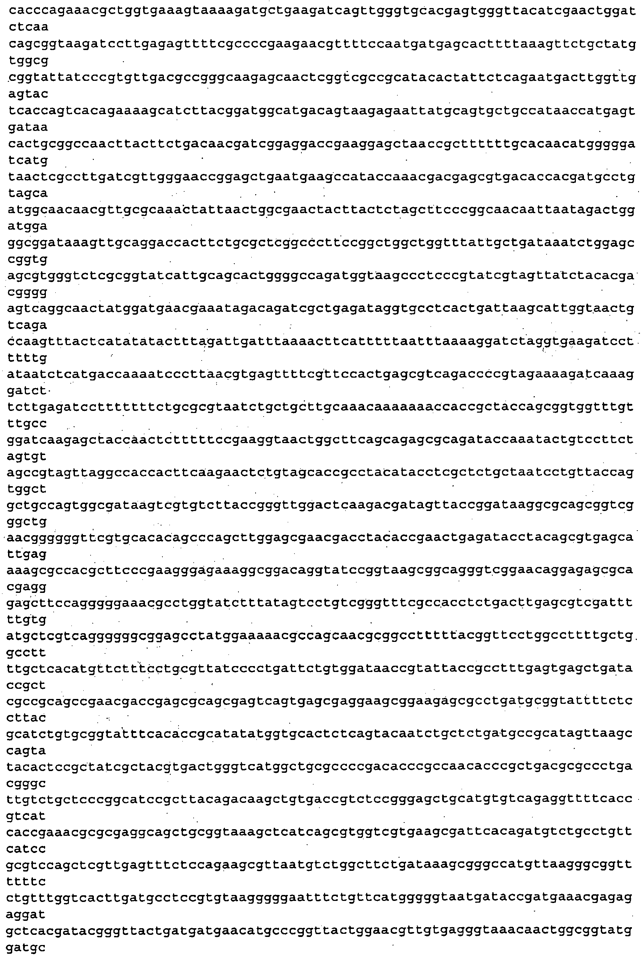

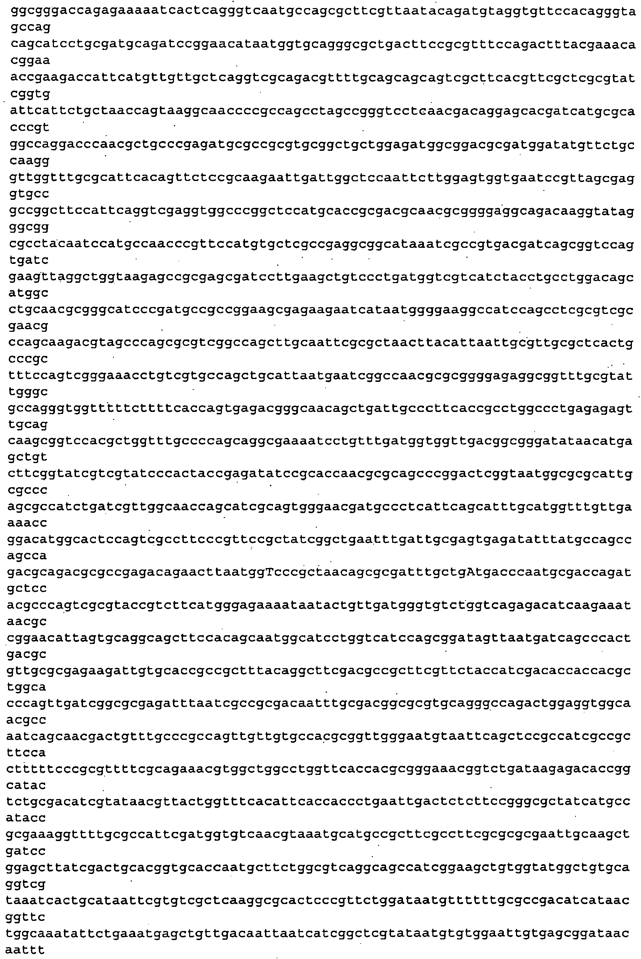

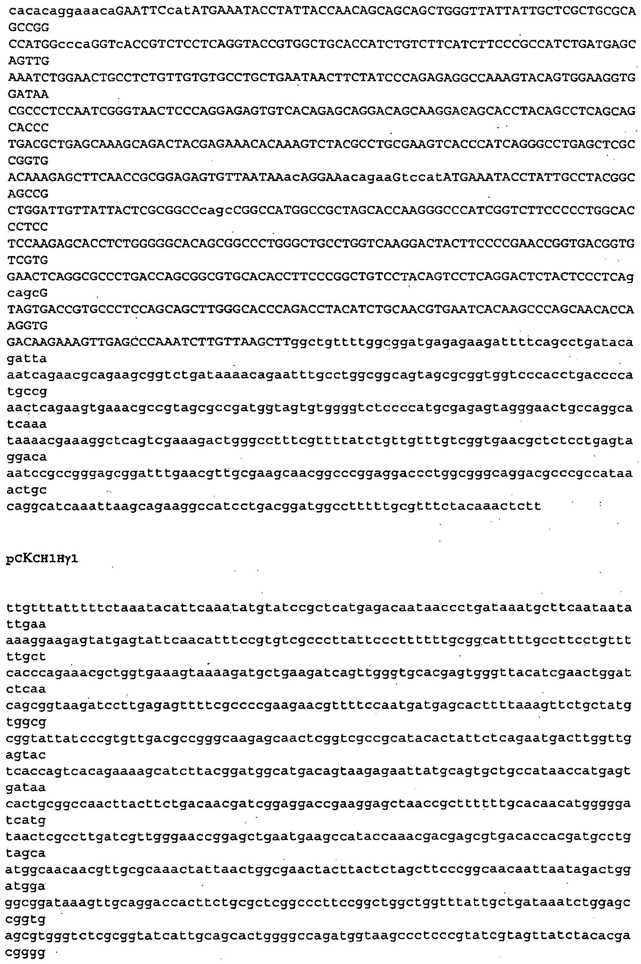

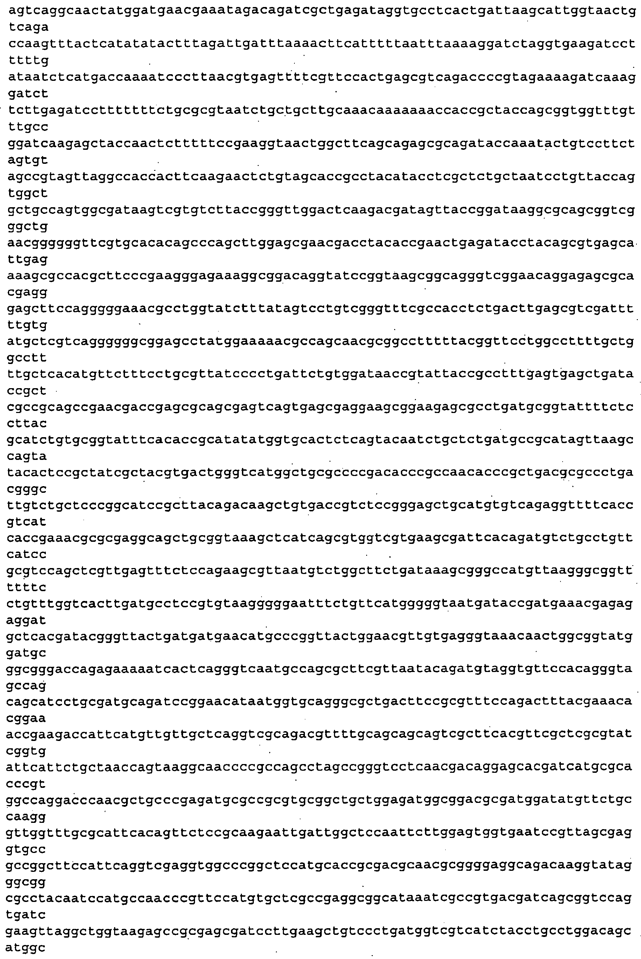

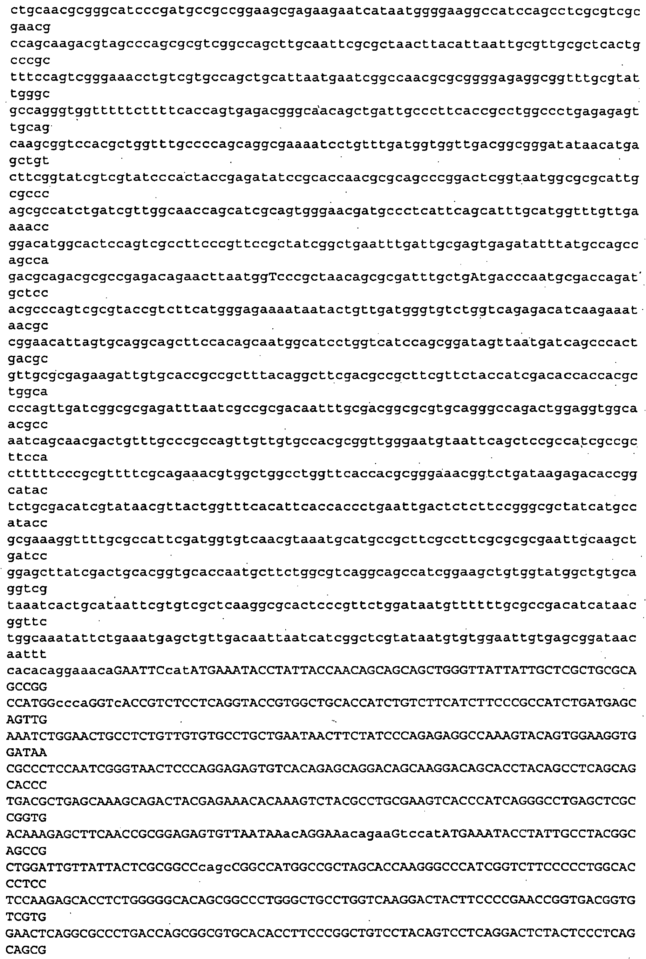

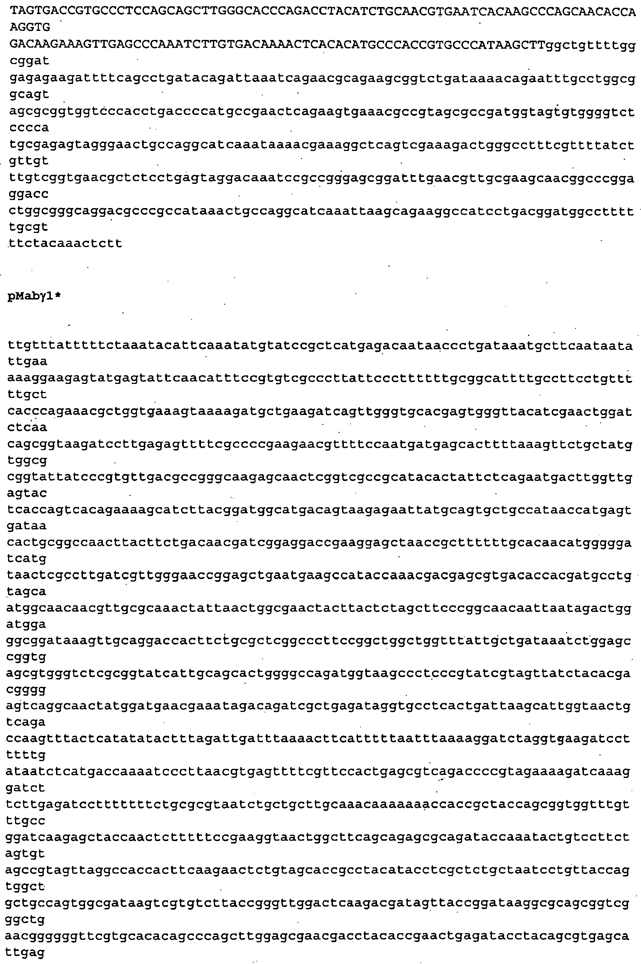

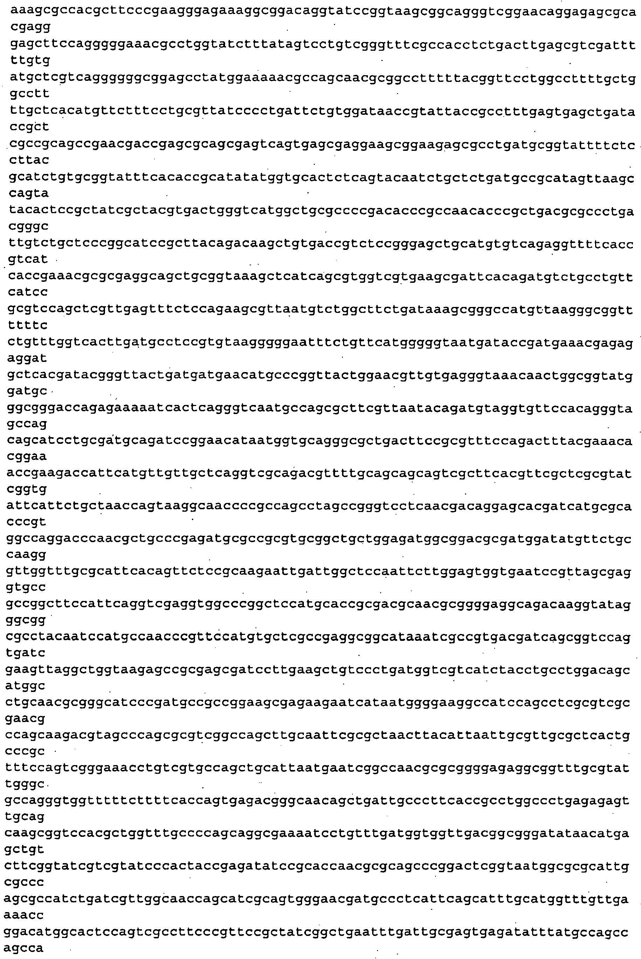

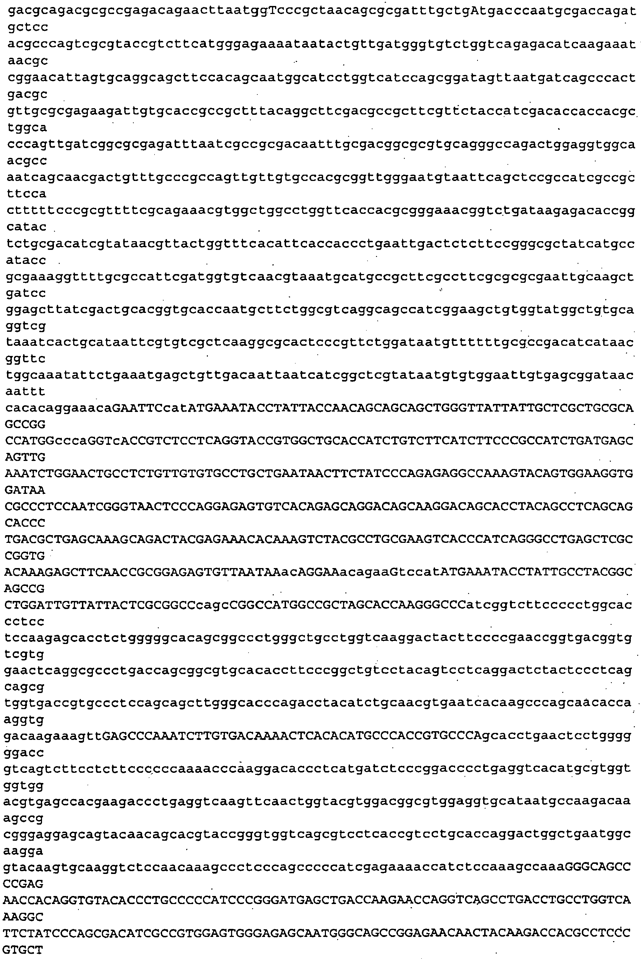

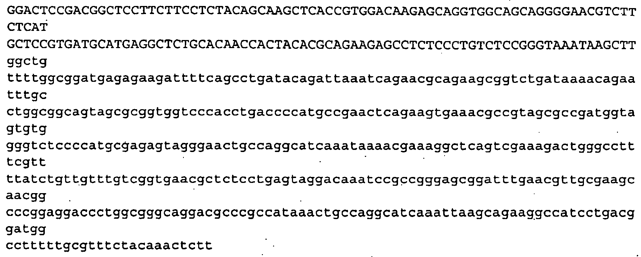

Les cellules cibles en culture sont remises en suspension (par réaction trypsique pour les cellules HeLa, mécaniquement pour les cellules MC38 et NKL) et comptées à l'aide de bleu de Trypan dans une cellule de Malassay. Deux mille cellules par puit sont incubées dans 100µL avec 3,7.10⁶ Bq de ⁵¹Cr et les différents formats de l'anticorps (à 200, 100 ou 50 µg/mL) 1h à 37°C. Les cellules sont ensuite lavées plusieurs fois pour éliminer le ⁵¹Cr restant dans le milieu ainsi que les anticorps non fixés. Les cellules NKL en suspension sont comptées et ajoutées aux cellules cibles avec des rapports effecteur/cible de 60:1 à 0,2:1. Après une incubation de 4h à 37°C, la radioactivité du Cr libéré dans le milieu est comptée à l'aide d'un compteur γ. Des exemples montrant la lyse cellulaire obtenue avec les anticorps bispécifiques anti-CEA 17 / anti-CD16 c21 et anti-CEA 17 / anti-CD16 c28 sous forme de Fab' et F(ab')₂ sont représentés en figure 9.

### Construction des différents plasmides (voir Figures 10A, 10B, 11, 12A et 12B).

- Tous les protocoles non détaillés sont décrits dans Sambrook, Fritsch et Maniatis, Molecular cloning a laboratory manual, 2nd ed, Cold Spring Harbor Laboratory Press, 1989.
- Les digestions avec les enzymes de restriction sont réalisées selon les recommandations des fournisseurs.
- Gènes d'origine humaine insérés : Les gènes codant pour les régions Cκ, CH1, H, CH2 et CH3 correspondent respectivement : aux domaines de gènes codant pour la région constante d'une chaîne légère kappa d'immunoglobuline humaine, pour la première région constante, pour la région charnière, pour la deuxième région constante et pour la troisième région constante d'une chaîne lourde d'immunoglobuline IgG1 humaine. Ces gènes ont été obtenus par RT-PCR à partir d'une poche du LFB (Laboratoire français du Fractionnement et des Biotechnologies). Ce matériel souscrit aux autorisations légales pour être utilisé pour les expériences décrites.
- La séquence de chaque plasmide est réalisée sur séquenceur ABI 310 en utilisant les oligonucléotides :
   EcoRI-90 de séquence SEQ ID N° 12 : GCGCCGACATCATAACGGTTCTGGC
   HindIII+88 de séquence SEQ ID N° 13 :CGCTACTGCCGCCAGGC
- Tous les vecteurs sont conçus pour permettre l'introduction entre 2 sites uniques d'enzymes de restriction de : différents promoteurs, différentes séquences signal de type PelB (ou autres), différentes séquences RBS, n'importe quel domaine VHH ou VHH humanisé, n'importe quel domaine Cλ ou domaines CH1, H, CH2 et CH3 de n'importe quel type d'immunoglobulines.

### pMCSPhoA'

### (Figure 10A)

La construction de ce plasmide est décrite dans Le Calvez et al. **(1995).REF**

Ce plasmide code pour la forme mature de la Phosphatase Alcaline (PhoA) démarrant au sixième résidu (Proline).

### p35PhoA'

### (Figure 10A)

La construction de ce plasmide est décrite dans le Calvez (1996).

Un fragment de gène (formé par des oligonucléotides dégénérés sur la troisième base des codons 2 à 14 codant pour la séquence signal de PelB) est inséré entre les sites Ndel et EagI du plasmide pMCSPhoA'. Les clones (1 à 60) présentant la meilleure activité phosphatase alcaline ont ensuite été ensuite sélectionnés.

### p35PhoA'/N⁻

### (Figures 10A et 11)

Suppression du site Nicol dans le gène phoA.

Une PCR de chevauchement est réalisée à partir de pMCSPhoA' avec les oligonucléotides : amont-RsrII, Ncol-sup, Ncol-inf et aval-EcoNI.

Séquences SEQ ID N° 14 à 17 des oligonucléotides utilisés :
amont-RsrII
   SEQ ID N° 14 : GGCACATGTGACCTCGCGC
NcoI-sup
   SEQ ID N° 15 : GCAACGTACCACGGCAATATCG
NcoI-inf
   SEQ ID N° 16: CGATATTGCCGTGGTACGTTGC
aval-EcoNI
   SEQ ID N° 17 : GCCATCTTTGGTATTTAGCGCC

### Conditions des PCR 1 et PCR 2 :

Un µl de plasmide (5 ng), 10 pmoles de chaque oligonucléotides (amont-Rsrll et NcoI-inf pour la PCR 1 et Ncol-sup et aval-EcoNI pour la PCR 2), 0,5 U Dynazyme (94°C, 3 min; 94°C, 45 s; 60°C, 45 s; 72°C, 45 s; pendant 25 cycles puis 72°C, 10 min) dans un volume final de 50 µl. Les produits de PCR sont purifiés à partir d'un gel d'agarose 2% (gel extraction Kit Quiagen, volume final 50 (µl)

### Conditions de la PCR 3 de chevauchement :

Un µl de chacune des PCR 1 et 2 et 0,5 U Deep Vent, dans un volume final de 50 µl. Après 5cycles (94°C, 3 min; 94°C, 1 min; 60°C, 1 min; 72°C, 1,5 min) on ajoute 10 pmoles de chaque oligonucléotides (amont-RsrII et aval-EcoNI) et la PCR est poursuivie pendant 35 cycles puis 72°C, 10 min. Le produit de la PCR 3 est purifié. à partir d'un gel d'agarose 2% (gel extraction Kit Quiagen, volume final 50 (µl).
La séquence du fragment de PCR est réalisée sur séquenceur ABI 310 en utilisant l'oligo 5' EcoRI-90.

### Clonage du fragrnent de PCR 3 dans le plasmides p35PhoA':

Trente cinq µl du fragment de PCR 3 et 5 µl (2,5 µg) de p35PhoA' sont digérés par 10 U de Rsrll et EcoNI. Après 16 h d'incubation les enzymes sont détruites 10 min à 65°C.
Chaque ADN est ensuite précipité et resuspendu avec 20 µl d'H₂O. La ligation est réalisée 16 h à 16°C avec 5 µl de fragment de PCR, 0,5 µl de vecteur et 3 U Weiss de T4 DNA ligase Biolabs dans un volume final de 10 µl. Des bactéries TG1 compétentes (technique CaCl₂) sont transformées avec 5 µl de ligation.

### p55PhoA6HisGS/N⁻

### (Figures 10A et 11)

Insertion du motif 6Histidine-Gly-Ser.

Une PCR est réalisée à partir de p35PhoA'/N⁻en utilisant les oligonucléotides Xhol-SacI et 6HisGS/HindIII.

Séquences SEQ ID N° 18 et 19 des oligonucléotides utilisés :
XhoI-SacI
   SEQ ID N° 18 : CCATGGCGGCCGATCCTCGAGAG
6HisGS/HindIII
   SEQ ID N° 19 :

### Conditions de PCR :

Une PCR est réalisée avec 5 ng de vecteur p35PhoA'/N⁻, 10 pmoles de chaque oligonucléotides et 0,5 U Dynazyme (94°C, 3, min ; 94°C, 1 min ; 70°C, 1 min ; 72°C, 1 min ; 35 cycles puis 72°C, 10 min) dans un volume final de 50 µl. Le produit de PCR est purifié à partir d'un gel d'agarose 1% (gel extraction Kit Quiagen, volume final de 50 µl).

### Clonage du fragmentXhoI-HindIII :

Vingt µl du fragment de PCR et 5 µl (2,5 µg) de vecteur p55PhoA' (Le Calvez et al. Gene 1996, 170, 51-55) sont digérés par 10 U de Xhol et HindIII en présence de BSA. Après 16 h d'incubation, les enzymes sont détruites 10 min à 65°C. Chaque ADN est ensuite précipité et resuspendu avec 20 µl d'H₂O. La ligation est réalisée 16 h à 16°C avec 5 µl de fragment de PCR, 0,5 µl de vecteur et 3 U Weiss de T4 DNA ligase Biolabs dans un volume final de 10 µl. Des bactéries TG1 compétentes (technique CaCl₂) sont transformées avec 5 µl de ligation.

### p55PhoA6HisGS/NAB⁻

### (Figures 10A et 11)

Suppression des sites ApaI et BstEII et p55PhoA6HisGS/N⁻.

Une PCR de chevauchement est réalisée à partir de p55PhoA6HisGS/N⁻ avec les oligonucléotides : amont-EcoRV, ApaI-BstEII-sup, BstEII-ApaI-inf et AvaI-MluI. Séquences SEQ ID N° 20 à 23 des oligonucléotides utilisés :
amont-EcoRV
   SEQ ID N° 20 : CATGAGCTGTCTTCGGTATC
ApaI-BstEII-sup
   SEQ ID N° 21 : TAATGGTCCCGCTAACAGCGCGATTTGCTGATGACCCA
BstEII-ApaI-inf
   SEQ ID N° 22 : TGGGTCATCAGCAAATCGCGCTGTTAGCGGGACCATTA
avaI-MluI
   SEQ ID N° 23 : GAACGAAGCGGCGTCGAAG

### Conditions des PCR1 et PCR 2 :

Un µl (5 ng) de plasmide p55PhoA6HisGS/N⁻, 10 pmoles de chaque oligonucléotides (amont-EcoRV et BstEII-ApaI-inf pour PCR 1 et ApaI-BstEII-sup et avaI-MluI pour PCR 2), 0,5 U Dynazyme (94°C, 3 min ; 94°C, 45 s ; 60°C, 45 s ; 72°C, 45 s ; 25 cycles puis 72°C, 10 min. Les produits de PCR sont purifiés à partir d'un gel d'agarose 2% (gel extraction Kit Quiagen, volume final 50 µl).

### Conditions de la PCR 3 de chevauchement :

Un µl de chacune des PCR 1 et 2 et 0,5 U Deep Vent, dans un volume final de 50 µl. Après 5cycles (94°C, 3 min; 94°C, 1 min; 60°C, 1 min; 72°C, 1,5 min) on ajoute 10 pmoles de chaque oligonucléotides (amont-EcoRV et avaI-MluI) et la PCR est poursuivie pendant 35 cycles puis 72°C, 10 min. Le produit de la PCR 3 est purifié à partir d'un gel d'agarose 2% (gel extraction Kit Quiagen, volume final 50 µl).

La séquence du fragment de PCR est réalisée sur séquenceur ABI 310 en utilisant l'oligo 5' EcoRI-90.

### Clonage du fragment de PCR 3 dans le plasmide p55PhoA6HisGS/N⁻:

Trente cinq (µl du fragment de PCR 3 et 5 µl (2,5 µg) de p55PhoA6HisGS/N⁻ sont digérés par 10 U de EcoRV et MluI. Après 16 h d'incubation les enzymes sont détruites 10 min à 65°C. Chaque ADN est ensuite précipité et resuspendu avec 20 µl d'H₂O.
La ligation est réalisée 16 h à 16°C avec 5 µl de fragment de PCR, 0,5 µl de vecteur et 3 U Weiss de T4 DNA ligase Biolabs dans un volume final de 10 µl. Des bactéries TG1 compétentes (technique CaCl₂) sont transformées avec 5 µl de ligation.

### p55PhoA6HisGS⁻/NAB⁻

### (Figures 10A et 11)

### Déphasage du gène PhoA au niveau du site EagI.

Ce déphasage crée un site unique FseI.

Cinq µl (2,5 µg) de p55PhoA6HisGS/NAB⁻ sont digérés par 10 U de EagI. Après 16 h d'incubation l'enzyme est détruite 10 min à 65°C. Le mélange réactionnel est ensuite précipité et resuspendu avec 20 µl d'H₂O. On ajoute un mélange équimolaire de dGTP et dCTP (33 µM final) et 2,5 U de fragment Klenow exo- (Biolabs) dans un volume final de 50 µl, 15 min à 25°C. On arrête la réaction avec 2 µl d'EDTA à 500 mM, 20 min à 75°C. Le mélange réactionnel est précipité à l'éthanol, repris avec 5 µl d'H₂O et ligaturer avec 3 U Weiss de T4 DNA ligase Biolabs dans un volume final de 10 µl. Des bactéries TG1 compétentes (technique CaCl₂) sont transformées avec 5 µl de ligation.

Ce plasmide permet le clonage et la sélection des fragments d'anticorps VH humains les mieux sécrétés.

### p55/MCS1

### (Figures 10A et 11)

Insertion du MCS1 dans p55PhoA6HisGS/NAB⁻ entre les sites NcoI et HindIII en utilisant les oligonucléotides appariés 5' MCS1 et 3' MCS 1.

Séquences SEQ ID N° 24 et 25 des oligonucléotides utilisés :
5' MCS1
   SEQ ID N°24 :
3' MCS1
   SEQ ID N° 25 :

Cinq µl (2,5 µg) de vecteur p55PhoA6HisGS/NAB⁻ sont digérés 16 h par 10 U de chaque enzyme NcoI et HindIII. Dix pmoles de chacun des oligonucléotides 5' MCSI et 3' MCSI sont incubés 5 min à 80°C, puis la solution est redescendue lentement à température ambiante.
Ligaturer 5 µl de vecteur avec 1,2 µl de la cassette hybridée (5' MCSI + 3' MCSI) en présence de 3 U Weiss deT4 DNA ligase Biolabs 1 h à température ambiante. La ligase est détruite en incubant 10 min à 65°C. On rajoute un mélange réactionnel (2 h) de 90 µl, contenant 10 U de EagI pour détruire le vecteur d'origine. Ce mélange est précipité à l'ethanol et repris avec 10 µl d'H2O. Des bactéries TG1 compétentes (technique CaCl₂) sont transformées avec 5 µl de mélange.

### p55Flag/RBS/35

### (Figures 10A et 11)

Insertion du motif Flag/RBS/PelB35 dans p55/MCS1 entre les sites SfiI et Sac2 en utilisant les oligonucléotides appariés 5' Flag/RBS/35-sup et 3' Flag/RBS/35-inf. Séquences SEQ ID N° 26 et 27 des oligonucléotides utilisés :
5' Flag/RBS/35-sup
   SEQ ID N°26 : SEQ ID N° 117 :
3' Flag/RBS/35-inf
   SEQ ID N° 27 : SEQ ID N° 118 :

Le clonage s'effectue exactement selon les conditions décrites précédemment pour l'insertion du MCSI. Après ligature le mélange réactionnel est digéré par 10 U d'enzyme XhoI.

### p55Flag/RBS/35cmyc6HisGS

### (Figures 10B et 11)

Insertion du motif c-myc-6HisHS dans p55Flag/RBS/35 entre les sites NotI et HindIII en utilisant les oligonucléotides appariés 5' c-myc-6HisGS et 3' c-myc-6HisGS. Séquences SEQ ID N° 28 et 29 des oligonucléotides utilisés :
5' c-myc-6HisGS
   SEQ ID N° 28: SEQ ID N° 29:
3'c-myc-6HisGS

Le clonage s'effectue exactement selon les conditions décrites précédemment pour l'insertion du MCSI. Le mélange de ligature est digéré par 10 U d'enzyme XbaI.

### p55CκFlag/RBS/35cmyc6HisGS

### (Figures 10B et 11)

Insertion de la région constante légère Ckappa d'une immunoglobuline dans p55Flag/RBS/35cmyc6HisGS.

Séquences SEQ ID N° 30 ET 31 des oligonucléotides utilisés :
5'Cκ
   SEQ ID N° 30 : SEQ ID N° 119 :
3'Cκ
   SEQ ID N°31 :

### Amplification du domaine Cκ :

Des lymphocytes B humains sont purifiés par gradient de Ficoll à partir de la poche fournie par LFB. L'ARN total est ensuite préparé selon le protocole décrit au paragraphe 1.3.2.

### Hybridation:

Un µl d'ARN total est préincubé avec 1 pmole d'oligonucléotide 3' Cκ pendant 10 min à 70°C dans un volume total de 8 µl. La température est diminuée lentement (45 min) jusqu'à 37°C.

### Transcription inverse :

Aux 8 µl, on ajoute 0,5 µl de RNAsine (20 U), 3 µl de tampon 5X (SuperScriptII, Invitrogen), 1 µl DTT, 100 mM, 2 µl dNTP 10 mM et on incube 10 min à 50°C. On ajoute ensuite 0,75 µl de SuperScript (150 U) et l'incubation est poursuivie 30 min à 50°C et 15 min à 70°C.

L'ADNc obtenu est purifié sur billes (BioMag Carboxyl Terminated, Polysciences) selon les recommandations du fournisseur. L'élution finale est réalisée avec 15 µl de Tris-acétate 10 mM pH 7,8.

### Conditions de PCR 1 et 2 :

La PCR1 est réalisée avec 1 µl d'ADNc, 10 pmoles de chaque oligonucléotides 5' Cκ et 3' Cκ, 0,5 U Dynazyme (94°C, 3 min ; 94°C, 1 min ; 60°C, 1 min ; 72°C, 1,5 min ; 30 cycles puis 72°C, 10 min) dans un volume final de 50 µl.

La PCR2 est faite à partir de 1 µl de PCR1 en utilisant 0,5 U de Deep-Vent, (94°C, 3 min ; 94°C, 45 s ; 60°C, 45 s ; 72°C, 45 s ; 25 cycles puis 72°C, 5 min) dans un volume final de 50 µl. Le produit de PCR est purifié à partir d'un gel d'agarose 2% (gel extraction Kit Quiagen, volume final 50 µl).

Le fragment de PCR est séquencé avant clonage sur ABI310 avec les oligonucléotides 5' Cκ et 3' Cκ.

Le clonage du domaine Cκ est effectué entre les sites BstEII et SacII de p55Flag/RBS/35cmyc6HisGS :
20 µl du fragment de PCR 2 et 5 µl (2,5 µg) de p55Flag/RBS/35cmyc6HisGS sont digérés par 10 U de BstEII et SacII. Après 16 h d'incubation les enzymes sont détruites 10 min à 65°C. Chaque ADN est ensuite précipité et resuspendu avec 20 µl d'H₂O.

La ligature est réalisée 16 h à 16°C avec 5 µl de fragment de PCR 2, 0,5 µl de vecteur et 3 U Weiss de T4 DNA ligase Biolabs dans un volume final de 10 µl. Des bactéries TG1 compétentes (technique CaCl₂) sont transformées avec 5 µl de ligation. **p55CκFlag**/**RBS**/**35CH1γ1cmyc6HisGS (*pCκCH1γ1-TAG*)**

### (Figures 10B et 11)

Insertion de la région constante lourde CH1 d'une immunoglobuline de type IgG1 dans p55CκFlag/RBS/35cmyc6HisGS.

Séquences SEQ ID N°32 à 35 des oligonucléotides utilisés :
5' CH1γ1
   SEQ ID N°32:
   CTCGAGGCGGCCCAGCCGGCCATGGCCGCTAGCACCAAGGGCCCATCGG
3'CH1 γ1
   SEQ ID N°33:
   AAGCTTAATCTAGAGCGGCCGCACAAGATTTGGGCTCAACTTTC
BstEII-sup
   SEQ ID N°34:
   CCCTCAGCAGCGTAGTGACCGTGCCCTCC
BstEII-inf
   SEQ ID N°35:
   GGAGGGCACGGTCACTACGCTGCTGAGGG

L'amplification du domaine CH1γ1 est réalisée par PCR de chevauchement pour détruire le site BstEII.

La transcription inverse est réalisée exactement comme décrite ci-dessus pour la Cκ, mais en utilisant l'oligonucléotide 3' CH1γ1.

La PCR 1 après RT est réalisée avec 1 µl d'ADNc, 10 pmoles de chaque oligonucléotides 5' CH1 γ1 et 3' CH1 γ1, 0,5 U de Dynazyme (94°C, 3 min ; 94°C, 1 min ; 60°C, 1 min ; 72°C, 1,5 min ; 30 cycles puis 72°C, 10 min) dans un volume final de 50 µl.

La PCR 2a est réalisée à partir de 1 µl la PCR 1, avec les oligonucléotides 5' CH1 γ1 et BstEII-inf en utilisant 0,5 U de Dynazyme (94°C, 3 min ; 94°C, 45 s ; 60°C, 45 s ; 72°C, 45 s ; 25 cycles puis 72°C, 5 min) dans un volume final de 50 µl.

La PCR 2b est réalisée à partir de 1 µl la PCR 1, avec les oligonucléotides BstEII-sup et 3' CH1 γ1 en utilisant 0,5 U de Dynazyme (94°C, 3 min ; 94°C, 45 s ; 60°C, 45 s ; 72°C, 45 s ; 25 cycles puis 72°C, 5 min) dans un volume final de 50 µl.

La PCR 3 est réalisée à partir d'un µl de chaque PCR 2a et PCR 2b, avec les oligonucléotides 5' CH1γ1 et 3' CH1γ1, en utilisant 0,5 U de Deep-Vent (94°C, 3 min ; 94°C, 45 s ; 60°C, 45 s ; 72°C, 45 s ; 25 cycles puis 72°C, 5 min) dans un volume final de 50 µl.

Le produit de PCR 3 est purifié à partir d'un gel d'agarose 2% (gel extraction Kit Quiagen, volume final 50 µl). La séquence du fragment de PCR 3 est réalisée sur séquenceur ABI 310 en utilisant les oligonucléotides : 5' CH1γ1 et 3' CH1γ1.

Le clonage est réalisé comme décrit pour le domaine Cκ mais entre les sites SfiI et NotI de p55CκFlag/RBS/35cmyc6HisGS.

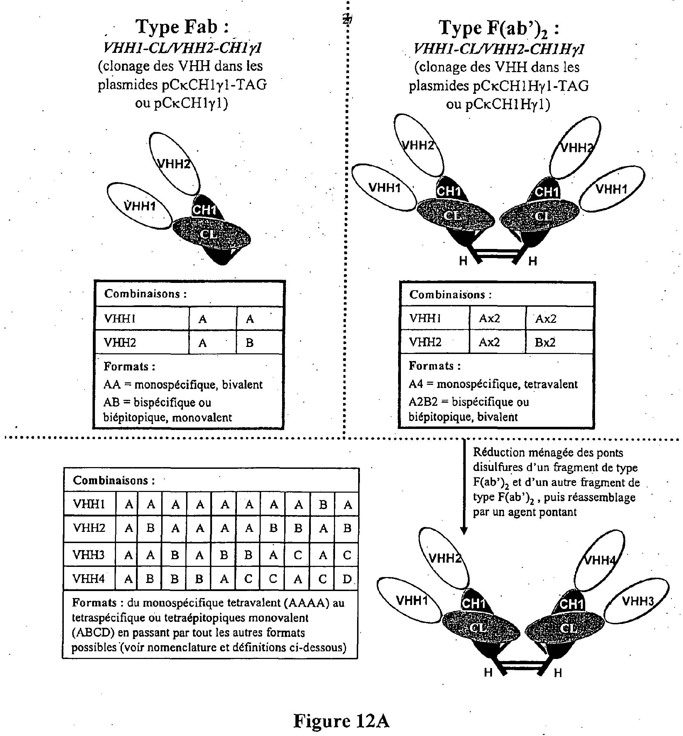

Le plasmide résultant est appelé plus communément: pCκCH1γ1-TAG, il permet la production de fragments d'anticorps de type Fab dont chaque chaîne possède une étiquette (Figure 12A).

### p55CκFlag/RBS/35CH1Hγ1cmyc6HisGS (pCκCH1Hγ1-TAG)

### (Figures 10B et 11)

Insertion de la région constante lourde CH1 et de la région Hinge (H) d'une immunoglobuline de type IgG1 dans p55CκFlag/RBS/35cmyc6HisGS.

Les PCR1, 2a, 2b et 3 sont réalisées exactement comme pour l'amplification du domaine CH1 décrite ci-dessus en remplaçant l'oligonucléotide 3' Cγ1 par l'oligonucléotide 3' CH1Hγ1 dont la séquence SEQ ID N° 36 est indiquée ci-dessous :
3' CH1Hγ1
   SEQ ID N° 36 :

Le clonage est réalisé comme décrit pour le domaine CH1γ1 entre les sites SfiI et NotI de p55CκFlag/RBS/35cmyc6HisGS.

Le plasmide résultant est appelé plus communément : pCκCH1Hγ1-TAG, il permet la production de fragments d'anticorps de type F(ab')₂ dont chaque chaîne possède une étiquette (Figure 12A).

### p55Cκ/RBS/35CH1γ1 (pCκCH1γ1)

### (Figures 10B et 11)

Elimination des étiquettes Flag et c-myc-6hisGS du plasmide p55CκFlag/RBS/35CH1γ1cmyc6HisGS par remplacement du fragment d'ADN compris entre SacII et SfiI par une nouvelle cassette en utilisant les oligonucléotides appariés 5' RBS/35-sup et 3' RBS/35-inf.

Séquences SEQ ID N°37 et 38 des oligonucléotides utilisés :
5' RBS/35-sup
   SEQ ID N°37 : SEQ ID N° 120 :
3' RBS/35-inf
   SEQ ID N°38 : SEQ ID N° 121 :

Le clonage s'effectue exactement selon les conditions décrites pour l'insertion du MCSI. Le plasmide résultant intermédiaire est appelé : p55Cκ/RBS/35CH1γ1cmyc6HisGS.

Le motif c-myc-6HisGS est enlevé en reclonant le domaine CH1γ1 dans p55Cκ/RBS/35CH1γ1cmyc6HisGS.

Une PCR est utilisée en amplifiant à partir de 5 ng de plasmide p55CκFlag/RBS/35CH1γ1cmyc6HisGS le domaine CH1γ1 avec les oligonucléotides 5' CH1γ1 et 3' CH1γ1-STOP.

Séquence SEQ ID N°39 de l'oligonucléotide utilisé :
3' CH1γ1-STOP
   SEQ ID N°39 :
   CATGCAGTCCCAAGCTTAACAAGATTTGGGCTCAACTTTC

Le clonage du fragment de PCR est réalisé comme décrit pour le domaine Cκ mais entre les sites SfiI et et HindIII du plasmide p55Cκ/RBS/35CH1γ1cmyc6HisGS.

Le plasmide résultant est appelé plus communément : pCκCH1γ1, il permet la production de fragments d'anticorps de type Fab (Figure 12A).

### p55Cκ/RBS/35CH1Hγ1 (pCκCH1Hγ1)

### (Figures 10B et 11)

Elimination des étiquettes Flag et c-myc-6hisGS du plasmide p55CκFlag/RBS/35CH1Hγ1cmyc6HisGS par remplacement du fragment d'ADN compris entre SacII et SfiI par une nouvelle cassette en utilisant les oligonucléotides appariés 5' RBS/35-sup et 3' RBS/35-inf décrits précédemment.

Le clonage s'effectue exactement selon les conditions décrites pour l'insertion du MCSI. Le plasmide résultant intermédiaire est appelé : p55Cκ/RBS/35CH1Hγ1cmyc6HisGS.

Le motif c-myc-6HisGS est enlevé en reclonant le domaine CH1Hγ1 dans p55Cκ/RBS/35CH1Hγ1cmyc6HisGS.

Une PCR est utilisé en amplifiant à partir à partir de 5 ng de plasmide p55CκFlag/RBS/35CH1Hγ1cmyc6HisGS le domaine CH1Hγ1 avec les oligonucléotides 5' CH1γ1 et 3' CH1Hγ1-STOP.
Séquence SEQ ID N°40 de l'oligonucléotide utilisé :
3' CH1Hγ1-STOP
   SEQ ID N°40 :
   CATGCAGTCCCAAGCTTATGGGCACGGTGGGCATGTGTG

Le clonage du fragment de PCR est réalisé entre les sites SfiI et HindIII comme décrit précédemment mais à partir du plasmide p55Cκ/RBS/35CH1Hγ1cmyc6HisGS.

Le plasmide résultant est appelé plus communément : pCκCH1Hγ1, il permet la production de fragments d'anticorps de type F(ab')₂ (Figure 12A).

### p55Cκ/RBS/35CH1HCH2CH3γ1 (pMaby1*)

### (Figures 10B et 11)

Insertion de la région constante lourde CH1, de la région Hinge (H) et des régions constantes CH2 et CH3 d'une immunoglobuline de type IgG1 dans p55CκFlag/RBS/35CH1γ1cmyc6HisGS.

Les PCR1, 2a, 2b et 3 sont réalisées exactement comme pour l'amplification du domaine CH1 décrite ci-dessus en remplaçant l'oligonucléotide 3' CH1γ1 par l'oligonucléotide 3' HindIII/H-CH2-CH3 dont la séquence SEQ I D N°41 est indiquée ci-dessous :
3' HindIII/H-CH2-CH3
   SEQ ID N°41 :
   CCGCCAAAACAGCCAAGCTTATTTACCCGGAGACAGGGAG

Le clonage est réalisé comme décrit pour le domaine CH1γ entre les sites SfiI et HindIII de p55CκFlag/RBS/35CH1γ1cmyc6HisGS.

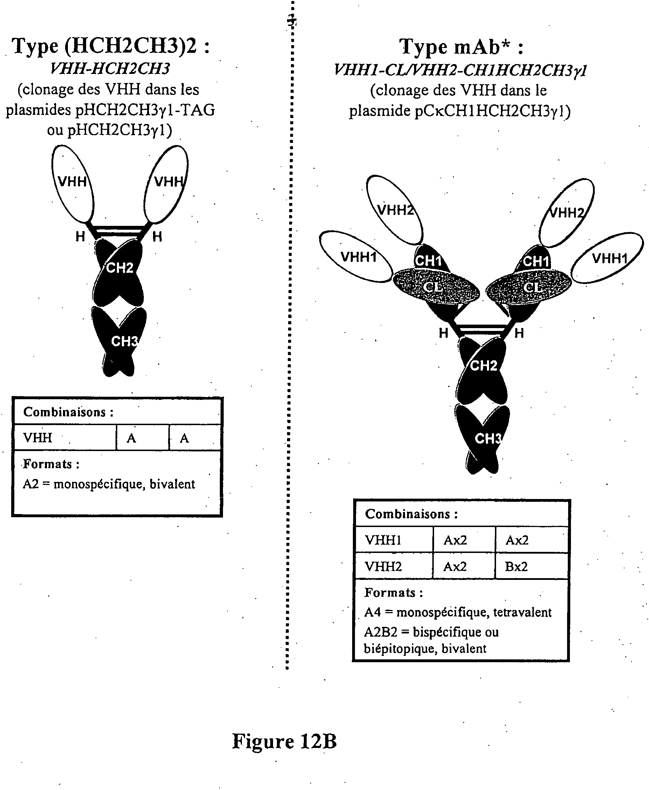

Le plasmide résultant est appelé plus communément : pMAbγ1*, il permet la production de fragments d'anticorps de type mAb* (Figure 12B).

### p55HCH2CH3γ1cmyc6HisGS (pHCH2CH3γ1-TAG)

### (Figures 10B et 11)

Insertion de la région Hinge (H) et des régions constantes CH2 et CH3 d'une immunoglobuline de type IgG1 entre les sites BstEII et NotI de p55Flag/RBS/35cmyc6HisGS.

La transcription inverse est réalisée exactement comme décrite pour la Cκ, mais en utilisant l'oligonucléotide 3' NotI/H-CH2-CH3.
Séquences SEQ ID N°42 et 43 des oligonucléotides utilisés :
5' BstE2/H-CH2-CH3
   SEQ ID N°42 :
   CCGGCCATGGCCCAGGTCACCGTCTCCTCAGACAAAACTCACACATGCCC
3' NotI/H-CH2-CH3
   SEQ ID N°43:
   AAGCTTAATCTAGAGCGGCCGCTTTACCCGGAGACAGGGAG

La PCR après RT est réalisée avec 1 µl d'ADNc, 10 pmoles de chaque oligonucléotides 5' BstE2/H-CH2-CH3 et 3' NotI/H-CH2-CH3, 0,5 U de Dynazyme (94°C, 3 min ; 94°C, 1 min ; 60°C, 1 min ; 72°C, 1,5 min ; 30 cycles puis 72°C, 10 min) dans un volume final de 50 µl.

Le plasmide résultant est appelé plus communément : pHCH2CH3γ1-TAG, il permet la production de fragments d'anticorps de type (HCH2CH3)₂ avec une étiquette à l'extrémité de CH3 (Figure 12B).

### p55HCH2CH3γ1 (pHCH2CH3γ1)

### (Figures 10B et 11)

Insertion de la région Hinge (H) et des régions constantes CH2 et CH3 d'une immunoglobuline de type IgG1 entre les sites BstEII et HindIII de p55Flag/RBS/35cmyc6HisGS.

La transcription inverse est réalisée exactement comme décrite pour la Cκ, mais en utilisant l'oligonucléotide 3' HindIII/H-CH2-CH3.
Séquences SEQ ID N°44 et 45 des oligonucléotides utilisés :
5' BstE2/H-CH2-CH3
   SEQ ID N°44 :
   CCGGCCATGGCCCAGGTCACCGTCTCCTCAGACAAAACTCACACATGCCC
3' HindIII/H-CH2-CH3
   SEQ ID N°45 : CCGCCAAAACAGCCAAGCTTATTTACCCGGAGACAGGGAG

La PCR après RT est réalisée avec 1 µl d'ADNc, 10 pmoles de chaque oligonucléotides 5' BstE2/H-CH2-CH3 et 3' HindIII/H-CH2-CH3, 0,5 U de Dynazyme (94°C, 3 min ; 94°C, 1 min ; 60°C, 1 min ; 72°C, 1,5 min ; 30 cycles puis 72°C, 10 min) dans un volume final de 50 µl.

Le plasmide résultant est appelé plus communément : pHCH2CH3γ1, il permet la production de fragments d'anticorps de type (HCH2CH3)₂ (Figure 12B).

### Clonage des VHH

Dans les différents formats n'importe quel VHH peut être introduit entre les sites uniques.
En amont de la Ck : entre EcoRI et BstEII (ou KpnI)
En amont de CH 1 : entre SfiI et Nhe1
En amont de H : EcoRI et BstEII

A cet effet, on amplifie par PCR les différents VHH avec les couples d'oligonucléotides 5' et 3' décrits ci -dessous :
Séquences SEQ ID N°46 à 52 des oligonucléotides utilisés :
   5' EcoR1-Pe1B55-Pe1BPHen
      SEQ ID N°46:
   5'VH1-Sfi
      SEQ ID N°47:
   5'VH2-Sfi
      SEQ ID N°48:
   5'VH3-Sfi
      SEQ ID N°49:
   5'VH4-Sfi
      SEQ ID N°50:
   3' BstEII/KpnI
      SEQ ID N°51:
      GGTGCAGCCACGGTACCTGAGGAGACGGTGACCTG
   3' BstE2/NheI
      SEQ ID N°52:
      GGGCCCTTGGTGCTAGCTGAGGAGACGGTGACCTG

### Production, purification et caractérisation des anticorps bio-spécifiques

La production et la purification des différents fragments d'anticorps possédant l'étiquette 6HisGS sont réalisées comme décrit en précédemment. Pour la purification des fragments d'anticorps sans étiquette, l'étape de chromatographie sur talon est remplacée par une colonne échangeuse d'ions dont les caractéristiques (anions ou cations) dépendent des caractéristiques du fragment d'anticorps.

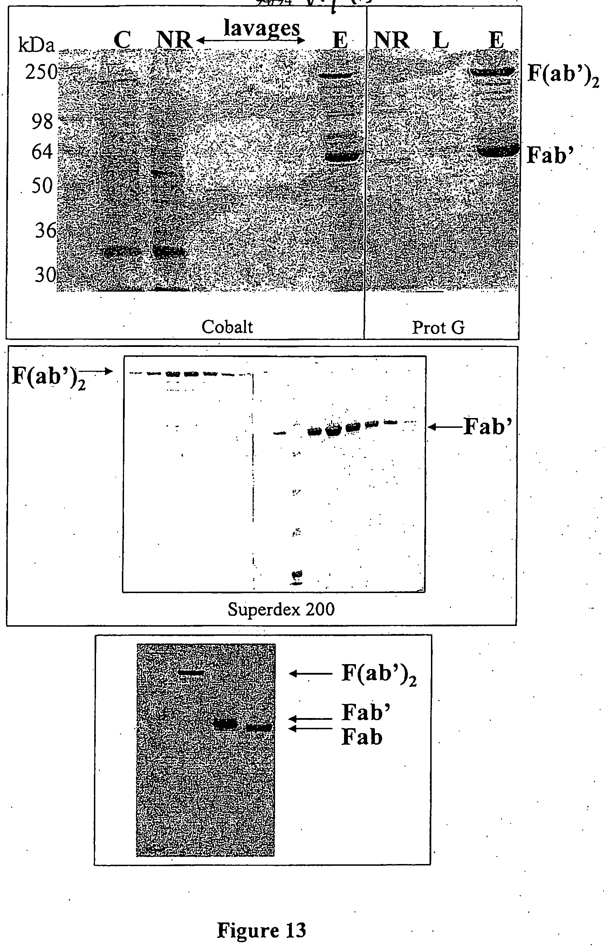

Des gels d'électrophorèse sont montrés dans la Figure 13. Les Fab' et F(ab')₂ sont purifiés sur collone de cobalt puis sur protéine G. Les différents fragments d'anticorps sont ensuite séparés sur supedex 200 (ou éventuellemnt superdex 75).

### Méthode pour isoler les VH humains et construction des vecteurs.

Le principe de la méthode consiste à cloner des domaines VH humains (isolés par RT-PCR à partir de la poche du LFB) dans le plasmide p55PhoA6HisGS⁻/NAB⁻ (Figures 10A et 11). Ce plasmide possède le gène codant pour la phosphatase alcaline dans une phase de lecture ne permettant pas son expression. Le clonage des VH restaure la phase de lecture de la phosphatase alcaline et permet la production des VH fusionnés en amont de la phosphatase alcaline (banque VH-PhoA clonée dans des bactéries TG1). Les différents clones sont ensuite produits en microplaques 96 puits et la cinétique de croissance des différents clones est mesuré toutes les 30 min (DO 620 nm) directement à partir des microplaques. On sélectionne ainsi les clones dont la croissance n'est pas altérée par la présence des VH. Les clones des microplaques sont répliqués et conservés à -80°C. Après 2 heures d'induction à 37°C, ou 16 heures d'induction à 30°C, 24°C, voire 18°C, la croissance est arrêtée et l'activité phosphatase est directement mesurée à partir des surnageants des milieux de culture. L'activité phosphatase est ensuite directement corrélée au nombre de bactéries présentes dans chaque puits de microplaques.

La phosphatase alcaline n'est active que si elle est sécrétée dans le périplasme bactérien, sous forme de dimère avec ses ponts disulfures correctement formés. Cette approche permet donc de sélectionner les clones produisant la plus grande quantité de protéine fusion VH-PhoA sécrétée dans le milieu de culture bactérien. Il est ainsi possible de sélectionner les VH qui sont correctement repliés et dont le pont disulfure est correctement formé, donc solubles. Les VH sélectionnés serviront de matrice pour échanger les CDR des VH humains par les CDR des VHH de lamas précédemment décrits. Le choix du VH sera réalisé en choisissant le VH dont les acides aminés des jonctions des CDR sont équivalents à ceux des VHH.

### Conditions de RT-PCR et PCR :

### Hybridation:

Un µl d'ARN total (purification décrite dans le paragraphe 1.3.2) est préincubé avec 1 pmole d'oligonucléotides (mélange de: 3' JH1-4-5; 3' JH2; 3' JH3 et 3' JH6) pendant 10 min à 70°C dans un volume final de 8 µl. La température est diminuée lentement (45 min) jusqu'à 37°C.

### Transcription inverse :

Aux 8 µl, on ajoute 0,5 µl de RNAsine (20 U), 3 µl de tampon 5X (SuperScriptII, Invitrogen), 1 µl DTT, 100 mM, 2 µl dNTP 10 mM et on incube 10 min à 50°C. On ajoute ensuite 0,75 µl de SuperScript (150 U) et l'incubation est poursuivie 30 min à 50°C et 15 min à 70°C.

L'ADNc obtenu est purifié sur billes (Biomag Carboxyl Terminated, Polysciences) selon les recommandations du fournisseur. L'élution finale est réalisée avec 15 µl de Tris-acétate 10 mM pH 7,8.

La PCR1 est réalisée avec 1 µl d'ADNc (obtenu par RT-PCR), 10 pmoles d'oligonucléotides 5' (0,625 pmole de chaque oligonucléotides 5' dont les séquences sont indiquées ci-dessous) et 3' (2,5 pmoles de chaque oligonucléotides 3' dont les séquences sont indiquées ci-dessous), 0,5 U de Dynazyme (95°C, 3 min; puis 95°C, 1 min; 58°C, 1 min; 72°C, 1 min; pendant 35 cycles puis 72°C, 10 min). Les produits de PCR sont déposés sur un gel d'agarose 2% et les bandes correspondant aux VH sont purifiées (gel extraction Kit Qiagen).

La PCR2 est faite à partir de 1 µl de PCR1 diluée au 1/100éme, la même quantité d'oligonucléotides décrits précédemment, 0,5 U Deep-Vent pour un volume final de 50 µl. (94°C, 3 min; puis 94°C, 1 min; 70°C, 1 min; 72°C, 1,5 min; pendant 40 cycles puis 72°C, 10 min). Les fragments sont purifiés à partir de gel d'agarose 2% comme précédemment décrit.
Séquences SEQ ID N°53 à SEQ ID N°72des oligonucléotides utilisés :
5' VH1a
   SEQ ID N°53 :
   CG GCC CAG CCG GCC ATG GCC CAG GTG CAG CTG GTG CAG TCT GG
5' VH1b
   SEQ ID N°54 :
   CG GCC CAG CCG GCC ATG GCC CAG GT(CT) CAG CT(GT) GTG CAG TCT GG
5' VH1c
   SEQ ID N°55 :
   CG GCC CAG CCG GCC ATG GCC (CG)AG GTC CAG CTG GTA CAG TCT GG
5' VH1d
   SEQ ID N°56 :
   CG GCC CAG CCG GCC ATG GCC CA(GA) ATG CAG CTG GTG CAG TCT GG
5' VH2a
   SEQ ID N°57 :
   CG GCC CAG CCG GCC ATG GCC CAG GTC ACC TTG AAG GAG TCT GG
5' VH2b
   SEQ ID N°58 :
   CG GCC CAG CCG GCC ATG GCC CAG ATC ACC TTG AAG GAG TCT GG
5' VH3a
   SEQ ID N°59 :
   CG GCC CAG CCG GCC ATG GCC GAG GTG CAG CTG GTG GAG TCT GG
5' VH3b
   SEQ ID N°60 :
   CG GCC CAG CCG GCC ATG GCC GAA GTG CAG CTG GTG GAG TCT GG
5' VH3c
   SEQ ID N°61 :
   CG GCC CAG CCG GCC ATG GCC CAG GTG CAG CTG GTG GAG TCT GG
5' VH3d SEQ ID N°62:
5' VH4a
   SEQ ID N°63:
   CG GCC CAG CCG GCC ATG GCC CAG GTG CAG CTG CAG GAG TCG GG
5' VH4b
   SEQ ID N°64:
   CG GCC CAG CCG GCC ATG GCC CAG CTG CAG CTG CAG GAG TC(GC) GG
5' VH4c
   SEQ ID N°65:
   CG GCC CAG CCG GCC ATG GCC CAG GTG CAG CTA CAG CAG TGG GG
5' VH5a
   SEQ ID N°66:
   CG GCC CAG CCG GCC ATG GCC GA(GA) GTG CAG CTG GTG CAG TCT GG
5' VH6a
   SEQ ID N°67:
   CG GCC CAG CCG GCC ATG GCC CAG) GTA CAG CTG CAG CAG TCA GG
5' VH7a
   SEQ ID N°68:
   CG GCC CAG CCG GCC ATG GCC CAG GTG CAG CTG GTG CAA TCT GG
3' JH1-4-5 SEQ ID N°69:
   GTC TAG ACG TCC CCC CGG GGA GGA GAC GGT GAC CAG GG
3' JH2 SEQ ID N°70: GTC TAG ACG TCC CCC CGG GGA GGA GAC AGT GAC CAG GG
3' JH3
   SEQ ID N°71:
   GTC TAG ACG TCC CCC CGG GGA AGA GAC GGT GAC CAT TG
3' JH6
   SEQ ID N°72:
   GTC TAG ACG TCC CCC CGG GGA GGA GAC GGT GAC CGT GG

Les différents fragments de PCR purifiés sont digérés par 10 U de Nicol et XmaI et insérés dans le vecteur de clonage p55/PhoA6HisGS⁻/NAB⁻ par ligature. Le mélange de ligature est digéré par FseI avant transformation des bactéries. La transformation est réalisée par électroporation avec de bactéries TG1 électrocompétentes. Les clones possédant un domaine VH inséré restaure l'activité phosphatase (colonies bleues).

### Production en microplaque 96 ou 384 puits :

Témoins: contrôle négatif milieu (2YT/ ampicilline 100 µg/ml), contrôle négatif du vecteur p55/PhoA6HisGS⁻/NAB⁻, contrôle positif du vecteur p55PhoA6HisGS/NAB⁻.

Distribution de 150 ou 40 µl de milieu de cutlure (2YT/ ampicilline 100 µg/ml) par puits (Nunclon Surface, Nunc). On inocule chaque puits avec une colonie isolée bleue avec un cure-dent ou un piqueur de cellule (Qpix) puis on scelle la plaque avec une feuille adhésive stérile. On fait pousser 16 h soit à 37°C ou 30°C dans un incubateur de plaques IEMS Thermo sous agitation à 900 rpm puis faire une réplique de la microplaque "mère" avec un réplicateur 96 ou 384 puits dans une microplaque contenant 150 ou 40 µl de 2YT/ ampicilline 100 µg/ml puis sceller avec une feuille adhésive stérile (Après la réplique, on ajoute dans la microplaque mère 37,5 ou 10 µl de glycérol 80% par puits et conserver à - 80°C). Après 3 h de culture (environ DO 620nm 0,5) on induit 16 h avec 100 µM d'ITPG final. La DO à 620 nm est mesurée en fin d'induction.

### Dosage de l'activité phosphatase alcaline :

Prendre 10 µl de la culture totale (cellules + milieu de culture) et 10 µl de surnageant de culture (pour cela centrifuger 3 min à 910 g). A chaque échantillon on ajoute 65 µl Tris-HC1 10mM pH 8,0 et on ajoute 25 µl de PNPP (paranitrophényl phosphate) à 1 mg/ml dans (diéthanolamine pH 9,8 (HCl); MgCl2 0,5 mM). Après 30 min de réaction sous agitation, on mesure la DO à 405nm.

L'activité phosphatase alcaline mesurée à 405 nm est ramenée au nombre de cellules (mesure DO 620 nm) contenues dans chaque puits en fin d'induction. Chaque activité phosphatase, des clones exprimant un VH fusionné à la phosphatase alcaline, est comparée à celle du contrôle positif (Phosphatase alcaline non fusionnée produite par p55PhoA6HisGS/NAB⁻). Seuls les clones présentant une activité égale ou supérieure au contrôle sont pris en considération et séquencés avec les oligonucléotides 5' EcoRI-90 et 3' inf-PstI+71 (séquence de l'oligonucléotide 3' inf-PstI+71: GTTAAACGGCGAGCACCG).

### REFERENCES BIBLIOGRAPHIQUES

1- Hamers-Casterman C, Atarhouch T, Muyldermans S, Robinson G, Hamers C, Songa EB, Bendahman N, Hamers R. Naturally occurring antibodies devoid of light chains. Nature 1993; 363 : 446-448.
2- Teillaud C, Galon J, Zilber MT, Mazieres N, Spagnoli R, Kurrle R, Fridman WH, Sautes C. Soluble CD16 binds peripheral blood mononuclear cells and inhibits pokeweed-mitogen-induced responses. Blood, 1993, 82 : 3081-3090).
3- Terskikh, A, Mach, JP, and Pèlegrin A. Marked increase in the secretion of a fully antigenic recombinant CEA obtained by deletion of its hydrophobic tail. Mol Immunol, 1993, 30:921-927.
4- Chomczynski P, Sacchi N. Single-step method of RNA isolation by acid guanidinium thiocyanate-phenol-chloroform extraction. Anal Biochem, 1987, 162 : 156-159.
5- Arbabi Ghahroudi M, Desmyter A, Wyns L, Hamers R, Muyldermans S. Selection and identification of single domain antibody fragments from camel heavy-chain antibodies. FEBS Lett, 1997, 414 : 521-526.
6- Vivier E, Rochet N, Ackerly M, Petrini J, Levine H, Daley J, Anderson P. Signaling function of reconstituted CD16: zeta: gamma receptor complex isoforms. Int Immunol, 1992, 4 : 1313-1323.
7- Vély F, Gruel N, Moncuit J, Cochet O, Rouard H, Daré S, Galon J, Sautès C, Fridman WH, Teillaud J-L. A new set of monoclonal antibodies against human FcgammaRII (CD32) and Fcgamma RIII (CD16): characterization and use in various assays. Hybridoma, 1997, 16 : 519-528.
8- Le Calvez H, Fieschi J, Green JM, Marchesi N, Chauvreau J, Baty D. Paratope characterisation by structural modelling of two anti-cortisol single-chain variable fragments produced in E. coli. Mol. Immunol. 1995, 32 : 185-198.
9- Le Calvez H, Green JM, Baty D. Increased efficiency of alkaline phosphatase production levels in Escherichia coli using a degenerate PelB signal sequence. Gene, 1996, 170 : 51-55.

## Revendications

1. Domaine d'anticorps VHH de camélidé anti-CD16 ayant une séquence en acides aminés choisie dans le groupe comprenant les séquences SEQ ID N°74, SEQ ID N°75, SEQ ID N°76 et SEQ ID N°104 ou codé par une séquence nucléotidique choisie dans le groupe comprenant les séquences SEQ ID N°82, SEQ ID N°83, SEQ ID N°84, et SEQ ID N°107.

2. Format d'anticorps comprenant un domaine selon la revendication 1, fusionné à une région constante d'anticorps humains.

3. Format d'anticorps selon la revendication 2, **caractérisé en ce qu'**il s'agit d'un format d'anticorps chimérisé ou humanisé, multispécifique et/ou multivalent.

4. Format d'anticorps selon la revendication 3, **caractérisé en ce qu'**il est de type Fab et **caractérisé par** l'association de deux domaines VHH identiques ou différents, l'un des domaines étant fusionné à la région constante Cκ ou Cλ d'une immunoglobuline humaine, l'autre à la région constante CH1 d'une immunoglobuline humaine.

5. Format d'anticorps selon la revendication 3, **caractérisé en ce qu'**il est de type Fab' et **caractérisé par** l'association de deux domaines VHH identiques ou différents, l'un des domaines étant fusionné à la région constante Cκ ou Cλ d'une immunoglobuline humaine, l'autre à la région constante CH1 d'une immunoglobuline humaine suivie d'une région charnière H d'une immunoglobuline humaine.

6. Format d'anticorps selon la revendication 4 ou 5, **caractérisé en ce qu'**il est bispécifique/monovalent.

7. Format d'anticorps selon la revendication 3, **caractérisé en ce qu'**il est de type F(ab')₂ et qu'il est obtenu par l'association de deux formats de type Fab' identiques ou différents tels que définis dans la revendication 5.

8. Format d'anticorps selon la revendication 7, **caractérisé en ce qu'**il est bispécifique/bivalent.

## Patentansprüche

1. Antikörper-Domäne VHH des Camelide-anti-CD 16-Antikörpers, der eine Aminosäuresequenz aufweist, die ausgewählt ist aus der Gruppe, umfassend die Sequenzen SEQ-ID-Nr. 74, SEQ-ID-Nr. 75, SEQ-ID-Nr. 76 und SEQ-ID-Nr. 104 oder für eine Nukleotidsequenz kodiert, ausgewählt aus der Gruppe, umfassend die Sequenzen SEQ-ID-Nr. 82, SEQ-ID-Nr. 83, SEQ-ID-Nr. 84 und SEQ-ID-Nr. 107.

2. Antikörperformat, umfassend eine Domäne nach Anspruch 1, die an eine konstante Region von humanen Antikörpern fusioniert ist.

3. Antikörperformat nach Anspruch 2, **dadurch gekennzeichnet, dass** es sich um ein chimäres oder humanisiertes, multispezifisches und/oder multivalentes Antikörperformat handelt.

4. Antikörperformat nach Anspruch 3, **dadurch gekennzeichnet, dass** es vom Typ Fab ist und **gekennzeichnet ist durch** die Assoziation von zwei identischen oder unterschiedlichen VHH-Domänen, wobei eine der Domänen an die konstante Region Cκ oder Cλ eines humanen Immunglobulins fusioniert ist und wobei die andere Domäne an die konstante Region CH₁ eines humanen Immunglobulins fusioniert ist.

5. Antikörperformat nach Anspruch 3, **dadurch gekennzeichnet, dass** es vom Typ Fab'ist und durch die Assoziation von zwei identischen oder unterschiedlichen VHH-Domänen gekennzeichnet ist, wobei eine der Domänen an die konstante Region Cκ oder Cλ eines humanen Antikörpers fusioniert ist und wobei die andere Domäne an die konstante Region CH1 eines humanen Immunglobulins, gefolgt von einer Scharnierregion H eines humanen Antikörpers, fusioniert ist.

6. Antikörperformat nach Anspruch 4 oder 5, **dadurch gekennzeichnet, dass** es sich um ein bispezifisches/monovalentes Format handelt.

7. Antikörperformat nach Anspruch 3, **dadurch gekennzeichnet, dass** es vom F(ab')₂-Typ ist und erhalten wird aus der Assoziation von zwei identischen oder unterschiedlichen Formaten des Typs Fab', wie gemäß Anspruch 5 definiert.

8. Antikörperformat nach Anspruch 7, **dadurch gekennzeichnet, dass** es bispezifisches/bivalentes Format ist.

## Claims

1. Anti-CD16 camelid VHH antibody domain having a sequence of amino acids chosen from the group comprising the sequences SEQ ID N°74, SEQ ID N°75, SEQ ID N°76 and SEQ ID N°104 or coded by a nucleotide sequence chosen from the group comprising the sequences SEQ ID N°82, SEQ ID N°83, SEQ ID N°84 and SEQ ID N°107.

2. Antibody format comprising a domain according to claim 1, fused to a constant region of human antibodies.

3. Antibody format according to claim 2, **characterised in that** said antibody format is chimerised or humanised, multispecific and/or multivalent.

4. Antibody format according to claim 3, **characterised in that** said antibody format is of the Fab type and **characterised by** the association of two identical or different VHH domains, one of the domains being fused to the constant region Cκ or Cλ of a human immunoglobulin, the other to the constant region CH1 of a human immunoglobulin.

5. Antibody format according to claim 3, **characterised in that** said antibody format is of the Fab' type and **characterised by** the association of two identical or different VHH domains, one of the domains being fused to the constant region Cκ or Cλ of a human immunoglobulin, the other to the constant region CH1 of a human immunoglobulin followed by a hinge region H of a human immunoglobulin.

6. Antibody format according to claim 4 or 5, **characterised in that** said antibody format is bispecific/monovalent.

7. Antibody format according to claim 3, **characterised in that** said antibody format is of the F(ab')₂ type and is obtained by the association of two identical or different formats of the Fab' type as defined in claim 5.

8. Antibody format according to claim 7, **characterised in that** said antibody format is bispecific/bivalent.


### SEQUENCE LISTING

<110> CENTRE NATIONAL DE LA RECHERCHE SCIENTIFIQUE (CNRS) INSTITUT NATIONAL DE LA SANTE ET DE LA RECHERCHE MEDICALE (INSERM)
<120> PRODUCTION DE FORMATS D'ANTICORPS ET APPLICATIONS IMMUNOLOGIQUES DE CES
   FORMATS
<130> CP/BB 61296-2051
<140> FR 04 13 433
   <141> 2004-12-16
<160> 121
<170> PatentIn version 3.1
<210> 1
   <211> 21
   <212> DNA
   <213> Artificial sequence
<220>
   <223> oligonucleotide
<400> 1
   cgccatcaag gtaccagttg a 21
<210> 2
   <211> 23
   <212> DNA
   <213> Artificial sequence
<220>
   <223> oligonucleotide
<400> 2
   ggtacgtgct gttgaactgt tcc 23
<210> 3
   <211> 26
   <212> DNA
   <213> Artificial sequence
<220>
   <223> oligonucleotide
<400> 3
   ggagctgggg tcttcgctgt ggtgcg 26
<210> 4
   <211> 57
   <212> DNA
   <213> Artificial sequence
<220>
   <223> oligonucleotide
<400> 4
   catgccatga ctcgcggccc agccggccat ggcccaggtg cagctggtgc agtctgg 57
<210> 5
   <211> 26
   <212> DNA
   <213> Artificial sequence
<220>
   <223> oligonucleotide
<400> 5
   tggttgtggt tttggtgtct tgggtt 26
<210> 6
   <211> 57
   <212> DNA
   <213> Artificial sequence
<220>
   <223> oligonucleotide
<400> 6
   catgccatga ctcgcggccc agccggccat ggcccaggtc accttgaagg agtctgg 57
<210> 7
   <211> 57
   <212> DNA
   <213> Artificial sequence
<220>
   <223> oligonucleotide
<400> 7
   catgccatga ctcgcggccc agccggccat ggccgaggtg cagctggtgg agtctgg 57
<210> 8
   <211> 57
   <212> DNA
   <213> Artificial sequence
<220>
   <223> oligonucleotide
<400> 8
   catgccatga ctcgcggccc agccggccat ggcccaggtg cagctgcagg agtcggg 57
<210> 9
   <211> 41
   <212> DNA
   <213> Artificial sequence
<220>
   <223> oligonucleotide
<400> 9
   cacgattctg cggccgctga ggagacaggt gacctgggtc c 41
<210> 10
   <211> 44
   <212> DNA
   <213> Artificial sequence
<220>
   <223> oligonucleotide
<400> 10
   ctttactatt ctcacggcca tggcggccga ggtgcagctg gtgg 44
<210> 11
   <211> 72
   <212> DNA
   <213> Artificial sequence
<220>
   <223> oligonucleotide
<400> 11
   ccgcgcgcgc caagacccaa gcttgggcta gatggatgga tggatggatg gatgtgcggc 60
cccattcaga tc 72
<210> 12
   <211> 25
   <212> DNA
   <213> Artificial sequence
<220>
   <223> Plasmide
<400> 12
   gcgccgacat cataacggtt ctggc 25
<210> 13
   <211> 17
   <212> DNA
   <213> Artificial sequence
<220>
   <223> Plasmide
<400> 13
   cgctactgcc gccaggc 17
<210> 14
   <211> 19
   <212> DNA
   <213> Artificial sequence
<220>
   <223> oligonucleotide
<400> 14
   ggcacatgtg acctcgcgc 19
<210> 15
   <211> 22
   <212> DNA
   <213> Artificial sequence
<220>
   <223> oligonucleotide
<400> 15
   gcaacgtacc acggcaatat cg 22
<210> 16
   <211> 22
   <212> DNA
   <213> Artificial sequence
<220>
   <223> oligonucleotide
<400> 16
   cgatattgcc gtggtacgtt gc 22
<210> 17
   <211> 22
   <212> DNA
   <213> Artificial sequence
<220>
   <223> oligonucleotide
<400> 17
   gccatctttg gtatttagcg cc 22
<210> 18
   <211> 23
   <212> DNA
   <213> Artificial sequence
<220>
   <223> Oligonucleotide
<400> 18
   ccatggcggc cgatcctcga gag 23
<210> 19
   <211> 67
   <212> DNA
   <213> Artificial sequence
<220>
   <223> oligonucleotide
<400> 19
   catgcagtcc caagcttatt agctcccgtg atggtgatga tgatgtttca gccccagagc 60
ggctttc 67
<210> 20
   <211> 20
   <212> DNA
   <213> Artificial sequence
<220>
   <223> oligonucleotide
<400> 20
   catgagctgt cttcggtatc 20
<210> 21
   <211> 38
   <212> DNA
   <213> Artificial sequence
<220>
   <223> oligonucleotide
<400> 21
   taatggtccc gctaacagcg cgatttgctg atgaccca 38
<210> 22
   <211> 38
   <212> DNA
   <213> Artificial sequence
<220>
   <223> oligonucleotide
<400> 22
   tgggtcatca gcaaatcgcg ctgttagcgg gaccatta 38
<210> 23
   <211> 19
   <212> DNA
   <213> Artificial sequence
<220>
   <223> oligonucleotide
<400> 23
   gaacgaagcg gcgtcgaag 19
<210> 24
   <211> 84
   <212> DNA
   <213> Artificial sequence
<220>
   <223> oligonucleotide
<400> 24
   catggcccag gtcaccgtct cctcaaaccg cggactcgag gcggcccagc cggccatggc 60
   cgctagcgcg gccgctctag atta 84
<210> 25
   <211> 84
   <212> DNA
   <213> Artificial sequence
<220>
   <223> oligonucleotide
<400> 25
   agcttaatct agagcggccg cgctagcggc catggccggc tgggccgcct cgagtccgcg 60
   gtttgaggag acggtgacct gggc 84
<210> 26
   <211> 119
   <212> DNA
   <213> Artificial sequence
<220>
   <223> oligonucleotide
<400> 26
   ggagagtgtg caggtgatta caaagacgat gacgataagt aataaacagg aaacagaagt 60
   ccatatgaaa tacctattgc ctacggcagc cgctggattg ttattactcg cggcccagc 119
<210> 27
   <211> 118
   <212> DNA
   <213> Artificial sequence
<220>
   <223> oligonucleotide
<400> 27
   gggccgcgag taataacaat ccagcggctg ccgtaggcaa taggtatttc atatggactt 60
   ctgtttcctg tttattactt atcgtcatcg tctttgtaat cacctgcaca ctctccgc 118
<210> 28
   <211> 75
   <212> DNA
   <213> Artificial sequence
<220>
   <223> oligonucleotide
<400> 28
   ggccgcagaa caaaaactca tctcagaaga ggatctgaat ggggccgtac atcaccacca 60
   tcaccatggg agcta 75
<210> 29
   <211> 75
   <212> DNA
   <213> Artificial sequence
<220>
   <223> oligonucleotide
<400> 29
   agcttagctc ccatggtgat ggtggtgatg tacggcccca ttcagatcct cttctgagat 60
   gagtttttgt tctgc 75
<210> 30
   <211> 59
   <212> DNA
   <213> Artificial sequence
<220>
   <223> oligonucleotide
<400> 30
   ggggccaggg gacccaggtc accgtctcct caggtaccgt ggctgcacca tctgtcttc 59
<210> 31
   <211> 57
   <212> DNA
   <213> Artificial sequence
<220>
   <223> oligonucleotide
<400> 31
   cgtcatcgtc tttgtaatca cctgcacact ctccgcggtt gaagctcttt gtcaccg 57
<210> 32
   <211> 49
   <212> DNA
   <213> Artificial sequence
<220>
   <223> oligonucleotide
<400> 32
   ctcgaggcgg cccagccggc catggccgct agcaccaagg gcccatcgg 49
<210> 33
   <211> 44
   <212> DNA
   <213> Artificial sequence
<220>
   <223> oligonucleotide
<400> 33
   aagcttaatc tagagcggcc gcacaagatt tgggctcaac tttc 44
<210> 34
   <211> 29
   <212> DNA
   <213> Artificial sequence
<220>
   <223> oligonucleotide
<400> 34
   ccctcagcag cgtagtgacc gtgccctcc 29
<210> 35
   <211> 29
   <212> DNA
   <213> Artificial sequence
<220>
   <223> oligonucleotide
<400> 35
   ggagggcacg gtcactacgc tgctgaggg 29
<210> 36
   <211> 74
   <212> DNA
   <213> Artificial sequence
<220>
   <223> oligonucleotide
<400> 36
   aagcttaatc tagagcggcc gctgggcacg gtgggcatgt gtgagttttg tcacaagatt 60
   tgggctcaac tttc 74
<210> 37
   <211> 89
   <212> DNA
   <213> Artificial sequence
<220>
   <223> oligonucleotide
<400> 37
   ggagagtgtt aataaacagg aaacagaagt ccatatgaaa tacctattgc ctacggcagc 60
   cgctggattg ttattactcg cggcccagc 89
<210> 38
   <211> 88
   <212> DNA
   <213> Artificial sequence
<220>
   <223> oligonucleotide
<400> 38
   gggccgcgag taataacaat ccagcggctg ccgtaggcaa taggtatttc atatggactt 60
   ctgtttcctg tttattaaca ctctccgc 88
<210> 39
   <211> 40
   <212> DNA
   <213> Artificial sequence
<220>
   <223> Oligonucleotide
<400> 39
   catgcagtcc caagcttaac aagatttggg ctcaactttc 40
<210> 40
   <211> 39
   <212> DNA
   <213> Artificial sequence
<220>
   <223> oligonucleotide
<400> 40
   catgcagtcc caagcttatg ggcacggtgg gcatgtgtg 39
<210> 41
   <211> 40
   <212> DNA
   <213> Artificial sequence
<220>
   <223> oligonucleotide
<400> 41
   ccgccaaaac agccaagctt atttacccgg agacagggag 40
<210> 42
   <211> 50
   <212> DNA
   <213> Artificial sequence
<220>
   <223> Oligonucleotide
<400> 42
   ccggccatgg cccaggtcac cgtctcctca gacaaaactc acacatgccc 50
<210> 43
   <211> 41
   <212> DNA
   <213> Artificial sequence
<220>
   <223> Oligonucleotide
<400> 43
   aagcttaatc tagagcggcc gctttacccg gagacaggga g 41
<210> 44
   <211> 50
   <212> DNA
   <213> Artificial sequence
<220>
   <223> oligonucleotide
<400> 44
   ccggccatgg cccaggtcac cgtctcctca gacaaaactc acacatgccc 50
<210> 45
   <211> 40
   <212> DNA
   <213> Artificial sequence
<220>
   <223> oligonucleotide
<400> 45
   ccgccaaaac agccaagctt atttacccgg agacagggag 40
<210> 46
   <211> 95
   <212> DNA
   <213> Artificial sequence
<220>
   <223> Oligonucleotide
<400> 46
   cgacaccgga attccatatg aaatacctat taccaacagc agcagctggg ttattattgc 60
   tcgctgcgca gccggccatg gccgaggtgc agctg 95
<210> 47
   <211> 57
   <212> DNA
   <213> Artificial sequence
<220>
   <223> oligonucleotide
<400> 47
   catgccatga ctcgcggccc agccggccat ggcccaggtg cagctggtgc agtctgg 57
<210> 48
   <211> 57
   <212> DNA
   <213> Artificial sequence
<220>
   <223> Oligonucleotide
<400> 48
   catgccatga ctcgcggccc agccggccat ggcccaggtc accttgaagg agtctgg 57
<210> 49
   <211> 57
   <212> DNA
   <213> Artificial sequence
<220>
   <223> oligonucleotide
<400> 49
   catgccatga ctcgcggccc agccggccat ggccgaggtg cagctggtgg agtctgg 57
<210> 50
   <211> 57
   <212> DNA
   <213> Artificial sequence
<220>
   <223> oligonucleotide
<400> 50
   catgccatga ctcgcggccc agccggccat ggcccaggtg cagctgcagg agtcggg 57
<210> 51
   <211> 35
   <212> DNA
   <213> Artificial sequence
<220>
   <223> oligonucleotide
<400> 51
   ggtgcagcca cggtacctga ggagacggtg acctg 35
<210> 52
   <211> 35
   <212> DNA
   <213> Artificial sequence
<220>
   <223> Oligonucleotide
<400> 52
   gggcccttgg tgctagctga ggagacggtg acctg 35
<210> 53
   <211> 43
   <212> DNA
   <213> Artificial sequence
<220>
   <223> oligonucleotide
<400> 53
   cggcccagcc ggccatggcc caggtgcagc tggtgcagtc tgg 43
<210> 54
   <211> 45
   <212> DNA
   <213> Artificial sequence
<220>
   <223> oligonucleotide
<400> 54
   cggcccagcc ggccatggcc caggtctcag ctgtgtgcag tctgg 45
<210> 55
   <211> 44
   <212> DNA
   <213> Artificial sequence
<220>
   <223> oligonucleotide
<400> 55
   cggcccagcc ggccatggcc cgaggtccag ctggtacagt ctgg 44
<210> 56
   <211> 44
   <212> DNA
   <213> Artificial sequence
<220>
   <223> oligonucleotide
<400> 56
   cggcccagcc ggccatggcc cagaatgcag ctggtgcagt ctgg 44
<210> 57
   <211> 43
   <212> DNA
   <213> Artificial sequence
<220>
   <223> oligonucleotide
<400> 57
   cggcccagcc ggccatggcc caggtcacct tgaaggagtc tgg 43
<210> 58
   <211> 43
   <212> DNA
   <213> Artificial sequence
<220>
   <223> oligonucleotide
<400> 58
   cggcccagcc ggccatggcc cagatcacct tgaaggagtc tgg 43
<210> 59
   <211> 43
   <212> DNA
   <213> Artificial sequence
<220>
   <223> oligonucleotide
<400> 59
   cggcccagcc ggccatggcc gaggtgcagc tggtggagtc tgg 43
<210> 60
   <211> 43
   <212> DNA
   <213> Artificial sequence
<220>
   <223> oligonucleotide
<400> 60
   cggcccagcc ggccatggcc gaagtgcagc tggtggagtc tgg 43
<210> 61
   <211> 43
   <212> DNA
   <213> Artificial sequence
<220>
   <223> oligonucleotide
<400> 61
   cggcccagcc ggccatggcc caggtgcagc tggtggagtc tgg 43
<210> 62
   <211> 46
   <212> DNA
   <213> Artificial sequence
<220>
   <223> oligonucleotide
<400> 62
   cggcccagcc ggccatggcc gaggtgcagc tggtggagat ctcgcg 46
<210> 63
   <211> 43
   <212> DNA
   <213> Artificial sequence
<220>
   <223> oligonucleotide
<400> 63
   cggcccagcc ggccatggcc caggtgcagc tgcaggagtc ggg 43
<210> 64
   <211> 44
   <212> DNA
   <213> Artificial sequence
<220>
   <223> oligonucleotide
<400> 64
<210> 65
   <211> 43
   <212> DNA
   <213> Artificial sequence
<220>
   <223> oligonucleotide
<400> 65
   cggcccagcc ggccatggcc caggtgcagc tacagcagtg ggg 43
<210> 66
   <211> 44
   <212> DNA
   <213> Artificial sequence
<220>
   <223> oligonucleotide
<400> 66
   cggcccagcc ggccatggcc gagagtgcag ctggtgcagt ctgg 44
<210> 67
   <211> 43
   <212> DNA
   <213> Artificial sequence
<220>
   <223> oligonucleotide
<400> 67
   cggcccagcc ggccatggcc caggtacagc tgcagcagtc agg 43
<210> 68
   <211> 43
   <212> DNA
   <213> Artificial sequence
<220>
   <223> oligonucleotide
<400> 68
   cggcccagcc ggccatggcc caggtgcagc tggtgcaatc tgg 43
<210> 69
   <211> 38
   <212> DNA
   <213> Artificial sequence
<220>
   <223> oligonucleotide
<400> 69
   gtctagacgt ccccccgggg aggagacggt gaccaggg 38
<210> 70
   <211> 38
   <212> DNA
   <213> Artificial sequence
<220>
   <223> oligonucleotide
<400> 70
   gtctagacgt ccccccgggg aggagacagt gaccaggg 38
<210> 71
   <211> 38
   <212> DNA
   <213> Artificial sequence
<220>
   <223> oligonucleotide
<400> 71
   gtctagacgt ccccccgggg aagagacggt gaccattg 38
<210> 72
   <211> 38
   <212> DNA
   <213> Artificial sequence
<220>
   <223> oligonucleotide
<400> 72
   gtctagacgt ccccccgggg aggagacggt gaccgtgg 38
<210> 73
   <211> 126
   <212> PRT
   <213> Artificial sequence
<220>
   <223> Amino acid
<220>
   <221> MISC_FEATURE
   <222> (35)..(37)
   <223> Unknown amino acid
<220>
   <221> MISC_FEATURE
   <222> (55)..(57)
   <223> unknown amino acid
<220>
   <221> MISC_FEATURE
   <222> (109)..(113)
   <223> Unknown amino acid
<400> 73
   Glu Val Gln Leu Val Gln Ser Gly Gly Gly Leu Val Gln Pro Gly Gly
   1               5                   10                  15
   Ser Leu Arg Leu Ser Cys Ser Phe Pro Gly Ser Ile Phe Ser Leu Thr
               20                  25                  30
   Met Gly Xaa Xaa Xaa Trp Tyr Arg Gln Ala Pro Gly Lys Glu Arg Glu
           35                  40                  45
   Leu Val Thr Ser Ala Thr Xaa Xaa Xaa Pro Gly Gly Asp Thr Asn Tyr
       50                  55                  60
   Ala Asp Phe Val Lys Gly Arg Phe Thr Ile Ser Arg Asp Asn Ala Arg
   65                  70                  75                  80
   Ser Ile Ile Tyr Leu Gln Met Asn Ser Leu Lys Pro Glu Asp Thr Ala
                   85                  90                  95
   Val Tyr Tyr Cys Tyr Ala Arg Thr Arg Asn Trp Gly Xaa Xaa Xaa Xaa
               100                 105                 110
   Xaa Thr Val Trp Gly Gln Gly Thr Gln Val Thr Val Ser Ser
                   115                 120                 125
<210> 74
   <211> 126
   <212> PRT
   <213> Artificial sequence
<220>
   <223> Amino acid
<220>
   <221> MISC_FEATURE
   <222> (36)..(37)
   <223> unknown amino acid
<220>
   <221> MISC_FEATURE
   <222> (56)..(57)
   <223> Unknown amino acid
<400> 74
   Glu Val Gln Leu Val Gln Ser Gly Gly Glu Leu Val Gln Ala Gly Gly
   1               5                   10                  15
   Ser Leu Arg Leu Ser Cys Ala Ala Ser Gly Leu Thr Phe Ser Ser Tyr
               20                  25                  30
   Asn Met Gly Xaa Xaa Trp Phe Arg Arg Ala Pro Gly Lys Glu Arg Glu
           35                  40                  45
   Phe Val Ala Ser Ile Thr Trp Xaa Xaa Ser Gly Arg Asp Thr Phe Tyr
       50                  55                  60
   Ala Asp Ser Val Lys Gly Arg Phe Thr Ile Ser Arg Asp Asn Ala Lys
   65                  70                  75                  80
   Asn Thr Val Tyr Leu Gln Met Ser Ser Leu Lys Pro Glu Asp Thr Ala
                   85                  90                  95
   Val Tyr Tyr Cys Ala Ala Asn Pro Trp Pro Val Ala Ala Pro Arg Ser
               100                 105                 110
   Gly Thr Tyr Trp Gly Gln Gly Thr Gln Val Thr Val Ser Ser
                       115                 120                 125
<210> 75
   <211> 126
   <212> PRT
   <213> Artificiel sequence
<220>
   <223> Amino acid
<220>
   <221> MISC_FEATURE
   <222> (35)..(37)
   <223> Unknown amino acid
<220>
   <221> MISC_FEATURE
   <222> (55)..(57)
   <223> Unknown amino acid
<220>
   <221> MISC_FEATURE
   <222> (113)..(113)
   <223> unknown amino acid
<400> 75
   Glu Val Gln Leu Val Glu Ser Gly Gly Gly Leu Val Gln Pro Gly Glu
   1 5 10 15
   Ser Leu Thr Leu Ser Cys Val Val Ala Gly Ser Ile Phe Ser Phe Ala
   20 25 30
   Met Ser Xaa Xaa Xaa Trp Tyr Arg Gln Ala Pro Gly Lys Glu Arg Glu
   35 40 45
   Leu Val Ala Arg Ile Gly Xaa Xaa Xaa Ser Asp Asp Arg Val Thr Tyr
   50 55 60
   Ala Asp Ser Val Lys Gly Arg Phe Thr Ile Ser Arg Asp Asn Ile Lys
   65 70 75 80
   Arg Thr Ala Gly Leu Gln Met Asn Ser Leu Lys Pro Glu Asp Thr Ala
   85 90 95
   Val Tyr Tyr Cys Asn Ala Gln Thr Asp Leu Arg Asp Trp Thr Val Arg
   100 105 110
   Xaa Glu Tyr Trp Gly Gln Gly Thr Gln Val Thr Val Ser Ser
   115 120 125
<210> 76
   <211> 126
   <212> PRT
   <213> Artificial sequence
<220>
   <223> Amino acid
<220>
   <221> MISC_FEATURE
   <222> (35)..(37)
   <223> unknown amino acid
<220>
   <221> MISC_FEATURE
   <222> (55)..(57)
   <223> Unknown amino acid
<220>
   <221> MISC_FEATURE
   <222> (113)..(113)
   <223> unknown amino acid
<400> 76
   Glu Val Gln Leu Val Glu Ser Gly Gly Gly Leu Val Gln Pro Gly Gly
   1               5                   10                  15
   Ser Leu Thr Leu Ser Cys Val Ala Ala Gly Ser Ile Phe Thr Phe Ala
               20                  25                  30
   Met Ser Xaa Xaa Xaa Trp Tyr Arg Gln Ala Pro Arg Lys Glu Arg Glu
           35                  40                  45
   Leu Val Ala Arg Ile Gly Xaa Xaa Xaa Thr Asp Asp Glu Thr Met Tyr
       50                  55                  60
   Lys Asp Ser Val Lys Gly Arg Phe Thr Ile Ser Arg Asp Asn Val Lys
   65                  70                  75                  80
   Arg Thr Ala Gly Leu Gln Met Asn Asn Leu Lys Pro Glu Asp Thr Ala
                   85                  90                  95
   Val Tyr Tyr Cys Asn Ala Arg Thr Asp Tyr Arg Asp Trp Thr Val Arg
               100                 105                 110
   Xaa Glu Tyr Trp Gly Gln Gly Thr Gln Val Thr Val Ser Ser
           115                 120                 125
<210> 77
   <211> 126
   <212> PRT
   <213> Artificial sequence
<220>
   <223> Amino acid
<220>
   <221> MISC_FEATURE
   <222> (36)..(37)
   <223> unknown amino acid
<220>
   <221> MISC_FEATURE
   <222> (56)..(57)
   <223> unknown amino acid
<220>
   <221> MISC_FEATURE
   <222> (111)..(113)
   <223> Unknown amino acid
<400> 77
   Glu Val Gln Leu Val Glu Ser Gly Gly Gly Leu Val Gln Ala Gly Gly
   1               5                   10                  15
   Ser Leu Arg Leu Ser Cys Thr Ser Ser Thr Val Thr Phe Thr Pro Tyr
               20                  25                  30
   Gln Met Gly Xaa Xaa Trp Tyr Arg Gln Ala Pro Gly Lys Gln Arg Ala
           35                  40                  45
   Leu Val Ala Asp Ile Ser Thr Xaa Xaa Gly Gly Ser Arg Thr Asn Tyr
       50                  55                  60
   Ala Asp Phe Ala Lys Gly Arg Phe Thr Ile Ser Arg Asp Asp Val Lys
   65                  70                  75                  80
   Asn Thr Val Tyr Leu Gln Met Asn Asn Leu Lys Pro Glu Asp Thr Ala
                   85                  90                  95
   Val Tyr Tyr Cys Asn Thr Tyr Tyr Ala Met Ile Gly His Ala Xaa Xaa
               100                 105                 110
   Xaa Arg Asn Trp Gly Gln Gly Thr Gln Val Thr Val Ser Ser
           115                 120                 125
<210> 78
   <211> 126
   <212> PRT
   <213> Artificial sequence
<220>
   <223> Amino acid
<220>
   <221> MISC_FEATURE
   <222> (36)..(37)
   <223> Unknown amino acid
<220>
   <221> MISC_FEATURE
   <222> (57)..(57)
   <223> Unknown amino acid
<220>
   <221> MISC_FEATURE
   <222> (111)..(113)
   <223> unknown amino acid
<400> 78
   Glu Val Gln Leu Val Glu Ser Gly Gly Gly Phe Val Gln Ala Gly Glu
   1               5                   10                  15
   Ser Leu Thr Leu Ser Cys Thr Ser Ser Thr Leu Thr Phe Thr Pro Tyr
               20                  25                  30
   Arg Met Ala Xaa Xaa Trp Tyr Arg Gln Ala Pro Gly Lys Gln Arg Asp
           35                  40                  45
   Leu Val Ala Asp Ile Ser Ser Gly Xaa Asp Gly Arg Thr Thr Asn Tyr
       50                  55                  60
   Ala Asp Phe Ala Lys Gly Arg Phe Thr Ile Ser Arg Asp Asn Ile Lys
   65                  70                  75                  80
   Asn Thr Val Phe Leu Arg Met Thr Asn Leu Lys Pro Glu Asp Thr Ala
                   85                  90                  95
   Val Tyr Tyr Cys Asn Thr Phe Val Ser Phe Val Gly Ile Ala Xaa Xaa
               100                 105                 110
   Xaa Arg Ser Trp Gly Gln Gly Thr Gln Val Thr Val Ser Ser
           115                 120                 125
<210> 79
   <211> 126
   <212> PRT
   <213> Artificial sequence
<220>
   <223> Amino acid
<220>
   <221> MISC_FEATURE
   <222> (36)..(37)
   <223> Unknown amino acid
<220>
   <221> MISC_FEATURE
   <222> (57)..(57)
   <223> Unknown amino acid
<220>
   <221> MISC_FEATURE
   <222> (111)..(113)
   <223> unknown amino acid
<400> 79
   Glu Val Gln Leu Val Glu Ser Gly Gly Gly Leu Val Gln Ala Gly Asp
   1               5                   10                  15
   Ser Leu Thr Leu Thr Cys Thr Ser Pro Thr Leu Thr Phe Thr Pro Tyr
               20                  25                  30
   Arg Met Gly Xaa Xaa Trp Tyr Arg Gln Ala Pro Gly Lys Gln Arg Asp
           35                  40                  45
   Leu Val Ala Asp Ile Ser Gly Gly Xaa Asp Gly Arg Thr Thr Asn Tyr
       50                  55                  60
   Ala Asp Phe Ala Lys Gly Arg Phe Thr Ile Ser Arg Asp Asn Val Lys
   65                  70                  75                  80
   Asn Ala Val Tyr Leu Gln Met Asn Asn Leu Lys Pro Glu Asp Thr Ala
                   85                  90                  95
   Ile Tyr Tyr Cys Asn Thr Tyr Val Ala Ile Val Gly His Ala Xaa Xaa
               100                 105                 110
   Xaa Arg Ser Trp Gly Gln Gly Thr Gln Val Thr Val Ser Ser
           115                 120                 125
<210> 80
   <211> 126
   <212> PRT
   <213> Artificial sequence
<220>
   <223> Amino acid
<220>
   <221> MISC_FEATURE
   <222> (36)..(37)
   <223> unknown amino acid
<220>
   <221> MISC_FEATURE
   <222> (57)..(57)
   <223> Unknown amino acid
<220>
   <221> MISC_FEATURE
   <222> (111)..(113)
   <223> unknown amino acid
<400> 80
   Gln Val Gln Leu Gln Glu Ser Gly Gly Gly Leu Val Gln Ala Gly Gly
   1               5                   10                  15
   Ser Leu Thr Leu Ser Cys Thr Ser Ser Thr Leu Thr Phe Thr Pro Tyr
               20                  25                  30
   Arg Met Gly Xaa Xaa Trp Tyr Arg Gln Thr Pro Gly Lys Gln Arg Asp
           35                  40                  45
   Leu Val Ala Asp Ile Ser Pro Gly Xaa Asp Gly Ser Thr Lys Asn Tyr
       50                  55                  60
   Ala Gly Phe Ala Gln Gly Arg Phe Thr Ile Ser Arg Asp Asn Ile Lys
   65                  70                  75                  80
   Asn Thr Val Tyr Leu Gln Met Asn Asp Leu Lys Pro Glu Asp Thr Ala
                   85                  90                  95
   Val Tyr Tyr Cys Asn Thr Tyr Val Ala Phe Val Gly Arg Ala Xaa Xaa
               100                 105                 110
   Xaa Arg Thr Trp Gly Gln Gly Thr Gln Val Thr Val Thr Ser
           115                 120                 125
<210> 81
   <211> 345
   <212> DNA
   <213> Artificial sequence
<220>
   <223> Nucleotide
<400> 81
   gaggtgcagc tggtgcagtc tgggggaggc ttggtgcagc ctggggggtc tctgagactc    60
   tcctgttcat tccctggaag catcttcagt ctcaccatgg gctggtaccg tcaggctcca   120
   gggaaggagc gcgagttggt cacaagtgct actcctggtg gtgacacaaa ctatgcagac   180
   ttcgtgaagg gccgattcac catctccaga gacaacgcca ggagcatcat atatctacaa   240
   atgaatagcc tgaaacctga ggacacggcc gtctattatt gttatgcacg tacgaggaat   300
   tggggtacgg tctggggcca ggggacccag gtcaccgtct cctca                   345
<210> 82
   <211> 366
   <212> DNA
   <213> Artificial sequence
<220>
   <223> Nucleotide
<400> 82
   gaggtgcagc tggtgcagtc tgggggagag ttggtgcagg ctgggggctc tctgagactc 60
   tcctgtgcag cctctggcct caccttcagt agctataaca tgggctggtt ccgccgggct 120
   ccagggaagg agcgtgagtt tgtagcatct attacctgga gtggtcggga cacattctat 180
   gcagactccg tgaagggccg attcaccatc tccagagaca acgccaagaa cactgtttat 240
   ctgcaaatga gcagcctgaa acctgaggac acggccgttt attattgtgc tgcaaacccc 300
   tggccagtgg cggcgccacg tagtggcacc tactggggcc aagggaccca ggtcaccgtc 360
   tcctca 366
<210> 83
   <211> 357
   <212> DNA
   <213> Artificial sequence
<220>
   <223> Nucleotide
<400> 83
   gaggtgcagc tggtggagtc tgggggaggc ttagtgcagc ctggggagtc tctgacactc 60
   tcctgtgtag ttgctggaag catcttcagc ttcgccatga gctggtatcg ccaggctcca 120
   ggaaaagagc gcgaattggt cgcacgtatt ggttcggatg atcgggtaac gtacgcagat 180
   tccgtgaagg gccgatttac catctccaga gacaacatca agcgcacggc gggcctgcag 240
   atgaacagcc tgaaacctga ggacacggcc gtctactact gcaatgccca aacagatttg 300
   agggattgga ctgtgcgaga gtactggggc caggggaccc aggtcaccgt ctcctca 357
<210> 84
   <211> 357
   <212> DNA
   <213> Artificial sequence
<220>
   <223> Nucleotide
<400> 84
   gaggtgcagc tggtggagtc tgggggaggc ttggtgcagc ctggggggtc tctgacactc 60
   tcctgtgttg ccgctggaag catcttcacc ttcgccatga gctggtaccg ccaggctcca 120
   cgaaaagagc gcgaattggt cgcacgtatt ggtacggatg acgaaacaat gtacaaagac 180
   tccgtgaagg gtcgattcac catctccaga gacaacgtca agcgcacggc gggtctgcag 240
   atgaacaacc tgaaacccga ggacacggcc gtctactact gcaatgcccg gacagattat 300
   agggactgga ctgtccgtga gtactggggc caggggaccc aggtcaccgt ctcctca 357
<210> 85
   <211> 357
   <212> DNA
   <213> Artificial sequence
<220>
   <223> Nucleotide
<400> 85
   gaggtgcagc tggtggagtc tgggggaggc ttggtgcagg ctgggggctc tctgagactc     60
   tcctgtacca gctctacggt taccttcact ccgtatcaaa tgggctggta ccgccaggct    120
   ccagggaagc agcgtgcttt ggtcgcagat attagtacgg gtggtagccg cacaaattat    180
   gcggatttcg cgaagggccg attcaccatc tccagagacg acgttaagaa cacggtgtat    240
   ctgcaaatga acaacctgaa acctgaggac acggccgtct actactgtaa cacctactac    300
   gcgatgatag ggcatgcgcg taattggggc caggggaccc aggtcactgt ctcctca       357
<210> 86
   <211> 360
   <212> DNA
   <213> Artificial sequence
<220>
   <223> Nucleotide
<400> 86
   gaggtgcagc tggtggagtc tgggggaggc ttcgtgcagg cgggggaatc tctgacgctc     60
   tcctgtacaa gttctacact gaccttcact ccgtatcgca tggcctggta ccgccaggct    120
   ccagggaagc agcgtgattt agtcgcggat attagtagtg gtgatggtag gaccacaaac    180
   tatgcggact tcgcgaaggg ccgattcacc atctccagag acaacatcaa gaacacggtc    240
   tttctgcgaa tgactaacct gaaacctgag gacacggccg tctactactg taacaccttc    300
   gtttcgtttg tggggattgc gcgttcttgg ggccagggga cccaggtcac tgtctcctca    360
<210> 87
   <211> 360
   <212> DNA
   <213> Artificial sequence
<220>
   <223> Nucleotide
<400> 87
   gaggtgcagc tggtggagtc tgggggaggc ttggtgcagg cgggggactc tctgacactg     60
   acctgtacaa gccctacact taccttcact ccgtatcgca tgggctggta ccgccaagct    120
   ccagggaagc agcgtgattt ggtcgcagat attagtggtg gtgatggtcg taccacaaac    180
   tatgcagact tcgcgaaggg ccgattcacc atctccagag acaacgtcaa gaacgcggtc    240
   tatctgcaaa tgaacaacct gaaacctgaa gacacggcca tttattactg taacacctac    300
   gtcgcgattg tgggccatgc gcgttcctgg ggccagggga cccaggtcac cgtctcctca    360
<210> 88
   <211> 360
   <212> DNA
   <213> Artificial sequence
<220>
   <223> Nucleotide
<400> 88
   caggtgcagc tgcaggagtc tgggggaggc ttggtgcagg cggggggctc tctgacactc    60
   tcctgcacaa gttctacact taccttcact ccgtatcgca tgggctggta ccgccagact   120
   ccagggaagc agcgtgattt ggtcgcggac attagtcctg gtgatggtag taccaaaaat   180
   tatgcaggct tcgcgcaggg ccgattcacc atctccagag acaacatcaa gaacacggtg   240
   tatctgcaaa tgaacgacct gaaacctgag gacacggccg tctattactg caacacctac   300
   gtcgcgtttg tggggcgtgc gcgtacttgg ggccagggga cccaggtcac tgtcacctca   360
<210> 89
   <211> 6715
   <212> DNA
   <213> Artificial sequence
<220>
   <223> Plasmide
<400> 89
   ttgtttattt ttctaaatac attcaaatat gtatccgctc atgagacaat aaccctgata    60
   aatgcttcaa taatattgaa aaaggaagag tatgagtatt caacatttcc gtgtcgccct   120
   tattcccttt tttgcggcat tttgccttcc tgtttttgct cacccagaaa cgctggtgaa   180
   agtaaaagat gctgaagatc agttgggtgc acgagtgggt tacatcgaac tggatctcaa   240
   cagcggtaag atccttgaga gttttcgccc cgaagaacgt tttccaatga tgagcacttt   300
   taaagttctg ctatgtggcg cggtattatc ccgtgttgac gccgggcaag agcaactcgg   360
   tcgccgcata cactattctc agaatgactt ggttgagtac tcaccagtca cagaaaagca   420
   tcttacggat ggcatgacag taagagaatt atgcagtgct gccataacca tgagtgataa   480
   cactgcggcc aacttacttc tgacaacgat cggaggaccg aaggagctaa ccgctttttt   540
   gcacaacatg ggggatcatg taactcgcct tgatcgttgg gaaccggagc tgaatgaagc   600
   cataccaaac gacgagcgtg acaccacgat gcctgtagca atggcaacaa cgttgcgcaa   660
   actattaact ggcgaactac ttactctagc ttcccggcaa caattaatag actggatgga   720
   ggcggataaa gttgcaggac cacttctgcg ctcggccctt ccggctggct ggtttattgc   780
   tgataaatct ggagccggtg agcgtgggtc tcgcggtatc attgcagcac tggggccaga   840
   tggtaagccc tcccgtatcg tagttatcta cacgacgggg agtcaggcaa ctatggatga   900
   acgaaataga cagatcgctg agataggtgc ctcactgatt aagcattggt aactgtcaga   960
   ccaagtttac tcatatatac tttagattga tttaaaactt catttttaat ttaaaaggat  1020
   ctaggtgaag atcctttttg ataatctcat gaccaaaatc ccttaacgtg agttttcgtt  1080
   ccactgagcg tcagaccccg tagaaaagat caaaggatct tcttgagatc ctttttttct 1140
   gcgcgtaatc tgctgcttgc aaacaaaaaa accaccgcta ccagcggtgg tttgtttgcc 1200
   ggatcaagag ctaccaactc tttttccgaa ggtaactggc ttcagcagag cgcagatacc 1260
   aaatactgtc cttctagtgt agccgtagtt aggccaccac ttcaagaact ctgtagcacc 1320
   gcctacatac ctcgctctgc taatcctgtt accagtggct gctgccagtg gcgataagtc 1380
   gtgtcttacc gggttggact caagacgata gttaccggat aaggcgcagc ggtcgggctg 1440
   aacggggggt tcgtgcacac agcccagctt ggagcgaacg acctacaccg aactgagata 1500
   cctacagcgt gagcattgag aaagcgccac gcttcccgaa gggagaaagg cggacaggta 1560
   tccggtaagc ggcagggtcg gaacaggaga gcgcacgagg gagcttccag ggggaaacgc 1620
   ctggtatctt tatagtcctg tcgggtttcg ccacctctga cttgagcgtc gatttttgtg 1680
   atgctcgtca ggggggcgga gcctatggaa aaacgccagc aacgcggcct ttttacggtt 1740
   cctggccttt tgctggcctt ttgctcacat gttctttcct gcgttatccc ctgattctgt 1800
   ggataaccgt attaccgcct ttgagtgagc tgataccgct cgccgcagcc gaacgaccga 1860
   gcgcagcgag tcagtgagcg aggaagcgga agagcgcctg atgcggtatt ttctccttac 1920
   gcatctgtgc ggtatttcac accgcatata tggtgcactc tcagtacaat ctgctctgat 1980
   gccgcatagt taagccagta tacactccgc tatcgctacg tgactgggtc atggctgcgc 2040
   cccgacaccc gccaacaccc gctgacgcgc cctgacgggc ttgtctgctc ccggcatccg 2100
   cttacagaca agctgtgacc gtctccggga gctgcatgtg tcagaggttt tcaccgtcat 2160
   caccgaaacg cgcgaggcag ctgcggtaaa gctcatcagc gtggtcgtga agcgattcac 2220
   agatgtctgc ctgttcatcc gcgtccagct cgttgagttt ctccagaagc gttaatgtct 2280
   ggcttctgat aaagcgggcc atgttaaggg cggttttttc ctgtttggtc acttgatgcc 2340
   tccgtgtaag ggggaatttc tgttcatggg ggtaatgata ccgatgaaac gagagaggat 2400
   gctcacgata cgggttactg atgatgaaca tgcccggtta ctggaacgtt gtgagggtaa 2460
   acaactggcg gtatggatgc ggcgggacca gagaaaaatc actcagggtc aatgccagcg 2520
   cttcgttaat acagatgtag gtgttccaca gggtagccag cagcatcctg cgatgcagat 2580
   ccggaacata atggtgcagg gcgctgactt ccgcgtttcc agactttacg aaacacggaa 2640
   accgaagacc attcatgttg ttgctcaggt cgcagacgtt ttgcagcagc agtcgcttca 2700
   cgttcgctcg cgtatcggtg attcattctg ctaaccagta aggcaacccc gccagcctag 2760
   ccgggtcctc aacgacagga gcacgatcat gcgcacccgt ggccaggacc caacgctgcc 2820
   cgagatgcgc cgcgtgcggc tgctggagat ggcggacgcg atggatatgt tctgccaagg 2880
   gttggtttgc gcattcacag ttctccgcaa gaattgattg gctccaattc ttggagtggt 2940
   gaatccgtta gcgaggtgcc gccggcttcc attcaggtcg aggtggcccg gctccatgca 3000
   ccgcgacgca acgcggggag gcagacaagg tatagggcgg cgcctacaat ccatgccaac 3060
   ccgttccatg tgctcgccga ggcggcataa atcgccgtga cgatcagcgg tccagtgatc 3120
   gaagttaggc tggtaagagc cgcgagcgat ccttgaagct gtccctgatg gtcgtcatct 3180
   acctgcctgg acagcatggc ctgcaacgcg ggcatcccga tgccgccgga agcgagaaga 3240
   atcataatgg ggaaggccat ccagcctcgc gtcgcgaacg ccagcaagac gtagcccagc 3300
   gcgtcggcca gcttgcaatt cgcgctaact tacattaatt gcgttgcgct cactgcccgc 3360
   tttccagtcg ggaaacctgt cgtgccagct gcattaatga atcggccaac gcgcggggag 3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg ggcccgctaa cagcgcgatt tgctggtgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggcggc cgatcctcga gagctcccgg gctgcagccc 4920
   tgttctggaa aaccgggctg ctcagggcga tattactgca cccggcggtg ctcgccgttt 4980
   aacgggtgat cagactgccg ctctgcgtga ttctcttagc gataaacctg caaaaaatat 5040
   tattttgctg attggcgatg ggatggggga ctcggaaatt actgccgcac gtaattatgc 5100
   cgaaggtgcg ggcggctttt ttaaaggtat agatgcctta ccgcttaccg ggcaatacac 5160
   tcactatgcg ctgaataaaa aaaccggcaa accggactac gtcaccgact cggctgcatc 5220
   agcaaccgcc tggtcaaccg gtgtcaaaac ctataacggc gcgctgggcg tcgatattca 5280
   cgaaaaagat cacccaacga ttctggaaat ggcaaaagcc gcaggtctgg cgaccggtaa 5340
   cgtttctacc gcagagttgc aggatgccac gcccgctgcg ctggtggcac atgtgacctc 5400
   gcgcaaatgc tacggtccga gcgcgaccag tgaaaaatgt ccgggtaacg ctctggaaaa 5460
   aggcggaaaa ggatcgatta ccgaacagct gcttaacgct cgtgccgacg ttacgcttgg 5520
   cggcggcgca aaaacctttg ctgaaacggc aaccgctggt gaatggcagg gaaaaacgct 5580
   gcgtgaacag gcacaggcgc gtggttatca gttggtgagc gatgctgcct cactgaattc 5640
   ggtgacggaa gcgaatcagc aaaaacccct gcttggcctg tttgctgacg gcaatatgcc 5700
   agtgcgctgg ctaggaccga aagcaacgta ccacggcaat atcgataagc ccgcagtcac 5760
   ctgtacgcca aatccgcaac gtaatgacag tgtaccaacc ctggcgcaga tgaccgacaa 5820
   agccattgaa ttgttgagta aaaatgagaa aggctttttc ctgcaagttg aaggtgcgtc 5880
   aatcgataaa caggatcatg ctgcgaatcc ttgtgggcaa attggcgaga cggtcgatct 5940
   cgatgaagcc gtacaacggg cgctggaatt cgctaaaaag gagggtaaca cgctggtcat 6000
   agtcaccgct gatcacgccc acgccagcca gattgttgcg ccggatacca aagctccggg 6060
   cctcacccag gcgctaaata ccaaagatgg cgcagtgatg gtgatgagtt acgggaactc 6120
   cgaagaggat tcacaagaac ataccggcag tcagttgcgt attgcggcgt atggcccgca 6180
   tgccgccaat gttgttggac tgaccgacca gaccgatctc ttctacacca tgaaagccgc 6240
   tctggggctg aaacatcatc atcaccatca cgggagctaa taagcttctg ttttggcgga 6300
   tgagagaaga ttttcagcct gatacagatt aaatcagaac gcagaagcgg tctgataaaa 6360
   cagaatttgc ctggcggcag tagcgcggtg gtcccacctg accccatgcc gaactcagaa 6420
   gtgaaacgcc gtagcgccga tggtagtgtg gggtctcccc atgcgagagt agggaactgc 6480
   caggcatcaa ataaaacgaa aggctcagtc gaaagactgg gcctttcgtt ttatctgttg 6540
   tttgtcggtg aacgctctcc tgagtaggac aaatccgccg ggagcggatt tgaacgttgc 6600
   gaagcaacgg cccggaggac cctggcgggc aggacgcccg ccataaactg ccaggcatca 6660
   aattaagcag aaggccatcc tgacggatgg cctttttgcg tttctacaaa ctctt 6715
<210> 90
   <211> 6715
   <212> DNA
   <213> Artificial sequence
<220>
   <223> Plasmide
<400> 90
   ttgtttattt ttctaaatac attcaaatat gtatccgctc atgagacaat aaccctgata 60
   aatgcttcaa taatattgaa aaaggaagag tatgagtatt caacatttcc gtgtcgccct 120
   tattcccttt tttgcggcat tttgccttcc tgtttttgct cacccagaaa cgctggtgaa 180
   agtaaaagat gctgaagatc agttgggtgc acgagtgggt tacatcgaac tggatctcaa 240
   cagcggtaag atccttgaga gttttcgccc cgaagaacgt tttccaatga tgagcacttt 300
   taaagttctg ctatgtggcg cggtattatc ccgtgttgac gccgggcaag agcaactcgg 360
   tcgccgcata cactattctc agaatgactt ggttgagtac tcaccagtca cagaaaagca 420
   tcttacggat ggcatgacag taagagaatt atgcagtgct gccataacca tgagtgataa 480
   cactgcggcc aacttacttc tgacaacgat cggaggaccg aaggagctaa ccgctttttt 540
   gcacaacatg ggggatcatg taactcgcct tgatcgttgg gaaccggagc tgaatgaagc 600
   cataccaaac gacgagcgtg acaccacgat gcctgtagca atggcaacaa cgttgcgcaa 660
   actattaact ggcgaactac ttactctagc ttcccggcaa caattaatag actggatgga 720
   ggcggataaa gttgcaggac cacttctgcg ctcggccctt ccggctggct ggtttattgc 780
   tgataaatct ggagccggtg agcgtgggtc tcgcggtatc attgcagcac tggggccaga 840
   tggtaagccc tcccgtatcg tagttatcta cacgacgggg agtcaggcaa ctatggatga 900
   acgaaataga cagatcgctg agataggtgc ctcactgatt aagcattggt aactgtcaga 960
   ccaagtttac tcatatatac tttagattga tttaaaactt catttttaat ttaaaaggat 1020
   ctaggtgaag atcctttttg ataatctcat gaccaaaatc ccttaacgtg agttttcgtt 1080
   ccactgagcg tcagaccccg tagaaaagat caaaggatct tcttgagatc ctttttttct 1140
   gcgcgtaatc tgctgcttgc aaacaaaaaa accaccgcta ccagcggtgg tttgtttgcc 1200
   ggatcaagag ctaccaactc tttttccgaa ggtaactggc ttcagcagag cgcagatacc 1260
   aaatactgtc cttctagtgt agccgtagtt aggccaccac ttcaagaact ctgtagcacc 1320
   gcctacatac ctcgctctgc taatcctgtt accagtggct gctgccagtg gcgataagtc 1380
   gtgtcttacc gggttggact caagacgata gttaccggat aaggcgcagc ggtcgggctg 1440
   aacggggggt tcgtgcacac agcccagctt ggagcgaacg acctacaccg aactgagata 1500
   cctacagcgt gagcattgag aaagcgccac gcttcccgaa gggagaaagg cggacaggta 1560
   tccggtaagc ggcagggtcg gaacaggaga gcgcacgagg gagcttccag ggggaaacgc 1620
   ctggtatctt tatagtcctg tcgggtttcg ccacctctga cttgagcgtc gatttttgtg 1680
   atgctcgtca ggggggcgga gcctatggaa aaacgccagc aacgcggcct ttttacggtt 1740
   cctggccttt tgctggcctt ttgctcacat gttctttcct gcgttatccc ctgattctgt 1800
   ggataaccgt attaccgcct ttgagtgagc tgataccgct cgccgcagcc gaacgaccga 1860
   gcgcagcgag tcagtgagcg aggaagcgga agagcgcctg atgcggtatt ttctccttac 1920
   gcatctgtgc ggtatttcac accgcatata tggtgcactc tcagtacaat ctgctctgat 1980
   gccgcatagt taagccagta tacactccgc tatcgctacg tgactgggtc atggctgcgc 2040
   cccgacaccc gccaacaccc gctgacgcgc cctgacgggc ttgtctgctc ccggcatccg 2100
   cttacagaca agctgtgacc gtctccggga gctgcatgtg tcagaggttt tcaccgtcat 2160
   caccgaaacg cgcgaggcag ctgcggtaaa gctcatcagc gtggtcgtga agcgattcac 2220
   agatgtctgc ctgttcatcc gcgtccagct cgttgagttt ctccagaagc gttaatgtct 2280
   ggcttctgat aaagcgggcc atgttaaggg cggttttttc ctgtttggtc acttgatgcc 2340
   tccgtgtaag ggggaatttc tgttcatggg ggtaatgata ccgatgaaac gagagaggat 2400
   gctcacgata cgggttactg atgatgaaca tgcccggtta ctggaacgtt gtgagggtaa 2460
   acaactggcg gtatggatgc ggcgggacca gagaaaaatc actcagggtc aatgccagcg 2520
   cttcgttaat acagatgtag gtgttccaca gggtagccag cagcatcctg cgatgcagat 2580
   ccggaacata atggtgcagg gcgctgactt ccgcgtttcc agactttacg aaacacggaa 2640
   accgaagacc attcatgttg ttgctcaggt cgcagacgtt ttgcagcagc agtcgcttca 2700
   cgttcgctcg cgtatcggtg attcattctg ctaaccagta aggcaacccc gccagcctag 2760
   ccgggtcctc aacgacagga gcacgatcat gcgcacccgt ggccaggacc caacgctgcc 2820
   cgagatgcgc cgcgtgcggc tgctggagat ggcggacgcg atggatatgt tctgccaagg 2880
   gttggtttgc gcattcacag ttctccgcaa gaattgattg gctccaattc ttggagtggt 2940
   gaatccgtta gcgaggtgcc gccggcttcc attcaggtcg aggtggcccg gctccatgca 3000
   ccgcgacgca acgcggggag gcagacaagg tatagggcgg cgcctacaat ccatgccaac 3060
   ccgttccatg tgctcgccga ggcggcataa atcgccgtga cgatcagcgg tccagtgatc 3120
   gaagttaggc tggtaagagc cgcgagcgat ccttgaagct gtccctgatg gtcgtcatct 3180
   acctgcctgg acagcatggc ctgcaacgcg ggcatcccga tgccgccgga agcgagaaga 3240
   atcataatgg ggaaggccat ccagcctcgc gtcgcgaacg ccagcaagac gtagcccagc 3300
   gcgtcggcca gcttgcaatt cgcgctaact tacattaatt gcgttgcgct cactgcccgc 3360
   tttccagtcg ggaaacctgt cgtgccagct gcattaatga atcggccaac gcgcggggag 3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg gtcccgctaa cagcgcgatt tgctgatgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggcggc cgatcctcga gagctcccgg gctgcagccc 4920
   tgttctggaa aaccgggctg ctcagggcga tattactgca cccggcggtg ctcgccgttt 4980
   aacgggtgat cagactgccg ctctgcgtga ttctcttagc gataaacctg caaaaaatat 5040
   tattttgctg attggcgatg ggatggggga ctcggaaatt actgccgcac gtaattatgc 5100
   cgaaggtgcg ggcggctttt ttaaaggtat agatgcctta ccgcttaccg ggcaatacac 5160
   tcactatgcg ctgaataaaa aaaccggcaa accggactac gtcaccgact cggctgcatc 5220
   agcaaccgcc tggtcaaccg gtgtcaaaac ctataacggc gcgctgggcg tcgatattca 5280
   cgaaaaagat cacccaacga ttctggaaat ggcaaaagcc gcaggtctgg cgaccggtaa 5340
   cgtttctacc gcagagttgc aggatgccac gcccgctgcg ctggtggcac atgtgacctc 5400
   gcgcaaatgc tacggtccga gcgcgaccag tgaaaaatgt ccgggtaacg ctctggaaaa 5460
   aggcggaaaa ggatcgatta ccgaacagct gcttaacgct cgtgccgacg ttacgcttgg 5520
   cggcggcgca aaaacctttg ctgaaacggc aaccgctggt gaatggcagg gaaaaacgct 5580
   gcgtgaacag gcacaggcgc gtggttatca gttggtgagc gatgctgcct cactgaattc 5640
   ggtgacggaa gcgaatcagc aaaaacccct gcttggcctg tttgctgacg gcaatatgcc 5700
   agtgcgctgg ctaggaccga aagcaacgta ccacggcaat atcgataagc ccgcagtcac 5760
   ctgtacgcca aatccgcaac gtaatgacag tgtaccaacc ctggcgcaga tgaccgacaa 5820
   agccattgaa ttgttgagta aaaatgagaa aggctttttc ctgcaagttg aaggtgcgtc 5880
   aatcgataaa caggatcatg ctgcgaatcc ttgtgggcaa attggcgaga cggtcgatct 5940
   cgatgaagcc gtacaacggg cgctggaatt cgctaaaaag gagggtaaca cgctggtcat 6000
   agtcaccgct gatcacgccc acgccagcca gattgttgcg ccggatacca aagctccggg 6060
   cctcacccag gcgctaaata ccaaagatgg cgcagtgatg gtgatgagtt acgggaactc 6120
   cgaagaggat tcacaagaac ataccggcag tcagttgcgt attgcggcgt atggcccgca   6180
   tgccgccaat gttgttggac tgaccgacca gaccgatctc ttctacacca tgaaagccgc   6240
   tctggggctg aaacatcatc atcaccatca cgggagctaa taagcttctg ttttggcgga   6300
   tgagagaaga ttttcagcct gatacagatt aaatcagaac gcagaagcgg tctgataaaa   6360
   cagaatttgc ctggcggcag tagcgcggtg gtcccacctg accccatgcc gaactcagaa   6420
   gtgaaacgcc gtagcgccga tggtagtgtg gggtctcccc atgcgagagt agggaactgc   6480
   caggcatcaa ataaaacgaa aggctcagtc gaaagactgg gcctttcgtt ttatctgttg   6540
   tttgtcggtg aacgctctcc tgagtaggac aaatccgccg ggagcggatt tgaacgttgc   6600
   gaagcaacgg cccggaggac cctggcgggc aggacgcccg ccataaactg ccaggcatca   6660
   aattaagcag aaggccatcc tgacggatgg cctttttgcg tttctacaaa ctctt        6715
<210> 91
   <211> 6721
   <212> DNA
   <213> Artificial sequence
<220>
   <223> Plasmide
<400> 91
   ttgtttattt ttctaaatac attcaaatat gtatccgctc atgagacaat aaccctgata     60
   aatgcttcaa taatattgaa aaaggaagag tatgagtatt caacatttcc gtgtcgccct    120
   tattcccttt tttgcggcat tttgccttcc tgtttttgct cacccagaaa cgctggtgaa    180
   agtaaaagat gctgaagatc agttgggtgc acgagtgggt tacatcgaac tggatctcaa    240
   cagcggtaag atccttgaga gttttcgccc cgaagaacgt tttccaatga tgagcacttt    300
   taaagttctg ctatgtggcg cggtattatc ccgtgttgac gccgggcaag agcaactcgg    360
   tcgccgcata cactattctc agaatgactt ggttgagtac tcaccagtca cagaaaagca    420
   tcttacggat ggcatgacag taagagaatt atgcagtgct gccataacca tgagtgataa    480
   cactgcggcc aacttacttc tgacaacgat cggaggaccg aaggagctaa ccgctttttt    540
   gcacaacatg ggggatcatg taactcgcct tgatcgttgg gaaccggagc tgaatgaagc    600
   cataccaaac gacgagcgtg acaccacgat gcctgtagca atggcaacaa cgttgcgcaa    660
   actattaact ggcgaactac ttactctagc ttcccggcaa caattaatag actggatgga    720
   ggcggataaa gttgcaggac cacttctgcg ctcggccctt ccggctggct ggtttattgc    780
   tgataaatct ggagccggtg agcgtgggtc tcgcggtatc attgcagcac tggggccaga    840
   tggtaagccc tcccgtatcg tagttatcta cacgacgggg agtcaggcaa ctatggatga    900
   acgaaataga cagatcgctg agataggtgc ctcactgatt aagcattggt aactgtcaga    960
   ccaagtttac tcatatatac tttagattga tttaaaactt catttttaat ttaaaaggat 1020
   ctaggtgaag atcctttttg ataatctcat gaccaaaatc ccttaacgtg agttttcgtt 1080
   ccactgagcg tcagaccccg tagaaaagat caaaggatct tcttgagatc ctttttttct 1140
   gcgcgtaatc tgctgcttgc aaacaaaaaa accaccgcta ccagcggtgg tttgtttgcc 1200
   ggatcaagag ctaccaactc tttttccgaa ggtaactggc ttcagcagag cgcagatacc 1260
   aaatactgtc cttctagtgt agccgtagtt aggccaccac ttcaagaact ctgtagcacc 1320
   gcctacatac ctcgctctgc taatcctgtt accagtggct gctgccagtg gcgataagtc 1380
   gtgtcttacc gggttggact caagacgata gttaccggat aaggcgcagc ggtcgggctg 1440
   aacggggggt tcgtgcacac agcccagctt ggagcgaacg acctacaccg aactgagata 1500
   cctacagcgt gagcattgag aaagcgccac gcttcccgaa gggagaaagg cggacaggta 1560
   tccggtaagc ggcagggtcg gaacaggaga gcgcacgagg gagcttccag ggggaaacgc 1620
   ctggtatctt tatagtcctg tcgggtttcg ccacctctga cttgagcgtc gatttttgtg 1680
   atgctcgtca ggggggcgga gcctatggaa aaacgccagc aacgcggcct ttttacggtt 1740
   cctggccttt tgctggcctt ttgctcacat gttctttcct gcgttatccc ctgattctgt 1800
   ggataaccgt attaccgcct ttgagtgagc tgataccgct cgccgcagcc gaacgaccga 1860
   gcgcagcgag tcagtgagcg aggaagcgga agagcgcctg atgcggtatt ttctccttac 1920
   gcatctgtgc ggtatttcac accgcatata tggtgcactc tcagtacaat ctgctctgat 1980
   gccgcatagt taagccagta tacactccgc tatcgctacg tgactgggtc atggctgcgc 2040
   cccgacaccc gccaacaccc gctgacgcgc cctgacgggc ttgtctgctc ccggcatccg 2100
   cttacagaca agctgtgacc gtctccggga gctgcatgtg tcagaggttt tcaccgtcat 2160
   caccgaaacg cgcgaggcag ctgcggtaaa gctcatcagc gtggtcgtga agcgattcac 2220
   agatgtctgc ctgttcatcc gcgtccagct cgttgagttt ctccagaagc gttaatgtct 2280
   ggcttctgat aaagcgggcc atgttaaggg cggttttttc ctgtttggtc acttgatgcc 2340
   tccgtgtaag ggggaatttc tgttcatggg ggtaatgata ccgatgaaac gagagaggat 2400
   gctcacgata cgggttactg atgatgaaca tgcccggtta ctggaacgtt gtgagggtaa 2460
   acaactggcg gtatggatgc ggcgggacca gagaaaaatc actcagggtc aatgccagcg 2520
   cttcgttaat acagatgtag gtgttccaca gggtagccag cagcatcctg cgatgcagat 2580
   ccggaacata atggtgcagg gcgctgactt ccgcgtttcc agactttacg aaacacggaa 2640
   accgaagacc attcatgttg ttgctcaggt cgcagacgtt ttgcagcagc agtcgcttca 2700
   cgttcgctcg cgtatcggtg attcattctg ctaaccagta aggcaacccc gccagcctag 2760
   ccgggtcctc aacgacagga gcacgatcat gcgcacccgt ggccaggacc caacgctgcc 2820
   cgagatgcgc cgcgtgcggc tgctggagat ggcggacgcg atggatatgt tctgccaagg 2880
   gttggtttgc gcattcacag ttctccgcaa gaattgattg gctccaattc ttggagtggt 2940
   gaatccgtta gcgaggtgcc gccggcttcc attcaggtcg aggtggcccg gctccatgca 3000
   ccgcgacgca acgcggggag gcagacaagg tatagggcgg cgcctacaat ccatgccaac 3060
   ccgttccatg tgctcgccga ggcggcataa atcgccgtga cgatcagcgg tccagtgatc 3120
   gaagttaggc tggtaagagc cgcgagcgat ccttgaagct gtccctgatg gtcgtcatct 3180
   acctgcctgg acagcatggc ctgcaacgcg ggcatcccga tgccgccgga agcgagaaga 3240
   atcataatgg ggaaggccat ccagcctcgc gtcgcgaacg ccagcaagac gtagcccagc 3300
   gcgtcggcca gcttgcaatt cgcgctaact tacattaatt gcgttgcgct cactgcccgc 3360
   tttccagtcg ggaaacctgt cgtgccagct gcattaatga atcggccaac gcgcggggag 3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg gtcccgctaa cagcgcgatt tgctgatgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggcggc cggccgatcc tcgagagctc ccgggctgca 4920
   gccctgttct ggaaaaccgg gctgctcagg gcgatattac tgcacccggc ggtgctcgcc 4980
   gtttaacggg tgatcagact gccgctctgc gtgattctct tagcgataaa cctgcaaaaa 5040
   atattatttt gctgattggc gatgggatgg gggactcgga aattactgcc gcacgtaatt 5100
   atgccgaagg tgcgggcggc ttttttaaag gtatagatgc cttaccgctt accgggcaat 5160
   acactcacta tgcgctgaat aaaaaaaccg gcaaaccgga ctacgtcacc gactcggctg 5220
   catcagcaac cgcctggtca accggtgtca aaacctataa cggcgcgctg ggcgtcgata 5280
   ttcacgaaaa agatcaccca acgattctgg aaatggcaaa agccgcaggt ctggcgaccg 5340
   gtaacgtttc taccgcagag ttgcaggatg ccacgcccgc tgcgctggtg gcacatgtga 5400
   cctcgcgcaa atgctacggt ccgagcgcga ccagtgaaaa atgtccgggt aacgctctgg 5460
   aaaaaggcgg aaaaggatcg attaccgaac agctgcttaa cgctcgtgcc gacgttacgc 5520
   ttggcggcgg cgcaaaaacc tttgctgaaa cggcaaccgc tggtgaatgg cagggaaaaa 5580
   cgctgcgtga acaggcacag gcgcgtggtt atcagttggt gagcgatgct gcctcactga 5640
   attcggtgac ggaagcgaat cagcaaaaac ccctgcttgg cctgtttgct gacggcaata 5700
   tgccagtgcg ctggctagga ccgaaagcaa cgtaccacgg caatatcgat aagcccgcag 5760
   tcacctgtac gccaaatccg caacgtaatg acagtgtacc aaccctggcg cagatgaccg 5820
   acaaagccat tgaattgttg agtaaaaatg agaaaggctt tttcctgcaa gttgaaggtg 5880
   cgtcaatcga taaacaggat catgctgcga atccttgtgg gcaaattggc gagacggtcg 5940
   atctcgatga agccgtacaa cgggcgctgg aattcgctaa aaaggagggt aacacgctgg 6000
   tcatagtcac cgctgatcac gcccacgcca gccagattgt tgcgccggat accaaagctc 6060
   cgggcctcac ccaggcgcta aataccaaag atggcgcagt gatggtgatg agttacggga 6120
   actccgaaga ggattcacaa gaacataccg gcagtcagtt gcgtattgcg gcgtatggcc 6180
   cgcatgccgc caatgttgtt ggactgaccg accagaccga tctcttctac accatgaaag 6240
   ccgctctggg gctgaaacat catcatcacc atcacgggag ctaataagct tggctgtttt 6300
   ggcggatgag agaagatttt cagcctgata cagattaaat cagaacgcag aagcggtctg 6360
   ataaaacaga atttgcctgg cggcagtagc gcggtggtcc cacctgaccc catgccgaac 6420
   tcagaagtga aacgccgtag cgccgatggt agtgtggggt ctccccatgc gagagtaggg 6480
   aactgccagg catcaaataa aacgaaaggc tcagtcgaaa gactgggcct ttcgttttat 6540
   ctgttgtttg tcggtgaacg ctctcctgag taggacaaat ccgccgggag cggatttgaa 6600
   cgttgcgaag caacggcccg gagggtggcg gcgggcagga cgcccgccat aaactgccag 6660
   gcatcaaatt aagcagaagg ccatcctgac ggatggcctt tttgcgtttc tacaaactct 6720
   t 6721
<210> 92
   <211> 5400
   <212> DNA
   <213> Artificial sequence
<220>
   <223> plasmide
<400> 92
<210> 93
   <211> 5500
   <212> DNA
   <213> Artificial sequence
<220>
   <223> Plasmide
<400> 93
   ttgtttattt ttctaaatac attcaaatat gtatccgctc atgagacaat aaccctgata 60
   aatgcttcaa taatattgaa aaaggaagag tatgagtatt caacatttcc gtgtcgccct    120
   tattcccttt tttgcggcat tttgccttcc tgtttttgct cacccagaaa cgctggtgaa    180
   agtaaaagat gctgaagatc agttgggtgc acgagtgggt tacatcgaac tggatctcaa    240
   cagcggtaag atccttgaga gttttcgccc cgaagaacgt tttccaatga tgagcacttt    300
   taaagttctg ctatgtggcg cggtattatc ccgtgttgac gccgggcaag agcaactcgg    360
   tcgccgcata cactattctc agaatgactt ggttgagtac tcaccagtca cagaaaagca    420
   tcttacggat ggcatgacag taagagaatt atgcagtgct gccataacca tgagtgataa    480
   cactgcggcc aacttacttc tgacaacgat cggaggaccg aaggagctaa ccgctttttt    540
   gcacaacatg ggggatcatg taactcgcct tgatcgttgg gaaccggagc tgaatgaagc    600
   cataccaaac gacgagcgtg acaccacgat gcctgtagca atggcaacaa cgttgcgcaa    660
   actattaact ggcgaactac ttactctagc ttcccggcaa caattaatag actggatgga    720
   ggcggataaa gttgcaggac cacttctgcg ctcggccctt ccggctggct ggtttattgc    780
   tgataaatct ggagccggtg agcgtgggtc tcgcggtatc attgcagcac tggggccaga    840
   tggtaagccc tcccgtatcg tagttatcta cacgacgggg agtcaggcaa ctatggatga    900
   acgaaataga cagatcgctg agataggtgc ctcactgatt aagcattggt aactgtcaga    960
   ccaagtttac tcatatatac tttagattga tttaaaactt catttttaat ttaaaaggat   1020
   ctaggtgaag atcctttttg ataatctcat gaccaaaatc ccttaacgtg agttttcgtt   1080
   ccactgagcg tcagaccccg tagaaaagat caaaggatct tcttgagatc ctttttttct   1140
   gcgcgtaatc tgctgcttgc aaacaaaaaa accaccgcta ccagcggtgg tttgtttgcc   1200
   ggatcaagag ctaccaactc tttttccgaa ggtaactggc ttcagcagag cgcagatacc   1260
   aaatactgtc cttctagtgt agccgtagtt aggccaccac ttcaagaact ctgtagcacc   1320
   gcctacatac ctcgctctgc taatcctgtt accagtggct gctgccagtg gcgataagtc   1380
   gtgtcttacc gggttggact caagacgata gttaccggat aaggcgcagc ggtcgggctg   1440
   aacggggggt tcgtgcacac agcccagctt ggagcgaacg acctacaccg aactgagata   1500
   cctacagcgt gagcattgag aaagcgccac gcttcccgaa gggagaaagg cggacaggta   1560
   tccggtaagc ggcagggtcg gaacaggaga gcgcacgagg gagcttccag ggggaaacgc   1620
   ctggtatctt tatagtcctg tcgggtttcg ccacctctga cttgagcgtc gatttttgtg   1680
   atgctcgtca ggggggcgga gcctatggaa aaacgccagc aacgcggcct ttttacggtt   1740
   cctggccttt tgctggcctt ttgctcacat gttctttcct gcgttatccc ctgattctgt   1800
   ggataaccgt attaccgcct ttgagtgagc tgataccgct cgccgcagcc gaacgaccga   1860
   gcgcagcgag tcagtgagcg aggaagcgga agagcgcctg atgcggtatt ttctccttac   1920
   gcatctgtgc ggtatttcac accgcatata tggtgcactc tcagtacaat ctgctctgat   1980
   gccgcatagt taagccagta tacactccgc tatcgctacg tgactgggtc atggctgcgc   2040
   cccgacaccc gccaacaccc gctgacgcgc cctgacgggc ttgtctgctc ccggcatccg   2100
   cttacagaca agctgtgacc gtctccggga gctgcatgtg tcagaggttt tcaccgtcat   2160
   caccgaaacg cgcgaggcag ctgcggtaaa gctcatcagc gtggtcgtga agcgattcac   2220
   agatgtctgc ctgttcatcc gcgtccagct cgttgagttt ctccagaagc gttaatgtct   2280
   ggcttctgat aaagcgggcc atgttaaggg cggttttttc ctgtttggtc acttgatgcc   2340
   tccgtgtaag ggggaatttc tgttcatggg ggtaatgata ccgatgaaac gagagaggat   2400
   gctcacgata cgggttactg atgatgaaca tgcccggtta ctggaacgtt gtgagggtaa   2460
   acaactggcg gtatggatgc ggcgggacca gagaaaaatc actcagggtc aatgccagcg   2520
   cttcgttaat acagatgtag gtgttccaca gggtagccag cagcatcctg cgatgcagat   2580
   ccggaacata atggtgcagg gcgctgactt ccgcgtttcc agactttacg aaacacggaa   2640
   accgaagacc attcatgttg ttgctcaggt cgcagacgtt ttgcagcagc agtcgcttca   2700
   cgttcgctcg cgtatcggtg attcattctg ctaaccagta aggcaacccc gccagcctag   2760
   ccgggtcctc aacgacagga gcacgatcat gcgcacccgt ggccaggacc caacgctgcc   2820
   cgagatgcgc cgcgtgcggc tgctggagat ggcggacgcg atggatatgt tctgccaagg   2880
   gttggtttgc gcattcacag ttctccgcaa gaattgattg gctccaattc ttggagtggt   2940
   gaatccgtta gcgaggtgcc gccggcttcc attcaggtcg aggtggcccg gctccatgca   3000
   ccgcgacgca acgcggggag gcagacaagg tatagggcgg cgcctacaat ccatgccaac   3060
   ccgttccatg tgctcgccga ggcggcataa atcgccgtga cgatcagcgg tccagtgatc   3120
   gaagttaggc tggtaagagc cgcgagcgat ccttgaagct gtccctgatg gtcgtcatct   3180
   acctgcctgg acagcatggc ctgcaacgcg ggcatcccga tgccgccgga agcgagaaga   3240
   atcataatgg ggaaggccat ccagcctcgc gtcgcgaacg ccagcaagac gtagcccagc   3300
   gcgtcggcca gcttgcaatt cgcgctaact tacattaatt gcgttgcgct cactgcccgc   3360
   tttccagtcg ggaaacctgt cgtgccagct gcattaatga atcggccaac gcgcggggag   3420
   aggcggtttg cgtattgggc gccagggtgg tttttctttt caccagtgag acgggcaaca   3480
   gctgattgcc cttcaccgcc tggccctgag agagttgcag caagcggtcc acgctggttt   3540
   gccccagcag gcgaaaatcc tgtttgatgg tggttgacgg cgggatataa catgagctgt   3600
   cttcggtatc gtcgtatccc actaccgaga tatccgcacc aacgcgcagc ccggactcgg   3660
   taatggcgcg cattgcgccc agcgccatct gatcgttggc aaccagcatc gcagtgggaa   3720
   cgatgccctc attcagcatt tgcatggttt gttgaaaacc ggacatggca ctccagtcgc   3780
   cttcccgttc cgctatcggc tgaatttgat tgcgagtgag atatttatgc cagccagcca   3840
   gacgcagacg cgccgagaca gaacttaatg gtcccgctaa cagcgcgatt tgctgatgac   3900
   ccaatgcgac cagatgctcc acgcccagtc gcgtaccgtc ttcatgggag aaaataatac   3960
   tgttgatggg tgtctggtca gagacatcaa gaaataacgc cggaacatta gtgcaggcag   4020
   cttccacagc aatggcatcc tggtcatcca gcggatagtt aatgatcagc ccactgacgc   4080
   gttgcgcgag aagattgtgc accgccgctt tacaggcttc gacgccgctt cgttctacca   4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggccca ggtcaccgtc tcctcaaacc gcggagagtg 4920
   tgcaggtgat tacaaagacg atgacgataa gtaataaaca ggaaacagaa gtccatatga 4980
   aatacctatt gcctacggca gccgctggat tgttattact cgcggcccag ccggccatgg 5040
   ccgctagcgc ggccgctcta gattaagctt ggctgttttg gcggatgaga gaagattttc 5100
   agcctgatac agattaaatc agaacgcaga agcggtctga taaaacagaa tttgcctggc 5160
   ggcagtagcg cggtggtccc acctgacccc atgccgaact cagaagtgaa acgccgtagc 5220
   gccgatggta gtgtggggtc tccccatgcg agagtaggga actgccaggc atcaaataaa 5280
   acgaaaggct cagtcgaaag actgggcctt tcgttttatc tgttgtttgt cggtgaacgc 5340
   tctcctgagt aggacaaatc cgccgggagc ggatttgaac gttgcgaagc aacggcccgg 5400
   aggaccctgg cgggcaggac gcccgccata aactgccagg catcaaatta agcagaaggc 5460
   catcctgacg gatggccttt ttgcgtttct acaaactctt 5500
<210> 94
   <211> 5560
   <212> DNA
   <213> Artificial sequence
<220>
   <223> Plasmide
<400> 94
   ttgtttattt ttctaaatac attcaaatat gtatccgctc atgagacaat aaccctgata 60
   aatgcttcaa taatattgaa aaaggaagag tatgagtatt caacatttcc gtgtcgccct 120
   tattcccttt tttgcggcat tttgccttcc tgtttttgct cacccagaaa cgctggtgaa 180
   agtaaaagat gctgaagatc agttgggtgc acgagtgggt tacatcgaac tggatctcaa 240
   cagcggtaag atccttgaga gttttcgccc cgaagaacgt tttccaatga tgagcacttt 300
   taaagttctg ctatgtggcg cggtattatc ccgtgttgac gccgggcaag agcaactcgg 360
   tcgccgcata cactattctc agaatgactt ggttgagtac tcaccagtca cagaaaagca 420
   tcttacggat ggcatgacag taagagaatt atgcagtgct gccataacca tgagtgataa 480
   cactgcggcc aacttacttc tgacaacgat cggaggaccg aaggagctaa ccgctttttt 540
   gcacaacatg ggggatcatg taactcgcct tgatcgttgg gaaccggagc tgaatgaagc 600
   cataccaaac gacgagcgtg acaccacgat gcctgtagca atggcaacaa cgttgcgcaa 660
   actattaact ggcgaactac ttactctagc ttcccggcaa caattaatag actggatgga 720
   ggcggataaa gttgcaggac cacttctgcg ctcggccctt ccggctggct ggtttattgc 780
   tgataaatct ggagccggtg agcgtgggtc tcgcggtatc attgcagcac tggggccaga 840
   tggtaagccc tcccgtatcg tagttatcta cacgacgggg agtcaggcaa ctatggatga 900
   acgaaataga cagatcgctg agataggtgc ctcactgatt aagcattggt aactgtcaga 960
   ccaagtttac tcatatatac tttagattga tttaaaactt catttttaat ttaaaaggat 1020
   ctaggtgaag atcctttttg ataatctcat gaccaaaatc ccttaacgtg agttttcgtt 1080
   ccactgagcg tcagaccccg tagaaaagat caaaggatct tcttgagatc ctttttttct 1140
   gcgcgtaatc tgctgcttgc aaacaaaaaa accaccgcta ccagcggtgg tttgtttgcc 1200
   ggatcaagag ctaccaactc tttttccgaa ggtaactggc ttcagcagag cgcagatacc 1260
   aaatactgtc cttctagtgt agccgtagtt aggccaccac ttcaagaact ctgtagcacc 1320
   gcctacatac ctcgctctgc taatcctgtt accagtggct gctgccagtg gcgataagtc 1380
   gtgtcttacc gggttggact caagacgata gttaccggat aaggcgcagc ggtcgggctg 1440
   aacggggggt tcgtgcacac agcccagctt ggagcgaacg acctacaccg aactgagata 1500
   cctacagcgt gagcattgag aaagcgccac gcttcccgaa gggagaaagg cggacaggta 1560
   tccggtaagc ggcagggtcg gaacaggaga gcgcacgagg gagcttccag ggggaaacgc 1620
   ctggtatctt tatagtcctg tcgggtttcg ccacctctga cttgagcgtc gatttttgtg 1680
   atgctcgtca ggggggcgga gcctatggaa aaacgccagc aacgcggcct ttttacggtt 1740
   cctggccttt tgctggcctt ttgctcacat gttctttcct gcgttatccc ctgattctgt 1800
   ggataaccgt attaccgcct ttgagtgagc tgataccgct cgccgcagcc gaacgaccga 1860
   gcgcagcgag tcagtgagcg aggaagcgga agagcgcctg atgcggtatt ttctccttac 1920
   gcatctgtgc ggtatttcac accgcatata tggtgcactc tcagtacaat ctgctctgat 1980
   gccgcatagt taagccagta tacactccgc tatcgctacg tgactgggtc atggctgcgc 2040
   cccgacaccc gccaacaccc gctgacgcgc cctgacgggc ttgtctgctc ccggcatccg 2100
   cttacagaca agctgtgacc gtctccggga gctgcatgtg tcagaggttt tcaccgtcat 2160
   caccgaaacg cgcgaggcag ctgcggtaaa gctcatcagc gtggtcgtga agcgattcac 2220
   agatgtctgc ctgttcatcc gcgtccagct cgttgagttt ctccagaagc gttaatgtct 2280
   ggcttctgat aaagcgggcc atgttaaggg cggttttttc ctgtttggtc acttgatgcc 2340
   tccgtgtaag ggggaatttc tgttcatggg ggtaatgata ccgatgaaac gagagaggat 2400
   gctcacgata cgggttactg atgatgaaca tgcccggtta ctggaacgtt gtgagggtaa 2460
   acaactggcg gtatggatgc ggcgggacca gagaaaaatc actcagggtc aatgccagcg 2520
   cttcgttaat acagatgtag gtgttccaca gggtagccag cagcatcctg cgatgcagat 2580
   ccggaacata atggtgcagg gcgctgactt ccgcgtttcc agactttacg aaacacggaa 2640
   accgaagacc attcatgttg ttgctcaggt cgcagacgtt ttgcagcagc agtcgcttca 2700
   cgttcgctcg cgtatcggtg attcattctg ctaaccagta aggcaacccc gccagcctag 2760
   ccgggtcctc aacgacagga gcacgatcat gcgcacccgt ggccaggacc caacgctgcc 2820
   cgagatgcgc cgcgtgcggc tgctggagat ggcggacgcg atggatatgt tctgccaagg 2880
   gttggtttgc gcattcacag ttctccgcaa gaattgattg gctccaattc ttggagtggt 2940
   gaatccgtta gcgaggtgcc gccggcttcc attcaggtcg aggtggcccg gctccatgca 3000
   ccgcgacgca acgcggggag gcagacaagg tatagggcgg cgcctacaat ccatgccaac 3060
   ccgttccatg tgctcgccga ggcggcataa atcgccgtga cgatcagcgg tccagtgatc 3120
   gaagttaggc tggtaagagc cgcgagcgat ccttgaagct gtccctgatg gtcgtcatct 3180
   acctgcctgg acagcatggc ctgcaacgcg ggcatcccga tgccgccgga agcgagaaga 3240
   atcataatgg ggaaggccat ccagcctcgc gtcgcgaacg ccagcaagac gtagcccagc 3300
   gcgtcggcca gcttgcaatt cgcgctaact tacattaatt gcgttgcgct cactgcccgc 3360
   tttccagtcg ggaaacctgt cgtgccagct gcattaatga atcggccaac gcgcggggag 3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg gtcccgctaa cagcgcgatt tgctgatgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca    4320
   ctttttcccg cgttttcgca gaaacgtggc tggcctggtt caccacgcgg gaaacggtct     4380
   gataagagac accggcatac tctgcgacat cgtataacgt tactggtttc acattcacca    4440
   ccctgaattg actctcttcc gggcgctatc atgccatacc gcgaaaggtt ttgcgccatt    4500
   cgatggtgtc aacgtaaatg catgccgctt cgccttcgcg cgcgaattgc aagctgatcc    4560
   ggagcttatc gactgcacgg tgcaccaatg cttctggcgt caggcagcca tcggaagctg    4620
   tggtatggct gtgcaggtcg taaatcactg cataattcgt gtcgctcaag gcgcactccc    4680
   gttctggata atgttttttg cgccgacatc ataacggttc tggcaaatat tctgaaatga     4740
   gctgttgaca attaatcatc ggctcgtata atgtgtggaa ttgtgagcgg ataacaattt    4800
   cacacaggaa acagaattcc atatgaaata cctattacca acagcagcag ctgggttatt    4860
   attgctcgct gcgcagccgg ccatggccca ggtcaccgtc tcctcaaacc gcggagagtg    4920
   tgcaggtgat tacaaagacg atgacgataa gtaataaaca ggaaacagaa gtccatatga    4980
   aatacctatt gcctacggca gccgctggat tgttattact cgcggcccag ccggccatgg    5040
   ccgctagcgc ggccgcagaa caaaaactca tctcagaaga ggatctgaat ggggccgtac    5100
   atcaccacca tcatcatggg agctaagctt ggctgttttg gcggatgaga gaagattttc    5160
   agcctgatac agattaaatc agaacgcaga agcggtctga taaaacagaa tttgcctggc    5220
   ggcagtagcg cggtggtccc acctgacccc atgccgaact cagaagtgaa acgccgtagc    5280
   gccgatggta gtgtggggtc tccccatgcg agagtaggga actgccaggc atcaaataaa    5340
   acgaaaggct cagtcgaaag actgggcctt tcgttttatc tgttgtttgt cggtgaacgc    5400
   tctcctgagt aggacaaatc cgccgggagc ggatttgaac gttgcgaagc aacggcccgg    5460
   aggaccctgg cgggcaggac gcccgccata aactgccagg catcaaatta agcagaaggc    5520
   catcctgacg gatggccttt ttgcgtttct acaaactctt                          5560
<210> 95
   <211> 6099
   <212> DNA
   <213> Artificial sequence
<220>
   <223> Plasmide
<400> 95
   ttgtttattt ttctaaatac attcaaatat gtatccgctc atgagacaat aaccctgata      60
   aatgcttcaa taatattgaa aaaggaagag tatgagtatt caacatttcc gtgtcgccct     120
   tattcccttt tttgcggcat tttgccttcc tgtttttgct cacccagaaa cgctggtgaa     180
   agtaaaagat gctgaagatc agttgggtgc acgagtgggt tacatcgaac tggatctcaa     240
   cagcggtaag atccttgaga gttttcgccc cgaagaacgt tttccaatga tgagcacttt    300
   taaagttctg ctatgtggcg cggtattatc ccgtgttgac gccgggcaag agcaactcgg    360
   tcgccgcata cactattctc agaatgactt ggttgagtac tcaccagtca cagaaaagca    420
   tcttacggat ggcatgacag taagagaatt atgcagtgct gccataacca tgagtgataa    480
   cactgcggcc aacttacttc tgacaacgat cggaggaccg aaggagctaa ccgctttttt    540
   gcacaacatg ggggatcatg taactcgcct tgatcgttgg gaaccggagc tgaatgaagc    600
   cataccaaac gacgagcgtg acaccacgat gcctgtagca atggcaacaa cgttgcgcaa    660
   actattaact ggcgaactac ttactctagc ttcccggcaa caattaatag actggatgga    720
   ggcggataaa gttgcaggac cacttctgcg ctcggccctt ccggctggct ggtttattgc    780
   tgataaatct ggagccggtg agcgtgggtc tcgcggtatc attgcagcac tggggccaga    840
   tggtaagccc tcccgtatcg tagttatcta cacgacgggg agtcaggcaa ctatggatga    900
   acgaaataga cagatcgctg agataggtgc ctcactgatt aagcattggt aactgtcaga    960
   ccaagtttac tcatatatac tttagattga tttaaaactt catttttaat ttaaaaggat   1020
   ctaggtgaag atcctttttg ataatctcat gaccaaaatc ccttaacgtg agttttcgtt   1080
   ccactgagcg tcagaccccg tagaaaagat caaaggatct tcttgagatc ctttttttct   1140
   gcgcgtaatc tgctgcttgc aaacaaaaaa accaccgcta ccagcggtgg tttgtttgcc   1200
   ggatcaagag ctaccaactc tttttccgaa ggtaactggc ttcagcagag cgcagatacc   1260
   aaatactgtc cttctagtgt agccgtagtt aggccaccac ttcaagaact ctgtagcacc   1320
   gcctacatac ctcgctctgc taatcctgtt accagtggct gctgccagtg gcgataagtc   1380
   gtgtcttacc gggttggact caagacgata gttaccggat aaggcgcagc ggtcgggctg   1440
   aacggggggt tcgtgcacac agcccagctt ggagcgaacg acctacaccg aactgagata   1500
   cctacagcgt gagcattgag aaagcgccac gcttcccgaa gggagaaagg cggacaggta   1560
   tccggtaagc ggcagggtcg gaacaggaga gcgcacgagg gagcttccag ggggaaacgc   1620
   ctggtatctt tatagtcctg tcgggtttcg ccacctctga cttgagcgtc gatttttgtg   1680
   atgctcgtca ggggggcgga gcctatggaa aaacgccagc aacgcggcct ttttacggtt   1740
   cctggccttt tgctggcctt ttgctcacat gttctttcct gcgttatccc ctgattctgt   1800
   ggataaccgt attaccgcct ttgagtgagc tgataccgct cgccgcagcc gaacgaccga   1860
   gcgcagcgag tcagtgagcg aggaagcgga agagcgcctg atgcggtatt ttctccttac   1920
   gcatctgtgc ggtatttcac accgcatata tggtgcactc tcagtacaat ctgctctgat   1980
   gccgcatagt taagccagta tacactccgc tatcgctacg tgactgggtc atggctgcgc   2040
   cccgacaccc gccaacaccc gctgacgcgc cctgacgggc ttgtctgctc ccggcatccg   2100
   cttacagaca agctgtgacc gtctccggga gctgcatgtg tcagaggttt tcaccgtcat   2160
   caccgaaacg cgcgaggcag ctgcggtaaa gctcatcagc gtggtcgtga agcgattcac   2220
   agatgtctgc ctgttcatcc gcgtccagct cgttgagttt ctccagaagc gttaatgtct   2280
   ggcttctgat aaagcgggcc atgttaaggg cggttttttc ctgtttggtc acttgatgcc 2340
   tccgtgtaag ggggaatttc tgttcatggg ggtaatgata ccgatgaaac gagagaggat 2400
   gctcacgata cgggttactg atgatgaaca tgcccggtta ctggaacgtt gtgagggtaa 2460
   acaactggcg gtatggatgc ggcgggacca gagaaaaatc actcagggtc aatgccagcg 2520
   cttcgttaat acagatgtag gtgttccaca gggtagccag cagcatcctg cgatgcagat 2580
   ccggaacata atggtgcagg gcgctgactt ccgcgtttcc agactttacg aaacacggaa 2640
   accgaagacc attcatgttg ttgctcaggt cgcagacgtt ttgcagcagc agtcgcttca 2700
   cgttcgctcg cgtatcggtg attcattctg ctaaccagta aggcaacccc gccagcctag 2760
   ccgggtcctc aacgacagga gcacgatcat gcgcacccgt ggccaggacc caacgctgcc 2820
   cgagatgcgc cgcgtgcggc tgctggagat ggcggacgcg atggatatgt tctgccaagg 2880
   gttggtttgc gcattcacag ttctccgcaa gaattgattg gctccaattc ttggagtggt 2940
   gaatccgtta gcgaggtgcc gccggcttcc attcaggtcg aggtggcccg gctccatgca 3000
   ccgcgacgca acgcggggag gcagacaagg tatagggcgg cgcctacaat ccatgccaac 3060
   ccgttccatg tgctcgccga ggcggcataa atcgccgtga cgatcagcgg tccagtgatc 3120
   gaagttaggc tggtaagagc cgcgagcgat ccttgaagct gtccctgatg gtcgtcatct 3180
   acctgcctgg acagcatggc ctgcaacgcg ggcatcccga tgccgccgga agcgagaaga 3240
   atcataatgg ggaaggccat ccagcctcgc gtcgcgaacg ccagcaagac gtagcccagc 3300
   gcgtcggcca gcttgcaatt cgcgctaact tacattaatt gcgttgcgct cactgcccgc 3360
   tttccagtcg ggaaacctgt cgtgccagct gcattaatga atcggccaac gcgcggggag 3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg gtcccgctaa cagcgcgatt tgctgatgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggccca ggtcaccgtc tcctcagaca aaactcacac 4920
   atgcccaccg tgcccagcac ctgaactcct ggggggaccg tcagtcttcc tcttcccccc 4980
   aaaacccaag gacaccctca tgatctcccg gacccctgag gtcacatgcg tggtggtgga 5040
   cgtgagccac gaagaccctg aggtcaagtt caactggtac gtggacggcg tggaggtgca 5100
   taatgccaag acaaagccgc gggaggagca gtacaacagc acgtaccggg tggtcagcgt 5160
   cctcaccgtc ctgcaccagg actggctgaa tggcaaggag tacaagtgca aggtctccaa 5220
   caaagccctc ccagccccca tcgagaaaac catctccaaa gccaaagggc agccccgaga 5280
   accacaggtg tacaccctgc ccccatcccg ggatgagctg accaagaacc aggtcagcct 5340
   gacctgcctg gtcaaaggct tctatcccag cgacatcgcc gtggagtggg agagcaatgg 5400
   gcagccggag aacaactaca agaccacgcc tcccgtgctg gactccgacg gctccttctt 5460
   cctctacagc aagctcaccg tggacaagag caggtggcag caggggaacg tcttctcatg 5520
   ctccgtgatg catgaggctc tgcacaacca ctacacgcag aagagcctct ccctgtctcc 5580
   gggtaaagcg gccgcagaac aaaaactcat ctcagaagag gatctgaatg gggccgtaca 5640
   tcaccaccat catcatggga gctaagcttg gctgttttgg cggatgagag aagattttca 5700
   gcctgataca gattaaatca gaacgcagaa gcggtctgat aaaacagaat ttgcctggcg 5760
   gcagtagcgc ggtggtccca cctgacccca tgccgaactc agaagtgaaa cgccgtagcg 5820
   ccgatggtag tgtggggtct ccccatgcga gagtagggaa ctgccaggca tcaaataaaa 5880
   cgaaaggctc agtcgaaaga ctgggccttt cgttttatct gttgtttgtc ggtgaacgct 5940
   ctcctgagta ggacaaatcc gccgggagcg gatttgaacg ttgcgaagca acggcccgga 6000
   ggaccctggc gggcaggacg cccgccataa actgccaggc atcaaattaa gcagaaggcc 6060
   atcctgacgg atggcctttt tgcgtttcta caaactctt 6099
<210> 96
   <211> 6024
   <212> DNA
   <213> Artificial sequence
<220>
   <223> Plasmide
<400> 96
   ttgtttattt ttctaaatac attcaaatat gtatccgctc atgagacaat aaccctgata 60
   aatgcttcaa taatattgaa aaaggaagag tatgagtatt caacatttcc gtgtcgccct 120
   tattcccttt tttgcggcat tttgccttcc tgtttttgct cacccagaaa cgctggtgaa 180
   agtaaaagat gctgaagatc agttgggtgc acgagtgggt tacatcgaac tggatctcaa 240
   cagcggtaag atccttgaga gttttcgccc cgaagaacgt tttccaatga tgagcacttt 300
   taaagttctg ctatgtggcg cggtattatc ccgtgttgac gccgggcaag agcaactcgg 360
   tcgccgcata cactattctc agaatgactt ggttgagtac tcaccagtca cagaaaagca 420
   tcttacggat ggcatgacag taagagaatt atgcagtgct gccataacca tgagtgataa 480
   cactgcggcc aacttacttc tgacaacgat cggaggaccg aaggagctaa ccgctttttt 540
   gcacaacatg ggggatcatg taactcgcct tgatcgttgg gaaccggagc tgaatgaagc 600
   cataccaaac gacgagcgtg acaccacgat gcctgtagca atggcaacaa cgttgcgcaa 660
   actattaact ggcgaactac ttactctagc ttcccggcaa caattaatag actggatgga 720
   ggcggataaa gttgcaggac cacttctgcg ctcggccctt ccggctggct ggtttattgc 780
   tgataaatct ggagccggtg agcgtgggtc tcgcggtatc attgcagcac tggggccaga 840
   tggtaagccc tcccgtatcg tagttatcta cacgacgggg agtcaggcaa ctatggatga 900
   acgaaataga cagatcgctg agataggtgc ctcactgatt aagcattggt aactgtcaga 960
   ccaagtttac tcatatatac tttagattga tttaaaactt catttttaat ttaaaaggat 1020
   ctaggtgaag atcctttttg ataatctcat gaccaaaatc ccttaacgtg agttttcgtt 1080
   ccactgagcg tcagaccccg tagaaaagat caaaggatct tcttgagatc ctttttttct 1140
   gcgcgtaatc tgctgcttgc aaacaaaaaa accaccgcta ccagcggtgg tttgtttgcc 1200
   ggatcaagag ctaccaactc tttttccgaa ggtaactggc ttcagcagag cgcagatacc    1260
   aaatactgtc cttctagtgt agccgtagtt aggccaccac ttcaagaact ctgtagcacc    1320
   gcctacatac ctcgctctgc taatcctgtt accagtggct gctgccagtg gcgataagtc 1380
   gtgtcttacc gggttggact caagacgata gttaccggat aaggcgcagc ggtcgggctg 1440
   aacggggggt tcgtgcacac agcccagctt ggagcgaacg acctacaccg aactgagata 1500
   cctacagcgt gagcattgag aaagcgccac gcttcccgaa gggagaaagg cggacaggta 1560
   tccggtaagc ggcagggtcg gaacaggaga gcgcacgagg gagcttccag ggggaaacgc 1620
   ctggtatctt tatagtcctg tcgggtttcg ccacctctga cttgagcgtc gatttttgtg 1680
   atgctcgtca ggggggcgga gcctatggaa aaacgccagc aacgcggcct ttttacggtt 1740
   cctggccttt tgctggcctt ttgctcacat gttctttcct gcgttatccc ctgattctgt 1800
   ggataaccgt attaccgcct ttgagtgagc tgataccgct cgccgcagcc gaacgaccga 1860
   gcgcagcgag tcagtgagcg aggaagcgga agagcgcctg atgcggtatt ttctccttac 1920
   gcatctgtgc ggtatttcac accgcatata tggtgcactc tcagtacaat ctgctctgat 1980
   gccgcatagt taagccagta tacactccgc tatcgctacg tgactgggtc atggctgcgc 2040
   cccgacaccc gccaacaccc gctgacgcgc cctgacgggc ttgtctgctc ccggcatccg 2100
   cttacagaca agctgtgacc gtctccggga gctgcatgtg tcagaggttt tcaccgtcat 2160
   caccgaaacg cgcgaggcag ctgcggtaaa gctcatcagc gtggtcgtga agcgattcac 2220
   agatgtctgc ctgttcatcc gcgtccagct cgttgagttt ctccagaagc gttaatgtct 2280
   ggcttctgat aaagcgggcc atgttaaggg cggttttttc ctgtttggtc acttgatgcc 2340
   tccgtgtaag ggggaatttc tgttcatggg ggtaatgata ccgatgaaac gagagaggat 2400
   gctcacgata cgggttactg atgatgaaca tgcccggtta ctggaacgtt gtgagggtaa 2460
   acaactggcg gtatggatgc ggcgggacca gagaaaaatc actcagggtc aatgccagcg 2520
   cttcgttaat acagatgtag gtgttccaca gggtagccag cagcatcctg cgatgcagat 2580
   ccggaacata atggtgcagg gcgctgactt ccgcgtttcc agactttacg aaacacggaa 2640
   accgaagacc attcatgttg ttgctcaggt cgcagacgtt ttgcagcagc agtcgcttca 2700
   cgttcgctcg cgtatcggtg attcattctg ctaaccagta aggcaacccc gccagcctag 2760
   ccgggtcctc aacgacagga gcacgatcat gcgcacccgt ggccaggacc caacgctgcc 2820
   cgagatgcgc cgcgtgcggc tgctggagat ggcggacgcg atggatatgt tctgccaagg 2880
   gttggtttgc gcattcacag ttctccgcaa gaattgattg gctccaattc ttggagtggt 2940
   gaatccgtta gcgaggtgcc gccggcttcc attcaggtcg aggtggcccg gctccatgca 3000
   ccgcgacgca acgcggggag gcagacaagg tatagggcgg cgcctacaat ccatgccaac 3060
   ccgttccatg tgctcgccga ggcggcataa atcgccgtga cgatcagcgg tccagtgatc 3120
   gaagttaggc tggtaagagc cgcgagcgat ccttgaagct gtccctgatg gtcgtcatct 3180
   acctgcctgg acagcatggc ctgcaacgcg ggcatcccga tgccgccgga agcgagaaga 3240
   atcataatgg ggaaggccat ccagcctcgc gtcgcgaacg ccagcaagac gtagcccagc   3300
   gcgtcggcca gcttgcaatt cgcgctaact tacattaatt gcgttgcgct cactgcccgc   3360
   tttccagtcg ggaaacctgt cgtgccagct gcattaatga atcggccaac gcgcggggag 3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg gtcccgctaa cagcgcgatt tgctgatgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggccca ggtcaccgtc tcctcagaca aaactcacac 4920
   atgcccaccg tgcccagcac ctgaactcct ggggggaccg tcagtcttcc tcttcccccc 4980
   aaaacccaag gacaccctca tgatctcccg gacccctgag gtcacatgcg tggtggtgga 5040
   cgtgagccac gaagaccctg aggtcaagtt caactggtac gtggacggcg tggaggtgca 5100
   taatgccaag acaaagccgc gggaggagca gtacaacagc acgtaccggg tggtcagcgt 5160
   cctcaccgtc ctgcaccagg actggctgaa tggcaaggag tacaagtgca aggtctccaa 5220
   caaagccctc ccagccccca tcgagaaaac catctccaaa gccaaagggc agccccgaga 5280
   accacaggtg tacaccctgc ccccatcccg ggatgagctg accaagaacc aggtcagcct 5340
   gacctgcctg gtcaaaggct tctatcccag cgacatcgcc gtggagtggg agagcaatgg 5400
   gcagccggag aacaactaca agaccacgcc tcccgtgctg gactccgacg gctccttctt 5460
   cctctacagc aagctcaccg tggacaagag caggtggcag caggggaacg tcttctcatg 5520
   ctccgtgatg catgaggctc tgcacaacca ctacacgcag aagagcctct ccctgtctcc 5580
   gggtaaataa gcttggctgt tttggcggat gagagaagat tttcagcctg atacagatta 5640
   aatcagaacg cagaagcggt ctgataaaac agaatttgcc tggcggcagt agcgcggtgg 5700
   tcccacctga ccccatgccg aactcagaag tgaaacgccg tagcgccgat ggtagtgtgg 5760
   ggtctcccca tgcgagagta gggaactgcc aggcatcaaa taaaacgaaa ggctcagtcg 5820
   aaagactggg cctttcgttt tatctgttgt ttgtcggtga acgctctcct gagtaggaca 5880
   aatccgccgg gagcggattt gaacgttgcg aagcaacggc ccggaggacc ctggcgggca 5940
   ggacgcccgc cataaactgc caggcatcaa attaagcaga aggccatcct gacggatggc 6000
   ctttttgcgt ttctacaaac tctt 6024
<210> 97
   <211> 5866
   <212> DNA
   <213> Artificial sequence
<220>
   <223> Plasmide
<400> 97
   ttgtttattt ttctaaatac attcaaatat gtatccgctc atgagacaat aaccctgata     60
   aatgcttcaa taatattgaa aaaggaagag tatgagtatt caacatttcc gtgtcgccct    120
   tattcccttt tttgcggcat tttgccttcc tgtttttgct cacccagaaa cgctggtgaa    180
   agtaaaagat gctgaagatc agttgggtgc acgagtgggt tacatcgaac tggatctcaa    240
   cagcggtaag atccttgaga gttttcgccc cgaagaacgt tttccaatga tgagcacttt    300
   taaagttctg ctatgtggcg cggtattatc ccgtgttgac gccgggcaag agcaactcgg    360
   tcgccgcata cactattctc agaatgactt ggttgagtac tcaccagtca cagaaaagca    420
   tcttacggat ggcatgacag taagagaatt atgcagtgct gccataacca tgagtgataa    480
   cactgcggcc aacttacttc tgacaacgat cggaggaccg aaggagctaa ccgctttttt    540
   gcacaacatg ggggatcatg taactcgcct tgatcgttgg gaaccggagc tgaatgaagc    600
   cataccaaac gacgagcgtg acaccacgat gcctgtagca atggcaacaa cgttgcgcaa    660
   actattaact ggcgaactac ttactctagc ttcccggcaa caattaatag actggatgga    720
   ggcggataaa gttgcaggac cacttctgcg ctcggccctt ccggctggct ggtttattgc    780
   tgataaatct ggagccggtg agcgtgggtc tcgcggtatc attgcagcac tggggccaga    840
   tggtaagccc tcccgtatcg tagttatcta cacgacgggg agtcaggcaa ctatggatga    900
   acgaaataga cagatcgctg agataggtgc ctcactgatt aagcattggt aactgtcaga    960
   ccaagtttac tcatatatac tttagattga tttaaaactt catttttaat ttaaaaggat   1020
   ctaggtgaag atcctttttg ataatctcat gaccaaaatc ccttaacgtg agttttcgtt   1080
   ccactgagcg tcagaccccg tagaaaagat caaaggatct tcttgagatc ctttttttct  1140
   gcgcgtaatc tgctgcttgc aaacaaaaaa accaccgcta ccagcggtgg tttgtttgcc   1200
   ggatcaagag ctaccaactc tttttccgaa ggtaactggc ttcagcagag cgcagatacc   1260
   aaatactgtc cttctagtgt agccgtagtt aggccaccac ttcaagaact ctgtagcacc   1320
   gcctacatac ctcgctctgc taatcctgtt accagtggct gctgccagtg gcgataagtc   1380
   gtgtcttacc gggttggact caagacgata gttaccggat aaggcgcagc ggtcgggctg    1440
   aacggggggt tcgtgcacac agcccagctt ggagcgaacg acctacaccg aactgagata    1500
   cctacagcgt gagcattgag aaagcgccac gcttcccgaa gggagaaagg cggacaggta    1560
   tccggtaagc ggcagggtcg gaacaggaga gcgcacgagg gagcttccag ggggaaacgc    1620
   ctggtatctt tatagtcctg tcgggtttcg ccacctctga cttgagcgtc gatttttgtg    1680
   atgctcgtca ggggggcgga gcctatggaa aaacgccagc aacgcggcct ttttacggtt    1740
   cctggccttt tgctggcctt ttgctcacat gttctttcct gcgttatccc ctgattctgt    1800
   ggataaccgt attaccgcct ttgagtgagc tgataccgct cgccgcagcc gaacgaccga    1860
   gcgcagcgag tcagtgagcg aggaagcgga agagcgcctg atgcggtatt ttctccttac    1920
   gcatctgtgc ggtatttcac accgcatata tggtgcactc tcagtacaat ctgctctgat    1980
   gccgcatagt taagccagta tacactccgc tatcgctacg tgactgggtc atggctgcgc    2040
   cccgacaccc gccaacaccc gctgacgcgc cctgacgggc ttgtctgctc ccggcatccg    2100
   cttacagaca agctgtgacc gtctccggga gctgcatgtg tcagaggttt tcaccgtcat    2160
   caccgaaacg cgcgaggcag ctgcggtaaa gctcatcagc gtggtcgtga agcgattcac    2220
   agatgtctgc ctgttcatcc gcgtccagct cgttgagttt ctccagaagc gttaatgtct    2280
   ggcttctgat aaagcgggcc atgttaaggg cggttttttc ctgtttggtc acttgatgcc    2340
   tccgtgtaag ggggaatttc tgttcatggg ggtaatgata ccgatgaaac gagagaggat    2400
   gctcacgata cgggttactg atgatgaaca tgcccggtta ctggaacgtt gtgagggtaa    2460
   acaactggcg gtatggatgc ggcgggacca gagaaaaatc actcagggtc aatgccagcg    2520
   cttcgttaat acagatgtag gtgttccaca gggtagccag cagcatcctg cgatgcagat    2580
   ccggaacata atggtgcagg gcgctgactt ccgcgtttcc agactttacg aaacacggaa    2640
   accgaagacc attcatgttg ttgctcaggt cgcagacgtt ttgcagcagc agtcgcttca    2700
   cgttcgctcg cgtatcggtg attcattctg ctaaccagta aggcaacccc gccagcctag    2760
   ccgggtcctc aacgacagga gcacgatcat gcgcacccgt ggccaggacc caacgctgcc    2820
   cgagatgcgc cgcgtgcggc tgctggagat ggcggacgcg atggatatgt tctgccaagg    2880
   gttggtttgc gcattcacag ttctccgcaa gaattgattg gctccaattc ttggagtggt    2940
   gaatccgtta gcgaggtgcc gccggcttcc attcaggtcg aggtggcccg gctccatgca    3000
   ccgcgacgca acgcggggag gcagacaagg tatagggcgg cgcctacaat ccatgccaac    3060
   ccgttccatg tgctcgccga ggcggcataa atcgccgtga cgatcagcgg tccagtgatc    3120
   gaagttaggc tggtaagagc cgcgagcgat ccttgaagct gtccctgatg gtcgtcatct    3180
   acctgcctgg acagcatggc ctgcaacgcg ggcatcccga tgccgccgga agcgagaaga    3240
   atcataatgg ggaaggccat ccagcctcgc gtcgcgaacg ccagcaagac gtagcccagc    3300
   gcgtcggcca gcttgcaatt cgcgctaact tacattaatt gcgttgcgct cactgcccgc    3360
   tttccagtcg ggaaacctgt cgtgccagct gcattaatga atcggccaac gcgcggggag    3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg gtcccgctaa cagcgcgatt tgctgatgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggccca ggtcaccgtc tcctcaggta ccgtggctgc    4920
   accatctgtc ttcatcttcc cgccatctga tgagcagttg aaatctggaa ctgcctctgt    4980
   tgtgtgcctg ctgaataact tctatcccag agaggccaaa gtacagtgga aggtggataa 5040
   cgccctccaa tcgggtaact cccaggagag tgtcacagag caggacagca aggacagcac 5100
   ctacagcctc agcagcaccc tgacgctgag caaagcagac tacgagaaac acaaagtcta 5160
   cgcctgcgaa gtcacccatc agggcctgag ctcgccggtg acaaagagct tcaaccgcgg 5220
   agagtgtgca ggtgattaca aagacgatga cgataagtaa taaacaggaa acagaagtcc 5280
   atatgaaata cctattgcct acggcagccg ctggattgtt attactcgcg gcccagccgg 5340
   ccatggccgc tagcgcggcc gcagaacaaa aactcatctc agaagaggat ctgaatgggg 5400
   ccgtacatca ccaccatcat catgggagct aagcttggct gttttggcgg atgagagaag 5460
   attttcagcc tgatacagat taaatcagaa cgcagaagcg gtctgataaa acagaatttg 5520
   cctggcggca gtagcgcggt ggtcccacct gaccccatgc cgaactcaga agtgaaacgc 5580
   cgtagcgccg atggtagtgt ggggtctccc catgcgagag tagggaactg ccaggcatca 5640
   aataaaacga aaggctcagt cgaaagactg ggcctttcgt tttatctgtt gtttgtcggt 5700
   gaacgctctc ctgagtagga caaatccgcc gggagcggat ttgaacgttg cgaagcaacg 5760
   gcccggagga ccctggcggg caggacgccc gccataaact gccaggcatc aaattaagca 5820
   gaaggccatc ctgacggatg gcctttttgc gtttctacaa actctt 5866
<210> 98
   <211> 6109
   <212> DNA
   <213> Artificial sequence
<220>
   <223> Plasmide
<400> 98
   ttgtttattt ttctaaatac attcaaatat gtatccgctc atgagacaat aaccctgata 60
   aatgcttcaa taatattgaa aaaggaagag tatgagtatt caacatttcc gtgtcgccct 120
   tattcccttt tttgcggcat tttgccttcc tgtttttgct cacccagaaa cgctggtgaa 180
   agtaaaagat gctgaagatc agttgggtgc acgagtgggt tacatcgaac tggatctcaa 240
   cagcggtaag atccttgaga gttttcgccc cgaagaacgt tttccaatga tgagcacttt 300
   taaagttctg ctatgtggcg cggtattatc ccgtgttgac gccgggcaag agcaactcgg 360
   tcgccgcata cactattctc agaatgactt ggttgagtac tcaccagtca cagaaaagca 420
   tcttacggat ggcatgacag taagagaatt atgcagtgct gccataacca tgagtgataa 480
   cactgcggcc aacttacttc tgacaacgat cggaggaccg aaggagctaa ccgctttttt 540
   gcacaacatg ggggatcatg taactcgcct tgatcgttgg gaaccggagc tgaatgaagc    600
   cataccaaac gacgagcgtg acaccacgat gcctgtagca atggcaacaa cgttgcgcaa    660
   actattaact ggcgaactac ttactctagc ttcccggcaa caattaatag actggatgga 720
   ggcggataaa gttgcaggac cacttctgcg ctcggccctt ccggctggct ggtttattgc 780
   tgataaatct ggagccggtg agcgtgggtc tcgcggtatc attgcagcac tggggccaga 840
   tggtaagccc tcccgtatcg tagttatcta cacgacgggg agtcaggcaa ctatggatga 900
   acgaaataga cagatcgctg agataggtgc ctcactgatt aagcattggt aactgtcaga 960
   ccaagtttac tcatatatac tttagattga tttaaaactt catttttaat ttaaaaggat 1020
   ctaggtgaag atcctttttg ataatctcat gaccaaaatc ccttaacgtg agttttcgtt 1080
   ccactgagcg tcagaccccg tagaaaagat caaaggatct tcttgagatc ctttttttct 1140
   gcgcgtaatc tgctgcttgc aaacaaaaaa accaccgcta ccagcggtgg tttgtttgcc   1200
   ggatcaagag ctaccaactc tttttccgaa ggtaactggc ttcagcagag cgcagatacc   1260
   aaatactgtc cttctagtgt agccgtagtt aggccaccac ttcaagaact ctgtagcacc   1320
   gcctacatac ctcgctctgc taatcctgtt accagtggct gctgccagtg gcgataagtc   1380
   gtgtcttacc gggttggact caagacgata gttaccggat aaggcgcagc ggtcgggctg   1440
   aacggggggt tcgtgcacac agcccagctt ggagcgaacg acctacaccg aactgagata   1500
   cctacagcgt gagcattgag aaagcgccac gcttcccgaa gggagaaagg cggacaggta   1560
   tccggtaagc ggcagggtcg gaacaggaga gcgcacgagg gagcttccag ggggaaacgc   1620
   ctggtatctt tatagtcctg tcgggtttcg ccacctctga cttgagcgtc gatttttgtg   1680
   atgctcgtca ggggggcgga gcctatggaa aaacgccagc aacgcggcct ttttacggtt   1740
   cctggccttt tgctggcctt ttgctcacat gttctttcct gcgttatccc ctgattctgt   1800
   ggataaccgt attaccgcct ttgagtgagc tgataccgct cgccgcagcc gaacgaccga   1860
   gcgcagcgag tcagtgagcg aggaagcgga agagcgcctg atgcggtatt ttctccttac   1920
   gcatctgtgc ggtatttcac accgcatata tggtgcactc tcagtacaat ctgctctgat   1980
   gccgcatagt taagccagta tacactccgc tatcgctacg tgactgggtc atggctgcgc   2040
   cccgacaccc gccaacaccc gctgacgcgc cctgacgggc ttgtctgctc ccggcatccg   2100
   cttacagaca agctgtgacc gtctccggga gctgcatgtg tcagaggttt tcaccgtcat   2160
   caccgaaacg cgcgaggcag ctgcggtaaa gctcatcagc gtggtcgtga agcgattcac   2220
   agatgtctgc ctgttcatcc gcgtccagct cgttgagttt ctccagaagc gttaatgtct   2280
   ggcttctgat aaagcgggcc atgttaaggg cggttttttc ctgtttggtc acttgatgcc   2340
   tccgtgtaag ggggaatttc tgttcatggg ggtaatgata ccgatgaaac gagagaggat   2400
   gctcacgata cgggttactg atgatgaaca tgcccggtta ctggaacgtt gtgagggtaa   2460
   acaactggcg gtatggatgc ggcgggacca gagaaaaatc actcagggtc aatgccagcg   2520
   cttcgttaat acagatgtag gtgttccaca gggtagccag cagcatcctg cgatgcagat   2580
   ccggaacata atggtgcagg gcgctgactt ccgcgtttcc agactttacg aaacacggaa   2640
   accgaagacc attcatgttg ttgctcaggt cgcagacgtt ttgcagcagc agtcgcttca   2700
   cgttcgctcg cgtatcggtg attcattctg ctaaccagta aggcaacccc gccagcctag   2760
   ccgggtcctc aacgacagga gcacgatcat gcgcacccgt ggccaggacc caacgctgcc   2820
   cgagatgcgc cgcgtgcggc tgctggagat ggcggacgcg atggatatgt tctgccaagg   2880
   gttggtttgc gcattcacag ttctccgcaa gaattgattg gctccaattc ttggagtggt   2940
   gaatccgtta gcgaggtgcc gccggcttcc attcaggtcg aggtggcccg gctccatgca   3000
   ccgcgacgca acgcggggag gcagacaagg tatagggcgg cgcctacaat ccatgccaac   3060
   ccgttccatg tgctcgccga ggcggcataa atcgccgtga cgatcagcgg tccagtgatc   3120
   gaagttaggc tggtaagagc cgcgagcgat ccttgaagct gtccctgatg gtcgtcatct   3180
   acctgcctgg acagcatggc ctgcaacgcg ggcatcccga tgccgccgga agcgagaaga 3240
   atcataatgg ggaaggccat ccagcctcgc gtcgcgaacg ccagcaagac gtagcccagc 3300
   gcgtcggcca gcttgcaatt cgcgctaact tacattaatt gcgttgcgct cactgcccgc 3360
   tttccagtcg ggaaacctgt cgtgccagct gcattaatga atcggccaac gcgcggggag 3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg gtcccgctaa cagcgcgatt tgctgatgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc    4680
   gttctggata atgttttttg cgccgacatc ataacggttc tggcaaatat tctgaaatga    4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggccca ggtcaccgtc tcctcaggta ccgtggctgc 4920
   accatctgtc ttcatcttcc cgccatctga tgagcagttg aaatctggaa ctgcctctgt 4980
   tgtgtgcctg ctgaataact tctatcccag agaggccaaa gtacagtgga aggtggataa 5040
   cgccctccaa tcgggtaact cccaggagag tgtcacagag caggacagca aggacagcac 5100
   ctacagcctc agcagcaccc tgacgctgag caaagcagac tacgagaaac acaaagtcta 5160
   cgcctgcgaa gtcacccatc agggcctgag ctcgccggtg acaaagagct tcaaccgcgg 5220
   agagtgtgca ggtgattaca aagacgatga cgataagtaa taaacaggaa acagaagtcc 5280
   atatgaaata cctattgcct acggcagccg ctggattgtt attactcgcg gcccagccgg 5340
   ccatggccgc tagcaccaag ggcccatcgg tcttccccct ggcaccctcc tccaagagca 5400
   cctctggggg cacagcggcc ctgggctgcc tggtcaagga ctacttcccc gaaccggtga 5460
   cggtgtcgtg gaactcaggc gccctgacca gcggcgtgca caccttcccg gctgtcctac 5520
   agtcctcagg actctactcc ctcagcagcg tagtgaccgt gccctccagc agcttgggca 5580
   cccagaccta catctgcaac gtgaatcaca agcccagcaa caccaaggtg gacaagaaag 5640
   ttgagcccaa atcttgtgcg gccgcagaac aaaaactcat ctcagaagag gatctgaatg 5700
   gggccgtaca tcaccaccat catcatggga gctaagcttg gctgttttgg cggatgagag 5760
   aagattttca gcctgataca gattaaatca gaacgcagaa gcggtctgat aaaacagaat 5820
   ttgcctggcg gcagtagcgc ggtggtccca cctgacccca tgccgaactc agaagtgaaa 5880
   cgccgtagcg ccgatggtag tgtggggtct ccccatgcga gagtagggaa ctgccaggca 5940
   tcaaataaaa cgaaaggctc agtcgaaaga ctgggccttt cgttttatct gttgtttgtc 6000
   ggtgaacgct ctcctgagta ggacaaatcc gccgggagcg gatttgaacg ttgcgaagca 6060
   gcagaaggcc atcctgacgg atggcctttt tgcgtttcta caaactctt 6109
<210> 99
   <211> 6199
   <212> DNA
   <213> Artificial sequence
<220>
   <223> Plasmide
<400> 99
   ttgtttattt ttctaaatac attcaaatat gtatccgctc atgagacaat aaccctgata 60
   aatgcttcaa taatattgaa aaaggaagag tatgagtatt caacatttcc gtgtcgccct 120
   tattcccttt tttgcggcat tttgccttcc tgtttttgct cacccagaaa cgctggtgaa 180
   agtaaaagat gctgaagatc agttgggtgc acgagtgggt tacatcgaac tggatctcaa 240
   cagcggtaag atccttgaga gttttcgccc cgaagaacgt tttccaatga tgagcacttt 300
   taaagttctg ctatgtggcg cggtattatc ccgtgttgac gccgggcaag agcaactcgg 360
   tcgccgcata cactattctc agaatgactt ggttgagtac tcaccagtca cagaaaagca 420
   tcttacggat ggcatgacag taagagaatt atgcagtgct gccataacca tgagtgataa 480
   cactgcggcc aacttacttc tgacaacgat cggaggaccg aaggagctaa ccgctttttt 540
   gcacaacatg ggggatcatg taactcgcct tgatcgttgg gaaccggagc tgaatgaagc 600
   cataccaaac gacgagcgtg acaccacgat gcctgtagca atggcaacaa cgttgcgcaa 660
   actattaact ggcgaactac ttactctagc ttcccggcaa caattaatag actggatgga 720
   ggcggataaa gttgcaggac cacttctgcg ctcggccctt ccggctggct ggtttattgc 780
   tgataaatct ggagccggtg agcgtgggtc tcgcggtatc attgcagcac tggggccaga 840
   tggtaagccc tcccgtatcg tagttatcta cacgacgggg agtcaggcaa ctatggatga 900
   acgaaataga cagatcgctg agataggtgc ctcactgatt aagcattggt aactgtcaga 960
   ccaagtttac tcatatatac tttagattga tttaaaactt catttttaat ttaaaaggat 1020
   ctaggtgaag atcctttttg ataatctcat gaccaaaatc ccttaacgtg agttttcgtt 1080
   ccactgagcg tcagaccccg tagaaaagat caaaggatct tcttgagatc ctttttttct 1140
   gcgcgtaatc tgctgcttgc aaacaaaaaa accaccgcta ccagcggtgg tttgtttgcc 1200
   ggatcaagag ctaccaactc tttttccgaa ggtaactggc ttcagcagag cgcagatacc 1260
   aaatactgtc cttctagtgt agccgtagtt aggccaccac ttcaagaact ctgtagcacc 1320
   gcctacatac ctcgctctgc taatcctgtt accagtggct gctgccagtg gcgataagtc 1380
   gtgtcttacc gggttggact caagacgata gttaccggat aaggcgcagc ggtcgggctg 1440
   aacggggggt tcgtgcacac agcccagctt ggagcgaacg acctacaccg aactgagata 1500
   cctacagcgt gagcattgag aaagcgccac gcttcccgaa gggagaaagg cggacaggta 1560
   tccggtaagc ggcagggtcg gaacaggaga gcgcacgagg gagcttccag ggggaaacgc 1620
   ctggtatctt tatagtcctg tcgggtttcg ccacctctga cttgagcgtc gatttttgtg 1680
   atgctcgtca ggggggcgga gcctatggaa aaacgccagc aacgcggcct ttttacggtt 1740
   cctggccttt tgctggcctt ttgctcacat gttctttcct gcgttatccc ctgattctgt 1800
   ggataaccgt attaccgcct ttgagtgagc tgataccgct cgccgcagcc gaacgaccga 1860
   gcgcagcgag tcagtgagcg aggaagcgga agagcgcctg atgcggtatt ttctccttac 1920
   gcatctgtgc ggtatttcac accgcatata tggtgcactc tcagtacaat ctgctctgat 1980
   gccgcatagt taagccagta tacactccgc tatcgctacg tgactgggtc atggctgcgc 2040
   cccgacaccc gccaacaccc gctgacgcgc cctgacgggc ttgtctgctc ccggcatccg 2100
   cttacagaca agctgtgacc gtctccggga gctgcatgtg tcagaggttt tcaccgtcat 2160
   caccgaaacg cgcgaggcag ctgcggtaaa gctcatcagc gtggtcgtga agcgattcac 2220
   agatgtctgc ctgttcatcc gcgtccagct cgttgagttt ctccagaagc gttaatgtct 2280
   ggcttctgat aaagcgggcc atgttaaggg cggttttttc ctgtttggtc acttgatgcc 2340
   tccgtgtaag ggggaatttc tgttcatggg ggtaatgata ccgatgaaac gagagaggat 2400
   gctcacgata cgggttactg atgatgaaca tgcccggtta ctggaacgtt gtgagggtaa 2460
   acaactggcg gtatggatgc ggcgggacca gagaaaaatc actcagggtc aatgccagcg 2520
   cttcgttaat acagatgtag gtgttccaca gggtagccag cagcatcctg cgatgcagat 2580
   ccggaacata atggtgcagg gcgctgactt ccgcgtttcc agactttacg aaacacggaa 2640
   accgaagacc attcatgttg ttgctcaggt cgcagacgtt ttgcagcagc agtcgcttca 2700
   cgttcgctcg cgtatcggtg attcattctg ctaaccagta aggcaacccc gccagcctag 2760
   ccgggtcctc aacgacagga gcacgatcat gcgcacccgt ggccaggacc caacgctgcc 2820
   cgagatgcgc cgcgtgcggc tgctggagat ggcggacgcg atggatatgt tctgccaagg 2880
   gttggtttgc gcattcacag ttctccgcaa gaattgattg gctccaattc ttggagtggt 2940
   gaatccgtta gcgaggtgcc gccggcttcc attcaggtcg aggtggcccg gctccatgca 3000
   ccgcgacgca acgcggggag gcagacaagg tatagggcgg cgcctacaat ccatgccaac 3060
   ccgttccatg tgctcgccga ggcggcataa atcgccgtga cgatcagcgg tccagtgatc 3120
   gaagttaggc tggtaagagc cgcgagcgat ccttgaagct gtccctgatg gtcgtcatct 3180
   acctgcctgg acagcatggc ctgcaacgcg ggcatcccga tgccgccgga agcgagaaga 3240
   atcataatgg ggaaggccat ccagcctcgc gtcgcgaacg ccagcaagac gtagcccagc 3300
   gcgtcggcca gcttgcaatt cgcgctaact tacattaatt gcgttgcgct cactgcccgc 3360
   tttccagtcg ggaaacctgt cgtgccagct gcattaatga atcggccaac gcgcggggag 3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg gtcccgctaa cagcgcgatt tgctgatgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggccca ggtcaccgtc tcctcaggta ccgtggctgc 4920
   accatctgtc ttcatcttcc cgccatctga tgagcagttg aaatctggaa ctgcctctgt 4980
   tgtgtgcctg ctgaataact tctatcccag agaggccaaa gtacagtgga aggtggataa 5040
   cgccctccaa tcgggtaact cccaggagag tgtcacagag caggacagca aggacagcac 5100
   ctacagcctc agcagcaccc tgacgctgag caaagcagac tacgagaaac acaaagtcta 5160
   cgcctgcgaa gtcacccatc agggcctgag ctcgccggtg acaaagagct tcaaccgcgg 5220
   agagtgtgca ggtgattaca aagacgatga cgataagtaa taaacaggaa acagaagtcc 5280
   atatgaaata cctattgcct acggcagccg ctggattgtt attactcgcg gcccagccgg 5340
   ccatggccgc tagcaccaag ggcccatcgg tcttccccct ggcaccctcc tccaagagca 5400
   cctctggggg cacagcggcc ctgggctgcc tggtcaagga ctacttcccc gaaccggtga 5460
   cggtgtcgtg gaactcaggc gccctgacca gcggcgtgca caccttcccg gctgtcctac 5520
   agtcctcagg actctactcc ctcagcagcg tagtgaccgt gccctccagc agcttgggca 5580
   cccagaccta catctgcaac gtgaatcaca agcccagcaa caccaaggtg gacaagaaag 5640
   ttgagcccaa atcttgtgac aaaactcaca catgcccacc gtgcccagcg gccgcagaac 5700
   aaaaactcat ctcagaagag gatctgaatg gggccgtaca tcaccaccat catcatggga 5760
   gctaagcttg gctgttttgg cggatgagag aagattttca gcctgataca gattaaatca 5820
   gaacgcagaa gcggtctgat aaaacagaat ttgcctggcg gcagtagcgc ggtggtccca 5880
   cctgacccca tgccgaactc agaagtgaaa cgccgtagcg ccgatggtag tgtggggtct 5940
   ccccatgcga gagtagggaa ctgccaggca tcaaataaaa cgaaaggctc agtcgaaaga 6000
   ctgggccttt cgttttatct gttgtttgtc ggtgaacgct ctcctgagta ggacaaatcc 6060
   gccgggagcg gatttgaacg ttgcgaagca acggcccgga ggaccctggc gggcaggacg 6120
   cccgccataa actgccaggc atcaaattaa gcagaaggcc atcctgacgg atggcctttt 6180
   tgcgtttcta caaactctt                                                6199
<210> 100
   <211> 6064
   <212> DNA
   <213> Artificial sequence
<220>
   <223> Plasmide
<400> 100
   ttgtttattt ttctaaatac attcaaatat gtatccgctc atgagacaat aaccctgata 60
   aatgcttcaa taatattgaa aaaggaagag tatgagtatt caacatttcc gtgtcgccct 120
   tattcccttt tttgcggcat tttgccttcc tgtttttgct cacccagaaa cgctggtgaa 180
   agtaaaagat gctgaagatc agttgggtgc acgagtgggt tacatcgaac tggatctcaa 240
   cagcggtaag atccttgaga gttttcgccc cgaagaacgt tttccaatga tgagcacttt 300
   taaagttctg ctatgtggcg cggtattatc ccgtgttgac gccgggcaag agcaactcgg 360
   tcgccgcata cactattctc agaatgactt ggttgagtac tcaccagtca cagaaaagca 420
   tcttacggat ggcatgacag taagagaatt atgcagtgct gccataacca tgagtgataa 480
   cactgcggcc aacttacttc tgacaacgat cggaggaccg aaggagctaa ccgctttttt 540
   gcacaacatg ggggatcatg taactcgcct tgatcgttgg gaaccggagc tgaatgaagc 600
   cataccaaac gacgagcgtg acaccacgat gcctgtagca atggcaacaa cgttgcgcaa 660
   actattaact ggcgaactac ttactctagc ttcccggcaa caattaatag actggatgga 720
   ggcggataaa gttgcaggac cacttctgcg ctcggccctt ccggctggct ggtttattgc 780
   tgataaatct ggagccggtg agcgtgggtc tcgcggtatc attgcagcac tggggccaga 840
   tggtaagccc tcccgtatcg tagttatcta cacgacgggg agtcaggcaa ctatggatga 900
   acgaaataga cagatcgctg agataggtgc ctcactgatt aagcattggt aactgtcaga 960
   ccaagtttac tcatatatac tttagattga tttaaaactt catttttaat ttaaaaggat 1020
   ctaggtgaag atcctttttg ataatctcat gaccaaaatc ccttaacgtg agttttcgtt 1080
   ccactgagcg tcagaccccg tagaaaagat caaaggatct tcttgagatc ctttttttct 1140
   gcgcgtaatc tgctgcttgc aaacaaaaaa accaccgcta ccagcggtgg tttgtttgcc 1200
   ggatcaagag ctaccaactc tttttccgaa ggtaactggc ttcagcagag cgcagatacc 1260
   aaatactgtc cttctagtgt agccgtagtt aggccaccac ttcaagaact ctgtagcacc 1320
   gcctacatac ctcgctctgc taatcctgtt accagtggct gctgccagtg gcgataagtc 1380
   gtgtcttacc gggttggact caagacgata gttaccggat aaggcgcagc ggtcgggctg 1440
   aacggggggt tcgtgcacac agcccagctt ggagcgaacg acctacaccg aactgagata 1500
   cctacagcgt gagcattgag aaagcgccac gcttcccgaa gggagaaagg cggacaggta 1560
   tccggtaagc ggcagggtcg gaacaggaga gcgcacgagg gagcttccag ggggaaacgc 1620
   ctggtatctt tatagtcctg tcgggtttcg ccacctctga cttgagcgtc gatttttgtg 1680
   atgctcgtca ggggggcgga gcctatggaa aaacgccagc aacgcggcct ttttacggtt 1740
   cctggccttt tgctggcctt ttgctcacat gttctttcct gcgttatccc ctgattctgt 1800
   ggataaccgt attaccgcct ttgagtgagc tgataccgct cgccgcagcc gaacgaccga 1860
   gcgcagcgag tcagtgagcg aggaagcgga agagcgcctg atgcggtatt ttctccttac 1920
   gcatctgtgc ggtatttcac accgcatata tggtgcactc tcagtacaat ctgctctgat 1980
   gccgcatagt taagccagta tacactccgc tatcgctacg tgactgggtc atggctgcgc 2040
   cccgacaccc gccaacaccc gctgacgcgc cctgacgggc ttgtctgctc ccggcatccg 2100
   cttacagaca agctgtgacc gtctccggga gctgcatgtg tcagaggttt tcaccgtcat   2160
   caccgaaacg cgcgaggcag ctgcggtaaa gctcatcagc gtggtcgtga agcgattcac   2220
   agatgtctgc ctgttcatcc gcgtccagct cgttgagttt ctccagaagc gttaatgtct   2280
   ggcttctgat aaagcgggcc atgttaaggg cggttttttc ctgtttggtc acttgatgcc   2340
   tccgtgtaag ggggaatttc tgttcatggg ggtaatgata ccgatgaaac gagagaggat   2400
   gctcacgata cgggttactg atgatgaaca tgcccggtta ctggaacgtt gtgagggtaa   2460
   acaactggcg gtatggatgc ggcgggacca gagaaaaatc actcagggtc aatgccagcg   2520
   cttcgttaat acagatgtag gtgttccaca gggtagccag cagcatcctg cgatgcagat   2580
   ccggaacata atggtgcagg gcgctgactt ccgcgtttcc agactttacg aaacacggaa   2640
   accgaagacc attcatgttg ttgctcaggt cgcagacgtt ttgcagcagc agtcgcttca   2700
   cgttcgctcg cgtatcggtg attcattctg ctaaccagta aggcaacccc gccagcctag   2760
   ccgggtcctc aacgacagga gcacgatcat gcgcacccgt ggccaggacc caacgctgcc   2820
   cgagatgcgc cgcgtgcggc tgctggagat ggcggacgcg atggatatgt tctgccaagg   2880
   gttggtttgc gcattcacag ttctccgcaa gaattgattg gctccaattc ttggagtggt   2940
   gaatccgtta gcgaggtgcc gccggcttcc attcaggtcg aggtggcccg gctccatgca   3000
   ccgcgacgca acgcggggag gcagacaagg tatagggcgg cgcctacaat ccatgccaac   3060
   ccgttccatg tgctcgccga ggcggcataa atcgccgtga cgatcagcgg tccagtgatc   3120
   gaagttaggc tggtaagagc cgcgagcgat ccttgaagct gtccctgatg gtcgtcatct   3180
   acctgcctgg acagcatggc ctgcaacgcg ggcatcccga tgccgccgga agcgagaaga   3240
   atcataatgg ggaaggccat ccagcctcgc gtcgcgaacg ccagcaagac gtagcccagc   3300
   gcgtcggcca gcttgcaatt cgcgctaact tacattaatt gcgttgcgct cactgcccgc   3360
   tttccagtcg ggaaacctgt cgtgccagct gcattaatga atcggccaac gcgcggggag   3420
   aggcggtttg cgtattgggc gccagggtgg tttttctttt caccagtgag acgggcaaca   3480
   gctgattgcc cttcaccgcc tggccctgag agagttgcag caagcggtcc acgctggttt   3540
   gccccagcag gcgaaaatcc tgtttgatgg tggttgacgg cgggatataa catgagctgt   3600
   cttcggtatc gtcgtatccc actaccgaga tatccgcacc aacgcgcagc ccggactcgg   3660
   taatggcgcg cattgcgccc agcgccatct gatcgttggc aaccagcatc gcagtgggaa   3720
   cgatgccctc attcagcatt tgcatggttt gttgaaaacc ggacatggca ctccagtcgc   3780
   cttcccgttc cgctatcggc tgaatttgat tgcgagtgag atatttatgc cagccagcca   3840
   gacgcagacg cgccgagaca gaacttaatg gtcccgctaa cagcgcgatt tgctgatgac   3900
   ccaatgcgac cagatgctcc acgcccagtc gcgtaccgtc ttcatgggag aaaataatac   3960
   tgttgatggg tgtctggtca gagacatcaa gaaataacgc cggaacatta gtgcaggcag   4020
   cttccacagc aatggcatcc tggtcatcca gcggatagtt aatgatcagc ccactgacgc   4080
   gttgcgcgag aagattgtgc accgccgctt tacaggcttc gacgccgctt cgttctacca   4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggccca ggtcaccgtc tcctcaggta ccgtggctgc 4920
   accatctgtc ttcatcttcc cgccatctga tgagcagttg aaatctggaa ctgcctctgt 4980
   tgtgtgcctg ctgaataact tctatcccag agaggccaaa gtacagtgga aggtggataa 5040
   cgccctccaa tcgggtaact cccaggagag tgtcacagag caggacagca aggacagcac 5100
   ctacagcctc agcagcaccc tgacgctgag caaagcagac tacgagaaac acaaagtcta 5160
   cgcctgcgaa gtcacccatc agggcctgag ctcgccggtg acaaagagct tcaaccgcgg 5220
   agagtgttaa taaacaggaa acagaagtcc atatgaaata cctattgcct acggcagccg 5280
   ctggattgtt attactcgcg gcccagccgg ccatggccgc tagcaccaag ggcccatcgg 5340
   tcttccccct ggcaccctcc tccaagagca cctctggggg cacagcggcc ctgggctgcc 5400
   tggtcaagga ctacttcccc gaaccggtga cggtgtcgtg gaactcaggc gccctgacca 5460
   gcggcgtgca caccttcccg gctgtcctac agtcctcagg actctactcc ctcagcagcg 5520
   tagtgaccgt gccctccagc agcttgggca cccagaccta catctgcaac gtgaatcaca 5580
   agcccagcaa caccaaggtg gacaagaaag ttgagcccaa atcttgttaa gcttggctgt 5640
   tttggcggat gagagaagat tttcagcctg atacagatta aatcagaacg cagaagcggt 5700
   ctgataaaac agaatttgcc tggcggcagt agcgcggtgg tcccacctga ccccatgccg 5760
   aactcagaag tgaaacgccg tagcgccgat ggtagtgtgg ggtctcccca tgcgagagta 5820
   gggaactgcc aggcatcaaa taaaacgaaa ggctcagtcg aaagactggg cctttcgttt 5880
   tatctgttgt ttgtcggtga acgctctcct gagtaggaca aatccgccgg gagcggattt 5940
   gaacgttgcg aagcaacggc ccggaggacc ctggcgggca ggacgcccgc cataaactgc 6000
   caggcatcaa attaagcaga aggccatcct gacggatggc ctttttgcgt ttctacaaac 6060
   tctt 6064
<210> 101
   <211> 6094
   <212> DNA
   <213> Artificial sequence
<220>
   <223> Plasmide
<400> 101
   ttgtttattt ttctaaatac attcaaatat gtatccgctc atgagacaat aaccctgata     60
   aatgcttcaa taatattgaa aaaggaagag tatgagtatt caacatttcc gtgtcgccct    120
   tattcccttt tttgcggcat tttgccttcc tgtttttgct cacccagaaa cgctggtgaa    180
   agtaaaagat gctgaagatc agttgggtgc acgagtgggt tacatcgaac tggatctcaa    240
   cagcggtaag atccttgaga gttttcgccc cgaagaacgt tttccaatga tgagcacttt    300
   taaagttctg ctatgtggcg cggtattatc ccgtgttgac gccgggcaag agcaactcgg    360
   tcgccgcata cactattctc agaatgactt ggttgagtac tcaccagtca cagaaaagca    420
   tcttacggat ggcatgacag taagagaatt atgcagtgct gccataacca tgagtgataa    480
   cactgcggcc aacttacttc tgacaacgat cggaggaccg aaggagctaa ccgctttttt    540
   gcacaacatg ggggatcatg taactcgcct tgatcgttgg gaaccggagc tgaatgaagc    600
   cataccaaac gacgagcgtg acaccacgat gcctgtagca atggcaacaa cgttgcgcaa    660
   actattaact ggcgaactac ttactctagc ttcccggcaa caattaatag actggatgga    720
   ggcggataaa gttgcaggac cacttctgcg ctcggccctt ccggctggct ggtttattgc    780
   tgataaatct ggagccggtg agcgtgggtc tcgcggtatc attgcagcac tggggccaga    840
   tggtaagccc tcccgtatcg tagttatcta cacgacgggg agtcaggcaa ctatggatga    900
   acgaaataga cagatcgctg agataggtgc ctcactgatt aagcattggt aactgtcaga    960
   ccaagtttac tcatatatac tttagattga tttaaaactt catttttaat ttaaaaggat   1020
   ctaggtgaag atcctttttg ataatctcat gaccaaaatc ccttaacgtg agttttcgtt   1080
   ccactgagcg tcagaccccg tagaaaagat caaaggatct tcttgagatc ctttttttct    1140
   gcgcgtaatc tgctgcttgc aaacaaaaaa accaccgcta ccagcggtgg tttgtttgcc   1200
   ggatcaagag ctaccaactc tttttccgaa ggtaactggc ttcagcagag cgcagatacc   1260
   aaatactgtc cttctagtgt agccgtagtt aggccaccac ttcaagaact ctgtagcacc   1320
   gcctacatac ctcgctctgc taatcctgtt accagtggct gctgccagtg gcgataagtc   1380
   gtgtcttacc gggttggact caagacgata gttaccggat aaggcgcagc ggtcgggctg   1440
   aacggggggt tcgtgcacac agcccagctt ggagcgaacg acctacaccg aactgagata   1500
   cctacagcgt gagcattgag aaagcgccac gcttcccgaa gggagaaagg cggacaggta   1560
   tccggtaagc ggcagggtcg gaacaggaga gcgcacgagg gagcttccag ggggaaacgc   1620
   ctggtatctt tatagtcctg tcgggtttcg ccacctctga cttgagcgtc gatttttgtg 1680
   atgctcgtca ggggggcgga gcctatggaa aaacgccagc aacgcggcct ttttacggtt 1740
   cctggccttt tgctggcctt ttgctcacat gttctttcct gcgttatccc ctgattctgt 1800
   ggataaccgt attaccgcct ttgagtgagc tgataccgct cgccgcagcc gaacgaccga 1860
   gcgcagcgag tcagtgagcg aggaagcgga agagcgcctg atgcggtatt ttctccttac 1920
   gcatctgtgc ggtatttcac accgcatata tggtgcactc tcagtacaat ctgctctgat 1980
   gccgcatagt taagccagta tacactccgc tatcgctacg tgactgggtc atggctgcgc 2040
   cccgacaccc gccaacaccc gctgacgcgc cctgacgggc ttgtctgctc ccggcatccg 2100
   cttacagaca agctgtgacc gtctccggga gctgcatgtg tcagaggttt tcaccgtcat 2160
   caccgaaacg cgcgaggcag ctgcggtaaa gctcatcagc gtggtcgtga agcgattcac 2220
   agatgtctgc ctgttcatcc gcgtccagct cgttgagttt ctccagaagc gttaatgtct 2280
   ggcttctgat aaagcgggcc atgttaaggg cggttttttc ctgtttggtc acttgatgcc 2340
   tccgtgtaag ggggaatttc tgttcatggg ggtaatgata ccgatgaaac gagagaggat 2400
   gctcacgata cgggttactg atgatgaaca tgcccggtta ctggaacgtt gtgagggtaa 2460
   acaactggcg gtatggatgc ggcgggacca gagaaaaatc actcagggtc aatgccagcg 2520
   cttcgttaat acagatgtag gtgttccaca gggtagccag cagcatcctg cgatgcagat 2580
   ccggaacata atggtgcagg gcgctgactt ccgcgtttcc agactttacg aaacacggaa 2640
   accgaagacc attcatgttg ttgctcaggt cgcagacgtt ttgcagcagc agtcgcttca 2700
   cgttcgctcg cgtatcggtg attcattctg ctaaccagta aggcaacccc gccagcctag 2760
   ccgggtcctc aacgacagga gcacgatcat gcgcacccgt ggccaggacc caacgctgcc 2820
   cgagatgcgc cgcgtgcggc tgctggagat ggcggacgcg atggatatgt tctgccaagg 2880
   gttggtttgc gcattcacag ttctccgcaa gaattgattg gctccaattc ttggagtggt 2940
   gaatccgtta gcgaggtgcc gccggcttcc attcaggtcg aggtggcccg gctccatgca 3000
   ccgcgacgca acgcggggag gcagacaagg tatagggcgg cgcctacaat ccatgccaac 3060
   ccgttccatg tgctcgccga ggcggcataa atcgccgtga cgatcagcgg tccagtgatc 3120
   gaagttaggc tggtaagagc cgcgagcgat ccttgaagct gtccctgatg gtcgtcatct 3180
   acctgcctgg acagcatggc ctgcaacgcg ggcatcccga tgccgccgga agcgagaaga 3240
   atcataatgg ggaaggccat ccagcctcgc gtcgcgaacg ccagcaagac gtagcccagc 3300
   gcgtcggcca gcttgcaatt cgcgctaact tacattaatt gcgttgcgct cactgcccgc 3360
   tttccagtcg ggaaacctgt cgtgccagct gcattaatga atcggccaac gcgcggggag 3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg gtcccgctaa cagcgcgatt tgctgatgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggccca ggtcaccgtc tcctcaggta ccgtggctgc 4920
   accatctgtc ttcatcttcc cgccatctga tgagcagttg aaatctggaa ctgcctctgt 4980
   tgtgtgcctg ctgaataact tctatcccag agaggccaaa gtacagtgga aggtggataa 5040
   cgccctccaa tcgggtaact cccaggagag tgtcacagag caggacagca aggacagcac 5100
   ctacagcctc agcagcaccc tgacgctgag caaagcagac tacgagaaac acaaagtcta 5160
   cgcctgcgaa gtcacccatc agggcctgag ctcgccggtg acaaagagct tcaaccgcgg 5220
   agagtgttaa taaacaggaa acagaagtcc atatgaaata cctattgcct acggcagccg 5280
   ctggattgtt attactcgcg gcccagccgg ccatggccgc tagcaccaag ggcccatcgg 5340
   tcttccccct ggcaccctcc tccaagagca cctctggggg cacagcggcc ctgggctgcc 5400
   tggtcaagga ctacttcccc gaaccggtga cggtgtcgtg gaactcaggc gccctgacca 5460
   gcggcgtgca caccttcccg gctgtcctac agtcctcagg actctactcc ctcagcagcg 5520
   tagtgaccgt gccctccagc agcttgggca cccagaccta catctgcaac gtgaatcaca 5580
   agcccagcaa caccaaggtg gacaagaaag ttgagcccaa atcttgtgac aaaactcaca 5640
   catgcccacc gtgcccataa gcttggctgt tttggcggat gagagaagat tttcagcctg 5700
   atacagatta aatcagaacg cagaagcggt ctgataaaac agaatttgcc tggcggcagt 5760
   agcgcggtgg tcccacctga ccccatgccg aactcagaag tgaaacgccg tagcgccgat 5820
   ggtagtgtgg ggtctcccca tgcgagagta gggaactgcc aggcatcaaa taaaacgaaa 5880
   ggctcagtcg aaagactggg cctttcgttt tatctgttgt ttgtcggtga acgctctcct 5940
   gagtaggaca aatccgccgg gagcggattt gaacgttgcg aagcaacggc ccggaggacc 6000
   ctggcgggca ggacgcccgc cataaactgc caggcatcaa attaagcaga aggccatcct 6060
   gacggatggc ctttttgcgt ttctacaaac tctt 6094
<210> 102
   <211> 6745
   <212> DNA
   <213> Artificial sequence
<220>
   <223> Plasmide
<400> 102
   ttgtttattt ttctaaatac attcaaatat gtatccgctc atgagacaat aaccctgata 60
   aatgcttcaa taatattgaa aaaggaagag tatgagtatt caacatttcc gtgtcgccct 120
   tattcccttt tttgcggcat tttgccttcc tgtttttgct cacccagaaa cgctggtgaa 180
   agtaaaagat gctgaagatc agttgggtgc acgagtgggt tacatcgaac tggatctcaa 240
   cagcggtaag atccttgaga gttttcgccc cgaagaacgt tttccaatga tgagcacttt 300
   taaagttctg ctatgtggcg cggtattatc ccgtgttgac gccgggcaag agcaactcgg 360
   tcgccgcata cactattctc agaatgactt ggttgagtac tcaccagtca cagaaaagca 420
   tcttacggat ggcatgacag taagagaatt atgcagtgct gccataacca tgagtgataa 480
   cactgcggcc aacttacttc tgacaacgat cggaggaccg aaggagctaa ccgctttttt 540
   gcacaacatg ggggatcatg taactcgcct tgatcgttgg gaaccggagc tgaatgaagc    600
   cataccaaac gacgagcgtg acaccacgat gcctgtagca atggcaacaa cgttgcgcaa    660
   actattaact ggcgaactac ttactctagc ttcccggcaa caattaatag actggatgga 720
   ggcggataaa gttgcaggac cacttctgcg ctcggccctt ccggctggct ggtttattgc 780
   tgataaatct ggagccggtg agcgtgggtc tcgcggtatc attgcagcac tggggccaga 840
   tggtaagccc tcccgtatcg tagttatcta cacgacgggg agtcaggcaa ctatggatga 900
   acgaaataga cagatcgctg agataggtgc ctcactgatt aagcattggt aactgtcaga 960
   ccaagtttac tcatatatac tttagattga tttaaaactt catttttaat ttaaaaggat 1020
   ctaggtgaag atcctttttg ataatctcat gaccaaaatc ccttaacgtg agttttcgtt 1080
   ccactgagcg tcagaccccg tagaaaagat caaaggatct tcttgagatc ctttttttct 1140
   gcgcgtaatc tgctgcttgc aaacaaaaaa accaccgcta ccagcggtgg tttgtttgcc 1200
   ggatcaagag ctaccaactc tttttccgaa ggtaactggc ttcagcagag cgcagatacc 1260
   aaatactgtc cttctagtgt agccgtagtt aggccaccac ttcaagaact ctgtagcacc 1320
   gcctacatac ctcgctctgc taatcctgtt accagtggct gctgccagtg gcgataagtc 1380
   gtgtcttacc gggttggact caagacgata gttaccggat aaggcgcagc ggtcgggctg 1440
   aacggggggt tcgtgcacac agcccagctt ggagcgaacg acctacaccg aactgagata 1500
   cctacagcgt gagcattgag aaagcgccac gcttcccgaa gggagaaagg cggacaggta 1560
   tccggtaagc ggcagggtcg gaacaggaga gcgcacgagg gagcttccag ggggaaacgc 1620
   ctggtatctt tatagtcctg tcgggtttcg ccacctctga cttgagcgtc gatttttgtg 1680
   atgctcgtca ggggggcgga gcctatggaa aaacgccagc aacgcggcct ttttacggtt 1740
   cctggccttt tgctggcctt ttgctcacat gttctttcct gcgttatccc ctgattctgt 1800
   ggataaccgt attaccgcct ttgagtgagc tgataccgct cgccgcagcc gaacgaccga 1860
   gcgcagcgag tcagtgagcg aggaagcgga agagcgcctg atgcggtatt ttctccttac 1920
   gcatctgtgc ggtatttcac accgcatata tggtgcactc tcagtacaat ctgctctgat 1980
   gccgcatagt taagccagta tacactccgc tatcgctacg tgactgggtc atggctgcgc 2040
   cccgacaccc gccaacaccc gctgacgcgc cctgacgggc ttgtctgctc ccggcatccg 2100
   cttacagaca agctgtgacc gtctccggga gctgcatgtg tcagaggttt tcaccgtcat 2160
   caccgaaacg cgcgaggcag ctgcggtaaa gctcatcagc gtggtcgtga agcgattcac 2220
   agatgtctgc ctgttcatcc gcgtccagct cgttgagttt ctccagaagc gttaatgtct 2280
   ggcttctgat aaagcgggcc atgttaaggg cggttttttc ctgtttggtc acttgatgcc 2340
   tccgtgtaag ggggaatttc tgttcatggg ggtaatgata ccgatgaaac gagagaggat 2400
   gctcacgata cgggttactg atgatgaaca tgcccggtta ctggaacgtt gtgagggtaa 2460
   acaactggcg gtatggatgc ggcgggacca gagaaaaatc actcagggtc aatgccagcg 2520
   cttcgttaat acagatgtag gtgttccaca gggtagccag cagcatcctg cgatgcagat 2580
   ccggaacata atggtgcagg gcgctgactt ccgcgtttcc agactttacg aaacacggaa 2640
   accgaagacc attcatgttg ttgctcaggt cgcagacgtt ttgcagcagc agtcgcttca 2700
   cgttcgctcg cgtatcggtg attcattctg ctaaccagta aggcaacccc gccagcctag 2760
   ccgggtcctc aacgacagga gcacgatcat gcgcacccgt ggccaggacc caacgctgcc 2820
   cgagatgcgc cgcgtgcggc tgctggagat ggcggacgcg atggatatgt tctgccaagg 2880
   gttggtttgc gcattcacag ttctccgcaa gaattgattg gctccaattc ttggagtggt 2940
   gaatccgtta gcgaggtgcc gccggcttcc attcaggtcg aggtggcccg gctccatgca 3000
   ccgcgacgca acgcggggag gcagacaagg tatagggcgg cgcctacaat ccatgccaac 3060
   ccgttccatg tgctcgccga ggcggcataa atcgccgtga cgatcagcgg tccagtgatc 3120
   gaagttaggc tggtaagagc cgcgagcgat ccttgaagct gtccctgatg gtcgtcatct 3180
   acctgcctgg acagcatggc ctgcaacgcg ggcatcccga tgccgccgga agcgagaaga 3240
   atcataatgg ggaaggccat ccagcctcgc gtcgcgaacg ccagcaagac gtagcccagc 3300
   gcgtcggcca gcttgcaatt cgcgctaact tacattaatt gcgttgcgct cactgcccgc 3360
   tttccagtcg ggaaacctgt cgtgccagct gcattaatga atcggccaac gcgcggggag 3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg gtcccgctaa cagcgcgatt tgctgatgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggccca ggtcaccgtc tcctcaggta ccgtggctgc 4920
   accatctgtc ttcatcttcc cgccatctga tgagcagttg aaatctggaa ctgcctctgt 4980
   tgtgtgcctg ctgaataact tctatcccag agaggccaaa gtacagtgga aggtggataa 5040
   cgccctccaa tcgggtaact cccaggagag tgtcacagag caggacagca aggacagcac 5100
   ctacagcctc agcagcaccc tgacgctgag caaagcagac tacgagaaac acaaagtcta 5160
   cgcctgcgaa gtcacccatc agggcctgag ctcgccggtg acaaagagct tcaaccgcgg 5220
   agagtgttaa taaacaggaa acagaagtcc atatgaaata cctattgcct acggcagccg   5280
   ctggattgtt attactcgcg gcccagccgg ccatggccgc tagcaccaag ggcccatcgg   5340
   tcttccccct ggcaccctcc tccaagagca cctctggggg cacagcggcc ctgggctgcc   5400
   tggtcaagga ctacttcccc gaaccggtga cggtgtcgtg gaactcaggc gccctgacca   5460
   gcggcgtgca caccttcccg gctgtcctac agtcctcagg actctactcc ctcagcagcg   5520
   tggtgaccgt gccctccagc agcttgggca cccagaccta catctgcaac gtgaatcaca   5580
   agcccagcaa caccaaggtg gacaagaaag ttgagcccaa atcttgtgac aaaactcaca   5640
   catgcccacc gtgcccagca cctgaactcc tggggggacc gtcagtcttc ctcttccccc   5700
   caaaacccaa ggacaccctc atgatctccc ggacccctga ggtcacatgc gtggtggtgg   5760
   acgtgagcca cgaagaccct gaggtcaagt tcaactggta cgtggacggc gtggaggtgc   5820
   ataatgccaa gacaaagccg cgggaggagc agtacaacag cacgtaccgg gtggtcagcg   5880
   tcctcaccgt cctgcaccag gactggctga atggcaagga gtacaagtgc aaggtctcca   5940
   acaaagccct cccagccccc atcgagaaaa ccatctccaa agccaaaggg cagccccgag   6000
   aaccacaggt gtacaccctg cccccatccc gggatgagct gaccaagaac caggtcagcc   6060
   tgacctgcct ggtcaaaggc ttctatccca gcgacatcgc cgtggagtgg gagagcaatg   6120
   ggcagccgga gaacaactac aagaccacgc ctcccgtgct ggactccgac ggctccttct   6180
   tcctctacag caagctcacc gtggacaaga gcaggtggca gcaggggaac gtcttctcat   6240
   gctccgtgat gcatgaggct ctgcacaacc actacacgca gaagagcctc tccctgtctc   6300
   cgggtaaata agcttggctg ttttggcgga tgagagaaga ttttcagcct gatacagatt   6360
   aaatcagaac gcagaagcgg tctgataaaa cagaatttgc ctggcggcag tagcgcggtg   6420
   gtcccacctg accccatgcc gaactcagaa gtgaaacgcc gtagcgccga tggtagtgtg   6480
   gggtctcccc atgcgagagt agggaactgc caggcatcaa ataaaacgaa aggctcagtc   6540
   gaaagactgg gcctttcgtt ttatctgttg tttgtcggtg aacgctctcc tgagtaggac   6600
   aaatccgccg ggagcggatt tgaacgttgc gaagcaacgg cccggaggac cctggcgggc   6660
   aggacgcccg ccataaactg ccaggcatca aattaagcag aaggccatcc tgacggatgg   6720
   cctttttgcg tttctacaaa ctctt                                         6745
<210> 103
   <211> 126
   <212> PRT
   <213> Artificial sequence
<220>
   <223> Amino acid
<220>
   <221> MISC_FEATURE
   <222> (35)..(37)
   <223> unknown amino acid
<220>
   <221> MISC_FEATURE
   <222> (55)..(57)
   <223> Unknown amino acid
<220>
   <221> MISC_FEATURE
   <222> (109)..(113)
   <223> unknown amino acid
<400> 103
   Glu Val Gln Leu Val Glu Ser Gly Gly Gly Leu Val Gln Pro Gly Gly
   1               5                   10                  15
   Ser Leu Arg Leu Ser Cys Ser Phe Pro Gly Ser Ile Phe Ser Leu Thr
               20                  25                  30
   Met Gly Xaa Xaa Xaa Trp Tyr Arg Gln Ala Pro Gly Lys Glu Arg Glu
           35                  40                  45
   Leu Val Thr Ser Ala Thr Xaa Xaa Xaa Pro Gly Gly Asp Thr Asn Tyr
       50                  55                  60
   Ala Asp Phe Val Lys Gly Arg Phe Thr Ile Ser Arg Asp Asn Ala Arg
   65                  70                  75                  80
   Ser Ile Ile Tyr Leu Gln Met Asn Ser Leu Lys Pro Glu Asp Thr Ala
                   85                  90                  95
   Val Tyr Tyr Cys Tyr Ala Arg Thr Arg Asn Trp Gly Xaa Xaa Xaa Xaa
               100                 105                 110
   Xaa Thr Val Trp Gly Gln Gly Thr Gln Val Thr Val Ser Ser
           115                 120                 125
<210> 104
   <211> 126
   <212> PRT
   <213> Artificial sequence
<220>
   <223> Amino acid
<220>
   <221> MISC_FEATURE
   <222> (36)..(37)
   <223> unknown amino acid
<220>
   <221> MISC_FEATURE
   <222> (56)..(57)
   <223> unknown amino acid
<400> 104
   Glu Val Gln Leu Val Glu Ser Gly Gly Glu Leu Val Gln Ala Gly Gly
   1 5 10 15
   Ser Leu Arg Leu Ser Cys Ala Ala Ser Gly Leu Thr Phe Ser Ser Tyr
   20 25 30
   Asn Met Gly Xaa Xaa Trp Phe Arg Arg Ala Pro Gly Lys Glu Arg Glu
   35 40 45
   Phe Val Ala Ser Ile Thr Trp Xaa Xaa Ser Gly Arg Asp Thr Phe Tyr
   50 55 60
   Ala Asp Ser Val Lys Gly Arg Phe Thr Ile Ser Arg Asp Asn Ala Lys
   65 70 75 80
   Asn Thr Val Tyr Leu Gln Met Ser Ser Leu Lys Pro Glu Asp Thr Ala
   85 90 95
   Val Tyr Tyr Cys Ala Ala Asn Pro Trp Pro Val Ala Ala Pro Arg Ser
   100 105 110
   Gly Thr Tyr Trp Gly Gln Gly Thr Gln Val Thr Val Ser Ser
   115 120 125
<210> 105
   <211> 126
   <212> PRT
   <213> Artificial sequence
<220>
   <223> Amino acid
<220>
   <221> MISC_FEATURE
   <222> (36)..(37)
   <223> unknown amino acid
<220>
   <221> MISC_FEATURE
   <222> (57)..(57)
   <223> unknown amino acid
<220>
   <221> MISC_FEATURE
   <222> (111)..(113)
   <223> Unknown amino acid
<400> 105
   Glu Val Gln Leu Val Glu Ser Gly Gly Gly Leu Val Gln Ala Gly Gly
   1               5                   10                  15
   Ser Leu Thr Leu Ser Cys Thr Ser Ser Thr Leu Thr Phe Thr Pro Tyr
               20                  25                  30
   Arg Met Gly Xaa Xaa Trp Tyr Arg Gln Thr Pro Gly Lys Gln Arg Asp
           35                  40                  45
   Leu Val Ala Asp Ile Ser Pro Gly Xaa Asp Gly Ser Thr Lys Asn Tyr
       50                  55                  60
   Ala Gly Phe Ala Gln Gly Arg Phe Thr Ile Ser Arg Asp Asn Ile Lys
   65                  70                  75                  80
   Asn Thr Val Tyr Leu Gln Met Asn Asp Leu Lys Pro Glu Asp Thr Ala
                   85                  90                  95
   Val Tyr Tyr Cys Asn Thr Tyr Val Ala Phe Val Gly Arg Ala Xaa Xaa
               100                 105                 110
   Xaa Arg Thr Trp Gly Gln Gly Thr Gln Val Thr Val Thr Ser
           115                 120                 125
<210> 106
   <211> 345
   <212> DNA
   <213> Artificial sequence
<220>
   <223> Nucleotide
<400> 106
   gaggtgcagc tggtggagtc tgggggaggc ttggtgcagc ctggggggtc tctgagactc 60
   tcctgttcat tccctggaag catcttcagt ctcaccatgg gctggtaccg tcaggctcca 120
   gggaaggagc gcgagttggt cacaagtgct actcctggtg gtgacacaaa ctatgcagac 180
   ttcgtgaagg gccgattcac catctccaga gacaacgcca ggagcatcat atatctacaa 240
   atgaatagcc tgaaacctga ggacacggcc gtctattatt gttatgcacg tacgaggaat 300
   tggggtacgg tctggggcca ggggacccag gtcaccgtct cctca 345
<210> 107
   <211> 366
   <212> DNA
   <213> Artificial sequence
<220>
   <223> Nucleotide
<400> 107
   gaggtgcagc tggtggagtc tgggggagag ttggtgcagg ctgggggctc tctgagactc     60
   tcctgtgcag cctctggcct caccttcagt agctataaca tgggctggtt ccgccgggct    120
   ccagggaagg agcgtgagtt tgtagcatct attacctgga gtggtcggga cacattctat    180
   gcagactccg tgaagggccg attcaccatc tccagagaca acgccaagaa cactgtttat    240
   ctgcaaatga gcagcctgaa acctgaggac acggccgttt attattgtgc tgcaaacccc    300
   tggccagtgg cggcgccacg tagtggcacc tactggggcc aagggaccca ggtcaccgtc    360
   tcctca                                                                366
<210> 108
   <211> 360
   <212> DNA
   <213> Artificial sequence
<220>
   <223> Nucleotide
<400> 108
   gaggtgcagc tggtggagtc tgggggaggc ttggtgcagg cggggggctc tctgacactc     60
   tcctgcacaa gttctacact taccttcact ccgtatcgca tgggctggta ccgccagact    120
   ccagggaagc agcgtgattt ggtcgcggac attagtcctg gtgatggtag taccaaaaat    180
   tatgcaggct tcgcgcaggg ccgattcacc atctccagag acaacatcaa gaacacggtg    240
   tatctgcaaa tgaacgacct gaaacctgag gacacggccg tctattactg caacacctac    300
   gtcgcgtttg tggggcgtgc gcgtacttgg ggccagggga cccaggtcac tgtcacctca    360
<210> 109
   <211> 5500
   <212> DNA
   <213> Artificial sequence
<220>
   <223> Plasmide
<400> 109
   ttgtttattt ttctaaatac attcaaatat gtatccgctc atgagacaat aaccctgata 60
   aatgcttcaa taatattgaa aaaggaagag tatgagtatt caacatttcc gtgtcgccct 120
   tattcccttt tttgcggcat tttgccttcc tgtttttgct cacccagaaa cgctggtgaa 180
   agtaaaagat gctgaagatc agttgggtgc acgagtgggt tacatcgaac tggatctcaa 240
   cagcggtaag atccttgaga gttttcgccc cgaagaacgt tttccaatga tgagcacttt 300
   taaagttctg ctatgtggcg cggtattatc ccgtgttgac gccgggcaag agcaactcgg 360
   tcgccgcata cactattctc agaatgactt ggttgagtac tcaccagtca cagaaaagca 420
   tcttacggat ggcatgacag taagagaatt atgcagtgct gccataacca tgagtgataa 480
   cactgcggcc aacttacttc tgacaacgat cggaggaccg aaggagctaa ccgctttttt 540
   gcacaacatg ggggatcatg taactcgcct tgatcgttgg gaaccggagc tgaatgaagc 600
   cataccaaac gacgagcgtg acaccacgat gcctgtagca atggcaacaa cgttgcgcaa 660
   actattaact ggcgaactac ttactctagc ttcccggcaa caattaatag actggatgga 720
   ggcggataaa gttgcaggac cacttctgcg ctcggccctt ccggctggct ggtttattgc 780
   tgataaatct ggagccggtg agcgtgggtc tcgcggtatc attgcagcac tggggccaga 840
   tggtaagccc tcccgtatcg tagttatcta cacgacgggg agtcaggcaa ctatggatga 900
   acgaaataga cagatcgctg agataggtgc ctcactgatt aagcattggt aactgtcaga 960
   ccaagtttac tcatatatac tttagattga tttaaaactt catttttaat ttaaaaggat 1020
   ctaggtgaag atcctttttg ataatctcat gaccaaaatc ccttaacgtg agttttcgtt 1080
   ccactgagcg tcagaccccg tagaaaagat caaaggatct tcttgagatc ctttttttct 1140
   gcgcgtaatc tgctgcttgc aaacaaaaaa accaccgcta ccagcggtgg tttgtttgcc 1200
   ggatcaagag ctaccaactc tttttccgaa ggtaactggc ttcagcagag cgcagatacc 1260
   aaatactgtc cttctagtgt agccgtagtt aggccaccac ttcaagaact ctgtagcacc 1320
   gcctacatac ctcgctctgc taatcctgtt accagtggct gctgccagtg gcgataagtc 1380
   gtgtcttacc gggttggact caagacgata gttaccggat aaggcgcagc ggtcgggctg 1440
   aacggggggt tcgtgcacac agcccagctt ggagcgaacg acctacaccg aactgagata 1500
   cctacagcgt gagcattgag aaagcgccac gcttcccgaa gggagaaagg cggacaggta 1560
   tccggtaagc ggcagggtcg gaacaggaga gcgcacgagg gagcttccag ggggaaacgc 1620
   ctggtatctt tatagtcctg tcgggtttcg ccacctctga cttgagcgtc gatttttgtg 1680
   atgctcgtca ggggggcgga gcctatggaa aaacgccagc aacgcggcct ttttacggtt 1740
   cctggccttt tgctggcctt ttgctcacat gttctttcct gcgttatccc ctgattctgt 1800
   ggataaccgt attaccgcct ttgagtgagc tgataccgct cgccgcagcc gaacgaccga 1860
   gcgcagcgag tcagtgagcg aggaagcgga agagcgcctg atgcggtatt ttctccttac 1920
   gcatctgtgc ggtatttcac accgcatata tggtgcactc tcagtacaat ctgctctgat 1980
   gccgcatagt taagccagta tacactccgc tatcgctacg tgactgggtc atggctgcgc 2040
   cccgacaccc gccaacaccc gctgacgcgc cctgacgggc ttgtctgctc ccggcatccg 2100
   cttacagaca agctgtgacc gtctccggga gctgcatgtg tcagaggttt tcaccgtcat 2160
   caccgaaacg cgcgaggcag ctgcggtaaa gctcatcagc gtggtcgtga agcgattcac 2220
   agatgtctgc ctgttcatcc gcgtccagct cgttgagttt ctccagaagc gttaatgtct 2280
   ggcttctgat aaagcgggcc atgttaaggg cggttttttc ctgtttggtc acttgatgcc 2340
   tccgtgtaag ggggaatttc tgttcatggg ggtaatgata ccgatgaaac gagagaggat 2400
   gctcacgata cgggttactg atgatgaaca tgcccggtta ctggaacgtt gtgagggtaa 2460
   acaactggcg gtatggatgc ggcgggacca gagaaaaatc actcagggtc aatgccagcg 2520
   cttcgttaat acagatgtag gtgttccaca gggtagccag cagcatcctg cgatgcagat 2580
   ccggaacata atggtgcagg gcgctgactt ccgcgtttcc agactttacg aaacacggaa 2640
   accgaagacc attcatgttg ttgctcaggt cgcagacgtt ttgcagcagc agtcgcttca 2700
   cgttcgctcg cgtatcggtg attcattctg ctaaccagta aggcaacccc gccagcctag 2760
   ccgggtcctc aacgacagga gcacgatcat gcgcacccgt ggccaggacc caacgctgcc 2820
   cgagatgcgc cgcgtgcggc tgctggagat ggcggacgcg atggatatgt tctgccaagg 2880
   gttggtttgc gcattcacag ttctccgcaa gaattgattg gctccaattc ttggagtggt 2940
   gaatccgtta gcgaggtgcc gccggcttcc attcaggtcg aggtggcccg gctccatgca 3000
   ccgcgacgca acgcggggag gcagacaagg tatagggcgg cgcctacaat ccatgccaac 3060
   ccgttccatg tgctcgccga ggcggcataa atcgccgtga cgatcagcgg tccagtgatc 3120
   gaagttaggc tggtaagagc cgcgagcgat ccttgaagct gtccctgatg gtcgtcatct 3180
   acctgcctgg acagcatggc ctgcaacgcg ggcatcccga tgccgccgga agcgagaaga 3240
   atcataatgg ggaaggccat ccagcctcgc gtcgcgaacg ccagcaagac gtagcccagc 3300
   gcgtcggcca gcttgcaatt cgcgctaact tacattaatt gcgttgcgct cactgcccgc 3360
   tttccagtcg ggaaacctgt cgtgccagct gcattaatga atcggccaac gcgcggggag 3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg gtcccgctaa cagcgcgatt tgctgatgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggccca ggtcaccgtc tcctcaaacc gcggagagtg 4920
   tgcaggtgat tacaaagacg atgacgataa gtaataaaca ggaaacagaa gtccatatga 4980
   aatatctttt acctacggca gccgcaggtt tgttgttact cgcggcccag ccggccatgg   5040
   ccgctagcgc ggccgctcta gattaagctt ggctgttttg gcggatgaga gaagattttc   5100
   agcctgatac agattaaatc agaacgcaga agcggtctga taaaacagaa tttgcctggc   5160
   ggcagtagcg cggtggtccc acctgacccc atgccgaact cagaagtgaa acgccgtagc   5220
   gccgatggta gtgtggggtc tccccatgcg agagtaggga actgccaggc atcaaataaa   5280
   acgaaaggct cagtcgaaag actgggcctt tcgttttatc tgttgtttgt cggtgaacgc   5340
   tctcctgagt aggacaaatc cgccgggagc ggatttgaac gttgcgaagc aacggcccgg   5400
   aggaccctgg cgggcaggac gcccgccata aactgccagg catcaaatta agcagaaggc   5460
   catcctgacg gatggccttt ttgcgtttct acaaactctt                          5500
<210> 110
   <211> 5560
   <212> DNA
   <213> Artificial sequence
<220>
   <223> Plasmide
<400> 110
   ttgtttattt ttctaaatac attcaaatat gtatccgctc atgagacaat aaccctgata     60
   aatgcttcaa taatattgaa aaaggaagag tatgagtatt caacatttcc gtgtcgccct    120
   tattcccttt tttgcggcat tttgccttcc tgtttttgct cacccagaaa cgctggtgaa    180
   agtaaaagat gctgaagatc agttgggtgc acgagtgggt tacatcgaac tggatctcaa    240
   cagcggtaag atccttgaga gttttcgccc cgaagaacgt tttccaatga tgagcacttt    300
   taaagttctg ctatgtggcg cggtattatc ccgtgttgac gccgggcaag agcaactcgg    360
   tcgccgcata cactattctc agaatgactt ggttgagtac tcaccagtca cagaaaagca    420
   tcttacggat ggcatgacag taagagaatt atgcagtgct gccataacca tgagtgataa    480
   cactgcggcc aacttacttc tgacaacgat cggaggaccg aaggagctaa ccgctttttt    540
   gcacaacatg ggggatcatg taactcgcct tgatcgttgg gaaccggagc tgaatgaagc    600
   cataccaaac gacgagcgtg acaccacgat gcctgtagca atggcaacaa cgttgcgcaa    660
   actattaact ggcgaactac ttactctagc ttcccggcaa caattaatag actggatgga    720
   ggcggataaa gttgcaggac cacttctgcg ctcggccctt ccggctggct ggtttattgc    780
   tgataaatct ggagccggtg agcgtgggtc tcgcggtatc attgcagcac tggggccaga    840
   tggtaagccc tcccgtatcg tagttatcta cacgacgggg agtcaggcaa ctatggatga    900
   acgaaataga cagatcgctg agataggtgc ctcactgatt aagcattggt aactgtcaga    960
   ccaagtttac tcatatatac tttagattga tttaaaactt catttttaat ttaaaaggat   1020
   ctaggtgaag atcctttttg ataatctcat gaccaaaatc ccttaacgtg agttttcgtt 1080
   ccactgagcg tcagaccccg tagaaaagat caaaggatct tcttgagatc ctttttttct 1140
   gcgcgtaatc tgctgcttgc aaacaaaaaa accaccgcta ccagcggtgg tttgtttgcc 1200
   ggatcaagag ctaccaactc tttttccgaa ggtaactggc ttcagcagag cgcagatacc 1260
   aaatactgtc cttctagtgt agccgtagtt aggccaccac ttcaagaact ctgtagcacc 1320
   gcctacatac ctcgctctgc taatcctgtt accagtggct gctgccagtg gcgataagtc 1380
   gtgtcttacc gggttggact caagacgata gttaccggat aaggcgcagc ggtcgggctg 1440
   aacggggggt tcgtgcacac agcccagctt ggagcgaacg acctacaccg aactgagata 1500
   cctacagcgt gagcattgag aaagcgccac gcttcccgaa gggagaaagg cggacaggta 1560
   tccggtaagc ggcagggtcg gaacaggaga gcgcacgagg gagcttccag ggggaaacgc 1620
   ctggtatctt tatagtcctg tcgggtttcg ccacctctga cttgagcgtc gatttttgtg 1680
   atgctcgtca ggggggcgga gcctatggaa aaacgccagc aacgcggcct ttttacggtt 1740
   cctggccttt tgctggcctt ttgctcacat gttctttcct gcgttatccc ctgattctgt 1800
   ggataaccgt attaccgcct ttgagtgagc tgataccgct cgccgcagcc gaacgaccga 1860
   gcgcagcgag tcagtgagcg aggaagcgga agagcgcctg atgcggtatt ttctccttac 1920
   gcatctgtgc ggtatttcac accgcatata tggtgcactc tcagtacaat ctgctctgat 1980
   gccgcatagt taagccagta tacactccgc tatcgctacg tgactgggtc atggctgcgc 2040
   cccgacaccc gccaacaccc gctgacgcgc cctgacgggc ttgtctgctc ccggcatccg 2100
   cttacagaca agctgtgacc gtctccggga gctgcatgtg tcagaggttt tcaccgtcat 2160
   caccgaaacg cgcgaggcag ctgcggtaaa gctcatcagc gtggtcgtga agcgattcac 2220
   agatgtctgc ctgttcatcc gcgtccagct cgttgagttt ctccagaagc gttaatgtct 2280
   ggcttctgat aaagcgggcc atgttaaggg cggttttttc ctgtttggtc acttgatgcc 2340
   tccgtgtaag ggggaatttc tgttcatggg ggtaatgata ccgatgaaac gagagaggat 2400
   gctcacgata cgggttactg atgatgaaca tgcccggtta ctggaacgtt gtgagggtaa 2460
   acaactggcg gtatggatgc ggcgggacca gagaaaaatc actcagggtc aatgccagcg 2520
   cttcgttaat acagatgtag gtgttccaca gggtagccag cagcatcctg cgatgcagat 2580
   ccggaacata atggtgcagg gcgctgactt ccgcgtttcc agactttacg aaacacggaa 2640
   accgaagacc attcatgttg ttgctcaggt cgcagacgtt ttgcagcagc agtcgcttca 2700
   cgttcgctcg cgtatcggtg attcattctg ctaaccagta aggcaacccc gccagcctag 2760
   ccgggtcctc aacgacagga gcacgatcat gcgcacccgt ggccaggacc caacgctgcc 2820
   cgagatgcgc cgcgtgcggc tgctggagat ggcggacgcg atggatatgt tctgccaagg 2880
   gttggtttgc gcattcacag ttctccgcaa gaattgattg gctccaattc ttggagtggt 2940
   gaatccgtta gcgaggtgcc gccggcttcc attcaggtcg aggtggcccg gctccatgca 3000
   ccgcgacgca acgcggggag gcagacaagg tatagggcgg cgcctacaat ccatgccaac 3060
   ccgttccatg tgctcgccga ggcggcataa atcgccgtga cgatcagcgg tccagtgatc 3120
   gaagttaggc tggtaagagc cgcgagcgat ccttgaagct gtccctgatg gtcgtcatct 3180
   acctgcctgg acagcatggc ctgcaacgcg ggcatcccga tgccgccgga agcgagaaga 3240
   atcataatgg ggaaggccat ccagcctcgc gtcgcgaacg ccagcaagac gtagcccagc 3300
   gcgtcggcca gcttgcaatt cgcgctaact tacattaatt gcgttgcgct cactgcccgc 3360
   tttccagtcg ggaaacctgt cgtgccagct gcattaatga atcggccaac gcgcggggag 3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg gtcccgctaa cagcgcgatt tgctgatgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggccca ggtcaccgtc tcctcaaacc gcggagagtg 4920
   tgcaggtgat tacaaagacg atgacgataa gtaataaaca ggaaacagaa gtccatatga 4980
   aatatctttt acctacggca gccgcaggtt tgttgttact cgcggcccag ccggccatgg 5040
   ccgctagcgc ggccgcagaa caaaaactca tctcagaaga ggatctgaat ggggccgtac 5100
   atcaccacca tcatcatggg agctaagctt ggctgttttg gcggatgaga gaagattttc 5160
   agcctgatac agattaaatc agaacgcaga agcggtctga taaaacagaa tttgcctggc 5220
   ggcagtagcg cggtggtccc acctgacccc atgccgaact cagaagtgaa acgccgtagc 5280
   gccgatggta gtgtggggtc tccccatgcg agagtaggga actgccaggc atcaaataaa 5340
   acgaaaggct cagtcgaaag actgggcctt tcgttttatc tgttgtttgt cggtgaacgc 5400
   tctcctgagt aggacaaatc cgccgggagc ggatttgaac gttgcgaagc aacggcccgg 5460
   aggaccctgg cgggcaggac gcccgccata aactgccagg catcaaatta agcagaaggc 5520
   catcctgacg gatggccttt ttgcgtttct acaaactctt 5560
<210> 111
   <211> 5866
   <212> DNA
   <213> Artificial sequence
<220>
   <223> Plasmide
<400> 111
   ttgtttattt ttctaaatac attcaaatat gtatccgctc atgagacaat aaccctgata 60
   aatgcttcaa taatattgaa aaaggaagag tatgagtatt caacatttcc gtgtcgccct 120
   tattcccttt tttgcggcat tttgccttcc tgtttttgct cacccagaaa cgctggtgaa 180
   agtaaaagat gctgaagatc agttgggtgc acgagtgggt tacatcgaac tggatctcaa 240
   cagcggtaag atccttgaga gttttcgccc cgaagaacgt tttccaatga tgagcacttt 300
   taaagttctg ctatgtggcg cggtattatc ccgtgttgac gccgggcaag agcaactcgg 360
   tcgccgcata cactattctc agaatgactt ggttgagtac tcaccagtca cagaaaagca 420
   tcttacggat ggcatgacag taagagaatt atgcagtgct gccataacca tgagtgataa 480
   cactgcggcc aacttacttc tgacaacgat cggaggaccg aaggagctaa ccgctttttt 540
   gcacaacatg ggggatcatg taactcgcct tgatcgttgg gaaccggagc tgaatgaagc 600
   cataccaaac gacgagcgtg acaccacgat gcctgtagca atggcaacaa cgttgcgcaa 660
   actattaact ggcgaactac ttactctagc ttcccggcaa caattaatag actggatgga 720
   ggcggataaa gttgcaggac cacttctgcg ctcggccctt ccggctggct ggtttattgc 780
   tgataaatct ggagccggtg agcgtgggtc tcgcggtatc attgcagcac tggggccaga 840
   tggtaagccc tcccgtatcg tagttatcta cacgacgggg agtcaggcaa ctatggatga 900
   acgaaataga cagatcgctg agataggtgc ctcactgatt aagcattggt aactgtcaga 960
   ccaagtttac tcatatatac tttagattga tttaaaactt catttttaat ttaaaaggat 1020
   ctaggtgaag atcctttttg ataatctcat gaccaaaatc ccttaacgtg agttttcgtt 1080
   ccactgagcg tcagaccccg tagaaaagat caaaggatct tcttgagatc ctttttttct 1140
   gcgcgtaatc tgctgcttgc aaacaaaaaa accaccgcta ccagcggtgg tttgtttgcc 1200
   ggatcaagag ctaccaactc tttttccgaa ggtaactggc ttcagcagag cgcagatacc 1260
   aaatactgtc cttctagtgt agccgtagtt aggccaccac ttcaagaact ctgtagcacc 1320
   gcctacatac ctcgctctgc taatcctgtt accagtggct gctgccagtg gcgataagtc 1380
   gtgtcttacc gggttggact caagacgata gttaccggat aaggcgcagc ggtcgggctg 1440
   aacggggggt tcgtgcacac agcccagctt ggagcgaacg acctacaccg aactgagata 1500
   cctacagcgt gagcattgag aaagcgccac gcttcccgaa gggagaaagg cggacaggta 1560
   tccggtaagc ggcagggtcg gaacaggaga gcgcacgagg gagcttccag ggggaaacgc 1620
   ctggtatctt tatagtcctg tcgggtttcg ccacctctga cttgagcgtc gatttttgtg 1680
   atgctcgtca ggggggcgga gcctatggaa aaacgccagc aacgcggcct ttttacggtt 1740
   cctggccttt tgctggcctt ttgctcacat gttctttcct gcgttatccc ctgattctgt 1800
   ggataaccgt attaccgcct ttgagtgagc tgataccgct cgccgcagcc gaacgaccga 1860
   gcgcagcgag tcagtgagcg aggaagcgga agagcgcctg atgcggtatt ttctccttac 1920
   gcatctgtgc ggtatttcac accgcatata tggtgcactc tcagtacaat ctgctctgat 1980
   gccgcatagt taagccagta tacactccgc tatcgctacg tgactgggtc atggctgcgc 2040
   cccgacaccc gccaacaccc gctgacgcgc cctgacgggc ttgtctgctc ccggcatccg 2100
   cttacagaca agctgtgacc gtctccggga gctgcatgtg tcagaggttt tcaccgtcat 2160
   caccgaaacg cgcgaggcag ctgcggtaaa gctcatcagc gtggtcgtga agcgattcac 2220
   agatgtctgc ctgttcatcc gcgtccagct cgttgagttt ctccagaagc gttaatgtct 2280
   ggcttctgat aaagcgggcc atgttaaggg cggttttttc ctgtttggtc acttgatgcc 2340
   tccgtgtaag ggggaatttc tgttcatggg ggtaatgata ccgatgaaac gagagaggat 2400
   gctcacgata cgggttactg atgatgaaca tgcccggtta ctggaacgtt gtgagggtaa 2460
   acaactggcg gtatggatgc ggcgggacca gagaaaaatc actcagggtc aatgccagcg 2520
   cttcgttaat acagatgtag gtgttccaca gggtagccag cagcatcctg cgatgcagat 2580
   ccggaacata atggtgcagg gcgctgactt ccgcgtttcc agactttacg aaacacggaa 2640
   accgaagacc attcatgttg ttgctcaggt cgcagacgtt ttgcagcagc agtcgcttca 2700
   cgttcgctcg cgtatcggtg attcattctg ctaaccagta aggcaacccc gccagcctag 2760
   ccgggtcctc aacgacagga gcacgatcat gcgcacccgt ggccaggacc caacgctgcc 2820
   cgagatgcgc cgcgtgcggc tgctggagat ggcggacgcg atggatatgt tctgccaagg 2880
   gttggtttgc gcattcacag ttctccgcaa gaattgattg gctccaattc ttggagtggt 2940
   gaatccgtta gcgaggtgcc gccggcttcc attcaggtcg aggtggcccg gctccatgca 3000
   ccgcgacgca acgcggggag gcagacaagg tatagggcgg cgcctacaat ccatgccaac 3060
   ccgttccatg tgctcgccga ggcggcataa atcgccgtga cgatcagcgg tccagtgatc 3120
   gaagttaggc tggtaagagc cgcgagcgat ccttgaagct gtccctgatg gtcgtcatct 3180
   acctgcctgg acagcatggc ctgcaacgcg ggcatcccga tgccgccgga agcgagaaga 3240
   atcataatgg ggaaggccat ccagcctcgc gtcgcgaacg ccagcaagac gtagcccagc 3300
   gcgtcggcca gcttgcaatt cgcgctaact tacattaatt gcgttgcgct cactgcccgc 3360
   tttccagtcg ggaaacctgt cgtgccagct gcattaatga atcggccaac gcgcggggag 3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg gtcccgctaa cagcgcgatt tgctgatgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggccca ggtcaccgtc tcctcacgta cggtggctgc 4920
   accatctgtc ttcatcttcc cgccatctga tgagcagttg aaatctggaa ctgcctctgt 4980
   tgtgtgcctg ctgaataact tctatcccag agaggccaaa gtacagtgga aggtggataa 5040
   cgccctccaa tcgggtaact cccaggagag tgtcacagag caggacagca aggacagcac 5100
   ctacagcctc agcagcaccc tgacgctgag caaagcagac tacgagaaac acaaagtcta 5160
   cgcctgcgaa gtcacccatc agggcctgag ctcgccggtg acaaagagct tcaaccgcgg 5220
   agagtgtgca ggtgattaca aagacgatga cgataagtaa taaacaggaa acagaagtcc 5280
   atatgaaata tcttttacct acggcagccg caggtttgtt gttactcgcg gcccagccgg 5340
   ccatggccgc tagcgcggcc gcagaacaaa aactcatctc agaagaggat ctgaatgggg 5400
   ccgtacatca ccaccatcat catgggagct aagcttggct gttttggcgg atgagagaag 5460
   attttcagcc tgatacagat taaatcagaa cgcagaagcg gtctgataaa acagaatttg 5520
   cctggcggca gtagcgcggt ggtcccacct gaccccatgc cgaactcaga agtgaaacgc 5580
   cgtagcgccg atggtagtgt ggggtctccc catgcgagag tagggaactg ccaggcatca 5640
   aataaaacga aaggctcagt cgaaagactg ggcctttcgt tttatctgtt gtttgtcggt 5700
   gaacgctctc ctgagtagga caaatccgcc gggagcggat ttgaacgttg cgaagcaacg 5760
   gcccggagga ccctggcggg caggacgccc gccataaact gccaggcatc aaattaagca 5820
   gaaggccatc ctgacggatg gcctttttgc gtttctacaa actctt 5866
<210> 112
   <211> 6169
   <212> DNA
   <213> Artificial sequence
<220>
   <223> Plasmide
<400> 112
   ttgtttattt ttctaaatac attcaaatat gtatccgctc atgagacaat aaccctgata 60
   aatgcttcaa taatattgaa aaaggaagag tatgagtatt caacatttcc gtgtcgccct 120
   tattcccttt tttgcggcat tttgccttcc tgtttttgct cacccagaaa cgctggtgaa 180
   agtaaaagat gctgaagatc agttgggtgc acgagtgggt tacatcgaac tggatctcaa 240
   cagcggtaag atccttgaga gttttcgccc cgaagaacgt tttccaatga tgagcacttt 300
   taaagttctg ctatgtggcg cggtattatc ccgtgttgac gccgggcaag agcaactcgg 360
   tcgccgcata cactattctc agaatgactt ggttgagtac tcaccagtca cagaaaagca 420
   tcttacggat ggcatgacag taagagaatt atgcagtgct gccataacca tgagtgataa 480
   cactgcggcc aacttacttc tgacaacgat cggaggaccg aaggagctaa ccgctttttt 540
   gcacaacatg ggggatcatg taactcgcct tgatcgttgg gaaccggagc tgaatgaagc 600
   cataccaaac gacgagcgtg acaccacgat gcctgtagca atggcaacaa cgttgcgcaa 660
   actattaact ggcgaactac ttactctagc ttcccggcaa caattaatag actggatgga 720
   ggcggataaa gttgcaggac cacttctgcg ctcggccctt ccggctggct ggtttattgc 780
   tgataaatct ggagccggtg agcgtgggtc tcgcggtatc attgcagcac tggggccaga 840
   tggtaagccc tcccgtatcg tagttatcta cacgacgggg agtcaggcaa ctatggatga 900
   acgaaataga cagatcgctg agataggtgc ctcactgatt aagcattggt aactgtcaga 960
   ccaagtttac tcatatatac tttagattga tttaaaactt catttttaat ttaaaaggat 1020
   ctaggtgaag atcctttttg ataatctcat gaccaaaatc ccttaacgtg agttttcgtt 1080
   ccactgagcg tcagaccccg tagaaaagat caaaggatct tcttgagatc ctttttttct 1140
   gcgcgtaatc tgctgcttgc aaacaaaaaa accaccgcta ccagcggtgg tttgtttgcc 1200
   ggatcaagag ctaccaactc tttttccgaa ggtaactggc ttcagcagag cgcagatacc 1260
   aaatactgtc cttctagtgt agccgtagtt aggccaccac ttcaagaact ctgtagcacc 1320
   gcctacatac ctcgctctgc taatcctgtt accagtggct gctgccagtg gcgataagtc 1380
   gtgtcttacc gggttggact caagacgata gttaccggat aaggcgcagc ggtcgggctg 1440
   aacggggggt tcgtgcacac agcccagctt ggagcgaacg acctacaccg aactgagata 1500
   cctacagcgt gagcattgag aaagcgccac gcttcccgaa gggagaaagg cggacaggta 1560
   tccggtaagc ggcagggtcg gaacaggaga gcgcacgagg gagcttccag ggggaaacgc 1620
   ctggtatctt tatagtcctg tcgggtttcg ccacctctga cttgagcgtc gatttttgtg 1680
   atgctcgtca ggggggcgga gcctatggaa aaacgccagc aacgcggcct ttttacggtt 1740
   cctggccttt tgctggcctt ttgctcacat gttctttcct gcgttatccc ctgattctgt 1800
   ggataaccgt attaccgcct ttgagtgagc tgataccgct cgccgcagcc gaacgaccga 1860
   gcgcagcgag tcagtgagcg aggaagcgga agagcgcctg atgcggtatt ttctccttac 1920
   gcatctgtgc ggtatttcac accgcatata tggtgcactc tcagtacaat ctgctctgat 1980
   gccgcatagt taagccagta tacactccgc tatcgctacg tgactgggtc atggctgcgc 2040
   cccgacaccc gccaacaccc gctgacgcgc cctgacgggc ttgtctgctc ccggcatccg 2100
   cttacagaca agctgtgacc gtctccggga gctgcatgtg tcagaggttt tcaccgtcat 2160
   caccgaaacg cgcgaggcag ctgcggtaaa gctcatcagc gtggtcgtga agcgattcac 2220
   agatgtctgc ctgttcatcc gcgtccagct cgttgagttt ctccagaagc gttaatgtct 2280
   ggcttctgat aaagcgggcc atgttaaggg cggttttttc ctgtttggtc acttgatgcc 2340
   tccgtgtaag ggggaatttc tgttcatggg ggtaatgata ccgatgaaac gagagaggat 2400
   gctcacgata cgggttactg atgatgaaca tgcccggtta ctggaacgtt gtgagggtaa 2460
   acaactggcg gtatggatgc ggcgggacca gagaaaaatc actcagggtc aatgccagcg 2520
   cttcgttaat acagatgtag gtgttccaca gggtagccag cagcatcctg cgatgcagat 2580
   ccggaacata atggtgcagg gcgctgactt ccgcgtttcc agactttacg aaacacggaa 2640
   accgaagacc attcatgttg ttgctcaggt cgcagacgtt ttgcagcagc agtcgcttca 2700
   cgttcgctcg cgtatcggtg attcattctg ctaaccagta aggcaacccc gccagcctag 2760
   ccgggtcctc aacgacagga gcacgatcat gcgcacccgt ggccaggacc caacgctgcc 2820
   cgagatgcgc cgcgtgcggc tgctggagat ggcggacgcg atggatatgt tctgccaagg 2880
   gttggtttgc gcattcacag ttctccgcaa gaattgattg gctccaattc ttggagtggt   2940
   gaatccgtta gcgaggtgcc gccggcttcc attcaggtcg aggtggcccg gctccatgca   3000
   ccgcgacgca acgcggggag gcagacaagg tatagggcgg cgcctacaat ccatgccaac   3060
   ccgttccatg tgctcgccga ggcggcataa atcgccgtga cgatcagcgg tccagtgatc   3120
   gaagttaggc tggtaagagc cgcgagcgat ccttgaagct gtccctgatg gtcgtcatct   3180
   acctgcctgg acagcatggc ctgcaacgcg ggcatcccga tgccgccgga agcgagaaga   3240
   atcataatgg ggaaggccat ccagcctcgc gtcgcgaacg ccagcaagac gtagcccagc   3300
   gcgtcggcca gcttgcaatt cgcgctaact tacattaatt gcgttgcgct cactgcccgc   3360
   tttccagtcg ggaaacctgt cgtgccagct gcattaatga atcggccaac gcgcggggag   3420
   aggcggtttg cgtattgggc gccagggtgg tttttctttt caccagtgag acgggcaaca   3480
   gctgattgcc cttcaccgcc tggccctgag agagttgcag caagcggtcc acgctggttt   3540
   gccccagcag gcgaaaatcc tgtttgatgg tggttgacgg cgggatataa catgagctgt   3600
   cttcggtatc gtcgtatccc actaccgaga tatccgcacc aacgcgcagc ccggactcgg   3660
   taatggcgcg cattgcgccc agcgccatct gatcgttggc aaccagcatc gcagtgggaa   3720
   cgatgccctc attcagcatt tgcatggttt gttgaaaacc ggacatggca ctccagtcgc   3780
   cttcccgttc cgctatcggc tgaatttgat tgcgagtgag atatttatgc cagccagcca   3840
   gacgcagacg cgccgagaca gaacttaatg gtcccgctaa cagcgcgatt tgctgatgac   3900
   ccaatgcgac cagatgctcc acgcccagtc gcgtaccgtc ttcatgggag aaaataatac   3960
   tgttgatggg tgtctggtca gagacatcaa gaaataacgc cggaacatta gtgcaggcag   4020
   cttccacagc aatggcatcc tggtcatcca gcggatagtt aatgatcagc ccactgacgc   4080
   gttgcgcgag aagattgtgc accgccgctt tacaggcttc gacgccgctt cgttctacca   4140
   tcgacaccac cacgctggca cccagttgat cggcgcgaga tttaatcgcc gcgacaattt   4200
   gcgacggcgc gtgcagggcc agactggagg tggcaacgcc aatcagcaac gactgtttgc   4260
   ccgccagttg ttgtgccacg cggttgggaa tgtaattcag ctccgccatc gccgcttcca   4320
   ctttttcccg cgttttcgca gaaacgtggc tggcctggtt caccacgcgg gaaacggtct   4380
   gataagagac accggcatac tctgcgacat cgtataacgt tactggtttc acattcacca   4440
   ccctgaattg actctcttcc gggcgctatc atgccatacc gcgaaaggtt ttgcgccatt   4500
   cgatggtgtc aacgtaaatg catgccgctt cgccttcgcg cgcgaattgc aagctgatcc   4560
   ggagcttatc gactgcacgg tgcaccaatg cttctggcgt caggcagcca tcggaagctg   4620
   tggtatggct gtgcaggtcg taaatcactg cataattcgt gtcgctcaag gcgcactccc   4680
   gttctggata atgttttttg cgccgacatc ataacggttc tggcaaatat tctgaaatga   4740
   gctgttgaca attaatcatc ggctcgtata atgtgtggaa ttgtgagcgg ataacaattt   4800
   cacacaggaa acagaattcc atatgaaata cctattacca acagcagcag ctgggttatt   4860
   attgctcgct gcgcagccgg ccatggccca ggtcaccgtc tcctcacgta cggtggctgc   4920
   accatctgtc ttcatcttcc cgccatctga tgagcagttg aaatctggaa ctgcctctgt 4980
   tgtgtgcctg ctgaataact tctatcccag agaggccaaa gtacagtgga aggtggataa 5040
   cgccctccaa tcgggtaact cccaggagag tgtcacagag caggacagca aggacagcac 5100
   ctacagcctc agcagcaccc tgacgctgag caaagcagac tacgagaaac acaaagtcta 5160
   cgcctgcgaa gtcacccatc agggcctgag ctcgccggtg acaaagagct tcaaccgcgg 5220
   agagtgtgca ggtgattaca aagacgatga cgataagtaa taaacaggaa acagaagtcc 5280
   atatgaaata tcttttacct acggcagccg caggtttgtt gttactcgcg gcccagccgg 5340
   ccatggccgc tagcaccaag ggcccatcgg tcttccccct ggcaccctcc tccaagagca 5400
   cctctggggg cacagcggcc ctgggctgcc tggtcaagga ctacttcccc gaaccggtga 5460
   cggtgtcgtg gaactcaggc gccctgacca gcggcgtgca caccttcccg gctgtcctac 5520
   agtcctcagg actctactcc ctcagcagcg tagtgaccgt gccctccagc agcttgggca 5580
   cccagaccta catctgcaac gtgaatcaca agcccagcaa caccaaggtg gacaagaaag 5640
   ttgagcccaa atcttgtgcg gccgcagaac aaaaactcat ctcagaagag gatctgaatg 5700
   gggccgtaca tcaccaccat catcatggga gctaagcttg gctgttttgg cggatgagag 5760
   aagattttca gcctgataca gattaaatca gaacgcagaa gcggtctgat aaaacagaat 5820
   ttgcctggcg gcagtagcgc ggtggtccca cctgacccca tgccgaactc agaagtgaaa 5880
   cgccgtagcg ccgatggtag tgtggggtct ccccatgcga gagtagggaa ctgccaggca 5940
   tcaaataaaa cgaaaggctc agtcgaaaga ctgggccttt cgttttatct gttgtttgtc 6000
   ggtgaacgct ctcctgagta ggacaaatcc gccgggagcg gatttgaacg ttgcgaagca 6060
   acggcccgga ggaccctggc gggcaggacg cccgccataa actgccaggc atcaaattaa 6120
   gcagaaggcc atcctgacgg atggcctttt tgcgtttcta caaactctt 6169
<210> 113
   <211> 6199
   <212> DNA
   <213> Artificial sequence
<220>
   <223> Plasmide
<400> 113
   ttgtttattt ttctaaatac attcaaatat gtatccgctc atgagacaat aaccctgata 60
   aatgcttcaa taatattgaa aaaggaagag tatgagtatt caacatttcc gtgtcgccct 120
   tattcccttt tttgcggcat tttgccttcc tgtttttgct cacccagaaa cgctggtgaa 180
   agtaaaagat gctgaagatc agttgggtgc acgagtgggt tacatcgaac tggatctcaa 240
   cagcggtaag atccttgaga gttttcgccc cgaagaacgt tttccaatga tgagcacttt 300
   taaagttctg ctatgtggcg cggtattatc ccgtgttgac gccgggcaag agcaactcgg 360
   tcgccgcata cactattctc agaatgactt ggttgagtac tcaccagtca cagaaaagca 420
   tcttacggat ggcatgacag taagagaatt atgcagtgct gccataacca tgagtgataa 480
   cactgcggcc aacttacttc tgacaacgat cggaggaccg aaggagctaa ccgctttttt 540
   gcacaacatg ggggatcatg taactcgcct tgatcgttgg gaaccggagc tgaatgaagc 600
   cataccaaac gacgagcgtg acaccacgat gcctgtagca atggcaacaa cgttgcgcaa 660
   actattaact ggcgaactac ttactctagc ttcccggcaa caattaatag actggatgga 720
   ggcggataaa gttgcaggac cacttctgcg ctcggccctt ccggctggct ggtttattgc 780
   tgataaatct ggagccggtg agcgtgggtc tcgcggtatc attgcagcac tggggccaga 840
   tggtaagccc tcccgtatcg tagttatcta cacgacgggg agtcaggcaa ctatggatga 900
   acgaaataga cagatcgctg agataggtgc ctcactgatt aagcattggt aactgtcaga 960
   ccaagtttac tcatatatac tttagattga tttaaaactt catttttaat ttaaaaggat 1020
   ctaggtgaag atcctttttg ataatctcat gaccaaaatc ccttaacgtg agttttcgtt 1080
   ccactgagcg tcagaccccg tagaaaagat caaaggatct tcttgagatc ctttttttct 1140
   gcgcgtaatc tgctgcttgc aaacaaaaaa accaccgcta ccagcggtgg tttgtttgcc 1200
   ggatcaagag ctaccaactc tttttccgaa ggtaactggc ttcagcagag cgcagatacc 1260
   aaatactgtc cttctagtgt agccgtagtt aggccaccac ttcaagaact ctgtagcacc 1320
   gcctacatac ctcgctctgc taatcctgtt accagtggct gctgccagtg gcgataagtc 1380
   gtgtcttacc gggttggact caagacgata gttaccggat aaggcgcagc ggtcgggctg 1440
   aacggggggt tcgtgcacac agcccagctt ggagcgaacg acctacaccg aactgagata 1500
   cctacagcgt gagcattgag aaagcgccac gcttcccgaa gggagaaagg cggacaggta 1560
   tccggtaagc ggcagggtcg gaacaggaga gcgcacgagg gagcttccag ggggaaacgc 1620
   ctggtatctt tatagtcctg tcgggtttcg ccacctctga cttgagcgtc gatttttgtg 1680
   atgctcgtca ggggggcgga gcctatggaa aaacgccagc aacgcggcct ttttacggtt 1740
   cctggccttt tgctggcctt ttgctcacat gttctttcct gcgttatccc ctgattctgt 1800
   ggataaccgt attaccgcct ttgagtgagc tgataccgct cgccgcagcc gaacgaccga 1860
   gcgcagcgag tcagtgagcg aggaagcgga agagcgcctg atgcggtatt ttctccttac 1920
   gcatctgtgc ggtatttcac accgcatata tggtgcactc tcagtacaat ctgctctgat 1980
   gccgcatagt taagccagta tacactccgc tatcgctacg tgactgggtc atggctgcgc 2040
   cccgacaccc gccaacaccc gctgacgcgc cctgacgggc ttgtctgctc ccggcatccg 2100
   cttacagaca agctgtgacc gtctccggga gctgcatgtg tcagaggttt tcaccgtcat 2160
   caccgaaacg cgcgaggcag ctgcggtaaa gctcatcagc gtggtcgtga agcgattcac 2220
   agatgtctgc ctgttcatcc gcgtccagct cgttgagttt ctccagaagc gttaatgtct 2280
   ggcttctgat aaagcgggcc atgttaaggg cggttttttc ctgtttggtc acttgatgcc 2340
   tccgtgtaag ggggaatttc tgttcatggg ggtaatgata ccgatgaaac gagagaggat 2400
   gctcacgata cgggttactg atgatgaaca tgcccggtta ctggaacgtt gtgagggtaa 2460
   acaactggcg gtatggatgc ggcgggacca gagaaaaatc actcagggtc aatgccagcg 2520
   cttcgttaat acagatgtag gtgttccaca gggtagccag cagcatcctg cgatgcagat 2580
   ccggaacata atggtgcagg gcgctgactt ccgcgtttcc agactttacg aaacacggaa 2640
   accgaagacc attcatgttg ttgctcaggt cgcagacgtt ttgcagcagc agtcgcttca 2700
   cgttcgctcg cgtatcggtg attcattctg ctaaccagta aggcaacccc gccagcctag 2760
   ccgggtcctc aacgacagga gcacgatcat gcgcacccgt ggccaggacc caacgctgcc 2820
   cgagatgcgc cgcgtgcggc tgctggagat ggcggacgcg atggatatgt tctgccaagg 2880
   gttggtttgc gcattcacag ttctccgcaa gaattgattg gctccaattc ttggagtggt 2940
   gaatccgtta gcgaggtgcc gccggcttcc attcaggtcg aggtggcccg gctccatgca 3000
   ccgcgacgca acgcggggag gcagacaagg tatagggcgg cgcctacaat ccatgccaac 3060
   ccgttccatg tgctcgccga ggcggcataa atcgccgtga cgatcagcgg tccagtgatc 3120
   gaagttaggc tggtaagagc cgcgagcgat ccttgaagct gtccctgatg gtcgtcatct 3180
   acctgcctgg acagcatggc ctgcaacgcg ggcatcccga tgccgccgga agcgagaaga 3240
   atcataatgg ggaaggccat ccagcctcgc gtcgcgaacg ccagcaagac gtagcccagc 3300
   gcgtcggcca gcttgcaatt cgcgctaact tacattaatt gcgttgcgct cactgcccgc 3360
   tttccagtcg ggaaacctgt cgtgccagct gcattaatga atcggccaac gcgcggggag 3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg ggcccgctaa cagcgcgatt tgctggtgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggccca ggtcaccgtc tcctcacgta cggtggctgc 4920
   accatctgtc ttcatcttcc cgccatctga tgagcagttg aaatctggaa ctgcctctgt 4980
   tgtgtgcctg ctgaataact tctatcccag agaggccaaa gtacagtgga aggtggataa 5040
   cgccctccaa tcgggtaact cccaggagag tgtcacagag caggacagca aggacagcac 5100
   ctacagcctc agcagcaccc tgacgctgag caaagcagac tacgagaaac acaaagtcta 5160
   cgcctgcgaa gtcacccatc agggcctgag ctcgccggtg acaaagagct tcaaccgcgg 5220
   agagtgtgca ggtgattaca aagacgatga cgataagtaa taaacaggaa acagaagtcc 5280
   atatgaaata tcttttacct acggcagccg caggtttgtt gttactcgcg gcccagccgg 5340
   ccatggccgc tagcaccaag ggcccatcgg tcttccccct ggcaccctcc tccaagagca 5400
   cctctggggg cacagcggcc ctgggctgcc tggtcaagga ctacttcccc gaaccggtga 5460
   cggtgtcgtg gaactcaggc gccctgacca gcggcgtgca caccttcccg gctgtcctac 5520
   agtcctcagg actctactcc ctcagcagcg tagtgaccgt gccctccagc agcttgggca 5580
   cccagaccta catctgcaac gtgaatcaca agcccagcaa caccaaggtg gacaagaaag 5640
   ttgagcccaa atcttgtgac aaaactcaca catgcccacc gtgcccagcg gccgcagaac 5700
   aaaaactcat ctcagaagag gatctgaatg gggccgtaca tcaccaccat catcatggga 5760
   gctaagcttg gctgttttgg cggatgagag aagattttca gcctgataca gattaaatca 5820
   gaacgcagaa gcggtctgat aaaacagaat ttgcctggcg gcagtagcgc ggtggtccca 5880
   cctgacccca tgccgaactc agaagtgaaa cgccgtagcg ccgatggtag tgtggggtct 5940
   ccccatgcga gagtagggaa ctgccaggca tcaaataaaa cgaaaggctc agtcgaaaga 6000
   ctgggccttt cgttttatct gttgtttgtc ggtgaacgct ctcctgagta ggacaaatcc 6060
   gccgggagcg gatttgaacg ttgcgaagca acggcccgga ggaccctggc gggcaggacg 6120
   cccgccataa actgccaggc atcaaattaa gcagaaggcc atcctgacgg atggcctttt 6180
   tgcgtttcta caaactctt 6199
<210> 114
   <211> 6064
   <212> DNA
   <213> Artificial sequence
<220>
   <223> Plasmide
<400> 114
   ttgtttattt ttctaaatac attcaaatat gtatccgctc atgagacaat aaccctgata     60
   aatgcttcaa taatattgaa aaaggaagag tatgagtatt caacatttcc gtgtcgccct    120
   tattcccttt tttgcggcat tttgccttcc tgtttttgct cacccagaaa cgctggtgaa    180
   agtaaaagat gctgaagatc agttgggtgc acgagtgggt tacatcgaac tggatctcaa    240
   cagcggtaag atccttgaga gttttcgccc cgaagaacgt tttccaatga tgagcacttt    300
   taaagttctg ctatgtggcg cggtattatc ccgtgttgac gccgggcaag agcaactcgg    360
   tcgccgcata cactattctc agaatgactt ggttgagtac tcaccagtca cagaaaagca    420
   tcttacggat ggcatgacag taagagaatt atgcagtgct gccataacca tgagtgataa    480
   cactgcggcc aacttacttc tgacaacgat cggaggaccg aaggagctaa ccgctttttt    540
   gcacaacatg ggggatcatg taactcgcct tgatcgttgg gaaccggagc tgaatgaagc    600
   cataccaaac gacgagcgtg acaccacgat gcctgtagca atggcaacaa cgttgcgcaa    660
   actattaact ggcgaactac ttactctagc ttcccggcaa caattaatag actggatgga    720
   ggcggataaa gttgcaggac cacttctgcg ctcggccctt ccggctggct ggtttattgc    780
   tgataaatct ggagccggtg agcgtgggtc tcgcggtatc attgcagcac tggggccaga    840
   tggtaagccc tcccgtatcg tagttatcta cacgacgggg agtcaggcaa ctatggatga    900
   acgaaataga cagatcgctg agataggtgc ctcactgatt aagcattggt aactgtcaga    960
   ccaagtttac tcatatatac tttagattga tttaaaactt catttttaat ttaaaaggat   1020
   ctaggtgaag atcctttttg ataatctcat gaccaaaatc ccttaacgtg agttttcgtt   1080
   ccactgagcg tcagaccccg tagaaaagat caaaggatct tcttgagatc ctttttttct   1140
   gcgcgtaatc tgctgcttgc aaacaaaaaa accaccgcta ccagcggtgg tttgtttgcc   1200
   ggatcaagag ctaccaactc tttttccgaa ggtaactggc ttcagcagag cgcagatacc   1260
   aaatactgtc cttctagtgt agccgtagtt aggccaccac ttcaagaact ctgtagcacc   1320
   gcctacatac ctcgctctgc taatcctgtt accagtggct gctgccagtg gcgataagtc   1380
   gtgtcttacc gggttggact caagacgata gttaccggat aaggcgcagc ggtcgggctg   1440
   aacggggggt tcgtgcacac agcccagctt ggagcgaacg acctacaccg aactgagata   1500
   cctacagcgt gagcattgag aaagcgccac gcttcccgaa gggagaaagg cggacaggta   1560
   tccggtaagc ggcagggtcg gaacaggaga gcgcacgagg gagcttccag ggggaaacgc   1620
   ctggtatctt tatagtcctg tcgggtttcg ccacctctga cttgagcgtc gatttttgtg   1680
   atgctcgtca ggggggcgga gcctatggaa aaacgccagc aacgcggcct ttttacggtt   1740
   cctggccttt tgctggcctt ttgctcacat gttctttcct gcgttatccc ctgattctgt 1800
   ggataaccgt attaccgcct ttgagtgagc tgataccgct cgccgcagcc gaacgaccga 1860
   gcgcagcgag tcagtgagcg aggaagcgga agagcgcctg atgcggtatt ttctccttac 1920
   gcatctgtgc ggtatttcac accgcatata tggtgcactc tcagtacaat ctgctctgat 1980
   gccgcatagt taagccagta tacactccgc tatcgctacg tgactgggtc atggctgcgc 2040
   cccgacaccc gccaacaccc gctgacgcgc cctgacgggc ttgtctgctc ccggcatccg 2100
   cttacagaca agctgtgacc gtctccggga gctgcatgtg tcagaggttt tcaccgtcat 2160
   caccgaaacg cgcgaggcag ctgcggtaaa gctcatcagc gtggtcgtga agcgattcac 2220
   agatgtctgc ctgttcatcc gcgtccagct cgttgagttt ctccagaagc gttaatgtct 2280
   ggcttctgat aaagcgggcc atgttaaggg cggttttttc ctgtttggtc acttgatgcc 2340
   tccgtgtaag ggggaatttc tgttcatggg ggtaatgata ccgatgaaac gagagaggat 2400
   gctcacgata cgggttactg atgatgaaca tgcccggtta ctggaacgtt gtgagggtaa 2460
   acaactggcg gtatggatgc ggcgggacca gagaaaaatc actcagggtc aatgccagcg 2520
   cttcgttaat acagatgtag gtgttccaca gggtagccag cagcatcctg cgatgcagat 2580
   ccggaacata atggtgcagg gcgctgactt ccgcgtttcc agactttacg aaacacggaa 2640
   accgaagacc attcatgttg ttgctcaggt cgcagacgtt ttgcagcagc agtcgcttca 2700
   cgttcgctcg cgtatcggtg attcattctg ctaaccagta aggcaacccc gccagcctag 2760
   ccgggtcctc aacgacagga gcacgatcat gcgcacccgt ggccaggacc caacgctgcc 2820
   cgagatgcgc cgcgtgcggc tgctggagat ggcggacgcg atggatatgt tctgccaagg 2880
   gttggtttgc gcattcacag ttctccgcaa gaattgattg gctccaattc ttggagtggt 2940
   gaatccgtta gcgaggtgcc gccggcttcc attcaggtcg aggtggcccg gctccatgca 3000
   ccgcgacgca acgcggggag gcagacaagg tatagggcgg cgcctacaat ccatgccaac 3060
   ccgttccatg tgctcgccga ggcggcataa atcgccgtga cgatcagcgg tccagtgatc 3120
   gaagttaggc tggtaagagc cgcgagcgat ccttgaagct gtccctgatg gtcgtcatct 3180
   acctgcctgg acagcatggc ctgcaacgcg ggcatcccga tgccgccgga agcgagaaga 3240
   atcataatgg ggaaggccat ccagcctcgc gtcgcgaacg ccagcaagac gtagcccagc 3300
   gcgtcggcca gcttgcaatt cgcgctaact tacattaatt gcgttgcgct cactgcccgc 3360
   tttccagtcg ggaaacctgt cgtgccagct gcattaatga atcggccaac gcgcggggag 3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg gtcccgctaa cagcgcgatt tgctgatgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggccca ggtcaccgtc tcctcacgta cggtggctgc 4920
   accatctgtc ttcatcttcc cgccatctga tgagcagttg aaatctggaa ctgcctctgt 4980
   tgtgtgcctg ctgaataact tctatcccag agaggccaaa gtacagtgga aggtggataa 5040
   cgccctccaa tcgggtaact cccaggagag tgtcacagag caggacagca aggacagcac 5100
   ctacagcctc agcagcaccc tgacgctgag caaagcagac tacgagaaac acaaagtcta 5160
   cgcctgcgaa gtcacccatc agggcctgag ctcgccggtg acaaagagct tcaaccgcgg 5220
   agagtgttaa taaacaggaa acagaagtcc atatgaaata tcttttacct acggcagccg 5280
   caggtttgtt gttactcgcg gcccagccgg ccatggccgc tagcaccaag ggcccatcgg 5340
   tcttccccct ggcaccctcc tccaagagca cctctggggg cacagcggcc ctgggctgcc 5400
   tggtcaagga ctacttcccc gaaccggtga cggtgtcgtg gaactcaggc gccctgacca 5460
   gcggcgtgca caccttcccg gctgtcctac agtcctcagg actctactcc ctcagcagcg 5520
   tagtgaccgt gccctccagc agcttgggca cccagaccta catctgcaac gtgaatcaca 5580
   agcccagcaa caccaaggtg gacaagaaag ttgagcccaa atcttgttaa gcttggctgt 5640
   tttggcggat gagagaagat tttcagcctg atacagatta aatcagaacg cagaagcggt 5700
   ctgataaaac agaatttgcc tggcggcagt agcgcggtgg tcccacctga ccccatgccg 5760
   aactcagaag tgaaacgccg tagcgccgat ggtagtgtgg ggtctcccca tgcgagagta 5820
   gggaactgcc aggcatcaaa taaaacgaaa ggctcagtcg aaagactggg cctttcgttt 5880
   tatctgttgt ttgtcggtga acgctctcct gagtaggaca aatccgccgg gagcggattt 5940
   gaacgttgcg aagcaacggc ccggaggacc ctggcgggca ggacgcccgc cataaactgc 6000
   caggcatcaa attaagcaga aggccatcct gacggatggc ctttttgcgt ttctacaaac 6060
   tctt 6064
<210> 115
   <211> 6094
   <212> DNA
   <213> Artificial sequence
<220>
   <223> Plasmide
<400> 115
   ttgtttattt ttctaaatac attcaaatat gtatccgctc atgagacaat aaccctgata 60
   aatgcttcaa taatattgaa aaaggaagag tatgagtatt caacatttcc gtgtcgccct 120
   tattcccttt tttgcggcat tttgccttcc tgtttttgct cacccagaaa cgctggtgaa 180
   agtaaaagat gctgaagatc agttgggtgc acgagtgggt tacatcgaac tggatctcaa 240
   cagcggtaag atccttgaga gttttcgccc cgaagaacgt tttccaatga tgagcacttt 300
   taaagttctg ctatgtggcg cggtattatc ccgtgttgac gccgggcaag agcaactcgg 360
   tcgccgcata cactattctc agaatgactt ggttgagtac tcaccagtca cagaaaagca 420
   tcttacggat ggcatgacag taagagaatt atgcagtgct gccataacca tgagtgataa 480
   cactgcggcc aacttacttc tgacaacgat cggaggaccg aaggagctaa ccgctttttt 540
   gcacaacatg ggggatcatg taactcgcct tgatcgttgg gaaccggagc tgaatgaagc 600
   cataccaaac gacgagcgtg acaccacgat gcctgtagca atggcaacaa cgttgcgcaa 660
   actattaact ggcgaactac ttactctagc ttcccggcaa caattaatag actggatgga 720
   ggcggataaa gttgcaggac cacttctgcg ctcggccctt ccggctggct ggtttattgc 780
   tgataaatct ggagccggtg agcgtgggtc tcgcggtatc attgcagcac tggggccaga 840
   tggtaagccc tcccgtatcg tagttatcta cacgacgggg agtcaggcaa ctatggatga 900
   acgaaataga cagatcgctg agataggtgc ctcactgatt aagcattggt aactgtcaga 960
   ccaagtttac tcatatatac tttagattga tttaaaactt catttttaat ttaaaaggat 1020
   ctaggtgaag atcctttttg ataatctcat gaccaaaatc ccttaacgtg agttttcgtt 1080
   ccactgagcg tcagaccccg tagaaaagat caaaggatct tcttgagatc ctttttttct 1140
   gcgcgtaatc tgctgcttgc aaacaaaaaa accaccgcta ccagcggtgg tttgtttgcc 1200
   ggatcaagag ctaccaactc tttttccgaa ggtaactggc ttcagcagag cgcagatacc 1260
   aaatactgtc cttctagtgt agccgtagtt aggccaccac ttcaagaact ctgtagcacc 1320
   gcctacatac ctcgctctgc taatcctgtt accagtggct gctgccagtg gcgataagtc 1380
   gtgtcttacc gggttggact caagacgata gttaccggat aaggcgcagc ggtcgggctg 1440
   aacggggggt tcgtgcacac agcccagctt ggagcgaacg acctacaccg aactgagata 1500
   cctacagcgt gagcattgag aaagcgccac gcttcccgaa gggagaaagg cggacaggta 1560
   tccggtaagc ggcagggtcg gaacaggaga gcgcacgagg gagcttccag ggggaaacgc 1620
   ctggtatctt tatagtcctg tcgggtttcg ccacctctga cttgagcgtc gatttttgtg 1680
   atgctcgtca ggggggcgga gcctatggaa aaacgccagc aacgcggcct ttttacggtt 1740
   cctggccttt tgctggcctt ttgctcacat gttctttcct gcgttatccc ctgattctgt 1800
   ggataaccgt attaccgcct ttgagtgagc tgataccgct cgccgcagcc gaacgaccga 1860
   gcgcagcgag tcagtgagcg aggaagcgga agagcgcctg atgcggtatt ttctccttac 1920
   gcatctgtgc ggtatttcac accgcatata tggtgcactc tcagtacaat ctgctctgat 1980
   gccgcatagt taagccagta tacactccgc tatcgctacg tgactgggtc atggctgcgc 2040
   cccgacaccc gccaacaccc gctgacgcgc cctgacgggc ttgtctgctc ccggcatccg 2100
   cttacagaca agctgtgacc gtctccggga gctgcatgtg tcagaggttt tcaccgtcat 2160
   caccgaaacg cgcgaggcag ctgcggtaaa gctcatcagc gtggtcgtga agcgattcac 2220
   agatgtctgc ctgttcatcc gcgtccagct cgttgagttt ctccagaagc gttaatgtct 2280
   ggcttctgat aaagcgggcc atgttaaggg cggttttttc ctgtttggtc acttgatgcc 2340
   tccgtgtaag ggggaatttc tgttcatggg ggtaatgata ccgatgaaac gagagaggat 2400
   gctcacgata cgggttactg atgatgaaca tgcccggtta ctggaacgtt gtgagggtaa 2460
   acaactggcg gtatggatgc ggcgggacca gagaaaaatc actcagggtc aatgccagcg 2520
   cttcgttaat acagatgtag gtgttccaca gggtagccag cagcatcctg cgatgcagat 2580
   ccggaacata atggtgcagg gcgctgactt ccgcgtttcc agactttacg aaacacggaa 2640
   accgaagacc attcatgttg ttgctcaggt cgcagacgtt ttgcagcagc agtcgcttca 2700
   cgttcgctcg cgtatcggtg attcattctg ctaaccagta aggcaacccc gccagcctag 2760
   ccgggtcctc aacgacagga gcacgatcat gcgcacccgt ggccaggacc caacgctgcc 2820
   cgagatgcgc cgcgtgcggc tgctggagat ggcggacgcg atggatatgt tctgccaagg 2880
   gttggtttgc gcattcacag ttctccgcaa gaattgattg gctccaattc ttggagtggt 2940
   gaatccgtta gcgaggtgcc gccggcttcc attcaggtcg aggtggcccg gctccatgca 3000
   ccgcgacgca acgcggggag gcagacaagg tatagggcgg cgcctacaat ccatgccaac 3060
   ccgttccatg tgctcgccga ggcggcataa atcgccgtga cgatcagcgg tccagtgatc 3120
   gaagttaggc tggtaagagc cgcgagcgat ccttgaagct gtccctgatg gtcgtcatct 3180
   acctgcctgg acagcatggc ctgcaacgcg ggcatcccga tgccgccgga agcgagaaga 3240
   atcataatgg ggaaggccat ccagcctcgc gtcgcgaacg ccagcaagac gtagcccagc 3300
   gcgtcggcca gcttgcaatt cgcgctaact tacattaatt gcgttgcgct cactgcccgc 3360
   tttccagtcg ggaaacctgt cgtgccagct gcattaatga atcggccaac gcgcggggag 3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg ggcccgctaa cagcgcgatt tgctgatgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggccca ggtcaccgtc tcctcacgta cggtggctgc 4920
   accatctgtc ttcatcttcc cgccatctga tgagcagttg aaatctggaa ctgcctctgt 4980
   tgtgtgcctg ctgaataact tctatcccag agaggccaaa gtacagtgga aggtggataa 5040
   cgccctccaa tcgggtaact cccaggagag tgtcacagag caggacagca aggacagcac 5100
   ctacagcctc agcagcaccc tgacgctgag caaagcagac tacgagaaac acaaagtcta 5160
   cgcctgcgaa gtcacccatc agggcctgag ctcgccggtg acaaagagct tcaaccgcgg 5220
   agagtgttaa taaacaggaa acagaagtcc atatgaaata tcttttacct acggcagccg 5280
   caggtttgtt gttactcgcg gcccagccgg ccatggccgc tagcaccaag ggcccatcgg 5340
   tcttccccct ggcaccctcc tccaagagca cctctggggg cacagcggcc ctgggctgcc 5400
   tggtcaagga ctacttcccc gaaccggtga cggtgtcgtg gaactcaggc gccctgacca 5460
   gcggcgtgca caccttcccg gctgtcctac agtcctcagg actctactcc ctcagcagcg 5520
   tagtgaccgt gccctccagc agcttgggca cccagaccta catctgcaac gtgaatcaca 5580
   agcccagcaa caccaaggtg gacaagaaag ttgagcccaa atcttgtgac aaaactcaca 5640
   catgcccacc gtgcccataa gcttggctgt tttggcggat gagagaagat tttcagcctg 5700
   atacagatta aatcagaacg cagaagcggt ctgataaaac agaatttgcc tggcggcagt 5760
   agcgcggtgg tcccacctga ccccatgccg aactcagaag tgaaacgccg tagcgccgat 5820
   ggtagtgtgg ggtctcccca tgcgagagta gggaactgcc aggcatcaaa taaaacgaaa 5880
   ggctcagtcg aaagactggg cctttcgttt tatctgttgt ttgtcggtga acgctctcct 5940
   gagtaggaca aatccgccgg gagcggattt gaacgttgcg aagcaacggc ccggaggacc 6000
   ctggcgggca ggacgcccgc cataaactgc caggcatcaa attaagcaga aggccatcct 6060
   gacggatggc ctttttgcgt ttctacaaac tctt 6094
<210> 116
   <211> 6745
   <212> DNA
   <213> Artificial sequence
<220>
   <223> Plasmide
<400> 116
   ttgtttattt ttctaaatac attcaaatat gtatccgctc atgagacaat aaccctgata 60
   aatgcttcaa taatattgaa aaaggaagag tatgagtatt caacatttcc gtgtcgccct 120
   tattcccttt tttgcggcat tttgccttcc tgtttttgct cacccagaaa cgctggtgaa 180
   agtaaaagat gctgaagatc agttgggtgc acgagtgggt tacatcgaac tggatctcaa 240
   cagcggtaag atccttgaga gttttcgccc cgaagaacgt tttccaatga tgagcacttt 300
   taaagttctg ctatgtggcg cggtattatc ccgtgttgac gccgggcaag agcaactcgg 360
   tcgccgcata cactattctc agaatgactt ggttgagtac tcaccagtca cagaaaagca 420
   tcttacggat ggcatgacag taagagaatt atgcagtgct gccataacca tgagtgataa 480
   cactgcggcc aacttacttc tgacaacgat cggaggaccg aaggagctaa ccgctttttt 540
   gcacaacatg ggggatcatg taactcgcct tgatcgttgg gaaccggagc tgaatgaagc 600
   cataccaaac gacgagcgtg acaccacgat gcctgtagca atggcaacaa cgttgcgcaa 660
   actattaact ggcgaactac ttactctagc ttcccggcaa caattaatag actggatgga 720
   ggcggataaa gttgcaggac cacttctgcg ctcggccctt ccggctggct ggtttattgc 780
   tgataaatct ggagccggtg agcgtgggtc tcgcggtatc attgcagcac tggggccaga 840
   tggtaagccc tcccgtatcg tagttatcta cacgacgggg agtcaggcaa ctatggatga 900
   acgaaataga cagatcgctg agataggtgc ctcactgatt aagcattggt aactgtcaga 960
   ccaagtttac tcatatatac tttagattga tttaaaactt catttttaat ttaaaaggat 1020
   ctaggtgaag atcctttttg ataatctcat gaccaaaatc ccttaacgtg agttttcgtt 1080
   ccactgagcg tcagaccccg tagaaaagat caaaggatct tcttgagatc ctttttttct 1140
   gcgcgtaatc tgctgcttgc aaacaaaaaa accaccgcta ccagcggtgg tttgtttgcc 1200
   ggatcaagag ctaccaactc tttttccgaa ggtaactggc ttcagcagag cgcagatacc 1260
   aaatactgtc cttctagtgt agccgtagtt aggccaccac ttcaagaact ctgtagcacc 1320
   gcctacatac ctcgctctgc taatcctgtt accagtggct gctgccagtg gcgataagtc 1380
   gtgtcttacc gggttggact caagacgata gttaccggat aaggcgcagc ggtcgggctg 1440
   aacggggggt tcgtgcacac agcccagctt ggagcgaacg acctacaccg aactgagata 1500
   cctacagcgt gagcattgag aaagcgccac gcttcccgaa gggagaaagg cggacaggta 1560
   tccggtaagc ggcagggtcg gaacaggaga gcgcacgagg gagcttccag ggggaaacgc 1620
   ctggtatctt tatagtcctg tcgggtttcg ccacctctga cttgagcgtc gatttttgtg 1680
   atgctcgtca ggggggcgga gcctatggaa aaacgccagc aacgcggcct ttttacggtt 1740
   cctggccttt tgctggcctt ttgctcacat gttctttcct gcgttatccc ctgattctgt 1800
   ggataaccgt attaccgcct ttgagtgagc tgataccgct cgccgcagcc gaacgaccga 1860
   gcgcagcgag tcagtgagcg aggaagcgga agagcgcctg atgcggtatt ttctccttac 1920
   gcatctgtgc ggtatttcac accgcatata tggtgcactc tcagtacaat ctgctctgat 1980
   gccgcatagt taagccagta tacactccgc tatcgctacg tgactgggtc atggctgcgc 2040
   cccgacaccc gccaacaccc gctgacgcgc cctgacgggc ttgtctgctc ccggcatccg 2100
   cttacagaca agctgtgacc gtctccggga gctgcatgtg tcagaggttt tcaccgtcat 2160
   caccgaaacg cgcgaggcag ctgcggtaaa gctcatcagc gtggtcgtga agcgattcac 2220
   agatgtctgc ctgttcatcc gcgtccagct cgttgagttt ctccagaagc gttaatgtct 2280
   ggcttctgat aaagcgggcc atgttaaggg cggttttttc ctgtttggtc acttgatgcc 2340
   tccgtgtaag ggggaatttc tgttcatggg ggtaatgata ccgatgaaac gagagaggat 2400
   gctcacgata cgggttactg atgatgaaca tgcccggtta ctggaacgtt gtgagggtaa 2460
   acaactggcg gtatggatgc ggcgggacca gagaaaaatc actcagggtc aatgccagcg 2520
   cttcgttaat acagatgtag gtgttccaca gggtagccag cagcatcctg cgatgcagat 2580
   ccggaacata atggtgcagg gcgctgactt ccgcgtttcc agactttacg aaacacggaa 2640
   accgaagacc attcatgttg ttgctcaggt cgcagacgtt ttgcagcagc agtcgcttca 2700
   cgttcgctcg cgtatcggtg attcattctg ctaaccagta aggcaacccc gccagcctag 2760
   ccgggtcctc aacgacagga gcacgatcat gcgcacccgt ggccaggacc caacgctgcc 2820
   cgagatgcgc cgcgtgcggc tgctggagat ggcggacgcg atggatatgt tctgccaagg 2880
   gttggtttgc gcattcacag ttctccgcaa gaattgattg gctccaattc ttggagtggt 2940
   gaatccgtta gcgaggtgcc gccggcttcc attcaggtcg aggtggcccg gctccatgca 3000
   ccgcgacgca acgcggggag gcagacaagg tatagggcgg cgcctacaat ccatgccaac 3060
   ccgttccatg tgctcgccga ggcggcataa atcgccgtga cgatcagcgg tccagtgatc 3120
   gaagttaggc tggtaagagc cgcgagcgat ccttgaagct gtccctgatg gtcgtcatct 3180
   acctgcctgg acagcatggc ctgcaacgcg ggcatcccga tgccgccgga agcgagaaga 3240
   atcataatgg ggaaggccat ccagcctcgc gtcgcgaacg ccagcaagac gtagcccagc 3300
   gcgtcggcca gcttgcaatt cgcgctaact tacattaatt gcgttgcgct cactgcccgc 3360
   tttccagtcg ggaaacctgt cgtgccagct gcattaatga atcggccaac gcgcggggag 3420
   aggcggtttg cgtattgggc gccagggtgg tttttctttt caccagtgag acgggcaaca 3480
   gctgattgcc cttcaccgcc tggccctgag agagttgcag caagcggtcc acgctggttt 3540
   gccccagcag gcgaaaatcc tgtttgatgg tggttgacgg cgggatataa catgagctgt 3600
   cttcggtatc gtcgtatccc actaccgaga tatccgcacc aacgcgcagc ccggactcgg 3660
   taatggcgcg cattgcgccc agcgccatct gatcgttggc aaccagcatc gcagtgggaa 3720
   cgatgccctc attcagcatt tgcatggttt gttgaaaacc ggacatggca ctccagtcgc 3780
   cttcccgttc cgctatcggc tgaatttgat tgcgagtgag atatttatgc cagccagcca 3840
   gacgcagacg cgccgagaca gaacttaatg gtcccgctaa cagcgcgatt tgctgatgac 3900
   ccaatgcgac cagatgctcc acgcccagtc gcgtaccgtc ttcatgggag aaaataatac 3960
   tgttgatggg tgtctggtca gagacatcaa gaaataacgc cggaacatta gtgcaggcag 4020
   cttccacagc aatggcatcc tggtcatcca gcggatagtt aatgatcagc ccactgacgc 4080
   gttgcgcgag aagattgtgc accgccgctt tacaggcttc gacgccgctt cgttctacca 4140
   tcgacaccac cacgctggca cccagttgat cggcgcgaga tttaatcgcc gcgacaattt 4200
   gcgacggcgc gtgcagggcc agactggagg tggcaacgcc aatcagcaac gactgtttgc 4260
   ccgccagttg ttgtgccacg cggttgggaa tgtaattcag ctccgccatc gccgcttcca 4320
   ctttttcccg cgttttcgca gaaacgtggc tggcctggtt caccacgcgg gaaacggtct 4380
   gataagagac accggcatac tctgcgacat cgtataacgt tactggtttc acattcacca 4440
   ccctgaattg actctcttcc gggcgctatc atgccatacc gcgaaaggtt ttgcgccatt 4500
   cgatggtgtc aacgtaaatg catgccgctt cgccttcgcg cgcgaattgc aagctgatcc 4560
   ggagcttatc gactgcacgg tgcaccaatg cttctggcgt caggcagcca tcggaagctg 4620
   tggtatggct gtgcaggtcg taaatcactg cataattcgt gtcgctcaag gcgcactccc 4680
   gttctggata atgttttttg cgccgacatc ataacggttc tggcaaatat tctgaaatga 4740
   gctgttgaca attaatcatc ggctcgtata atgtgtggaa ttgtgagcgg ataacaattt 4800
   cacacaggaa acagaattcc atatgaaata cctattacca acagcagcag ctgggttatt 4860
   attgctcgct gcgcagccgg ccatggccca ggtcaccgtc tcctcacgta cggtggctgc 4920
   accatctgtc ttcatcttcc cgccatctga tgagcagttg aaatctggaa ctgcctctgt 4980
   tgtgtgcctg ctgaataact tctatcccag agaggccaaa gtacagtgga aggtggataa 5040
   cgccctccaa tcgggtaact cccaggagag tgtcacagag caggacagca aggacagcac 5100
   ctacagcctc agcagcaccc tgacgctgag caaagcagac tacgagaaac acaaagtcta 5160
   cgcctgcgaa gtcacccatc agggcctgag ctcgccggtg acaaagagct tcaaccgcgg 5220
   agagtgttaa taaacaggaa acagaagtcc atatgaaata tcttttacct acggcagccg 5280
   caggtttgtt gttactcgcg gcccagccgg ccatggccgc tagcaccaag ggcccatcgg 5340
   tcttccccct ggcaccctcc tccaagagca cctctggggg cacagcggcc ctgggctgcc 5400
   tggtcaagga ctacttcccc gaaccggtga cggtgtcgtg gaactcaggc gccctgacca 5460
   gcggcgtgca caccttcccg gctgtcctac agtcctcagg actctactcc ctcagcagcg 5520
   tggtgaccgt gccctccagc agcttgggca cccagaccta catctgcaac gtgaatcaca 5580
   agcccagcaa caccaaggtg gacaagaaag ttgagcccaa atcttgtgac aaaactcaca 5640
   catgcccacc gtgcccagca cctgaactcc tggggggacc gtcagtcttc ctcttccccc 5700
   caaaacccaa ggacaccctc atgatctccc ggacccctga ggtcacatgc gtggtggtgg 5760
   acgtgagcca cgaagaccct gaggtcaagt tcaactggta cgtggacggc gtggaggtgc 5820
   ataatgccaa gacaaagccg cgggaggagc agtacaacag cacgtaccgg gtggtcagcg 5880
   tcctcaccgt cctgcaccag gactggctga atggcaagga gtacaagtgc aaggtctcca 5940
   acaaagccct cccagccccc atcgagaaaa ccatctccaa agccaaaggg cagccccgag 6000
   aaccacaggt gtacaccctg cccccatccc gggatgagct gaccaagaac caggtcagcc 6060
   tgacctgcct ggtcaaaggc ttctatccca gcgacatcgc cgtggagtgg gagagcaatg 6120
   ggcagccgga gaacaactac aagaccacgc ctcccgtgct ggactccgac ggctccttct 6180
   tcctctacag caagctcacc gtggacaaga gcaggtggca gcaggggaac gtcttctcat 6240
   gctccgtgat gcatgaggct ctgcacaacc actacacgca gaagagcctc tccctgtctc 6300
   cgggtaaata agcttggctg ttttggcgga tgagagaaga ttttcagcct gatacagatt 6360
   aaatcagaac gcagaagcgg tctgataaaa cagaatttgc ctggcggcag tagcgcggtg 6420
   gtcccacctg accccatgcc gaactcagaa gtgaaacgcc gtagcgccga tggtagtgtg 6480
   gggtctcccc atgcgagagt agggaactgc caggcatcaa ataaaacgaa aggctcagtc 6540
   gaaagactgg gcctttcgtt ttatctgttg tttgtcggtg aacgctctcc tgagtaggac 6600
   aaatccgccg ggagcggatt tgaacgttgc gaagcaacgg cccggaggac cctggcgggc 6660
   aggacgcccg ccataaactg ccaggcatca aattaagcag aaggccatcc tgacggatgg 6720
   cctttttgcg tttctacaaa ctctt 6745
<210> 117
   <211> 119
   <212> DNA
   <213> Artificial sequence
<220>
   <223> oligonucleotide
<400> 117
   ggagagtgtg caggtgatta caaagacgat gacgataagt aataaacagg aaacagaagt 60
   ccatatgaaa tatcttttac ctacggcagc cgcaggtttg ttgttactcg cggcccagc 119
<210> 118
   <211> 118
   <212> DNA
   <213> Artificial sequence
<220>
   <223> oligonucleotide
<400> 118
   gggccgcgag taacaacaaa cctgcggctg ccgtaggtaa aagatatttc atatggactt 60
   ctgtttcctg tttattactt atcgtcatcg tctttgtaat cacctgcaca ctctccgc 118
<210> 119
   <211> 59
   <212> DNA
   <213> Artificial sequence
<220>
   <223> oligonucleotide
<400> 119
   ggggccaggg gacccaggtc accgtctcct cacgtacggt ggctgcacca tctgtcttc 59
<210> 120
   <211> 89
   <212> DNA
   <213> Artificial sequence
<220>
   <223> oligonucleotide
<400> 120
   ggagagtgtt aataaacagg aaacagaagt ccatatgaaa tatcttttac ctacggcagc 60
   cgcaggtttg ttgttactcg cggcccagc 89
<210> 121
   <211> 88
   <212> DNA
   <213> Artificial sequence
<220>
   <223> oligonucleotide
<400> 121
   gggccgcgag taacaacaaa cctgcggctg ccgtaggtaa aagatatttc atatggactt    60
   ctgtttcctg tttattaaca ctctccgc                                       88